(19) Europäisches Patentamt
European Patent Office
Office européen des brevets

(11) EP 2 707 487 B1

(12) # EUROPEAN PATENT SPECIFICATION

(45) Date of publication and mention of the grant of the patent:
**04.10.2017 Bulletin 2017/40**

(21) Application number: **12724371.5**

(22) Date of filing: **10.05.2012**

(51) Int Cl.:
***C12N 15/115*** *(2010.01)* ***C12N 15/63*** *(2006.01)*
***C12N 15/67*** *(2006.01)*

(86) International application number:
**PCT/GB2012/051029**

(87) International publication number:
**WO 2012/153142 (15.11.2012 Gazette 2012/46)**

(54) **RIBOSWITCHES**

RIBOSWITCHES

RIBORÉGULATEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.05.2011 GB 201107768**

(43) Date of publication of application:
**19.03.2014 Bulletin 2014/12**

(73) Proprietor: **The University Of Manchester**
**Manchester M13 9PL (GB)**

(72) Inventors:
- **DIXON, Neil**
**Manchester M1 7DN (GB)**
- **MICKLEFIELD, Jason**
**Manchester M1 7DN (GB)**

(74) Representative: **HGF Limited**
**4th Floor**
**Merchant Exchange**
**17-19 Whitworth Street West**
**Manchester M1 5WG (GB)**

(56) References cited:
**WO-A1-2008/116223 WO-A2-2004/027035**
**WO-A2-2006/055351 US-A1- 2009 170 793**
**US-A1- 2010 226 901**

- **N. DIXON ET AL: "Reengineering orthogonally selective riboswitches", PROCEEDINGS OF THE NATIONAL ACADEMY OF SCIENCES, vol. 107, no. 7, 16 February 2010 (2010-02-16), pages 2830-2835, XP55034292, ISSN: 0027-8424, DOI: 10.1073/pnas.0911209107 cited in the application**
- **C. SCHEICH ET AL: "Vectors for co-expression of an unrestricted number of proteins", NUCLEIC ACIDS RESEARCH, vol. 35, no. 6, 1 January 2007 (2007-01-01), pages e43-e43, XP055046215, ISSN: 0305-1048, DOI: 10.1093/nar/gkm067**
- **BAUER G ET AL: "Engineered riboswitches as novel tools in molecular biology", JOURNAL OF BIOTECHNOLOGY, ELSEVIER SCIENCE PUBLISHERS, AMSTERDAM, NL, vol. 124, no. 1, 25 June 2006 (2006-06-25) , pages 4-11, XP024956748, ISSN: 0168-1656, DOI: 10.1016/J.JBIOTEC.2005.12.006 [retrieved on 2006-06-25]**
- **SUDARSAN NARASIMHAN ET AL: "Tandem riboswitch architectures exhibit complex gene control functions", SCIENCE, AMERICAN ASSOCIATION FOR THE ADVANCEMENT OF SCIENCE, US, vol. 314, no. 5797, 13 October 2006 (2006-10-13), pages 300-304, XP002514465, ISSN: 1095-9203, DOI: 10.1126/SCIENCE.1130716**
- **VANDANA SHARMA ET AL: "Engineering Complex Riboswitch Regulation by Dual Genetic Selection", JOURNAL OF THE AMERICAN CHEMICAL SOCIETY, vol. 130, no. 48, 3 December 2008 (2008-12-03), pages 16310-16315, XP055046200, ISSN: 0002-7863, DOI: 10.1021/ja805203w**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 707 487 B1

- YONG CHANG ET AL: "One-plasmid tunable coexpression for mycobacterial protein-protein interaction studies", PROTEIN SCIENCE, vol. 18, no. 11, 1 November 2009 (2009-11-01), pages 2316-2325, XP055046128, ISSN: 0961-8368, DOI: 10.1002/pro.242
- BRIAN F PFLEGER ET AL: "Combinatorial engineering of intergenic regions in operons tunes expression of multiple genes", NATURE BIOTECHNOLOGY, vol. 24, no. 8, 1 August 2006 (2006-08-01) , pages 1027-1032, XP055046133, ISSN: 1087-0156, DOI: 10.1038/nbt1226
- NEIL DIXON ET AL: "Orthogonal Riboswitches for Tuneable Coexpression in Bacteria", ANGEWANDTE CHEMIE INTERNATIONAL EDITION, vol. 51, no. 15, 10 April 2012 (2012-04-10), pages 3620-3624, XP55034291, ISSN: 1433-7851, DOI: 10.1002/anie.201109106
- Atsushi Ogawa ET AL: "An Artificial Aptazyme-Based Riboswitch and its Cascading System inE. coli", CHEMBIOCHEM - A EUROPEAN JOURNAL OF CHEMICAL BIOLOGY., vol. 9, no. 2, 20 December 2007 (2007-12-20), pages 206-209, XP055316770, DE ISSN: 1439-4227, DOI: 10.1002/cbic.200700478
- ROSA MORRA ET AL: "Dual transcriptional-translational cascade permits cellular level tuneable expression control", NUCLEIC ACIDS RESEARCH, vol. 44, no. 3, 23 September 2015 (2015-09-23), pages e21-e21, XP055316773, GB ISSN: 0305-1048, DOI: 10.1093/nar/gkv912

## Description

The present invention relates to systems comprising a genetic construct comprising a riboswitch for regulation of gene expression; kits comprising a system of the invention and optionally one or more ligands of a riboswitch; and methods of regulating expression of a coding sequence, using a system of the invention and a ligand of the riboswitch. Also provided are host cells or expression systems comprising a system of the invention and optionally a ligand of the riboswitch of the genetic construct in the system.

## BACKGROUND

A number of small-molecule inducible expression systems have been developed for bacteria. Notably, systems based on the lactose (*lac*), arabinose (*ara*) and tetracycline (*tet*) operons have been extensively utilised for the production of recombinant proteins, including biopharmaceuticals and industrial biocatalysts, and are also widely used for *in vivo* gene function analysis. For example, by placing a second copy of a gene under the control of an inducible promoter, then deleting the original gene from the chromosome, it is possible to create conditional mutants which can be used to explore the function of essential gene products in bacteria. Similarly, inducible expression systems have been used to control antisense RNA-mediated down-regulation of critical genes in pathogenic bacteria, allowing for the development of sensitive screens for new anti-microbial agents. Inducible expression systems have become indispensable tools in synthetic biology and have been used to control genetic circuits that can function as sensors, switches and oscillators. The expression of novel metabolic pathways, under the control of inducible promoters, has been employed for the production of fine chemicals as well as natural products including drug precursors and fatty acids for biofuels. Expression systems triggered by specific organic pollutants are also being developed for bioremediation applications.

Despite many notable successes, the commonly used regulatory systems do have limitations. Firstly, some of these systems exhibit all-or-none expression, where a subpopulation of cells are fully induced, whilst others remain un-induced. This can be problematic for many applications, including gene function analysis where tuneable gene expression, through titration of the inducer, is preferred. A number of the promoter based systems are also "leaky", exhibiting high basal levels of expression, which may be undesirable particularly for the expression of toxic genes. Some systems, such as *lac* and *ara*, exhibit cross-talk which limits their use in synthetic biology applications, where it is desirable to control the simultaneous and differential expression of multiple genes using distinct inducers. Some systems are limited by undesirable side-effects of the small molecule inducer, for example, the *tet* system is induced by tetracycline which can inhibit cell growth. Finally, whilst the common expression systems are up-regulated upon addition of small molecule ligands, there are few systems available where addition of ligand represses expression of the gene of interest. In generating conditional mutants, it is desirable for the essential gene of interest to be expressed at first, to allow growth to be established, and then down-regulated at the desired time point upon the addition of an effector molecule. Whilst some of these issues can be overcome by engineering improved host strains, this can be laborious. For example, it has taken several decades to engineer *E. coli* host strains that are compatible with the many different expression systems that are now commercially available. As a result, there are many bacterial species for which common expression systems are incompatible and where there are few or no alternative expression systems available. This is particularly relevant for pathogenic strains, where expression tools are crucial in the effort to develop new antimicrobial treatments.

Alternative methods have been sought to control gene expression with small molecules. For example, aptamers, generated by *in vitro* selection (e.g. SELEX), have been inserted into the 5'-untranslated regions (5'-UTRs) of bacterial mRNA to control translation in response to selected ligands. Notably, a theophylline aptamer inserted in the 5'-UTR of a β-galactosidase (*lacZ*) reporter was shown to sequester the ribosome binding site (rbs) in the absence of its ligand (Lynch et al. Chem Biol 2007, 14, 173). Upon binding theophylline, the rbs is released from the aptamer and translation is initiated. Whilst aptamers have significant potential for small molecule modulation of gene expression, there are currently very few aptamers available that are selective for ligands with desirable physiochemical and pharmacokinetic properties. Indeed, to date, only theophylline and a recently engineered atrazine aptamer have been shown to be effective at controlling gene expression in bacteria (Sinha et al. Nature Chem. Biol. 2010, 6, 464). However, nature has already evolved its own aptamers that provide exquisite control of gene expression through binding to a wide range of small molecule metabolites including amino acids, nucleobases, carbohydrates and co-enzymes. These so-called riboswitches are found in all domains of life, occurring with highest frequency in bacteria where they usually regulate genes involved in metabolite biosynthesis, catabolism, or transport. The bacterial riboswitches typically function through the binding of a metabolite to an aptamer domain, within the 5'-UTR of mRNA, which affects the conformation of an adjacent expression platform, thus modulating either the termination of transcription, initiation of translation or mRNA self-cleavage. This is a fundamentally different paradigm from protein-based regulatory systems. Riboswitches therefore offer an ideal platform to develop small-molecule controllable expression systems which address many of the limitations of the classical systems. However, the applicability of riboswitches as generic genetic control elements is limited by the fact that natural riboswitches have evolved to bind to primary metabolites that are common within cells. The aptamer domain within each category of

riboswitch is generally highly conserved, and as such they can be identified readily using homology based searches. In contrast, the expression platform of a riboswitch often varies in sequence and structure, and controls the expression of the coding sequence to which it is operably linked. For example, the TPP riboswitch in bacteria shares a highly conserved aptamer domain to which the ligand binds, but in different bacterial strains the expression platform varies: in *E.coli* it causes inhibition of translation by blocking the Shine-Dalgarno sequence, whereas in *B.subtilis* it causes termination of transcription.

The effects of a riboswitch on gene expression include transcriptional termination, inhibition of translation initiation, mRNA self-cleavage, and in eukaryotes, alteration of splicing pathways. Specifically, the following mechanisms of action have been observed: riboswitch-controlled formation of Rho-independent transcription termination hairpins, leading to premature transcription termination; riboswitch-mediated folding resulting in sequestration of the ribosome-binding site, thereby inhibiting translation; dual role of the riboswitch as a control mechanism and a ribozyme, the latter being cleaved in the presence of sufficient concentrations of ligand; and alternative structures of the riboswitch affecting the splicing of the pre-mRNA.

The mechanisms of action of the riboswitches can have higher levels of complexity. For example, a riboswitch in *Clostridium acetobutylicum* has been shown to regulate not the gene which it is linked to, but an adjacent gene which is not part of the same mRNA transcript. Another similar example has been observed in *Listeria monocytogenes,* where the riboswitch regulates the expression of a downstream gene. However, riboswitch-controlled transcripts can also subsequently modulate the expression of genes located elsewhere in the genome.

Known riboswitches are generally categorised by the ligand which binds them. Riboswitches discovered to date include:

- Cobalamin riboswitch (also *$B_{12}$-element*), which binds adenosylcobalamin (the coenzyme form of vitamin $B_{12}$) to regulate the biosynthesis and transport of cobalamin and similar metabolites.
- cyclic di-GMP riboswitches, which bind cyclic di-GMP to regulate a variety of genes. Two non-structurally related classes exist - cyclic di-GMP-I and cyclic di-GMP-II.
- FMN riboswitch (also *RFN-element*) which binds flavin mononucleotide (FMN) to regulate riboflavin biosynthesis and transport.
- *glmS* riboswitch, which cleaves itself when there is a sufficient concentration of glucosamine-6-phosphate.
- Glutamine riboswitches, which bind glutamine to regulate genes involved in glutamine and nitrogen metabolism. They also bind short peptides of unknown function. Such riboswitches fall into two classes, which are structurally related: the *glnA RNA motif* and *Downstream-peptide motif.*
- Glycine riboswitch, which binds glycine to regulate glycine metabolism genes. It comprises two adjacent aptamer domains in the same mRNA, and is the only known natural RNA that exhibits cooperative binding.
- Lysine riboswitch (also *L-box*), which binds lysine to regulate lysine biosynthesis, catabolism and transport.
- PreQ1 riboswitches, which bind pre-queuosine$_1$, to regulate genes involved in the synthesis or transport of this precursor to queuosine. Two entirely distinct classes of PreQ1 riboswitches are known: PreQ1-I riboswitches and PreQ1-II riboswitches. The binding domain of PreQ1-I riboswitches is unusually small among naturally occurring riboswitches. PreQ1-II riboswitches, which are only found in certain species in the genera *Streptococcus* and *Lactococcus,* have a completely different structure, and are larger.
- Purine riboswitches, which bind purines to regulate purine metabolism and transport. Different forms of the purine riboswitch bind guanine (a form originally known as the *G-box*) or adenine. The specificity for either guanine or adenine depends completely upon Watson-Crick interactions with a single pyrimidine in the riboswitch at position Y74. In the guanine riboswitch this residue is always a cytosine (i.e. C74), in the adenine residue it is always a uracil (i.e. U74). Homologous types of purine riboswitches bind deoxyguanosine, but have more significant differences than a single nucleotide mutation.
- SAH riboswitches, which bind S-adenosylhomocysteine to regulate genes involved in recycling this metabolite which is produced when S-adenosylmethionine is used in methylation reactions.
- SAM riboswitches, which bind S-adenosyl methionine (SAM) to regulate methionine and SAM biosynthesis and transport. Three distinct SAM riboswitches are known: SAM-I (originally called S-box), SAM-II and the $S_{MK}$ box riboswitch. SAM-I is widespread in bacteria, but SAM-II is found only in $\alpha$-, $\beta$- and a few $\gamma$-proteobacteria. The $S_{MK}$ box riboswitch is found only in the order *Lactobacillales.* These three varieties of riboswitch have no obvious similarities in terms of sequence or structure. A fourth variety, SAM-IV, appears to have a similar ligand-binding core to that of SAM-I, but in the context of a distinct scaffold.
- SAM-SAH riboswitches, which bind both SAM and SAH with similar affinities. Since they are always found in a position to regulate genes encoding methionine adenosyltransferase, it was proposed that only their binding to SAM is physiologically relevant.
- Tetrahydrofolate riboswitches, which bind tetrahydrofolate to regulate synthesis and transport genes.
- TPP riboswitches (also THI-box), which bind thiamine pyrophosphate (TPP) to regulate thiamine biosynthesis and

transport, as well as transport of similar metabolites. It is the only riboswitch found so far in eukaryotes.

- Moco riboswitch, which binds molybdenum cofactor, to regulate genes involved in biosynthesis and transport of this coenzyme, as well as enzymes that use it or its derivatives as a cofactor.
- Adenine sensing *add*-A riboswitch, found in the 5' UTR of the adenine deaminase encoding gene *(add)* of *Vibrio vulnificus*. Ogawa and Mizuo (2008), "An Artifical Aptazyme-Based Riboswitch and its Cascading System in E.coli", Chembiochem vol 9, no. 2, pp 206-209, discloses a system wherein the expression of luciferase is driven by a SP6 polymerase expressed from a second vector under translational control of an aptazyme-based riboswitch. Dixon et al (2010), "Reengineering orthogonally selective riboswitches", Proceedings of the National Academy of Sciences vol 107, no.7, pp 2830-2835, is concerned with riboswitches that are selective for synthetic non-natural small molecules and no longer respond to the natural intracellular ligands. WO 2008/116223 A1 is concerned with PreQ1 riboswitches and associated methods and compositions. WO 2004/027035 A2 is concerned with riboswitches, methods for their use and compositions for use with riboswitches. US 2010/226901 A1 is concerned with the genetic control of mammalian cells with synthetic RNA regulatory systems. US 2009/170793 A1 is concerned with an artificial riboswitch for controlling pre-mRNA splicing.

The present invention aims to provide novel genetic systems to regulate gene expression, using riboswitches as a regulatory tool. Such genetic systems, genetic constructs, and kits or methods utilising same, may be of use in controlling gene expression, for example in the production of recombinant proteins.

The disclosure also relates to novel ligands for riboswitches. A non-natural ligand for a riboswitch may be one which is generally not present in a cell, for example is drug-like, for example can penetrate the cell membrane, for example is non-toxic, for example is stable, cheap and for example shows high specificity.

Riboswitches represent a target for therapeutic intervention, particularly for the development of novel pharmaceuticals and anti-bacterial agents. Riboswitch-regulated systems of the invention may also be suitable for use in therapeutic indications. Systems or constructs for use in such applications will preferably be transferable, allowing simultaneous yet differential control of multiple genes. Preferably, it will be capable of activating and blocking gene expression, be capable of acting rapidly and in a dose-dependent manner, and/or have broad dynamic range and temporal control.

## BRIEF SUMMARY OF THE DISCLOSURE

Described herein, in a first aspect, is a genetic construct, comprising a first nucleic acid molecule encoding a riboswitch and a second nucleic acid molecule comprising a coding sequence, wherein the first and second nucleic acid molecules are operably linked to one another by a third nucleic acid molecule comprising a spacer sequence.

The spacer may be a nucleic acid molecule of low GC content, for example less than 65%, 60%, 55%, 50%, 55%, 50%, 45%, 40%, 39%, 38%, 37%, 36%, 35%, 34%, 33%, 32%, 31%, 30%, 29%, 28%, 27%, 26%, 25%, 24%, 23%, 22%, 20%, 19%, 18%, 17%, 16%, 15%, 14%, 13%, 12%, 11%, 10%, 9%, 8%, 7%, 6%, 5%, 4%, 3%, 2% or 1 %, across the full length of the spacer, or across at least 50%, 60%, 70%, 80%, 90%, 91%, 92%, 93%, 94%, 95%, 96%, 97%, 98% or 99% contiguous nucleic acid residues of the spacer. The spacer may be substantially free of secondary structure, for example comprising less than 40kcal/mol, for example less than -39, -38, -37, -36, -35, -34, -33, -32, -31, -30, -29, -28, -27, -26, -25, -24, -23, -22, -20, -19, -18, -17, -16, -15, -14, -13, -12, -11, -10, -9, -8,- 7, -6,-5, -4, -3, -2 or -1 kcal/mol. The spacer may be a nucleic acid, for example DNA or RNA.

The spacer sequence may encode an RNA sequence, and for example a protein or peptide sequence, including a secretion signal peptide.

The nucleic acid molecules of the genetic construct of the first aspect may be DNA molecules, but may be RNA molecules. Thus, also described herein is a genetic construct comprising a first RNA molecule comprising a riboswitch and a second RNA molecule comprising a coding sequence, wherein the first and second RNA molecules are operably linked by a third RNA molecule comprising a spacer sequence.

Within the genetic construct, the first nucleic acid molecule may be upstream of the third nucleic acid molecule, and the third nucleic acid molecule in turn upstream of the second nucleic acid molecule.

In the first aspect of the disclosure, two or more of the first, second and third nucleic acid molecules of the genetic construct may be operably linked to each other. For example, they may be structurally linked. They may be linked by nucleic acid residues. They may be contiguous, such that the last nucleic acid residue of one nucleic acid molecule is adjacent in sequence to the last nucleic acid residue of another of the first, second or third nucleic acid molecules.

Two or more of the first, second and third nucleic acid molecules may each be contiguous. Alternatively, two or more of the first, second or third nucleic acid molecules may be indirectly linked by intervening sequence. They may be operably linked. At least the third nucleic acid molecule comprising the spacer sequence and the second nucleic acid molecule comprising the coding sequence may be contiguous. The last nucleic acid residue of the spacer may be directly linked to, the first nucleic acid residue of the coding sequence. Where the spacer is a non-coding sequence, a start

codon may be provided in the coding sequence of the second molecule. In this case, it is envisaged that the first nucleic acid residue of the coding sequence of the second nucleic acid molecule may be the A residue of a start codon, such as AUG. Where the spacer encodes an RNA or protein or peptide sequence, a start codon may be provided in the spacer sequence. In instances where the third molecule is itself a coding sequence (as considered elsewhere in the present disclosure) the third molecule should be in-frame with the second molecule, such that an extended mRNA corresponding to both the second and third molecules is transcribed. In such cases a N-terminally tagged protein and/or mini-fusion protein may be expressed.

It is also envisaged that an intervening sequence may be provided between the third nucleic acid molecule and the second nucleic acid molecule, for example a nucleic acid molecule comprising a second riboswitch. Such a second riboswitch may be the same or different to the first riboswitch, as defined herein.

Such a genetic construct may comprise, in the following order:

RIBOSWITCH - SPACER - INTERVENING SEQ (e.g. RIBOSWITCH) - CODING SEQUENCE.

In this instance, the last nucleic acid residue of the spacer sequence of the third nucleic acid molecule may be contiguous with the intervening sequence. The last nucleic acid residue of the intervening molecule may be contiguous with the last nucleic acid residue of the second nucleic acid molecule, for example to the first nucleic acid residue of the coding sequence.

Genetic constructs of the first aspect of the disclosure may comprise an orthogonal riboswitch (herein referred to as "ORS"). Examples of riboswitches for use in the present disclosure include the M6" and addA riboswitches, for example operably linked to sequence encoding a T7 polymerase. The riboswitch-T7 polymerase sequence may be under the control of a *lac* promoter (though it will be appreciated that other promoters considered elsewhere in the specification may also be used). The genetic construct may also comprise a kanamycin and an ampicillin resistance marker genes. Examples of genetic constructs of the first aspect of the disclosure are shown in Figures 1, 2, 23, 24, 26 (pMOD3_spT7, operon, dual promoter and ORS_sT7), 41, 42, 43, 44, 45.

Also described herein is a kit of parts, comprising a genetic construct as herein described, and a ligand. The ligand may be a ligand of the fourth aspect of the disclosure.

Also described herein in the first aspect of the disclosure is a method of regulating expression of a coding sequence, the method comprising: contacting a cell or expression system comprising a genetic construct of the disclosure with a ligand capable of activating a riboswitch of the genetic construct. The method may be selected from a method of inhibiting bacterial cell growth, a method of increasing or decreasing the yield of a coding sequence product or products, a method of altering the physiological characteristics of a cell, a method of increasing gene production, a method altering cell morphology, a method of altering sensitivity to an antibiotic, use as a tool for drug discovery, use as a biosensor, or a method of increasing flux through a biosynthetic pathway. The ligand may be a ligand of the fourth aspect of the disclosure.

Also described herein in the first aspect of the disclosure is a cell or expression system comprising a genetic construct of the first aspect, and optionally a ligand capable of activating a riboswitch of the genetic construct of the first aspect. The ligand may be a ligand of the fourth aspect of the disclosure.

## DECOUPLED RIBOSWITCH AND GENE OF INTEREST

The present invention provides a system comprising

a) a first genetic construct comprising a promoter controlling transcription of first and second nucleic acid molecules, the first nucleic acid molecule encoding a riboswitch operably linked to the second nucleic acid molecule comprising a regulatory sequence encoding a polymerase; and
b) a second genetic construct comprising a nucleic acid molecule comprising a coding sequence whose expression is capable of being regulated by of the polymerase encoded by the second nucleic acid molecule;

wherein said riboswitch is a naturally occurring riboswitch or a modified riboswitch having at least 85% sequence identity to a naturally occurring riboswitch.

The regulatory sequence of the second nucleic acid molecule encodes a polymerase which serves to regulate expression of the coding sequence of the second genetic construct. Thus, the riboswitch acts indirectly on the coding sequence of the second genetic construct by regulating the expression of a component which effects the expression of the coding sequence. The polymerase may be any suitable polymerase, preferably a viral polymerase, and preferably T7, SP6, or T3 viral polymerase (pMLOST, and pMAST3).

Preferably, the first and second genetic constructs may independently be DNA or RNA. Preferably, the nucleic acid molecules are independently DNA molecules. Thus, the present invention also provides a system according to the

invention wherein the a first genetic construct comprises a first RNA molecule comprising a riboswitch operably linked to a second RNA molecule which is, or encodes, a regulatory sequence.

The nucleic acid molecule of the second construct may be DNA or RNA independently of the nature of the nucleic acid molecules of the first construct.

The regulated expression of the polymerase encoded in the first genetic construct in turn affects the expression of the coding sequence of the second genetic construct, which is transcribed by the polymerase of the first genetic construct. Thus, the expression of the coding sequence of the second construct is dependent upon the expression of the polymerase, for example in terms of the level of polymerase or its temporal expression.

In the first genetic construct the first and second nucleic acid molecules are operably linked to each other. More preferably, they are structurally linked. More preferably, they are contiguous.

Within the first genetic construct, preferably the first nucleic acid molecule is provided upstream of the second nucleic acid molecule.

In a preferred embodiment, the first genetic construct additionally comprises a nucleic acid molecule comprising a spacer sequence. The nucleic acid molecule comprising the spacer sequence is preferably provided upstream of the nucleic acid molecule comprising a regulatory sequence. The nucleic acid molecule comprising the spacer sequence may be operably linked to the nucleic acid molecule comprising the regulatory sequence, either contiguously or indirectly. Contiguous is preferred, as defined herein. Where the spacer is a non-coding sequence, a start codon may be provided in the coding sequence of the second molecule. In this case, it is envisaged that the first nucleic acid residue of the coding sequence of the second nucleic acid molecule may be the A residue of a start codon, such as AUG. Where the spacer encodes an RNA or protein or peptide sequence, a start codon may be provided in the spacer sequence.

Thus, for example, the first genetic construct of system of the invention may comprise:

RIBOSWITCH--(OPTIONAL SPACER)-REGULATORY SEQUENCE

In a preferred embodiment of the invention, the second genetic construct may independently comprise a nucleic acid molecule comprising a spacer sequence. The nucleic acid molecule comprising the spacer sequence is preferably provided upstream of the nucleic acid molecule comprising the coding sequence. The nucleic acid molecule comprising the spacer sequence may be operably linked to the nucleic acid molecule comprising the coding sequence, either contiguously or indirectly. Contiguous is preferred, as defined herein. More preferably, the last nucleic acid residue of the nucleic acid molecule comprising the spacer sequence is adjacent to the first nucleic acid residue of the nucleic acid molecule comprising the coding sequence. For example, the last nucleic acid residue of the spacer is immediately upstream, or adjacent to, the first nucleic acid residue of the coding sequence. Where the spacer is a non-coding sequence, a start codon may be provided in the coding sequence of the second molecule. In this case it is envisaged that the first nucleic acid residue of the coding sequence of the nucleic acid molecule may be the A residue of a start, for example AUG, for example where the spacer is a non-coding sequence. Where the spacer encodes an RNA or protein or peptide sequence, a start codon may be provided in the spacer sequence.

In a system of the invention, the promoter in the first genetic construct may in one instance be selected from the group consisting of T7, T3, Sp6, araC, araBAD, lac promoter; lacUCV5; tac; trc; trpR promoter; Omp; tet promoter; luxR; lambda Prm promoter; and pLac promoter.

The invention also provides a kit of parts, comprising a first genetic construct and a second genetic construct of the system of the invention, and optionally a ligand of the riboswitch of the first genetic construct. Preferably, the ligand is a ligand of the fourth aspect of the disclosure.

The invention also provides a method of regulating expression of a coding sequence, the method comprising: contacting a cell or expression system comprising a system of the invention in vitro with a ligand of the riboswitch, wherein the expression of said coding sequence is regulated by the polymerase encoded by the second nucleic acid molecule of the system. Preferably, the method may be a method of inhibiting bacterial cell growth, a method of increasing or decreasing the yield of a coding sequence product or multiple products, a method of altering the physiological characteristics of a cell, a method of increasing gene production, a method altering cell morphology, a method of altering sensitivity to an antibiotic, or a method of increasing flux through a biosynthetic pathway. The invention also provides use of a system according to the invention as a tool for drug discovery, or as a biosensor, wherein the use comprises regulating expression of a coding sequence by contacting a cell or expression system comprising a system of the invention in vitro with a ligand of the riboswitch, and wherein the expression of said coding sequence is regulated by the polymerase encoded by the second nucleic acid molecule of the system of the invention. The ligand may be a ligand of the fourth aspect of the disclosure.

The invention further provides a system of the invention for use in therapy.

The invention also provides a cell or expression system comprising a first genetic construct of the system of the invention, and a second genetic construct of the system of the invention, and optionally a ligand of the riboswitch of the first genetic construct. Preferably, the ligand is a ligand of the fourth aspect of the disclosure.

MULTIPLE RIBOSWITCHES CONTROLLING MULTIPLE GENES

In a third aspect of the present disclosure, described herein is a genetic construct comprising

a) a first nucleic acid molecule encoding a riboswitch, the first nucleic acid molecule being operably linked to a second nucleic acid molecule comprising a coding sequence; and
b) a third nucleic acid molecule encoding a second riboswitch, the third nucleic acid molecule being operably linked to a fourth nucleic acid molecule comprising a second coding sequence.

Optionally, the genetic construct may comprise further (3, 4, 5, 6 or more) nucleic acid molecules, each encoding a further riboswitch, and each being independently and operably linked to a further (2, 3, 4, 5, 6 or more) nucleic acid molecule comprising a coding sequence. This aspect of the disclosure allows variable co-expression of any multimeric or multi-domain protein or complex, such as heavy and light chain antibodies, or any ancillary protein e.g. chaperone, secretion protein or glycosylation proteins.
The genetic construct may comprise a transcriptional terminator between a coding sequence and a downstream second or further riboswitch. Thus, for example, a transcriptional terminator may be provided between the second nucleic acid molecule and the third nucleic acid molecule or between a fourth nucleic acid molecule and any fifth or further nucleic acid molecules. In any genetic construct, one or more transcriptional terminators may be provided, as appropriate.
The order of components in a genetic construct of the third aspect may be:

PROMOTER-RIBOSWITCH-CODING SEQUENCE-TRANSCRIPTIONAL TERMINATOR - PROMOTOR 2 - RIBOSWITCH 2 - CODING SEQUENCE 2

The presence of a transcriptional terminator enables two or more RNA molecules to be yielded.

RIBOSWITCH 1 - CODING SEQUENCE 1 and RIBOSWITCH 2 - CODING SQUENCE 2

Such a configuration is shown in Figure 42. In such a configuration, a genetic construct is provided which comprises an M6" riboswitch upstream of and operably linked to a T7RNAP coding sequence, and downstream thereof an AddA riboswitch upstream of and operably linked to a T3RNAP coding sequence. Both pairings are under the control of a *lac* promoter. Riboswitch mediated control of expression of the T7 and T3 polymerases serves to regulate, for example, expression of domains of a multimeric protein, the expression each domain or protein being under the control of a T3 or T7 promoter. This aspect of the disclosure is also useful in achieving variable co-expression of coding sequence products (such as pTTv7 and pTTv9, as described elsewhere in the specification).
Where there is no transcriptional terminator provided between a coding sequence and a downstream riboswitch, two or more riboswitches and coding sequences may be provided within a single RNA molecule, i.e.

RIBOSWITCH-CODING SEQUENCE 1-RIBOSWITCH2-CODING SEQUENCE2

All genetic molecules within a genetic construct may be of the same type, ie DNA or RNA. Thus, also described in the third aspect of the present disclosure is a genetic construct comprising a first RNA molecule comprising a riboswitch and a second RNA molecule comprising a coding sequence, and a third RNA molecule encoding a second riboswitch, the third RNA molecule being operably linked to a fourth RNA molecule comprising a second coding sequence.
Two or more riboswitches in the genetic construct may be the same or may be different. Optionally, two or more may be the same, and the remainder may each be the same or different thereto.
The coding sequence of the genetic constructs may be regulatory sequences, as defined herein, or may encode a non-regulatory RNA or protein or peptide sequence. Where regulatory, the product of a coding sequence may in turn control the expression of a further coding sequence within the construct, or within an additional genetic construct.
The coding sequences of the two or more nucleic acid molecules comprising the same may be the same or different. Two or more of the coding sequences may be the same, and the remainder may each be the same or different thereto. By the same is meant that they encode substantially the same product (RNA or protein or peptide), this being measured in terms of primary sequence, secondary structure and/or function.
In the genetic construct the first and second nucleic acid molecules may be operably linked to each other, and the third and fourth nucleic acid molecules may be operably linked to each other. The first, second, third and fourth nucleic acid molecules may be operably linked to one another. The nucleic acid molecules which are operably linked may be structurally linked. They may be linked by nucleic acid residues. They may be contiguous.
Within the genetic construct, the first and second nucleic acid molecules may be provided in any order. For example, the first nucleic acid molecule may be upstream of the second or further nucleic acid molecules, and in turn

the second may be upstream of the third and so on.

One or more nucleic acid molecules of the genetic construct may each independently additionally comprise a spacer sequence. For example, at least one nucleic acid molecule comprising a coding sequence may also comprise a spacer sequence. Two or more nucleic acid molecules comprising a coding sequence may each independently comprise a spacer sequence. Each nucleic acid molecule comprising a riboswitch may also comprise a spacer sequence. Where two or more nucleic acid molecules comprising spacer sequences are provided in the genetic construct of the third aspect, these spacer sequences may each be the same or different. Two or more may be the same, and the remainder may each, independently, be the same or different thereto.

A spacer sequence may be operably linked to a nucleic acid molecule comprising a coding sequence, either contiguously or indirectly linked. Contiguous is preferred, as defined herein. The last nucleic acid residue of the riboswitch may be immediately upstream, or adjacent to, the first nucleic acid residue of the riboswitch. Where the spacer is a non-coding sequence, a start codon may be provided in the coding sequence of the second molecule. In this case, it is envisaged that the first nucleic acid residue of the coding sequence of the second nucleic acid molecule may be the A residue of a start codon, such as AUG. Where the spacer encodes an RNA or protein or peptide sequence, a start codon may be provided in the spacer sequence, allowing expression of an expression tag and/or mini-fusion protein.

In one instance a genetic construct of the third aspect of the disclosure may comprise an M6" riboswitch operably linked to a DsRed coding sequence, and an AddA riboswitch operably linked to a eGFP coding sequence. The AddA-GFP sequence may be downstream of the M6"-DsRed sequence. The genetic construct may be pOperon, of Figure 2. An alternative instance comprises a transcription termination sequence downstream of the M6"-DsRed sequence, and upstream of the AddA-GFP sequence. The genetic construct may be pDual Promoter of Figure 2.

Also described in the third aspect of the disclosure is a kit of parts, comprising a genetic construct of the third aspect of the disclosure, and a ligand of a riboswitch of the construct. A ligand may be a ligand of the fourth aspect of the disclosure. The kit may also comprise additional ligands for additional riboswitches.

Also described in the third aspect of the disclosure is a method of regulating expression of a coding sequence, the method comprising: contacting a cell or expression system comprising a genetic construct of the third aspect of the disclosure with a ligand of a riboswitch of the construct. The method may be a method of inhibiting bacterial cell growth, a method of increasing or decreasing the yield of a coding sequence product or multiple products a method of altering the physiological characteristics of a cell, a method of increasing gene production, a method altering cell morphology, a method of altering sensitivity to an antibiotic, use as a tool for drug discovery, use as a biosensor, or a method of increasing flux through a biosynthetic pathway. The ligand may be a ligand of the fourth aspect of the disclosure.

Also described in the third aspect of the disclosure is a cell or expression system comprising a genetic construct of the third aspect of the disclosure, and optionally a ligand of a riboswitch of the genetic construct. The ligand may be a ligand of the fourth aspect of the disclosure.

LIGANDS

In a fourth aspect of the disclosure, described herein is a ligand defined by Formula I;

I

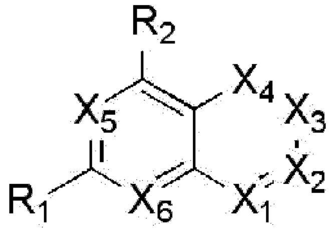

wherein

$R_1$ and $R_2$ are independently selected from a hydrogen bond donor or a hydrogen bond acceptor, for example H or $NH_2$, O, OH; and

$X_1$, $X_2$, $X_3$, $X_4$ $X_5$, and $X_6$ are independently selected from any hydrogen bond donor or hydrogen bond acceptor, or from CH or N.

In one instance, $R_1$ and $R_2$ are independently selected from O, OH, $NH_2$, $NHCOCH_3$, $NHCO_2CH_3$, NH-cCH($CH_2CH_2$), SH, $NHNHCOCH_3$, $NHOCH_3$, or NHOR (R=alkyl, aryl, or benzyl).

In one instance, the compound of Formula I is

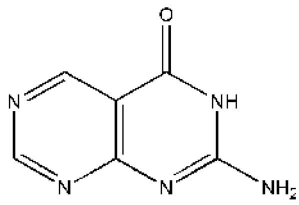
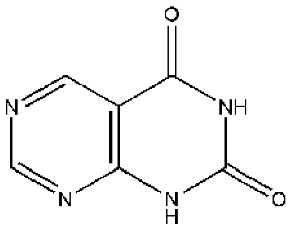
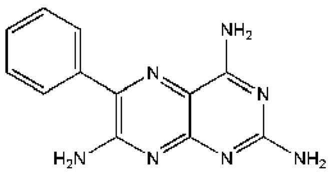
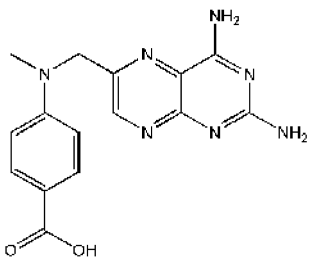
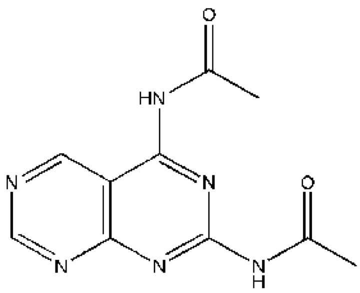
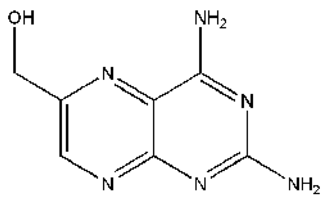
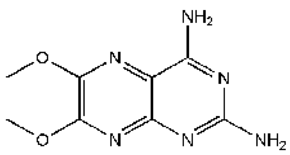
or
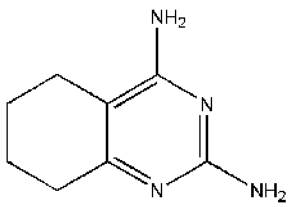
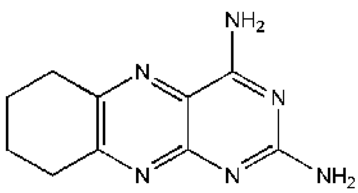

In one instance, both $R_1$ and $R_2$ are $NH_2$.

In one instance, the compound of Formula I is

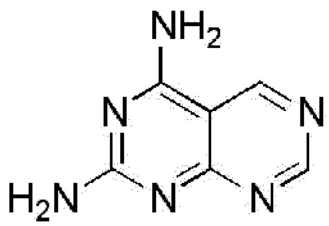

or

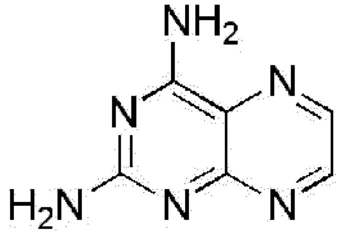

Also described in the fourth aspect of the disclosure is a ligand defined by Formula II

II

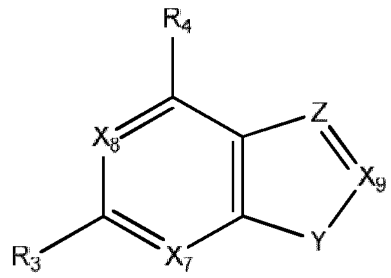

wherein

$R_3$ and $R_4$ are independently selected from a hydrogen bond donor or a hydrogen acceptor;

$X_7$, $X_8$ and $X_9$ are independently selected from CH or any hydrogen bond donor or any hydrogen bond acceptor.

Y is independently selected from N, O, S, NR, where R is independently selected from alkyl, ribose, deoxyribose, dideoxyribose, (hydroxymethyl)morpholino analogues of ribose or 5'-modified ribose;
Z is independently selected from any one of N, C attached to $-CH_2OH$, $-CH_2NH_2$, CN, or $CONH_2$
In one instance, Z is O.
In one instance, both $R_3$ and $R_4$ are $NH_2$.
In one instance, the compound of Formula II is:

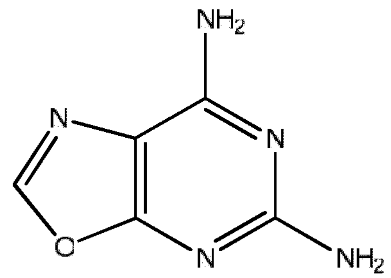

In one instance, $R_3$ and $R_4$ are independently selected from OH, $NH_2$, $NHCOCH_3$, $NHCO_2CH_3$, NH-cCH($CH_2CH_2$), SH, $NHNHCOCH_3$, $NHOCH_3$, NHCO $CH(CH_2)_3$ NHOR (where R=alkyl, aryl, or benzyl).
In one instance Formula II may be a compound of formula:

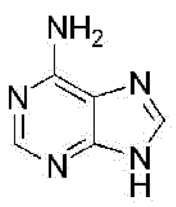
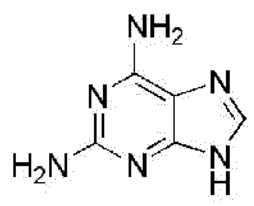
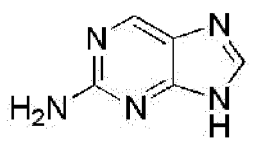

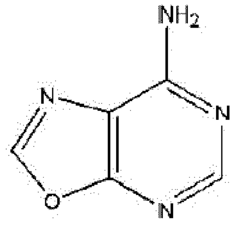
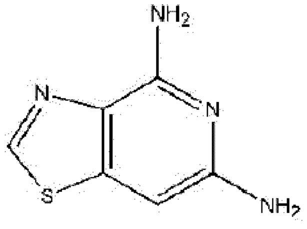
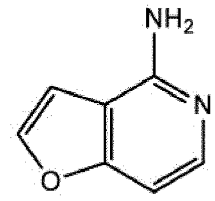

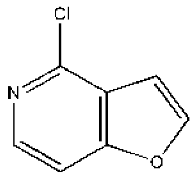
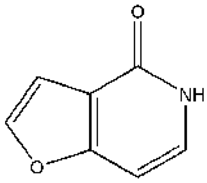
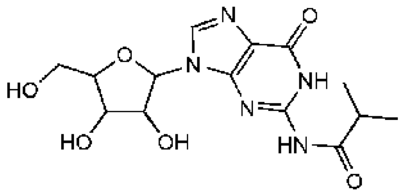
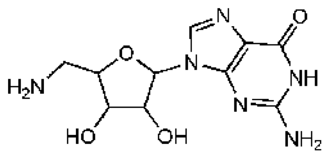

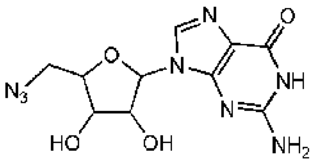
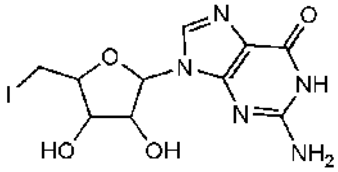

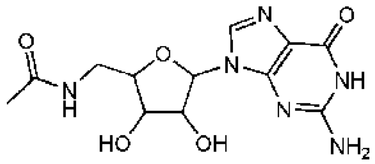
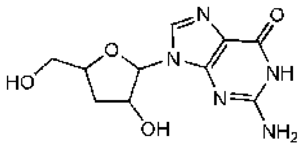
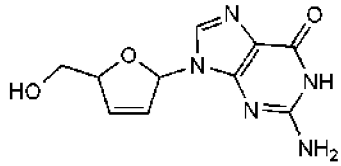

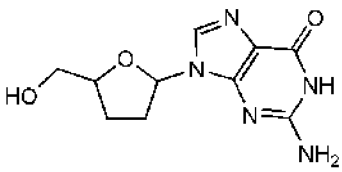
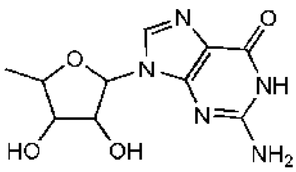
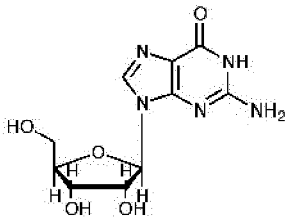

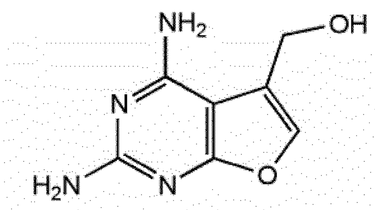
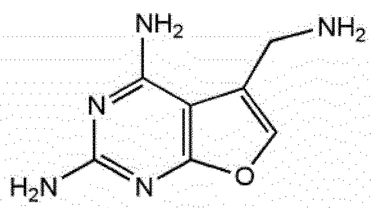
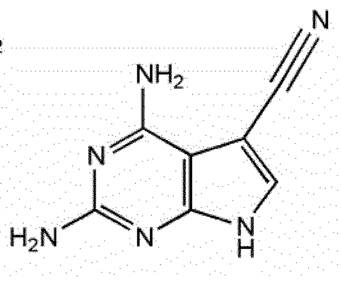

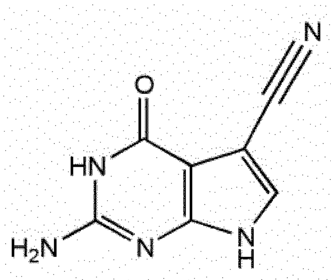
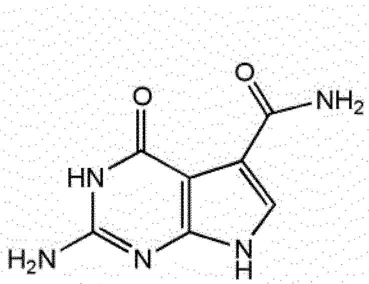
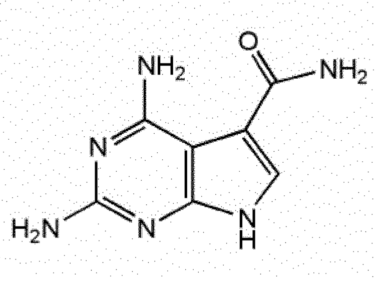

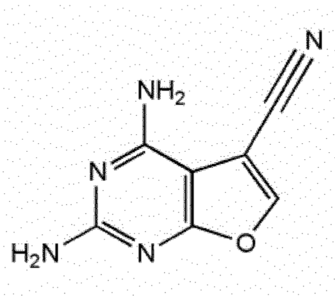 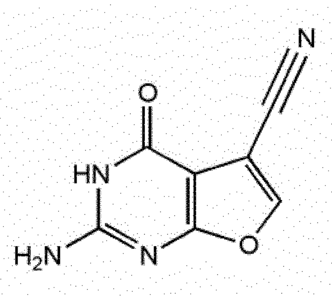 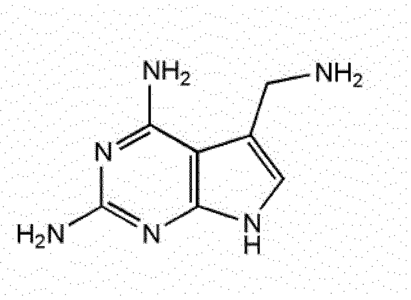

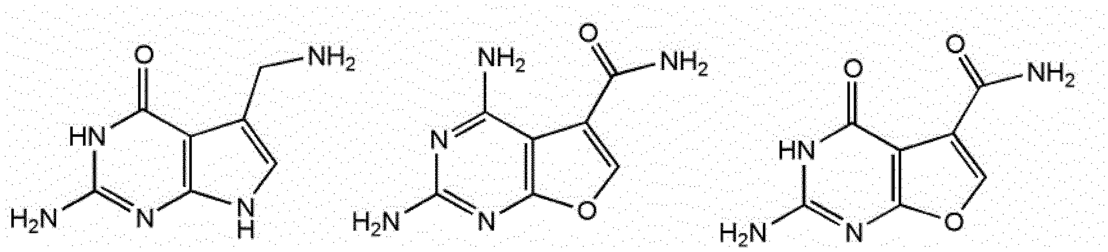

Also described herein in the fourth aspect of the disclosure is a ligand defined by Formula III

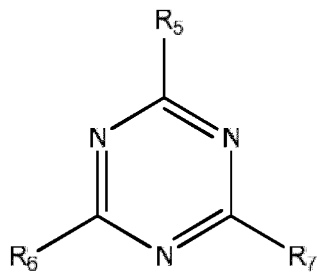

wherein

$R_5$ $R_6$ and $R_7$ is independently selected from aryl or heteroaryl; and $R_5$ is optionally substituted by $C(O)NH_2$, are independently selected from H, $NH_2$, O, OH, $NHCOCH_3$, $NHCO_2CH_3$, $NHcCH(CH_2CH_2)$, SH, $NHNHCOCH_3$, $NHOCH_3$, NHCO $CH(CH_2)_3$ NHOR (where R=alkyl, aryl, or benzyl).

In one instance, the compound of Formula III may be selected from:

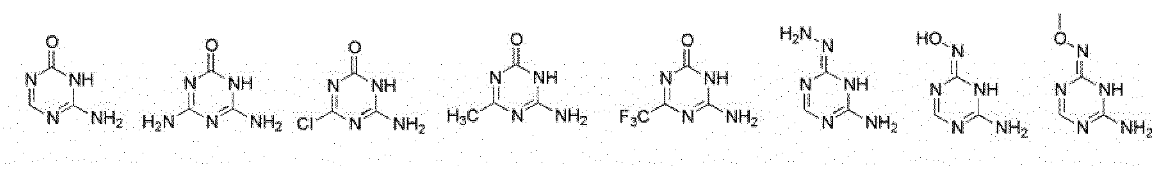

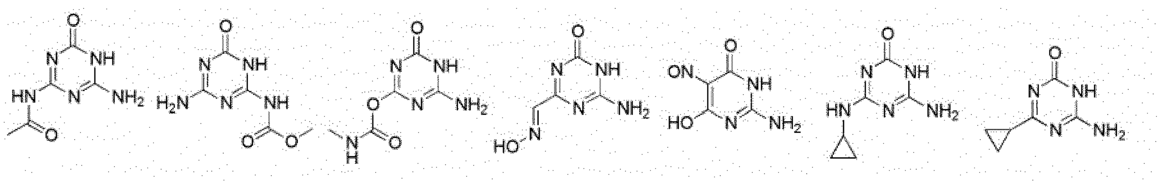

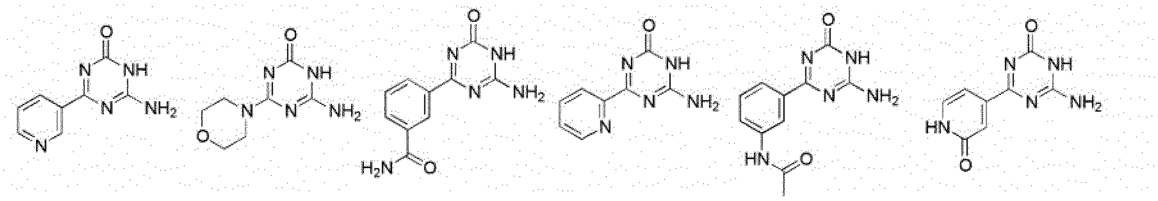

Suitably, a ligand for use in the present disclosure is a compound of any of Formulas I, II or III as herein defined.

Depending upon the groups that are selected, a first group can be incorporated within second group or, alternatively, the first group can be pendant (i.e. attached) to the second group. For example, with the phrase "an alkyl group comprising an amino group", the amino group can be attached to the backbone of the alkyl group. The nature of the group(s) that is (are) selected with determine if the first group is embedded or attached to the second group.

Unless stated to the contrary, a formula with chemical bonds shown only as solid lines are not as wedges or dashed lines contemplates each possible isomer, e.g. each enantiomer and diastereomer and a mixture of isomers such as a racemic or scalemic mixture.

A ligand of Formula I, II or III may be used in combination with any of the genetic constructs of the disclosure, as a system or kit to regulate gene expression.

In systems and kits comprising a ligand of Formula I, II or III of the fourth aspect of the disclosure, a riboswitch provided in the genetic construct may be a purine riboswitch (Kim Biol Cell 100:1 (2008)), an Add-A riboswitch, for example a modified Add-A riboswitch, for example a modified Add-A riboswitch known as M6, M6', M6" or M6C, M6C' or M6C"

(Dixon et al PNAS 107: 2830 (2010)), or a PreQ switch or a modified PreQ switch, for example the PreQ switch of Figure 14 or a riboswitch which is derived therefrom.

Alternatively, the riboswitch may be a chimeric or engineered riboswitch.

Thus, a ligand of the fourth aspect of the disclosure may be used in combination with a genetic construct comprising a riboswitch which comprises a ligand binding site of a purine riboswitch such as that of Figure 11, or one which is derived therefrom, for example having at least 50, 60, 70, 80, 85, 90, 95, 96, 97, 98 or 99% sequence identity thereto and in one instance, for example, being capable of binding a ligand of Formula I, II or III and causing a conformational change in the expression platform of the riboswitch. The conformational change may be sufficient to cause a change in expression of a coding sequence operably linked to the riboswitch. The ligand may be one of Formula I, II or III.

Thus, a ligand of the fourth aspect of the disclosure may for example be used in combination with a genetic construct comprising a riboswitch which comprises a ligand binding site of an Add-A riboswitch such as that of Figure 13, or one which is derived therefrom, such as the M6, M6', M6" or M6C, M6C' or M6C" (Dixon et al PNAS 107: 2830 (2010)). The riboswitch may be one having at least 50, 60, 70, 80, 85, 90, 95, 96, 97, 98 or 99% sequence identity thereto and may for example be capable of binding a ligand of Formula I, II or III and causing a conformational change in the expression platform of the riboswitch. The conformational change may be sufficient to cause a change in expression of a coding sequence operably linked to the riboswitch. The ligand may be be one of Formula I, II or III.

A ligand of the fourth aspect of the disclosure may also be used in combination with a genetic construct comprising a riboswitch which comprises a ligand binding site of a PreQ riboswitch such as that of Figure 14, or one which is derived therefrom, for example having at least 50, 60, 70, 80, 85, 90, 95, 96, 97, 98 or 99% sequence identity thereto and in one instance for example being capable of binding a ligand of Formula II and causing a conformational change in the expression platform of the riboswitch. The conformational change may be sufficient to cause a change in expression of a coding sequence operably linked to the riboswitch. The ligand may be one of Formula I, II or III.

Examples of ligands falling within the scope of Formula I, II or III are those having a $K_d$ of below 10$\mu$M, for example below 9, for example below 8, 7, 6, 5, 4, 3, 2 or 1.

## BRIEF DESCRIPTION OF THE DRAWINGS

Embodiments of the invention are further described hereinafter with reference to the accompanying drawings, in which:

Figure 1 is a diagrammatic illustration of examples of genetic constructs, comprising Origin of replication (ORI) Orthogonal riboswitch (ORS), T7 RNA polymerase (T7pol), T7 promoter (T7pro),
Multi-cloning site (MCS), lac promoter (lacP), chromosomal insertion site (CIS), Zeocin antibiotic resistance marker (Zeo);

Figure 2 is a diagrammatic illustration of further genetic constructs, comprising Orthogonal riboswitch (ORS), T7

RNA polymerase (T7), spacer T7 fusion protein (spT7). Lac promoter (lacP), and transcription terminator (term);

Figure 3 shows the ligand effects on eGFP expression regulated by lac promoter-operator and AddA riboswitch;

Figure 4 shows ligand effects on eGFP expression regulated by AddA riboswitch;

Figure 5 shows the ligand effects on eGFP expression regulated by M6" riboswitch;

Figure 6 shows the ligand effects on DsRED expression regulated by M6" riboswitch;

Figure 7 shows the ligand effects on eGFP expression regulated by M6" riboswitch;

Figure 8 shows the effect of a ligand and IPTG on eGFP expression regulated by an M6" riboswitch;

Figure 9 shows the ligand and IPTG effects on eGFP expression regulated by M6C" riboswitch;

Figure 10 shows the effect of ligand effects on eGFP expression regulated by M6C" riboswitch;

Figure 11 shows the consensus purine riboswitch aptamer sequence. Rfam accession number RF00167;

Figure 12 shows the nucleotide sequences of purine aptamer sequences;

Figure 13 shows the sequences of AddA derived riboswitches;

Figure 14 shows the consensus of a PreQ riboswitch and structural configuration of a PreQ riboswitch having RFMA accession number RF00522;

Figure 15 shows the upstream region of *queC* ORF. Pre Q1 riboswitch shown in bold;

Figure 16 shows a consensus mutant sequence of a PreQ riboswitch and specific mutants thereof. The present invention should be taken as encompassing any possible mutant sequence combinations from nucleotide substitutions at nucleotides R5, Y17, Y18, R29, R30, where R= purine (adenine or guanine) and Y = pyrimidine (cytosine and uracil (thymidine in DNA));

Figure 17 shows an example of anti-sense RNA sequence. Antisense complimenting the drug target sequence (RodZ);

Figure 18 shows an example of antibiotic drug target sequence controlled by PreQ riboswitch (PreQ1-MreB). PreQ1 riboswitch controlling the expression of the MreB gene, to create conditional mutant for drug candidate screening;

Figure 19 shows the examples of possible spacer sequences for use in the present invention, also showing Gibbs free energy calculations produced using DINAmelt (Markham 2005) (dG), GC content shown;

Figure 20 shows an example spacer used to separate tandem riboswitches;

Figure 21 shows an example spacer used to separate tandem riboswitches;

Figure 22 shows an example spacer used to separate tandem riboswitches;

Figure 23 shows an operon sequence, composed of lacUV5 promoter/operator, M6 riboswitch, DsRED gene, AddA riboswitch, eGFP gene;

Figure 24 shows a Dual Promoter sequence, composed of lacUV5 promoter/operator, M6 riboswitch, DsRED gene, lambda tR1 terminator, lacUV5 promoter/operator, AddA riboswitch, eGFP gene promoter;

Figure 25 shows an orthogonal Riboswitch T7 sequence, composed of lacUV5 promoter/operator, M6" riboswitch, T7 RNA polymerase;

Figure 26 shows orthogonal Riboswitch spacer T7 sequence, composed of lacUV5 promoter/operator, M6" riboswitch, spacer sequence, T7 RNA polymerase;

Figure 27 shows (A) Secondary structure model of the parental add A-riboswitch. In the OFF-state the ribosome binding site (RBS) is sequestered in a repressor stem loop blocking translation. When present at sufficient concentrations adenine binds to the aptamer domain releasing the RBS. (B) *add* A-riboswitch aptamer domain showing H-bonding contacts to the ligand adenine 1. (C) Model of the mutant M6" aptamer (U28G, G42C, U47C, U51C) H-bonding with ammeline 2. (D) 1.7 A X-ray crystal structure of the mutant M6C" (U28G, G42C, U47C, U51C, U74C) aptamer domain in complex with azacytosine 3. (E) Electron density contour map showing contacts between M6C" and azacytosine 3, with H-bonding patterns shown below (F). Note the C51 translocation relative to the addA structure;

Figure 28 shows the *lacZ* expression profiles of variant $preQ_1$ riboswitches. A Sequence of $preQ_1$ riboswitch constructs. The mutation sites are circled. The hydrogen bond interactions between $preQ_1$ binding residues and ligand $preQ_1$ are presented as Watson-Crick pairs (▬), Cis Watson-Crick/Hoogsteen (■—●), Trans Watson-Crick/Sugar Edge (o—▷) and single hydrogen bond interaction (▪ ▪ ▪). B The regulation of β-galactosidase activity by the wild-type $preQ_1$ riboswitch and by the $preQ_1$ riboswitch mutants. The β-galactosidase activities are presented as Miller Units (left axis indicated by the blue bars) and the error bars represent the mean±SD of four replicates. The derepression ratios (right axis indicated as brown squares) are calculated by comparing with the C17U, C18U mutant strain;

Figure 29 shows the effect of the $PreQ_0$ ligand on the *lacZ* expression of variant $preQ_1$ riboswitches at 16°C;

Figure 30 shows the effect of the ligand D6 on the *lacZ* expression of variant $preQ_1$ riboswitches;

Figure 31 shows the effect of the ligand D9 on the *lacZ* expression of variant $preQ_1$ riboswitches;

Figure 32 shows the dose-response curves and growth inhibition of the compound D9. The dose-response curve with $IC_{50}$ values 0.498 mM was obtained by the *in-vivo* lacZ activity assay with an initial concentration of 6.25 μM to a final concentration of 4 mM. The $IC_{50}$ value was calculated by non-linear regression analysis (four parameter logistic equation) using Sigmaplot 12.0;

Figure 33 shows PreQ Applications with Target genes *rodZ* and *mreB*;

Figure 34 shows multicomponent riboswitch gene expression systems. A Selective inducer molecules ammeline, and 2-aminiopurine. Dual Promoter construct comprised of promoter/operator (lacP/O), the M6 ammeline responsive riboswitch in front of the red fluorescent protein DsRed, lambda tR1 terminator, a second lacP/O lac promoter, the *add*-A 2-aminopurine responsive riboswitch in front of the green fluorescent protein eGFP. B DsRed and C eGFP Multi-variant dose matrix analysis of induction factors;

Figure 35 shows multicomponent riboswitch gene expression systems. A Selective inducer molecules ammeline, and 2-aminopurine. Operon construct comprised of promoter/operator (lacP/O), the M6" ammeline responsive riboswitch in front of the red fluorescent protein DsRed, the *add*-A 2-aminopurine responsive riboswitch in front of the green fluorescent protein eGFP. B DsRed and C eGFP multi-variant dose matrix analysis of induction factors;

Figure 36 shows dose-response curves for dual promoter construct: (A) DsRed; (B) eGFP expression;

Figure 37 shows dose-response curves for operon construct: (A) DsRed; (B) eGFP expression;

Figure 38 shows the results of fluorescent microscopy: A Dual promoter construct B Operon construct. Induced with IPTG (1mM), ammeline 1 (250μM), and 2-aminopurine 2 (250μM). (top left) GFP channel, (top right) DsRED channel, (bottom left) Phase contrast (bottom right) Overlay of channels. Scale bar equal to 5 μm for reference;

Figure 39 shows a multi-variant dose matrix analysis of absolute normalized protein expression levels showing dual promoter relative protein expression stoichiometry ratio. Gradient lines range from 1:5 on the left hand side of the matrix, to 4:1 on the lower right hand side;

Figure 40 shows a multi-variant dose matrix analysis of absolute normalized protein expression levels showing operon relative protein expression stoichiometry ratio. Gradient lines range from 1:5 on the left hand side of the matrix, to 4:1 on the lower right hand side;

Figure 41 shows genetic constructs containing M6" riboswitch, T7 RNAP, second construct containing T7 promoter ahead of any gene of interest. $P_{lac}$ = lac UV5 promoter, $O_{lac}$ = lac operator, $P_{T7}$ = T7 promoter, $P_{T3}$ = T3 promoter, MCS = Multi-cloning site, EP = expression plasmid e.g. pET, POI = protein of interest;

Figure 42 shows multi component genetic containing dicistronic M6" riboswitch-T7 RNAP, addA riboswitch-T3 RNAP and a second construct containing the T7 and T3 promoters sequence ahead of any genes of interest, (referred to as pTTv7). $P_{lac}$ = lac UV5 promoter, $O_{lac}$ = lac operator, $P_{T7}$ = T7 promoter, $P_{T3}$ = T3 promoter, MCS = Multi-cloning site, EP = expression plasmid e.g. pET, mPOI = multimeric protein of interest;

Figure 43 shows differences in lacZ induction by riboswitches (both addA and M6) in genetic constructs in which a spacer is absent, or in which a spacer is present (constituting examples of genetic constructs in accordance with the first aspect of the disclosure);

Figure 44 shows differences in induction of the fluorescent marker DsRed by riboswitches in genetic constructs in which a spacer is absent, or in which a spacer is present (constituting examples of genetic constructs in accordance with the first aspect of the disclosure);

Figure 45 shows differences in induction of T7 RNA polymerase by riboswitches in genetic constructs in which a spacer is absent, or in which a spacer is present (constituting examples of genetic constructs in accordance with the first aspect of the disclosure);

Figure 46 shows differences in T7 RNA polymerase expression in response to increasing amounts of a riboswitch ligand for comparator genetic constructs in which a spacer is absent, or for genetic constructs of the invention incorporating spacer sequences of variable lengths (30, 45, 60, 75, 90, 93, 96, 99, 102, 105, 120, 135, 150 or 165 nucleotide lengths) and for a control genetic construct lacking a spacer sequence (designated "nts"). Expression is induced using IPTG (0.5mM) and increasing concentrations of A43 (respectively 0,5, 10, 25, 50,100, 250uM) shown in the third to eighth columns;

Figure 47 shows three graphs representing differing induction of T7 RNA polymerase expression in response to increasing amounts of a riboswitch ligand for genetic constructs of the invention incorporating spacer sequences of variable lengths (as before) and for a control genetic construct lacking a spacer sequence;

Figure 48 compares T7 RNA polymerase expression in genetic constructs of the invention in which lac I is inserted in either the same or the opposite direction to the T7 RNA polymerase open reading frame;

Figure 49 demonstrates the decoupled concept where an inducible promoter coupled to a riboswitch permits dose dependent control over the expression pf T7 RNAP, and by using a 2$^{nd}$ generation construct with the T7 promoter sequence and a gene of interest cloned downstream from said promoter, dose dependent control of gene (of interest) expression is permitted in a de-coupled manner;

Figure 50 shows a systems containing two constructs, first a construct that permits control of the T7 RNAP (either BL21(DE3), or the low copy vector p15LOST (containing the lacl repressor, the orthogonal ricoswicth (M6"), a spacer sequence and the T7RNAP). The second construct a pET vector containing the 50kDa protein MppK. Here the de-couple riboswitch construct is shown to permit dose-dependent regulation of the gene of interest (MppK, upon addition of IPTG and riboswitch inducer A43);

Figure 51 shows a systems containing two constructs, first a construct that permits control of the T7 RNAP either BL21(DE3), or an E.coli strain containing the low copy vector p15LOST (containing the lacl repressor, the orthogonal ricoswicth (M6"), a spacer sequence and the T7RNAP). The second construct a pET vector containing the GFP protein Here the de-coupled riboswitch construct is shown to permit dose-dependent regulation of the gene of interest (GFP), upon addition of IPTG and riboswitch inducer A43);

Figure 52 shows GFP expression data for constructs with and without the riboswitch (AddA), with addition fo IPTG and riboswitch inducer (A1);

Figure 53 shows GFP expression data for constructs with and without the riboswitch (M6"), with addition fo IPTG and riboswitch inducer (A43);

Figure 54. shows the cellular distribution of GFP expression analysed by FACS;

Figure 55 shows the sensitivity of the B.subtilis MreB knockout, and the reconstituted MreB under the control of the repressible PreQ C17U riboswitch mutant, providing a conditional mutant upon addition of D9;

Figure 56 shows in a first panel the genetic construct pMAST3-h6, transcriptionally regulated by a lac promoter/operator containing the addA riboswitch - A1 ligand combination, controlling the expression if the T3 RNA polymerase-h6; in a second panel, SDS analysis of cell lysates expression induced with 0.5mM IPTG, and A1 at 0.8, 4, 20, 100, 250, 250uM is shown, illustrating dose-dependent control of expression; the third panel shows Western blot analysis with anti-his6 antibody. The construct is omposed of lacUV5 promoter/operator, AddA riboswitch, spacer, T3 RNA polymerase with a C-his6 tag;

Figure 57 shows, in panel "a", a dual promoter construct transcriptionally regulated by a lac promoter/operator, where the M6" riboswitch - A43 ligand combination control the expression of the spT7 RNA polymerase, separated by at tR1 transcriptional terminator, a second lac promoter/operator and the addA riboswitch - A1 ligand combination control the expression if the T3 RNA polymerase (pTTv9)(top); and a dicistronic operon transcriptionally regulated by the lac promoter/operator, where the M6" riboswitch - A43 ligand combination control the expression of the spT7 RNA polymerase, and the addA riboswitch - A1 ligand combination control the expression if the spT3 RNA polymerase (pTTv7) (below). Panel "b" shows SDS analysis of cell lysates; panel "c" shows Western blot analysis with anti-T7RNAP antibody; and panel "d" shows Western blot analysis with anti-his6 antibody;

Figure 58 shows the dose dependent control of eGFP expression from constructs WT (AddA) and M6"(ORS), with selective inducers Adenine (Ad) Ammeline (Am), pyrimido[4,5-*d*]pyrimidine-2,4-diamine (A43);

Figure 59 shows isothermal titration calorimetry plots between the riboswitch aptamers WT (AddA) and M6"(ORS), pyrimido[4,5-*d*]pyrimidine-2,4-diamine (A43);

Figure 60, shows isothermal titration calorimetry plots between the riboswitch aptamers WT (AddA) and M6"(ORS), 2,4-Diaminopteridine (A44);

Figure 61 Isothermal titration calorimetry plots between the riboswitch aptamers M6C" and ligands;

Figure 62 shows the structure and sequence of the AddA, M6" and M6C" aptamers;

Figure 63 shows additional examples of spacer sequences further to (Figure 19)
Gibbs free energy calculated using DINAmelt (Markham 2005) (dG), GC content shown;

Figure 64 shows pMAST-h6 sequence, composed of lacUV5 promoter/operator, AddA riboswitch, spacer, T3 RNA polymerase with a C-his6 tag;

Figure 65 shows the pTTv7 (operon) sequence, composed of lacUV5 promoter/operator, M6" riboswitch, spacer, T7 RNAP gene, AddA riboswitch, spacer T3 RNAP gene;

Figure 66 pTTv9 (dual promoter) sequence, composed of lacUV5 promoter/operator, M6" riboswitch, spacer, T7 RNAP gene, lambda tR1 terminator, lacUV5 promoter/operator, AddA riboswitch, spacer T3 RNAP gene;

Figure 67 shows the dose-response curves for the dual promoter construct (a) DsRed (b) eGFP expression (update of Figure 36), and for the operon construct (c) DsRed (d) eGFP expression (update of Figure 37).

## DETAILED DESCRIPTION

The present invention provides novel engineered expression systems comprising riboswitches, which allow orthogonally selective, tunable and/or dose dependent control of gene expression in response to a ligand. The different aspects of the present invention have applicability in a range of different fields, including for example functional genomics, target validation and drug development, systems chemical biology, NOR boolean logic gates, synthetic biology, plant

biology, gene therapy, recombinant protein production, pathway engineering and directed evolution, bioremediation and metabolic engineering.

DEFINITIONS

"Antisense sequence" nucleic acid molecules that are specifically hybridizable or specifically complementary to either RNA or the plus strand of DNA. In a cell, the antisense nucleic acids hybridize to the corresponding mRNA, forming a double-stranded molecule. The antisense nucleic acids interfere with the translation of the mRNA.

"Aptamer domain" is a part of a riboswitch which is capable of binding a ligand. Preferably, ligand binding results in a structural change in the expression platform of the riboswitch, which is operably linked to the aptamer domain, and effects regulation of the expression of the nucleic acid sequence which is regulated by the riboswitch. An aptamer domain is a nucleic acid sequence, preferably an RNA sequence, and preferably encoded by a DNA sequence. Binding of the aptamer domain to a ligand is preferably selective, to a particular class of type of ligands.

"Contiguous" such that the last nucleic acid residue of one nucleic acid molecule is adjacent to the first nucleic acid residue of another of the first, second or third nucleic acid molecules.

"Dynamic range" is defined as the difference between high and low protein levels. "Expression" herein means either transcription or translation or both, i.e. From DNA to RNA, and/or RNA to protein or peptide. A riboswitch used in the present invention may operate at either the transcriptional or translational level.

"Expression platform" Within the context of the present invention, the portion of the riboswitch which mediates the effect on the nucleic acid expression is known as the expression platform. Preferably, the expression platform is operably linked to the aptamer domain of the riboswitch, preferably structurally linked, most preferably by a nucleic acid linker. Most preferred is that the aptamer domain is linked to the expression platform by a nucleic acid sequence. Preferably, the stem configuration of the portion of the expression domain changes configuration upon binding of a ligand molecule, such that a change in the configuration of the stem structure results in a corresponding change in the structure of the expression platform, between a first configuration which enhances expression of the nucleic acid sequence and a second configuration which inhibits expression of the nucleic acid sequence.

"Genetic construct" can include nucleic acid sequences that permit it to replicate in the host cell, such as an origin of replication. Examples include, but are not limited to a plasmid, cosmid, bacteriophage, or virus that carries exogenous DNA into a cell. A genetic construct can also include one or more cytotoxic genes, antisense molecules, and/or selectable marker genes and other genetic elements known in the art. A genetic construct can preferably transduce, transform or infect a cell, thereby causing the cell to express the nucleic acids and/or proteins encoded by the vector. A genetic construct optionally includes materials to aid in achieving entry of the nucleic acid into the cell, such as a liposome, protein coating or the like.

"Isolated" biological component (such as a nucleic acid or protein) has been substantially separated, produced apart from, or purified away from other biological components in the cell of the organism in which the component naturally occurs, i.e., other chromosomal and extra chromosomal DNA and RNA, and proteins. Nucleic acids and proteins that have been "isolated" include nucleic acids and proteins purified by standard purification methods. The term also embraces nucleic acids and proteins prepared by recombinant expression in a host cell as well as chemically synthesized nucleic acids, proteins and peptides.

"Operably linked" a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame. Nucleic acid: a deoxyribonucleotide or ribonucleotide polymer in either single or double stranded form, and unless otherwise limited, encompasses known analogues of natural nucleotides that hybridize to nucleic acids in a manner similar to naturally occurring nucleotides.

"Orthogonal" in relation to the riboswitches disclosed herein means that they have greater selectivity for non-native, non-natural and/or synthetic ligands than for a natural, native ligand. More preferably, an orthogonal riboswitch responds to a non-native, non-natural and/or synthetic ligand to the same extent in terms of the effect on coding sequence expression as to a native, natural or intracellular ligand and shows substantially no response to a native, natural and/or intracellular ligand.

"Oligonucleotide": a linear polynucleotide (such as DNA or RNA) sequence of at least 9 to 35 nucleotides, for example at least 15, 18, 24, 25, 27, 30, 50, 100 or even 200 nucleotides.

"Peptide": a chain of amino acids of which is at least 4 amino acids in length. In one example, a peptide is from about 4 to about 30 amino acids in length, for example about 8 to about 25 amino acids in length, such as from about 9 to about 15 amino acids in length, for example about 9-10 amino acids in length.

"Promoter": an array of nucleic acid control sequences that directs transcription of a nucleic acid. A promoter includes necessary nucleic acid sequences near the start site of transcription, such as, in the case of a polymerase ii

type promoter, a TATA element. A promoter also optionally includes distal enhancer or repressor elements that can be located as much as several thousand base pairs from the start site of transcription. Both constitutive and inducible promoters are included.

"Riboswitch" means an expression control element, which is preferably in the form of an RNA molecule. A riboswitch comprises a ligand binding domain, also referred to herein and in the art as an aptamer domain, and a regulatory domain which influences gene expression, and is also known as the expression platform of the riboswitch. For a riboswitch to respond to the effects of a particular ligand by regulating gene expression, it must comprise a ligand binding domain and a regulatory domain.

"Regulatory sequence" is a sequence which serves to control an element of transcription or translation of a coding sequence. For example, it may encode an antisense RNA sequence which regulates expression of a coding sequence by binding thereto or to a transcript thereof. Alternatively, it may encode a regulatory element such as a promoter, enhancer or transcription factor binding site, or may encode a gene product which controls expression, such as a transcription factor, a polymerase, a repressor, a translation initiation factor.

"Secondary structure" in a nucleic acid sequence is the three dimensional structure formed by hydrogen bonds between the two bases. Secondary structure includes Watson and Crick base pairing, wobble pairings, stems and loops, hairpins, pseudoknots, duplexes, triplexes, tetraplexes. When measured using a predictive tool for secondary structure such as Genscan (Burge et al; J. Mol. Biol. 268, 78-94); Genviewer . Suitable folding packages for determining secondary structure include RNA Vienna (Hofacker NAR 1: 3429 (2003)), mfold (Zuker NAR 31: 3406 (2003), DINAMelt (Markham NAR 33: W577 (2005)), CMFinder (Yao et al BioInfo 22:445 (2006)).

"Sequence identity" is expressed as a percentage. The measurement of sequence identity of a nucleotide sequences is a method well known to those skilled in the art, using computer implementated mathematical algorithms such as ALIGN (Version 2.0), GAP, BESTFIT, BLAST (Altschul et al J. Mol. Biol. 215: 403 (1990)), FASTA and TFASTA (Wisconsin Genetic Software Package Version 8, available from Genetics Computer Group, Accelrys Inc. San Diego, California), and CLUSTAL (Higgins et al, Gene 73: 237-244 (1998)), using default parameters.

Nucleic acid molecules defined herein as having sequence identity with a reference sequence may alternatively be defined as being capable of hybridising under stringent conditions to the complement of the reference sequence. Stringent hybridisation conditions are defined as those conditions under which a nucleotide sequence will preferentially hybridize to a target sequence. Increasing the stringency of the hybridisation conditions enables sequences of higher sequence identity to be found. Typical hybridisation conditions are 30-60°C, pH 7.0 to 8.3 and a salt concentration of less than 1.5 M $Na^+$ ions. Preferred stringent hybridisation conditions hybridisation in 1M NaCl, 1% SDS at 37°C, and 50% formamide and washing in 0.1xSSC at 60 to 65°C.

"Structurally linked" herein means linked by a nucleic acid residue or protein or other component, so that they form part of the same structural entity, although they are not necessarily contiguous (i.e. adjacent) to one another in the structure.

"same or different riboswitches" - by the same means that they are the same riboswitch, from the same family and of the same subtype of that family, having substantially the same sequence and structure, and being able to mediate substantially the same regulatory effect on a coding sequence to which they are operably linked. Different riboswitches may be from different families or may be from the same family but be of different subtypes.

"heterologous" sequences are sequences which in nature are not operably linked to each other and/or are not found next to each other in a native sequence. In contrast, homologous sequences refer to sequences which share sequence similarity, which may be described as sequence homology. Homology is usually in a fragment of the sequence, typically in a functional domain of the sequence.

The term "substituted" herein includes all possible substituents or organic compounds. Preferably, the substituents include acyclic and cyclic, branched and unbranched, carbocyclic and heterocyclic, and aromatic and non-aromatic substituents of organic compounds. Preferred substituents include, for example, those described below. The substituents can be one or more and the same or different for appropriate organic compounds. In the present invention, the heteroatoms, such as nitrogen, can have hydrogen substituents and/or any permissible substituents of organic compounds described herein which satisfy the valences of the heteroatoms. In addition, the terms "substitution" or "substituted with" include the possibility that such substitution is in accordance with permitted valence of the substituted atom and the substituent, and that the substitution results in a stable compound, e.g. a compound that does not spontaneously undergo transformation such as by rearrangement, cyclization, elimination, etc.

"$R^1$," "$R^2$," "$R^3$,", "$R^4$", "$R^5$, "$R^6$, "$R^7$, X, Y and Z herein are generic symbols to represent various specific substituents. These symbols can be any substituent, and when they are defined to be certain substituents in one instance, they can, in another instance, be defined as some other substituents.

The term "alkyl" as used herein is a branched or unbranched saturated hydrocarbon group of 1 to 24 carbon atoms, such as methyl, ethyl, n-propyl, isopropyl, n-butyl, isobutyl, t-butyl, pentyl, hexyl heoptyl octyl, nonyl, decyl, dedecyl, tetradecyl, hexadecyl, eicosyl, tetracosyl, and the like. The alkyl group can also be substituted or unsubstituted. The alkyl group can be substituted with one or more groups including, but not limited to, alkyl, halogenated alkyl, alkoxy,

alkynyl, aryl, heteroaryl, aldehyde, amino, carboxylic acid, ester, ether, halide, hydroxy, ketone, sulfo-oxo, sulfonyl, sulfone, sulfoxide, or thiol, as described below. The term "lower alkyl" is an alkyl group with 6 or fewer carbon atoms, e.g., methyl, ethyl, propyl, isopropyl, butlyl, sec-butyl, iso-butyl, tert-butyl, pentyl, hexyl, and the like.

Herein "alkyl" is generally used to refer to both unsubstitued alkyl groups and substituted alkyl groups; however, substituted alkyl groups are also specifically referred to herein by identifying the specific substituents(s) on the alkyl group. For example, the term "halogented alkyl" specifically refers to an alkyl group that is substituted with one or more halide, e.g., fluorine, chlorine, bromine, or iodine. The term "alkoxyalkyl" specifically refers to an alkyl group that is substituted with one of more alkoxy groups, as described below. The term "alkylamino" specifically refers to an alkyl group that is substituted with one of more amino groups, as described below, and the like. When "alkyl" is used in one instance and a specific term such as "halogenated alkyl" is used in another, it is not meant to imply that the term "alkyll" does not also refer to specific terms such as "halogenated alkyl" and the like.

This is the same for other groups described herein. That is, while a term such as "cycloalkyl" refers to both unsubstituted and substituted cycloalkyl moieties, the substituted moieties can, in addition, be specifically identified herein; for example, a particular substituted cycloalkyl can be referred to as, e.g., a "alkylcycloalkyl". Similarly, a substituted alkoxy can be specifically referred to as, e.g. an "halogenated alkoxy," a particular substituted alkenyl can be, e.g., an "alkenylalcohol", and the like. Again, the practice of using a general term, such as "cycloalkyl", and a specific term, such as "alkylcycloalkyl", is not meant to imply that the general term does not also include the specific term.

The term "aryl" as used herein is a group that contains any carbon-based aromatic group including, but not limited to, benzene, naphthalene, phenyl, biphenyl, phenoxybenzene, and the like. The term "aryl" also includes "heteroaryl", which is defined as a group that contains an aromatic group that has at least one heteroatom incorporated within the ring of the aromatic group. Examples of heteroatoms include, but are not limited to, nitrogen, oxygen, sulphur and phosphorus. Likewise, the term "non-heteroaryl", which is also included in the term "aryl", defines a group that contains an aromatic group that does not contain a heteroatom. The aryl group can be substituted or unsubstituted. The aryl group can be substituted with one or more groups including, but not limited to alkyl, halogenated alkyl, alkoxy, alkenyl, aryl, heteroaryl, aledhyde, amino, carboxylic acid, ester, ether, halide, hydroxyl, ketone, sulfo-oxo, sulfonyl, sulfone, sulfoxide, or thiol as described herein. The term "biaryl" is a specific type of aryl group and is included in the definition of aryl. Biaryl refers to two aryl groups that are bound together *via* one or more carbon-carbon bonds, as in biphenyl.

The term "cylcoalkyl" as used herein is a non-aromatic carbon-based ring composed of at least three carbon atoms. Examples of cycloalkyl groups include, but are not limited to, cyclopropyl, cyclobutyl, cyclopentyl, cyclohexyl etc. The term "heterocycloalkyl" is a cycloalkyl group as defined above where at least one of the carbon atoms of the ring is substituted with a heteroatom such as, but not limited, to nitrogen, oxygen, sulphur or phosphorus. The cycloalkyl group and the heterocycloalkyl group can be substituted with one or more groups including, but not limited to alkyl alkoxy, alkenyl, alkenyl, aryl, heteroaryl, aldehyde, amino, carboxylic acid, ester, ether, halide, hydroxy, ketone, sulfo-oxo, sulfonyl, sulfone, sulfoxide, or thiol as described herein.

The term "cyclic group" is used herein to refer to either aryl groups, non-aryl groups (i.e. cylcoalkyl, heterocycloalkyl, cycloalkenyl, and heterocycloalkenyl groups) or both. Cyclic groups have one or more ring systems that can be substituted or unsubstituted. A cyclic group can contain one or more aryl groups, one or more non-aryl groups or one or more aryl groups and one or more non-aryl groups.

RIBOSWITCHES

Riboswitches in the present disclosure include isolated, purified and recombinant riboswitches. They may be naturally occurring riboswitches, or derived therefrom, or may be artificially created, engineered riboswitches. A naturally occurring riboswitch is one which is found in nature, typically from a bacteria, eukaryote or archea, and preferably has been isolated or purified therefrom. It may be an isolated, purified or recombinant form of a riboswitch found in nature. This means that it has been extracted from a naturally occurring source, and retains the same sequence, structure and function as the riboswitch found in nature, or if recombinant, it is the same riboswitch as found in nature, but has been derived from a new genetic source.

A riboswitch which is derived from a naturally occurring riboswitch may have modified sequence, structure or function to the naturally occurring riboswitch, but may retain substantially the same biological or functional activity (e.g. in terms of dynamic range and output). Modified sequence may mean a derivative of a naturally occurring riboswitch which has at least 50%, 60%, 70%, 75%, 80%, 85%, 90%, 95%, 96%, 97%, 98% or 99% sequence identity to the naturally occurring riboswitch, over a region of 20, 30, 40, 50, 100, 200, 300, 400, or 500, up to the full length of the naturally occurring riboswitch. "Modified structure" means that it retains substantially the same structure as the naturally occurring riboswitch, but may be modified, for example, in terms of the effectiveness or result of ligand binding, degree of interaction and effect on the expression platform, or degree of gene regulation upon ligand binding. By substantially the same means that the riboswitches ability to bind a particular ligand is not changed, although its affinity for the ligand

may be increased or decreased. Similarly, the downstream effect of the riboswitch remains the same in terms of its ability to fold and regulate coding sequence expression, but the parameters within which this is achieved may be altered. Thus, it may retain substantially the same binding properties and gene regulation activity as the native riboswitch. Suitable folding packages for determining secondary structure include RNA Vienna (Hofacker NAR 1: 3429 (2003)), mfold (Zuker NAR 31: 3406 (2003), DINAMelt (Markham NAR 33: W577 (2005)), CMFinder (Yao et al BioInfo 22:445 (2006)).

Also provided are engineered riboswitches. Engineered riboswitches have sequence alterations in terms of substitutions, additions or deletions which serve to affect their ability to bind natural, native or intracellular ligands and also their ability to bind non-natural, non-native and/or synthetic ligands. They may have a modified structure in terms of the effectiveness or result of ligand binding, degree of interaction and effect on the expression platform, or degree of gene regulation upon ligand binding. Engineered riboswitches may also show altered dynamic range, or output compared to the native riboswitch from which they are derived. Engineered riboswitches may be orthogonal. They may have an increased or decreased dynamic range compared to the native riboswitch from which they are derived. Engineered riboswitches include, for example, riboswitches which include the consensus sequences of native riboswitches, but the remainder of the riboswitch (the non-consensus portions) have been altered. Engineered riboswitches may retain at least 50%, 60%, 70%, 80%, 90%, 95%, 96%, 97%, 98% or 99% sequence identity over a region of 50%, 60%, 70%, 80%, 90%, 95%, 96%, 97%, 98% or 99% of contiguous nucleic acid residues of a consensus sequences of the native riboswitch, and/or has at least 50%, 60%, 70%, 80%, 90%, 95%, 96%, 97%, 98% or 99% similarity in terms of structure with the consensus sequence of the native riboswitch, when measured using packages such as those defined above.

Also included are chimeric riboswitches, which may comprise portions, domains or functionality from two or more riboswitches. For example, the aptamer domain may be provided from a first riboswitch, and the regulatory domain or expression platform from a second riboswitch. Chimeric riboswitches may also be consensus riboswitches, in that each domain may comprise the consensus sequence of the domain of the riboswitch from which is obtained. Chimeric riboswitches may comprise part of a riboswitch from a riboswitch of a particular class or type, and part of a riboswitch from the same class or type of riboswitch, or part of a riboswitch from a different class or type of riboswitch. Also included within the present disclosure are chimeric riboswitches which comprise part of a riboswitch and non-riboswitch sequence or parts. Also envisaged is that an aptamer domain and/or an expression platform may be non-riboswitch derived (i.e. may come from another source), and when placed with a heterologous aptamer domain or expression platform, forms a chimeric molecule which functions as a riboswitch. Each domain (i.e. aptamer domain or expression platform) may have a native structure, and therefore native activity in terms of effectiveness or result of ligand binding, degree of interaction and effect on the expression platform, or degree of gene regulation upon ligand binding Alternatively, the combination of domains in a chimeric riboswitch may exhibit altered effectiveness of ligand binding, degree of interaction and effect on the expression platform, or degree of gene regulation upon ligand binding. They may have an altered dynamic range, or output compared to the native riboswitch from which they are derived. Chimeric riboswitches may also be engineered. Chimeric riboswitches may be orthogonal.

The riboswitch may comprise one or more (e.g. 2, 3, 4, 5, or 6 or more) aptamer domains. Where two or more aptamer domains are present in a riboswitch, these may exhibit cooperative binding of ligands, or independent binding of ligands. The two or more aptamer domains may be the same or different, and may bind the same or different ligands to one another.

Also envisaged is a riboswitch comprising a single aptamer domain which is capable of binding two or more ligands simultaneously, which in some scenarios may be necessary to mediate the conformational change required to regulate coding sequence expression. Thus, reference herein to a ligand includes reference to the combined use of two or more ligands simultaneously.

Riboswitches for use in the present disclosure include purine riboswitches, for example those of Figure 11, an Add-A riboswitch or one which is derived therefrom, such as the M6, M6’, M6" or M6C, M6C’ or M6C" such as those of Figure 13 or Figure 62 (Dixon et al PNAS 107: 2830 (2010)), or a PreQ riboswitch such as that of Figure 14 or a PreQ mutant consensus sequence such as that of Figure 16.

A riboswitch for use in the present invention is a naturally occurring riboswitch or a modified riboswitch having at least 85% sequence identity to a naturally occurring riboswitch.

A riboswitch used in the present disclosure may be controlled by any suitable ligand. Suitable ligands are defined herein, and many examples are known to persons skilled in the art. Binding of a ligand to a riboswitch may cause activation (i.e. cause a conformational change in the expression platform leading to up-regulation of coding sequence expression); repression (i.e. cause a conformational change in the expression platform leading to down-regulation of coding sequence expression); inhibition (i.e. prevention or reduction in the conformational change of the expression platform, leading to an inhibition of the riboswitch mediated regulation of coding sequence expression) and blocking of the riboswitch (i.e. prevention of any conformational change in the expression platform). The effect of the ligand on the riboswitch is non-constitutive.

The effect of the ligand on the riboswitch may take place when the ligand reaches a threshold level in the expression system or host cell comprising the genetic construct of the disclosure.

The threshold level for a particular ligand will be the specific concentration within the expression system or host cell at which the riboswitch undergoes a conformational change. For any particular ligand/riboswitch combination, a threshold level can be determined by a person skilled in the art using standard methodology. Such methodology can be used to then produce a standard dose-expression curve for any particular ligand and riboswitch combination.

## APTAMER DOMAIN

The aptamer domain may be a naturally occurring aptamer domain, or may be derived, or re-engineered therefrom or artificially created. It may be isolated, purified or recombinant. Alternatively, it may not be derived from a native riboswitch, but may be a ligand binding domain of an RNA molecule, which when operably linked to an expression platform is capable of acting as an aptamer domain of a riboswitch.

The aptamer domain may have dual functionality, meaning that it is able to bind a ligand which mediates an effect on the conformational change in the expression platform (i.e. an activation or an inhibition or a blocking thereof), and also upon binding is able to mediate other downstream/upstream effects, such as control of other RNA molecules, stabilisation of mRNA, protection from mRNA degradation nuclease or chemical, or other genetic switches.

The aptamer domain may comprise a consensus sequence, which is a motif or common sequence shared by a particular type or class of riboswitches or aptamer domains, for example dictated by the class or type of ligands bound by the aptamer domain. Consensus sequences for aptamer domains of riboswitches will be known in the art.

The aptamer domain of a riboswitch of the disclosure may be chimeric, for example comprising a portion of an aptamer domain of one riboswitch, and a portion of an aptamer domain of one or more different riboswitches. In this way, different functionalities such as preference for ligand binding and effect on the expression platform can be engineered into a particular aptamer domain. Such aptamer domains may comprise at least a consensus sequence for a ligand binding portion of the aptamer domain, or a sequence which is derived therefrom and has at least 50, 60, 70, 80, 85, 90, 95, 96, 97, 98, or 99% sequence identity over at least 50, 60, 70, 80, 85, 90, 95, 96, 97, 98 or 99% of the length of the consensus sequence.

Examples of aptamer domains include those derived from purine riboswitches, for example those of Figure 12; those of an Add-A riboswitch or a riboswitch derived therefrom such as the M6, M6', M6" or M6C, M6C' or M6C" (Dixon et al PNAS 107: 2830 (2010)) riboswitches of Figure 13; or those of a PreQ riboswitch such as that of Figure 14 (Roth, Breaker. Annu Rev Biochem 78:305 (2009), Barrick, Breaker Genome Biol 8:R239 (2007)). Examples of specific aptamer domains for use in the present disclosure are the purine aptamer domains of Figure 12.

The aptamer domain may be operably linked to the expression platform of the riboswitch. It may be structurally linked, forming party of the same nucleic acid molecule. It may be linked thereto by a nucleic acid molecule linker, which may be an RNA coding sequence, such that when transcribed the aptamer domain and expression platform remain structurally linked by a nucleic acid molecule linker.

The riboswitches of the present disclosure include those comprising two or more aptamer domains operably linked to a single expression platform. The aptamer domains may be the same or different. The effect on the conformational change of the expression platform may be dictated by the binding of one, the other, or both of the ligands. The ligands may be the same or different. At least one of the aptamer domains and the expression platform are heterologous.

## EXPRESSION PLATFORM DOMAIN

The expression platform is a nucleic acid sequence, for example an RNA sequence. The expression platform may be a naturally occurring expression platform, or may be derived, re-engineered therefrom or artificially created. It may be isolated, purified or recombinant. It may not be derived from a native riboswitch, but may be a regulatory molecule which is able to adopt at least two different configurations, each of which being able to mediate a different (either inhibitory or activating) effect on expression of a nucleic acid sequence to which it is linked. It may be able to be change configuration upon binding of a ligand to an aptamer domain, to which the expression platform is operably, and in one instance, for example, structurally, linked.

The expression platform may comprise a consensus sequence, which is a motif or common sequence shared by a particular type or class of riboswitches or expression platforms, for example dictated by the class or type of ligands bound by the expression platforms. Consensus sequences for expression platforms of particular riboswitches are known in the art for example, *pbuE (ydhL), xpt*G, PreQ and *add*A.

The expression platform of a riboswitch of the disclosure may be chimeric, for example comprising a portion of an expression platform of one riboswitch, and a portion of an expression platform of one or more different riboswitches. In this way, different functionalities such as manner of regulating gene expression and folding, may be achieved. Such an expression platform may comprise at least a consensus sequence of the expression platform, or a sequence which is derived therefrom and has at least 50, 60, 70, 80, 85, 90, 95, 96, 97, 98, or 99% sequence identity over at least 50, 60, 70, 80, 85, 90, 95, 96, 97, 98 or 99% of the length of the consensus sequence.

Examples of expression platforms include those derived from Figure 27, an Add-A riboswitch such as that of Figure 13, or one which is derived therefrom, such as the M6, M6', M6" or M6C, M6C' or M6C" (Dixon et al Proc Natl Acad Sci USA 2010, 107, 2830), or a PreQ riboswitch such as that of Figure 14. (Roth, Breaker. Annu Rev Biochem 78:305 (2009), Barrick, Breaker Genome Biol 8:R239 (2007)). Examples of specific expression platforms for use in the present disclosure are the expression platforms of pbuE, xpt, ydhL, PreQ addA riboswitches.

The expression platform may be operably linked to the aptamer domain of the riboswitch. It may be structurally linked, forming part of the same nucleic acid molecule.

Chimeric riboswitches comprising heterologous aptamer domain and expression platforms may be produced using standard methodology known in the art, for example as described in Sambook et al 2000 Molecular Cloning; A Laboratory Manual (3rd edition).

SPACER

A spacer used in a genetic construct of the disclosure may be a nucleic acid molecule which separates the riboswitch and the gene of interest, and for example serves to enhance the ability of the riboswitch to regulate gene expression. The spacer may modulate the expression of a coding sequence when linked to the riboswitch, as compared to the regulation of expression when the coding sequence is regulated by the riboswitch in the absence of a spacer. By modulation means an increase or decrease in the output of the coding sequence, i.e. in terms of the yield of RNA transcripts or protein or peptide. The spacer may serve to increase the output of the coding sequence, in terms of RNA transcripts or protein or peptide yielded as compared to output when regulated by the same riboswitch and regulatory elements but no spacer.

The inventors have surprisingly found that the presence of a spacer in a genetic construct in accordance with the first aspect of the disclosure, as compared to an otherwise identical control genetic construct from which a spacer is absent, is able to markedly increase both the absolute amount of expression of a gene of interest (encoded by the coding sequence of the second nucleic acid molecule), and also to increase the ratio of expression levels in the presence or absence of the riboswitch's ligand. Thus the presence of the spacer sequence is able to increase the dynamic window of induction exhibited by a genetic construct in accordance with the present disclosure.

This is illustrated in Figures 43, 44, and 45, which illustrate differences in induction of various genes of interest (respectively lacZ, DsRed and T7 RNA polymerase) by riboswitches in genetic constructs in which a spacer is in which a spacer sequence is present (constituting examples of genetic constructs in accordance with the first aspect of the disclosure), or from which a spacer sequence is absent (these genetic constructs constituting control constructs used for comparison). In each of these examples the spacer sequences utilised were 105 nucleotides in length.

It can be seen from each of these Figures that the presence of a spacer sequence in the genetic construct leads to an increased dynamic range exhibited by the genetic constructs of the first aspect of the disclosure as opposed to the control constructs. Furthermore, as can readily be seen from

Figure 45, genetic constructs comprising a suitable spacer sequence exhibit a dose-dependent increase in induction of the gene of interest in response to the presence of the riboswitch's ligand, as compared to the "binary" response of the control constructs (in which presence of the ligand gives rise to expression of the gene of interest independent of the amount of the ligand present). The difference between the dose dependent and binary induction is clearly illustrated in the blot data presented in Figure 45.

As defined above, a spacer preferably has a low GC content. The GC content of a nucleic acid is the percentage of nitrogenous bases on a nucleic acid molecule that are either guanine or cytosine.

The GC content may be calculated as

$$\frac{G+C}{A+T+G+C} \times 100$$

whereas the AT/GC ratio is calculated as

$$\frac{A+T}{G+C}.$$

The GC-content percentages as well as GC-ratio can be measured by several means, preferably by measuring the melting temperature of the nucleic acid sequence by spectrophotometry. The absorbance of DNA at a wavelength

of 260 nm increases fairly sharply when the double-stranded DNA separates into two single strands when sufficiently heated. Another suitable method for non-sequenced nucleic acid molecules uses flow cytometry for large number of samples.

In alternative manner, if the DNA or RNA molecule under investigation has been sequenced then the GC-content can be accurately calculated by using the above formula.

The spacer may be positioned upstream of the gene of interest. It may be immediately upstream, such that the last nucleic acid residue of the spacer is adjacent to the first nucleic acid residue of the start codon of the gene of interest. Alternatively, the spacer may be separated from the start codon of the gene of interest by a non -coding and/or non-functional sequence. Alternatively or additionally, the spacer may be separated from the gene of interest by one or more upstream sequences such as additional regulatory sequences such as transcriptional or translational regulatory elements, additional coding or non coding sequences, ribosome binding sites, additional riboswitches, chromosomal integration sites, and transcription terminators, marker genes, reporter genes, coding sequences for ancillary regulators, repressors, and any other desired components, many of which will be known to persons skilled in the art.

Where two or more riboswitches are present regulating a nucleic acid molecule comprising a coding sequence, then a spacer may be provided upstream of the nucleic acid molecule comprising the coding sequence, and downstream of the riboswitches.

The spacer may be any size or sequence which serves to have the effect of enhancing regulation of the gene of interest by the riboswitch, preferably to increase or decrease the dynamic range of the riboswitch, or the output of the coding sequence in terms of yield of RNA transcripts or protein or peptide. The spacer may be a DNA molecule, which encodes an RNA molecule and which is operably linked to the RNA molecule which encodes the riboswitch, which in turn may preferably be operably linked to the gene of interest under its regulation. The spacer may be a coding sequence or a non-coding sequence, or a combination thereof. The spacer may be a naturally occurring sequence, or an artificial sequence. It may be isolated or purified from a sequence which exists in nature, or may be recombinantly produced, which means that it is derived from a new genetic source rather than being derived from a native sequence . The spacer may be naturally associated with the riboswitch i.e. it is operably linked thereto in nature, or may be heterologous to the riboswitch.

The spacer may be 5, 20, 15, 20, 25, 30, 35, 40, 45, 50, 55, 60, 65, 70, 75, 80, 85, 90, 95, 100, 105, 110, 120, 125, 130, 135, 140, 145, 150, 200, 250, 300 or more nucleotides in length. It will be appreciated that in order that nucleic acid molecules connected by a spacer may be kept in frame, the number of nucleotides incorporated in a spacer should preferably be a multiple of three. In keeping with this, the spacer may suitably be 5, 20, 15, 20, 25, 30, 35, 40, 45, 50, 55, 60, 65, 70, 75, 80, 85, 90, 95, 100, 105, 110, 120, 125, 130, 135, 140, 145, 150, 200, 250, 300 or more nucleotides in length.

More information regarding the impact of the length of spacer sequence on the ability to bring about dose-responsive induction of a gene of interest is shown in Figures 46 and 47, both of which report on induction of T7 RNA polymerase under riboswitch control in genetic constructs of the invention incorporating reporter sequences of different lengths.

From Figures 46 and 47 it can be seen that dose-dependent induction is seen when spacer sequence length varies between about 90 nucleotides and about 165 nucleotides. Below 90 nucleotides spacer length the dynamic range of expression upon the addition of 250uM A43 plus 0.5mM IPTG is <2fold (above the expression induced by IPTG alone), for constructs with spacer lengths of 90 nucleotides and above this value is generally >2 fold.

These graphs show the regulation afforded upon the addition of IPTG (0.5mM) varies for constructs of different spacer length from ~1-fold for sp120, to 44-fold for sp105. Upon addition of the riboswitch inducer A43 the induction factors above IPTG expression again vary greatly for constructs with different lengths, with sp120 exhibiting up to12-fold regulation. Overall the construct with 105nt spacer length permits the biggest dynamic range of control (max/un-induced) of 134-fold, upon the addition of IPTG (0.5mM) plus A43 (250uM).

Accordingly it will be recognised that a preferred length of the spacer is 105 nucleotides in length. As referred to above, the inventors have found that use of a spacer sequence of around 105 nucleotides length confers a number of advantages, which may include increased dynamic range in response to the riboswitch ligand, and dose-dependent induction of the gene of interest.

The spacer may be derived from a naturally occurring coding sequence, which may be eukaryotic or non-eukaryotic. It may be derived from a nucleotide sequence which encodes a protein or polypeptide. It may be derived from the coding sequence thereof, including from exons or introns of said coding sequence, or from an upstream sequence associated with the coding sequence or from a downstream region. It may be a translated, coding sequence or a non-translated sequence. It may comprise a combination, either naturally occurring or artificially created, of coding and non-coding sequence regulatory and non-regulatory, upstream, coding and downstream sequence, or sequences from heterologous sources.

In one instance, it is derived from a protein, for example a non-bacterial protein, for example a eukaryotic protein. It may be derived from a marker protein, for example enhanced green fluorescent protein (eGFP), or DsRed.

Other examples include T7, T3 or SP6.

Spacers for use in the present disclosure may be all or part of a spacer sequence provided in Figure 19 or a sequence which is derived therefrom. Thus, a spacer sequence for use in the present disclosure may be a spacer of GFP 165, GFP 150, GFP 135, GFP 120, GFP 105, GFP 102, GFP 99, GFP 96, GFP 93, GFP_90, GFP_75, GFP_60, GFP_45, GFP_30, GFP_codon optimized variant, DSRed, lacZ, T7, T7, T3 or SPG as shown in Figure 19, or fragments thereof. A fragment thereof may comprise at least 6, 9, 12, 15, 30, 45, 60, 75, 90 or 99 contiguous nucleotides of a sequence of Figure 19. A spacer may comprise a sequence which has at least 50, 60, 70, 80, 85, 90, 95, 96, 97, 98 or 99% sequence identity across at least 10, 20, 30, 40, 50, 60, 70, 80, 85, 90, 95, 96, 97, 98 or 99% of the sequence from which it is derived, that sequence preferably being a sequence of Figure 19 or a fragment thereof.

The spacer may be heterologous to the genetic construct in which it is placed, and may be distinguished therefrom by its ability to moderate the function and activity of the riboswitch with which it is associated.

GENETIC CONSTRUCT

A genetic construct of the disclosure may be DNA or may be RNA. In one instance, it is a DNA construct, which encodes an RNA riboswitch operably linked to a spacer and a gene of interest. Two or more of the components of the genetic construct may be heterologous, i.e. from different sources. Thus, they may be from different RNA molecules, different transcripts, different genes, different cells, different organisms or different species. When heterologous, they may each independently be naturally occurring, artificial or engineered sequences, and/or independently isolated, purified or recombinant.

In one instance a genetic construct may comprise one or more transcriptional or translational regulatory elements, additional coding or non coding sequences, ribosome binding sites, chromosomal integration sites, and transcription terminators, marker genes, reporter genes, coding sequences for ancillary regulators, repressors, and any other desired components, many of which will be known to persons skilled in the art. The position of such additional regulatory elements within the genetic construct will depend upon their role within the construct, and the nature of components or other elements or nucleic acid molecules they are operably linked to.

The genetic construct of the may be a vector, which is a carrier of exogenous nucleic acid. Any suitable vector may be used as a genetic construct. The vector may be eukaryotic or prokaryotic. Vectors include plasmids, viral vectors, phage, cosmids, artificial chromosomes and any others known to persons skilled in the art. A vector may be integrative. A vector may be replicative. It may be conjugative or non-conjugative, an expression vector, cloning vector or shuttle vector.

Suitable viral vectors include (but are not limited to), for example herpes virus, Vaccinia virus, polio virus, adenovirus, adeno-associated virus, AIDS virus, HIV virus, Pox virus, and other RNA viruses. Retroviral vectors include MMLV, pBADE, pADCMV, CMV, MoMLV. Viral vectors also comprise the vectors which share sufficient properties with the viral vectors making them suitable for use as a vector. Viral vectors may also include early and late genes, for example non structural early genes and structural late genes, inverted terminal repeats, and RNA polymerase transcript, and regulatory sequences such as a promoter. One or more of the early genes of the virus may have been removed and replaced by exogenous DNA, such as the nucleic acid comprising the coding region. In such a case, any necessary early genes may be provided by the host cell or in vitro expression system, as required. Viral vectors have the advantages of being able to carry a large load of exogenous nucleic acid (for example, up to 8kb) , being thermostable, and being capable of being stored at room temperature, as well as being able to transfect non-dividing cells. Viral vectors may have been modified such that they do not induce an immune response in the host organism. Viral vectors may be replication defective. Known viral vectors will be known to persons skilled in the art.

Other suitable vectors include plant vectors, such as plant shuttle vectors.

Specifically, examples of vectors include pGEXxx, pETxx, pMODxx, pBlueScriptxx, pDGxx, pACxx, pMODxx, pBRxx, pBC Phagemid Vectors, Lambda vectors, pCMV, pCR, pTOPO, pBAD, pEM7, pRSET, pTrc, pENTR, pDEST, pxxDuet, pCDFxx, pRSFxx, pCITExx, pFLAGxx, pTACxx, pPCV, pShuttlePHT43, pMUTIN4, pAMJ2008, pACYxx, p15xx.

The vector selected for use will be appropriate to the expression system in which it is placed, i.e. in vivo expression for example in a host cell, or in vitro expression. Where the disclosure uses two or more genetic constructs, these may be the same or different types, independently selected from the group consisting of plasmids, viral vectors, phage, cosmids, artificial chromosomes.

Examples of vector maps and genetic constructs described herein are shown in Figures 1 and 2.

CODING SEQUENCE

The riboswitch of the present disclosure may be operably linked to and used to regulate a variety of sequences, including nucleic acid sequences which comprise coding sequences (i.e. a nucleic acid molecule which comprises codons for amino acids, for example two or more contiguous codons for amino acids) and which in one instance for

example can be transcribed into RNA, and which in turn can in one instance for example be transcribed into protein or peptide; and nucleic acids comprising non-coding sequences (i.e. the nucleic acid sequence does not comprise two or more contiguous codons for amino acids). The nucleic acid sequence regulated by the riboswitch may complementary to the above mentioned coding sequences (i.e. the complementary strand where the nucleic acid molecule is double stranded). The nucleic acid molecules may comprise both coding and non-coding sequences, for example many mRNA molecules include both coding sequence and upstream a functional non-coding region such as rRNA and tRNA. The nucleic acid molecules regulated by the riboswitch may be DNA, RNA, PNA, LNA, GNA or TNA.

Regulation of a nucleic acid sequence by a riboswitch includes regulation of expression, wherein expression includes transcription, and translation.

Where two or more coding sequences are present in a genetic construct, these may be the same or they may be different. By the same, means that they comprise the same nucleic acid sequence as each other, at DNA level. Different coding sequences are those which have different nucleic acid sequences at the DNA level, and so may produce different RNA transcripts and different amino acid sequences, although it is possible that the different coding sequences encode the same product having the same functional and/or activity, but differing in sequence at one or more of the DNA, RNA or amino acid levels.

The coding sequence of a genetic construct of the disclosure may be heterologous to the riboswitch to which it is operably linked to. Heterologous means that in nature, the sequences are not operably linked. Thus, they may be from the same or different sources, but are not naturally associated with one another and do not naturally have any functional relationship with one another.

Examples of coding sequences for use in the present disclosure include antisense genes, repressors, oscillators, quorum sensing genes, transcription factors, motility genes, morphology genes, reporters, drug target homologs, and antibiotic genes. Specific examples include the genes coding for T7 RNA polymerase, T3 RNA polymerase, SP6 RNA polymerase, eGFP, LacZ and DsRed.

Examples of coding sequences include those encoding human proteins or parts thereof. Non-limiting examples of human proteins for expression in the present invention include Pim-1, Pim-2, MAPKAPK5 , TRIB1, FYN , MAPK1 , MAPK14 , MAPK8 , EMG1 , IFN gamma , IFN alpha , TNF alpha , NFKB1a , YY1 , TFIIB , Kif11 , CDC2 , Caveolin , CSF2 , HGF, G-CSF, GMCSF, Interferon, IL-4 , IL-6 , IL-7 , IL-10 , IFN alpha , TNF alpha , CCL5 , TFIIB and multimeric proteins including, but not limited to, Human Growth factors, erythropoietin, thrombopoietin, interferons, insulin-like growth factors, epidermal growth factors, platelet-derived growth factors, angiogenin, angiotensin, interleukins, GPCRs, taste receptors, odorant receptors, Leucine zippers, Chimeric Zinc Fingers, Heteromeric cytokines, T cell receptors, antibodies, heavy light chain, fragments, fab, fv, chemokines, and cytokines.

One of the many challenges of expressing recombinant antibodies in bacterial expression systems is the lack glycosylation machinery. Use of the co-expression systems demonstrated in this disclosure, to precisely control the expression of eukaryotic glycosylation machinery in E.coli cells as shown possible recently (Valderrama-Rincon et al Nature Chemical Biology 2012 8, 434-436.), would allow the expression of this glycosylation machinery to be regulated to aid the challenge of expressing antibodies in bacterial expression systems such as *E.coli, B. subtilis, P. fluorescens.*

An additional challenge of expressing antibodies and antibody fragment such as Fabs in bacterial expression systems, is the challenge of balancing the co-expressional requirements of the antibody heavy and light chains. It is very difficult to predict expression requirements based on promoter strength, codon usage, mRNA structure prediction, and cellular copy number of the gene(s) of interest. Current methods involve a combinatorial challenge of manually changing promoter strengths, and altering the copy number of the vector(s) containing the recombinant gene(s) of interest. Additional methods involve using intergenic sequences between the genes of interest within a discistronic expression cassette, again this intergenic sequence needs to be optimized and reselected for each co-expression requirement. The ability of the dual promoter expression and operon systems developed means that the optimal co-expression ratio(s) can be readily controlled by addition of differing inducer concentrations to the growth media. This has application for controlling the co-expression of heavy and light chains of antibodies and anti-body fragments.

REGULATORY SEQUENCES

A regulatory sequence as used in the invention is a sequence which may be used to regulate or control the expression of a coding sequenceprovided in a second genetic construct. The regulatory sequence in the first genetic construct in a system of the invention encodes a polymerase.

CONTROL ELEMENTS

The control elements provided in the genetic construct of the disclosure may be transcriptional or translational control elements, and may include promoters, enhancers, ribosome binding sites, transcription terminators, repressors and other ancillary regulators.

Promoters suitable for use in the genetic constructs will be those which can regulate transcription of the nucleic acid molecules to which it is operably linked, in the vector in which it provided. A promoter may be constitutive or inducible. It may be a positive or negative operator. A promoter may be one which is capable of directing transcription in the expression system of choice, and may be be appropriately selected. A promoter may be selected which is capable of functioning in a variety of expression systems or host cell types. A promoter may be provided in close proximity to the transcription start site of the nucleic acid molecule under its control. The promoter sequence may be a naturally occurring promoter sequence, isolated or purified from any suitable source, including for example eukaryotes (such as yeast, fungi, algae, protist, insect, plant, animal or human), prokaryotes such as bacteria and archaea, viruses and organelles such as chloroplasts and mitochondria. Alternatively, the promoter sequence may be an engineered sequence, for example derived from a naturally occurring sequence, but modified to comprise characteristics suitable for the desired expression of the nucleic acid molecule to which it is linked in the constructs of the disclosure.

T7, T3, Sp6, araC, araBAD, lac promoter, lacUV5, tac, trc, trpR promoter Omp, lac promoter/operator, tet promoter/operator, luxR, Lambda Prm promoter, pLac promoter.

In one instance, in each genetic construct of the disclosure, each nucleic acid molecule to be transcribed may be under the control of a promoter sequence. Where two or more nucleic acid molecules are operably linked, for example a riboswitch and a coding sequence, or two or more coding sequences, these may be transcribed under the control of a single promoter, preferably positioned upstream of the sequences to be transcribed. Alternatively, the two or more nucleic acid molecules of the genetic construct to be transcribed may each be under the control of individual promoters. Where more than two nucleic acid molecules within a construct are to be transcribed, two or more of these may be under the control of the same or different promoters. Where two or more promoters are present in a genetic construct, these may be the same promoter, or may be derived from the same source, or of the same type, or may be different. It may be desirable to use different promoters where different levels of regulation are required, for example, responsiveness to different transcription factors, environmental conditions such as temperature, light, chemicals, irradiation, pH, osmotic pressure, nutrient starvation, toxins, atmospheric pressure, $O_2$ levels, or temporal controls.

For example where a riboswitch is operably linked to a nucleic acid molecule comprising a coding sequence, in the same genetic construct, a promoter sequence may be provided which regulates transcription of both the riboswitch and the coding sequence. Alternatively, transcription of the riboswitch and the coding sequence may be under the control of separate promoters, which may be either the same or different type. In the invention where a first genetic construct comprises a first nucleic acid molecule encoding a riboswitch operably linked to a second nucleic acid molecule encoding a polymerase, the riboswitch and the polymerase are transcribed under the control of the same promoter. In the second construct provided in the invention, the coding sequence may be under the control of a promoter which is the same or different to the promoter of the first construct. In the third aspect of the disclosure, where a genetic construct comprises two or more riboswitches, each operably linked to a nucleic acid comprising a coding sequence, it is envisaged that one or more riboswitch/coding sequence pair may be under the control of a separate promoter, and/or that two or more pairs of riboswitch/coding sequence may be under the control of a single promoter.

For regulation of eukaryotic sequences, one or more enhancer sequences may also be provided in a genetic constructs of the disclosure. In the present disclosure, an enhancer sequence is a DNA sequence which regulates transcription of a coding sequence, in combination with a promoter sequence, and which may be positioned within no fixed distance from the coding sequence, and either 3' or 5' thereto. An enhancer sequence is typically between 10 and 300bp in length. In a genetic construct of the present disclosure, a promoter sequence may be optionally operably linked to an enhancer sequence. Two or more promoters may be operably linked to a single enhancer sequence. An enhancer may be derived from any suitable source. Examples of enhancers for use in the present disclosure include CMV, IGHM, TEF-1, TEF-2, TEF-3 enhancers.

In addition to promoter and/or enhancer sequences, a genetic construct may also comprise a regulatory element capable of terminating transcription. An example of such an element is a polyA segment in an untranslated region of an mRNA transcript. Other suitable transcription termination sequences are known to the person skilled in the art, and include for example Rho dependent (eg. Graham. NAR, Vol. 32:3093 (2004), Ciampi. Microbiol 152: 2515 (2006). Rho independent (intrinsic) terminators, natural and synthetic, eg. any hairpin loop, any CG rich dyad and specific terminator such as T7(TE) or T1 (rrnB). These elements must be compatible with the remainder of the genetic construct. These elements may not be necessary for the expression or function of the nucleic acid molecules but may serve to improve expression or functioning of the nucleic acid molecule by affecting transcription, stability of the mRNA, or the like. Therefore, such elements may be included in the expression construct to obtain the optimal expression and function of one or more of the nucleic acid molecules.

Additional regulatory sequences include any nucleotide sequence which is capable of influencing transcription or translation of a coding sequence product, for example in terms of initiation, rate, stability, downstream processing, secretion, and mobility. Examples of regulatory sequences include promoters, 5' and 3' UTR's, enhancers, transcription factor or protein binding sequences, start sites and termination sequences, ribosome binding sites, recombination sites and polyadenylation sequences.

Ribosome binding sites include optimized Shine Dalgarno/Kozak sequences. Examples of sequences are those comprising the consensus sequences (prokaryote) AGGAGG; (E. coli) AGGAGGU; (Kozak) CCCGCCGCCACCATGGAG.

Thus, in one instance, a genetic construct of the disclosure may comprise a transcription termination sequence associated with each transcription unit (i.e. the nucleic acid molecules under the control of any particular promoter and which are transcribed as a single RNA sequence).

MARKER GENES

The genetic construct of the disclosure may comprise one or more reporter or marker genes. Such genes are preferably included to indicate A) delivery of the genetic construct to the expression system or a host cell; B) expression of the riboswitch transcript; C) conformational change of the RNA sequence comprising the riboswitch; and D) the activity and quantification of activity of the riboswitch.

A. To indicate delivery of the genetic construct to the expression system, a reporter gene may be provided in the genetic construct. The reporter gene may be operably linked to one or more other nucleic acid molecules in the genetic construct, or may be provided within the genetic construct but independent from the other nucleic acid molecules of the construct, and under the control of its own regulatory sequences. Suitable reporter genes for determining delivery of a genetic construct to an expression system include E. Coli *lac*Z, and green fluorescent protein, luciferase or selectable reporters such as DHFR, thymidine kinase, mycophenolic acid; and resistance to antibiotics such as neomycin, neomycin analogue G418, hydromycin, and purocin, Kanamycin$^R$ , Tetracyclin, Zeocyin, ampicillin$^R$ , and hygromycin; ancillary regulators such as Laclq, arabinose repressor, Tet repressor, tryptophan repressor, LuxR, and lambda repressor (cl); and Chloramphenicol Acetyl Transferase. Methods of using such reporter genes are well known in the art.

B. To determine expression of a riboswitch (i.e. transcription/translation thereof), a nucleic acid molecule encoding a reporter gene may be provided, operably linked to a nucleic acid molecule encoding a riboswitch. Thus, the expression of the riboswitch can be monitored by observing expression of the reporter gene.

C. The genetic construct may also comprise reporter genes which provide a signal dependent upon a conformational change in the riboswitch, thus indicating activity of the riboswitch. For example, the fluorescent output or wavelength may change dependent upon the structural conformation of the riboswitch to which it is operably linked.

D. A reporter gene may also be provided, operably linked to a riboswitch, which enables monitoring of activity and optionally quantification of riboswitch activity. Thus, in one instance, the reporter gene for this purpose will be one whose output (e.g expression levels or activity) correlates to activity of the riboswitch.

A reporter gene for use in the present disclosure as described above may produce a detectable signal, being one which can be observed using standard techniques, such as immunoassay, radioimmunoassay, fluorescence detection, luminescence detection, western blot, absorbance, assays for protein activity. Suitable reporter genes include, for example molecules. Examples of these are known in the art. Examples of reporter genes are those which encode a protein which provides a detectable signal which is easy to observe, for example, luminescence or fluorescence, and which is observed even at low expression levels.

Other reporter molecules such as fluorescent molecules and radioactive isotopes may also be used.

A reporter gene may be used which is capable of combining two or more of functions A-D above. One or more suitable reporter genes may be used in a genetic construct.

Suitable reporter genes include E. Coli *lacZ,* and green fluorescent protein, luciferase or selectable reporters such as DHFR, thymidine kinase, mycophenolic acid; and resistance to antibiotics such as neomycin, neomycin analogue G418, hydromycin, and purocin, Kanamycin$^R$ , Tetracyclin, Zeocyin, ampicillin$^R$ , and hygromycin; ancillary regulators such as Laclq, arabinose repressor, Tet repressor, tryptophan repressor, LuxR, and lambda repressor (cl); and Chloramphenicol Acetyl Transferase.

LIGANDS

The ligands which activate the riboswitches used in the present disclosure may be any which are capable of binding to the aptamer domain of the selected riboswitch, and mediating the structural change in the confirmation of the expression platform, which enable the riboswitch to regulate nucleic acid sequence expression. The ligands may be naturally occurring or may be artificial or synthetic.. They may be isolated or purified. They may be the native ligand

which triggers the riboswitch in nature, or may be a different ligand to the native ligand.

The ligand may serve to activate, inhibit or block a riboswitch. Thus, a ligand may be a non-native ligand of a riboswitch, which serves to inhibit rather than activate the riboswitch. Alternatively, a ligand may be a modified form of a native ligand, or an analogue of a native ligand, which serves to block gene regulation by the riboswitch when bound to the aptamer domain.

Examples of ligands are those which interact with riboswitches including cyclic di-GMP, SAH, pre-Q, Moco and SAM-IV, add-A and purine riboswitches. The disclosure includes the use of such known ligands which have been modified in relation to their binding to the aptamer domain, for example either enhanced or decreased binding and a resulting effect on expression platform configuration and preferably on coding sequence expression.

Such compounds include, for example, cyclic di-GMP, pGpG, GpG or GpGpG, derivatives of cyclic di-GMP, pGpG, GpG or GpGpG which are capable of activating a cyclic di-GMP responsive riboswitch, S-adenosylhomocysteine, derivatives of S-adenosylhomocysteine that can activate an S-adenosylhomocysteine-responsive riboswitch, preQ1 &/or preQ0 , derivatives of preQ1 &/or preQ0 that can activate a pre-Q-responsive riboswitch, Moco, derivatives of Moco that can activate a Moco-responsive riboswitch, SAM, and derivatives of SAM that can activate a SAM responsive riboswitch, and purines or derivatives thereof which can activate a purine riboswitch.

It will be appreciated that the compounds of the disclosure will represent suitable ligands for use in many aspects disclosure.

For the avoidance of doubt, references to the compounds of the disclosure in the context of the present disclosure should be taken as encompassing the compounds *per se,* and also suitable salts or other derivatives thereof. Salts of the compounds of the disclosure may be of particular utility in contexts where the compound *per se* has relatively low solubility, or where it is otherwise desired to improve solubility of the compounds.

A suitable salt of the compound of the disclosure is, for example, an acid-addition salt of the compound formed with an acid such as hydrochloric, hydrobromic, sulfuric, phosphoric, trifluoroacetic, formic, citric or maleic acid. In a particular embodiment, the compound of the disclosure may be in the form of a hydrochloride salt.

METHODS

It is envisaged that the genetic constructs of the disclosure and the ligands which are provided to activate one or more of the riboswitches described herein may be used in a variety of gene expression based techniques, several examples of which are provided herein.

Also described herein is a method of detecting the presence or absence of an analyte, comprising providing in a cell or an expression system a genetic construct of the first aspect of the disclosure, wherein the coding sequence is a signalling molecule; and detecting the presence or absence of the signalling molecule, wherein presence of the signalling molecule indicates presence of the analyte which is a ligand of the riboswitch of the genetic construct.

HOST CELLS AND EXPRESSION SYSTEMS

The invention may be provided as an *in vitro* expression system or *in vivo,* within a host cell. Suitable *in vitro* expression systems include wells, test strip, chip, plate or *in vitro* cell-like expression systems.

Host cells may be eukaryotic, or prokaryotic. Preferred host cells include yeast, bacteria, plant, animal or mammalian cells. Preferred bacterial cells include, but are not limited to, Escherichia *coli, Bacillus Subtilis, Clostridium difficile, Clostridium botulinum, Staphylococcus aureus, lactococcus lactis, pseudomonas fluorescens, bacillus anthracis.* Non-bacterial expression systems include, for example, chloroplasts and cell-free systems.

KITS

Kits of the invention may additionally comprise instructions for use, and one or more additional components selected from buffers, deoxyribonucleic acids, ribonucleic acids, supports, enzymes, biological strains both engineered and non-engineered including, bacterial strains, cell lines, plasmids, components for transfection and transfection, inducer molecules.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification

(including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

EXAMPLES

Here we show how two mutually orthogonal riboswitches can be independently controlled by varying concentrations of two distinct synthetic inducers, allowing dynamic expression landscapes to be accessed and optimal co-expression stoichiometries to be established. In the first instance a dual promoter system was constructed, in the pBKS vector with the orthogonal M6 riboswitch selected previously (Dixon et al Proc Natl Acad Sci USA 2010, 107, 2830] or Dixon et al Angew. Chem. 2012, 51, 3620-3624. controlling translation of the red fluorescent protein DsRed [Bevis, Nat. Biotech. 20:83 (2002)] in response to the ligand ammeline (Am). The bacteriophage lambda tR1 transcription terminator [Graham. NAR, Vol. 32:3093 (2004] was inserted downstream of DsRed gene, followed by a second *lac* promoter and the 2-aminopurine (2AP)-responsive parental *add* A-riboswitch [Dixon et al Proc Natl Acad Sci USA 2010, 107, 2830 & Mandal, Nat Struct Mol Biol 11 : 29 (2004)] controlling translation of the green fluorescent protein eGFP (Figure 34A). *Escherichia coli* was transformed with this dual promoter construct and expression of DsRed and eGFP genes, was then determined by fluorescence as function of inducer concentrations.

To explore the capacity of the dual promoter system for controlling the differential expression of DsRed and eGFP, multi-dosage matrix expression analysis was performed where induction factors (IF), the ratio of the maximum ligand-induced protein expression over the basal protein expression level, are determined for a full range of inducer concentrations (Figure 34A & B). Following, Co-administration of Am and 2-AP, it can be seen that induction of DsRed expression with increasing concentrations of Am reaches a maximum induction factor of 9.3, whilst the simultaneous increase in 2AP has essentially no effect upon the DsRed induction (Figure 34B). Similarly, dose-dependent regulation of eGFP is observed upon increasing addition of 2AP, reaching an induction factor of 11.3, with changes in Am concentration having no effect upon the induction of eGFP expression (Figure 34C). Whilst the orthogonal selectivity of M6 and *add-A* has been previously determined *in vitro* [Dixon et al Proc Natl Acad Sci USA 2010, 107, 2830], these results show for the first time the two riboswitches, despite being similar in sequence and structure, possess excellent *in vivo* orthogonality and can be used to independently control co-expression of the two genes simultaneously.

In a second series of experiments, a synthetic operon was designed with one promoter driving transcription of a single bicistronic mRNA comprising the M6 riboswitch regulating DsRed, and an intergenic *add-A* riboswitch controlling a downstream eGFP gene (Figure 35A). Matrix analysis of the induction factors demonstrates differential control of DsRed can be achieved. Moreover, the maximum DsRed induction factor was 21.2, which is also more than 2-fold higher than the dual promoter system (Figure 35B). The organisation of M6-DsRed in an operon, permits differential control, but also gives rise to significant amplification of the dynamic range of gene expression (IF), compared with the dual promoter system. Analysis of eGFP expression also showed very interesting and unexpected results. In this case, eGFP output was clearly dependent on both ligands. A synergistic effect of the two ligands is observed in the induction factor matrix analysis, across a full range of Am and 2AP concentrations (Figure 35C), with the expression landscape reaching a maximum induction factor of 24.1 in the presence of the highest concentrations of both inducers. Our earlier *in vitro* studies, clearly show that there is no binding interaction with Am and *add* A or between 2AP and M6. Similarly no cross-talk is observed with the dual promoter system described above. This suggests that eGFP expression within operon system is not only regulated by the intergenic *add* A-riboswitch but also by the upstream M6 riboswitch.

A number of reasons could possibly account for this. Firstly, ribosomal read-through could result in a more efficient initiation of translation at the eGFP RBS. Secondly, riboswitches that control gene expression through changing the accessibility of the RBS (like *addA),* may also rely on Rho-dependent termination of transcription [Roth, Annu. Rev. Biochem. 78:305 (2009) & Peters Proc. Natl. Acad. Sci. USA 106:15406 (2009)]. In the case of the operon, when both riboswitches are activated by Am and 2AP, ribosomes will be recruited at both RBSs and will occupy the full length of the transcript, blocking Rho binding [Epshtein, Nature 463:245 (2010)]. Consequently the levels of full-length mRNA transcripts in the cell will be higher, than if either or both ligands are absent. Finally protection of the full-length transcript from nuclease degradation by actively translating ribosomes [Deana, Genes Dev. 19:2526 (2005)], recruited at both RBSs, would also serve to increase mRNA stability in the presence of both inducers. Whichever mechanism is prevailing it is clear that the presence of two riboswitches within an operon system affords greater digital control of gene expression, which is also demonstrated by dose dependent analysis (Figure 36-37). Titration of the M6 selective inducer Am (0→250 μM) against the dual promoter in either the absence or presence 2AP (0 or 250 μM) gives dose-dependent protein expression curves for DsRed that are very similar, with maximum normalized red fluorescence (fu/$OD_{620}$) of 1.1 x $10^5$ and 1.0 x $10^5$ respectively (Figure 36A). Dose-dependent control of eGFP expression is also observed upon addition of

the *add* A-selective inducer 2AP (Figure 36B) and eGFP expression is also clearly unaffected by the presence of the M6 ligand (Am), with maximum eGFP levels of ca. 55 x$10^6$fu/$OD_{620}$. In the case of the operon construct titration of Am (0→250 μM) in the presence or absence of 2AP (0 or 250 μM) produces essentially identical dose-response curves for DsRed expression, where a maximum output both of 2.9 x $10^6$ fu/$OD_{620}$ is observed (Figure 37A). However for the eGFP gene the synergistic effect observed above is also seen, where upon titration of 2AP alone produces maximum output of 14.8 x $10^6$ fu/$OD_{620}$, and in the presence Am (250 μM) an output 25.9 x $10^6$ fu/$OD_{620}$ (Figure 37B). Indicating that both inducers serve to amplify expression of the downstream eGFP gene, within the operon.

In nature, bacterial riboswitches are typically found in the 5'-UTR sequence of the mRNA, controlling expression of single genes and operons, to date no natural riboswitches have been found to function within intergenic regions (as in the synthetic operon Figure 34A). There are however several examples in nature where tandem riboswitches, within 5'-UTR sequences, have been shown to regulate a single gene output [Sudarsan, Science 314: 300 (2006) & Welz, RNA 13:573 (2007)], providing similar 'digital' control as the synthetic operon system (Figure 34A).

Fluorescent Microscopy was used to ascertain whether the observed independent expression of the two reporter genes occurs within individual bacterial cells. The microscopy images of *E. coli* cells transformed with the dual promoter or operon construct (Figure 38A-B) show that the individual cells emitting both green and red fluorescence upon co-administration of both Am and 2AP (250 μM) and that co-localisation of fluorescence is clearly seen in the overlay frame. This indicates co-expression of DsRed and eGFP occurs within individual *E. coli* cells, for both systems, and that the observed expression profiles are not due to differential expression of sub-populations of cells, which would be problematic for many of the applications described above.

To further demonstrate how mutually orthogonal riboswitches, can regulate the stochiometry of two gene products, the normalised gene expression outputs for the both the dual promoter and operon systems were used to generate 2D contour maps of the expression landscapes (Figure 39 & 40). From these contour maps it is possible to see how appropriate inducer concentrations can easily be selected so that the desired defined stoichiometry of the two gene products can be obtained.

In summary, mutually orthogonal riboswitches can be used control the differential expression of multiple genes, in the same cell, simultaneously. The Dual Promoter system permits simultaneous and independent control over two genes with no "cross-talk" between the genetic control elements. Using 2D-contour plots of the resulting dynamic expression landscapes, it is possible to select appropriate concentration of small molecule inducers required to obtain a specific stoichiometry of gene products. Moreover when two mutually orthogonal riboswitches are deployed in the 5'-UTR sequence and in the intergenic region of a bicistronic mRNA, significant amplification of expression of the downstream gene is observed, allowing more digital control over gene expression. A synthetic Operon system, in which riboswitches are deployed in both the 5'-UTR and intergenic region of a bicistronic mRNA, was shown to afford a greater level of control over the dynamic range of inducer response, wherein the dynamic range of control that is afforded by the downstream intergenic riboswitch is controlled by the upstream 5'-UTR riboswitch.

Given that there are very few reliable methods currently available to accurately balance expression of multiple protein outputs [Pfleger 2006], the method described here could have wide-ranging biotechnological applications and new gene expression tools.

Screening for new riboswitch ligands

Re-engineering the adenine responsive *add* A-riboswitch (addA) from *Vibrio vulnificus* (Figure. 27A & B) [Dixon et al Proc Natl Acad Sci USA 2010, 107, 2830]. By stabilising the P2 stem of the mutants, it was possible to engineer two improved riboswitches M6″ and M6C″, which produced higher *eGFP* expression levels than the wild-type. Crucially both mutant riboswitches are completely orthogonal to the parental riboswitch as neither responds to adenine or other purines present the cell, whilst addA does not respond to the synthetic ligands **2** and **3** (Figure 27C and F). Isothermal titration calorimetry (ITC) experiments confirmed that the M6" and M6C" aptamer domains have no affinity for adenine, but bind respectively to ligands 2 and 3 with $K_d$ values in the 1.0-1.2 μmol range. Finally, a 1.7 Å crystal structure of the M6C" aptamer domain in complex with azacytosine 3 (3LA5.pdb) was obtained, which shows how the riboswitch can adapt its structure to accommodate alternative synthetic ligands (Figure 27D-F). This structural information has been used to guide screening of a number of alternative heterocyclic compounds, from which we discovered a superior M6" ligand pyrimido[4,5-*d*]pyrimidine-2,4-diamine (A43) that affords a 12-fold induction of gene expression in *E. coli,* which is higher than the parental riboswitch. This ligand has been shown to bind to the M6" riboswitch by isothermal titration calorimetry with a $K_d$ = 189nM (Figure 59) Used in combination with an inducible promoter such as the lac promoter/operator this induction can be extended >400-fold (Figure 53). It was also shown how the orthogonally selective riboswitches can be used for the differential and simultaneous control of two genes in the same *E. coli cell.*

Controlled expression of toxic and insoluble proteins using orthogonal riboswitches.

The orthogonal riboswitches generated to date exhibit very low basal levels of expression, compared with the *lac*-IPTG system. The aim was to develop protein expression plasmids for *E. coli,* employing orthogonally-inducible single and tandem riboswitches, which are essentially silent in the absence of the inducer ligand. These expression plasmids can then be used to demonstrate the controlled production of selected toxic proteins, which have proven difficult to produce in *E. coli* by conventional means. Another major problem is the insolubility of some recombinant proteins when overproduced in bacteria. This can be alleviated, to some extent, by using an inducible promoter to reduce the rate of protein synthesis and promote proper folding. However, the all-or-none behaviour of the lac-IPTG system, means that even at low IPTG concentrations whilst some cells remain un-induced, others become fully induced and consequently rates of protein synthesis cannot be controlled at the cellular level. The orthogonal riboswitches developed will allow much more stringent and quantitative (dose-dependent) control of both the transcriptional and translational levels. The expression plasmids, carefully optimising concentrations of riboswitch ligands, were tested for production of proteins that are difficult to produce in soluble form in *E. coli.* In parallel, *E. coli* expression strains were engineered with a copy of the T7 viral RNA polymerase gene integrated into the chromosome under the control of an orthogonal riboswitch. Such *E. coli* strains would therefore be compatible with commercial expression plasmids such at the pET vectors.

Figure 48 illustrates induction of T7 RNA polymerase in response to increasing concentrations of a riboswitch ligand. As shown in the schematic representation of the construct the T7 RNA polymerase is under the control of a riboswitch downstream from a lac promoter/operator which is itself downstream from a lac I repressor. Two versions of this construct were created in which the lac I repressor was in either the same direction as the T7 RNA polymerase open reading frame (designated "f" for forward) or was in the opposite direction (designated "r" for reverse). As can be seen from the blot data presented, increasing concentrations of the riboswitch ligand A43 (provided at concentrations of 50μM, 100μM, 250μM, or 1000μM) gave rise to increased induction of T7RNA polymerase in a dose-dependent manner, irrespective of the direction of the lac I repressor.

Figure 49 illustrates a means by which modular gene expression may be achieved using a genetic construct in accordance with the present invention. T7 RNA polymerase (T7 RNAP) induction is controlled by both the lac promoter/operator (P/O lac), and the 5'UTR riboswitch (top). The blot data set out in this Figure illustrated that the gene construct allows dose-dependent control of T7 RNAP in response to the presence of IPTG and increasing concentrations of the riboswitch ligand A43. The riboswitch ligand controls induction of T7 RNA polymerase, and this, in turn, controls the expression of any gene of a further gene of interest cloned within a vector containing the T7 promoter (such as the PT7/pET vectors pictured on the right hand side of the Figure). Thus the riboswitch ligand indirectly controls expression of a gene of interest in this decoupled system.

Modular control of this sort is further demonstrated in Figure 50. Here a gene construct of the invention incorporates T7 RNA polymerase under the control of a riboswitch, with induction of the T7 RNA polymerase then controlling expression of a gene of interest coupled to a T7 promoter.

The blot data shows results of incorporation of these constructs in BL21(DE3) cells (lanes 2-3) and Top10F' cells (lanes 4-10) both strains having been transformed with pET45_MppK expression vector. Addition of IPTG to BL21(DE3) leads to the expected expression of the gene of interest, in this case the His-tagged 50kDa protein MppK. Upon addition of the addition of IPTG and the riboswitch inducer, the protein of interest is expressed in a dose-dependent manner.

Figure 51 shows data generated from a similar model in which T7 RNA polymerase under the control of a riboswitch and lac promoter/operator is used to control expression of green fluorescent protein in a pET-GFP vector. These expression data (shown in the right hand part of the graph) are compared with expression data from pET-GFP in BL21(DE3) cells (in the left hand part of the graph).

It can be seen that GFP expression in the BL21(DE3) cells is "leaky", whilst GFP expression in the BL21 p15_LOST1 cells comprising systems in accordance with the invention exhibit far tighter control of GFP expression. In these cells comprising a system of the invention IPTG provides transcriptional control of GFP expression, while the riboswitch ligand A43 provides translational control of GFP expression. Upon addition of both IPTG and A43 dose-dependent control of the gene of interest (GFP) from pET-GFP is observed.

Figures 52 and 53 illustrate the increased dynamic regulation of expression of a gene of interest (here exemplified by green fluorescent protein) that can be achieved using gene constructs in accordance with the present disclosure, as compared to comparator (control) constructs. In the control constructs GFP expression is controlled directly by a lac promoter/operator alone. In contrast, in the gene constructs of the disclosure GFP expression is controlled by both a lac promoter/operator, and a riboswitch. In this arrangement the lac promoter provides transcriptional control of expression, and the riboswitch provides translational control. Figures 52 and 53 respectively show data generated by gene constructs of the disclosure comprising first and second generation riboswitches (referred to respectively as "1$^{st}$ Generation" and "2$^{nd}$ Generation" constructs) when present on high copy plasmids in *E.coli* Top10F'. In this example, the first generation riboswitch comprises AddA (the ligand for which is adenine), and the second generation riboswitch

comprises M6" (the ligand for which is A43). These two riboswitches may generally represent preferred examples to be used when it is desired to utilise two orthogonal riboswitches in an instance of the various aspects of the disclosure.

Turning first to Figure 52, this shows results comparing GFP expression by cells comprising a control construct and by cells comprising the 1st Generation (addA-eGFP) construct of the disclosure. GFP induction factors (left axis indicated by the green bars) and absolute normalized GFP fluorescence signal (right axis) in the absence of inducer (open shape) and in the presence of inducer (closed shape).

The induction factor represents the degree to which gene expression is controlled, and is determined from the ratio of the maximum induced protein expression over the basal protein expression level. In the present example, the control lac P/O construct (results for which are shown in column 1) affords GFP expression of ca. ~$100x10^6$ fu/OD in the presence of 1 mM IPTG, and a basal expression of ca. ~$25x10^6$ fu/OD without IPTG. Hence the induction factor of this control construct is 4. The riboswitch containing genetic construct (results for which are set out in columns 2-6) achieves approximately the same maximum GFP expression of around ~$95x10^6$ fu/OD, in the presence of the riboswitch inducer A1. However, the basal level of expression for this 1st Generation genetic construct is ~$4x1O^6$ fu/OD, thus the induction factor achieved by this construct is approximately -22. These results demonstrate the tight control of the basal level of expression, and an increased dynamic window of expressional control, that may be achieved by the genetic constructs in accordance with the disclosure as the riboswitch inducer concentration is increased from 10uM-1mM.

Figure 53 illustrates expression control of transcription regulation (using a comparator genetic construct) as compared to dual transcription-translation regulation with a genetic construct in accordance with the disclosure utilising a second-generation riboswitch and inducer. As before, the comparator genetic construct comprises lac promoter/operator directly controlling the expression of GFP. This construct is shown schematically in the top left of Figure 53, and results obtained are shown in column 1 of the graph at the bottom of this Figure.

In contrast, the genetic construct of the disclosure comprises a lac promoter/operator upstream of a 2nd generation riboswitch M6"-eGFP the lac promoter/operator and riboswitch respectively providing direct control of the expression of GFP via transcriptional and translational control. This genetic construct is shown schematically at the top right of Figure 53 (where the riboswitch is identified as "RS"), and the results achieved using this genetic construct are shown in columns 2-5 of the graph at the bottom of this Figure, concentrations of riboswitch inducer A43 are shown.

Both the comparator construct and the genetic construct of the disclosure were provided on high copy plasmids in Top 10F'. Results shown in the graph illustrate GFP induction factors (left axis indicated by the green bars) and absolute normalized eGFP fluorescence signal (right axis) in the absence of inducer (open shape) and in the presence of inducer (closed shape).

The results achieved for the comparator construct, in which GFP expression is controlled solely by the lac promoter/operator, are shown for comparison in column 1, and are the same as referred to in the description of Figure 52. The results achieved by the genetic construct incorporating the second generation riboswitch differ from both those obtained using the comparator construct, and those obtained using the genetic construct comprising a first generation riboswitch. In these results, shown in columns 2-5, the maximum GFP expression observed is ~$120x1O^6$ fu/OD, as for the first generation construct. However, the basal level is ~$0.25x1O^6$ fu/OD, affording an induction factor of -450. This demonstrates much tighter control of the basal expression and a large dynamic 'widow' of expressional control as the riboswitch inducer (A43) concentration is increased 10uM-1mM.

When considering the lac P/O control alone (column 1) only 4-fold control is observed +/- IPTG, in comparison with the riboswitch containing construct +/- IPTG (column 2) 40-fold control is observed. This demonstrates that there is a 'regulatory synergy' when transcriptional and translational regulatory elements are coupled together, affording tight basal control with addition of precise 'tuneable' control over a wide dynamic gene expression range upon addition of the riboswitch inducer.

Figure 54, illustrates cell population control analyzed FACS of cells in which the genetic construct of the disclosure incorporating a 2nd generation riboswitch controls the expression of GFP. Fluorescence intensity (RFU) is shown on the x-axis, while cell counts with specific RFU are shown on the y-axis. The order of the peaks from left to right correspond to the order shown in the legend, in which "Lac O" refers to treatment with 0.5mM IPTG, and "1uM"-"1mM" refers to concentration of the riboswitch inducer A43 in addition to 0.5mM IPTG.

The graph illustrates that RFU intensity, indicative of GFP expression, increases along with inducer concentration. Furthermore, the uniform distribution achieved demonstrates that all the cells within the population are expressing GFP at approximately the 'same' level. This observation allows the skilled person to match the expression rate of the gene of interest to one or more of a number of different relevant cell characteristics; to synthetic capacity of the cell, to chaperone capacity, and potentially to secretion capacity. Finally, as all of the cells at any given dose of riboswitch inducer are uniform there should also be no difference between protein homogeneity across the cellular population.

Figure 55 illustrates use of genetic constructs comprising the riboswitch $PreQ_1$ to establish conditional mutants for MreB that are of use in target validation and drug screening.

Screening new compounds for antibacterial activity can be a challenging and laborious, and can lead to false positives due to the non-optimised physiochemical properties of compounds in the screening libraries. To aid this screening process methods have been implemented to increase the sensitivity of bacteria to compounds and to help increase the screening hit rate. Notably, where the antisense sequence for a drug target of interest has been placed under the control of an inducible promoter [Wang et al Nature 2006, 441, 358].

As described herein, the inventors have developed a novel method where an antibiotic target gene of interest has been put under control of a mutant PreQ-C17U riboswitch, using in combination a knock-out strain of that gene of interest This conditional mutant demonstrates a wild-type phenotype with respect to growth rate and morphology, but ideally results in a conditional 'sensitive' phenotype suitable for drug screening only upon addition of the PreQ-C17U riboswitch ligand D9.

The bacterial actin, MreB, forms helical cables underneath the cytoplasmic membrane and can interact with the enzymes for peptidoglycan biosynthesis [Errington et al Trends Cell Biol. 2003 13, 577-83]. It plays a key role in elongation of the cell wall in most rod-shaped bacteria. Accordingly MreB is a promising target for broad-spectrum antibiotics.

The inventors have developed a gene expression tool, the $preQ_1$ riboswitch, which can be used to control different level of MreB expression in response to variation of the concentration of the D9 ligand. In keeping with the inventors' expectations, this tool has utility in drug screening, as demonstrated by the present example.

*B.subtilis* 3725-ΔMreB, as employed in this study, is a magnesium-dependent null mutant which hardly grows in media without magnesium. A construct $preQ_1$C17U-mreB (in which MreB expression is controlled by the C17U mutant form of the $preQ_1$ riboswitch described above) was set up within *amyE* gene fragment as a linear DNA and integrated into *amyE* locus of *B.subtilis* 3725-AMreB, producing a conditional mutant *B.subtilis* 3725C. This resulting strain can grow well in media without magnesium, presenting a growth rate of 0.27 (OD/min) which is similar to that of the 3725 wt strain grows in the medium with magnesium (0.29 OD/min). This demonstrates that the $preQ_1$C17U-*mreB* construct overexpresses MreB and complements the phenotype of *B.subtilis* 3725. Furthermore, treatment of D9 (1.25 mM) can repress the growth rate of the 3725C strain to 0.15 OD/min, indiacting that D9 represses the overexpression of MreB, and thus provides a conditional mutant with potential drug screening applications.

In addition to applications of this method for use in antibacterial screening programme, this method also has application for pharmaceutical target validation for 'hit' compounds. Where the gene product believed to be target for a hit compound can be placed under a repressible mutant riboswitch construct as above and used to compare phenotype and confirm the hit's cellular target.

Figure 56 shows the addA riboswitch connected to the the T3 RNA polymerase optimally connected by the spacer detailed in this disclosure. With addition of IPTG (0.5mM) and upon increasing concentration of the riboswitch inducer A1 (0.8, 4, 20, 100, 250, 500uM) dose-dependent control of the polymerase is observed, this is visualised by anti-His6 western blot.

Figure 57 Dual Promoter construct transcriptionally regulated by a lac promoter/operator, where the M6" riboswitch - A43 ligand combination control the expression of the spT7 RNA polymerase, separated by at tR1 transcriptional terminator, a second lac promoter/operator and the addA riboswitch-A1 ligand combination control the expression of the T3 RNA polymerase (pTTv9).

Expression induced with A43 at 2, 10, 50, 250uM in the absence and presence (+) of A1 at 250uM, and A1 addition at 2, 10, 50, 250uM in the absence and presence (+) of A43 at 250uM (b). Addition of A43 (2→250uM) permits dose dependent regulation of the T7RNAP, in the absence and presence (+) of A1 (250uM) visualized by western blot with αT7 (c). Addition of A1 (2→250uM) permits dose-dependent regulation of the T3RNAP, in the absence and presence (+) of A43 (250uM) visualized by western blot with αHis (d).

By combining mutually-orthogonal riboswitches, this multicomponent Dual Promoter co-expression system has been constructed, which can be used in combination with distinct small-molecule inducer compounds to control the differential expression of multiple genes within the same cell. The Dual Promoter system containing two different viral RNA polymerases (T7 and T3) permits simultaneous and independent control over two genes with no "cross-talk" between the genetic control elements. As the two polymerase are additionally selective for their respective promoter sequences (T7: TAATACGACTCACTATAGG) (T3: AATTAACCCTCACTAAAGG) [Morris et al, Gene, 1986, 41, 193-200], using an expression plasmid such as a modified pET vector containing with two genes of interest under control of the T7 and T3 promoter allows the multicomponent-decoupled system to control the genes of interest as is shown for the single component systems (Figure 50, 51)

Improved 2nd generation ligands have been discovered both in terms of in vivo and in vitro binding affinity. Specifically the pyrimido[4,5-d]pyrimidine-2,4-diamine A43 ligand demonstrates greatly improved activity against the M6" when compared to the 1st generation ligands (Amm) this is demonstrated clearly in the eGFP expression assay (Figure 58), where dose-dependent control over eGFP expression is observed, further the maximum expression exceeds that of the WT (adddA) with adenine. The A43 ligand is again entirely selective for the M6" riboswitch and demonstrates no affinity toward to the AddA(wt) riboswitch, this is demonstrated by the Isothermal titration calorimetry measurements (Figure 59), where the A43 affinity for the aptamer of the M6" riboswitch = 189nM. An additional 2nd generation ligand discovered the 2,4-Diaminopteridine A44 interestingly shows approximately equal in vitro biding affinity to both the

addA(wt) and M6" riboswitch aptamers both ~2uM (Figure 60). This indicates that by placement of the heterocyclic nitrogens in either positions 1-3 (A43), and 1-4 (A44), the selectivity of the ligand can be changed from M6" selective to equally active against both addA and M6" aptamers.

The inventors further found from *in vivo* and *in vitro* analysis that a number of guanosine nucleotide analogues induced gene expression when assayed against E.coli cells bearing a plasmid containing the riboswitch-reporter gene construct M6C"-eGFP (Figure 9, 10). Isothermal titration calorimetry plots between the riboswitch aptamers M6C" and ligands indicate $K_d$ values range from 165-460uM for ligands A52, C44, C39 shown (Figure 61)

## Claims

1. A system comprising

   a) a first genetic construct comprising a promoter controlling transcription of first and second nucleic acid molecules, the first nucleic acid molecule encoding a riboswitch operably linked to the second nucleic acid molecule comprising a regulatory sequence encoding a polymerase; and
   b) a second genetic construct comprising a nucleic acid molecule comprising a coding sequence whose expression is capable of being regulated by the polymerase encoded by the second nucleic acid molecule;

   wherein said riboswitch is a naturally occurring riboswitch or a modified riboswitch having at least 85% sequence identity to a naturally occurring riboswitch.

2. A system according to claim 1 wherein the polymerase encoded by the regulatory sequence of the second nucleic acid molecule is a viral polymerase, which serves to regulate expression of the coding sequence of the second genetic construct.

3. A system according to claim 2 wherein the viral polymerase is selected from the group consisting of: T7, SP6, and T3 viral polymerases.

4. A system according to any one of claims 1 to 3 wherein the first genetic construct comprises a first RNA molecule comprising a riboswitch operably linked to a second RNA molecule which is, or encodes, a regulatory sequence.

5. A system according to any one of claims 1 to 4 wherein the second genetic construct is DNA or RNA.

6. A system according to any one of claims 1 to 5, wherein the first genetic construct comprises a spacer operably linked to the riboswitch.

7. A system according to claim 6 wherein the second genetic construct may independently comprise a nucleic acid molecule comprising a spacer sequence.

8. A system according to any preceding claim, wherein the promoter is selected from the group consisting of: T7, T3, Sp6, araC, araBAD, lac promoter; lacUCV5; tac; trc; trpR promoter; Omp; tet promoter; luxR; lambda Prm promoter; and pLac promoter.

9. A kit of parts, comprising a first genetic construct and a second genetic construct of the system according to any one of claims 1 to 8, and optionally a ligand of a riboswitch of a first genetic construct of the system.

10. A method of regulating expression of a coding sequence, the method comprising: contacting a cell or expression system comprising a system according to any one of claims 1 to 8 in vitro with a ligand of the riboswitch, wherein the expression of said coding sequence is regulated by the polymerase encoded by the second nucleic acid molecule of the system according to any one of claims 1 to 8.

11. A method of regulating expression of a coding sequence, thereby:

   (a) inhibiting bacterial cell growth;
   (b) increasing or decreasing the yield of a coding sequence product or products;
   (c) altering the physiological characteristics of a cell;
   (d) increasing gene production;

(e) altering cell morphology;
(f) altering sensitivity to an antibiotic; or
(g) increasing flux through a biosynthetic pathway;

wherein the method comprises contacting a cell or expression system comprising a system according to any one of claims 1 to 8 in vitro with a ligand of the riboswitch, and wherein the expression of said coding sequence is regulated by the polymerase encoded by the second nucleic acid molecule of the system according to any one of claims 1 to 8.

**12.** Use of a system according to any one of claims 1 to 8 as a tool for drug discovery, or as a biosensor, wherein the use comprises regulating expression of a coding sequence by contacting a cell or expression system comprising a system according to any one of claims 1 to 8 in vitro with a ligand of the riboswitch, and wherein the expression of said coding sequence is regulated by the polymerase encoded by the second nucleic acid molecule of the system according to any one of claims 1 to 8.

**13.** A system according to any one of claims 1 to 8 for use in therapy.

**14.** A cell or expression system comprising a first genetic construct of the system according to any one of claims 1 to 8, and a second genetic construct of a system according to any one of claims 1 to 8, and optionally a ligand of the riboswitch of the first genetic construct.

**15.** A kit or cell or expression system according to claim 9 or claim 14, wherein the ligand is a compound having a formula selected from the group consisting of:

A: a riboswitch ligand defined by Formula I:

**I**

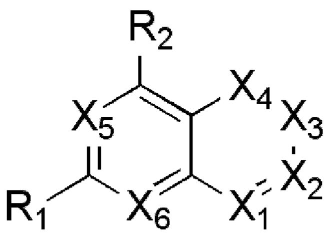

wherein

$R_1$ and $R_2$ are independently selected from a hydrogen bond donor or a hydrogen bond acceptor, for example H or $NH_2$, O, OH; and
$X_1$, $X_2$, $X_3$, $X_4$ $X_5$, and $X_6$ are independently selected from any hydrogen bond donor or hydrogen bond acceptor, or from CH or N;
optionally, such a ligand wherein $R_1$ and $R_2$ are independently selected from O, OH, $NH_2$, $NHCOCH_3$, $NHCO_2CH_3$ $NHcCH(CH_2CH_2)$, SH, $NHNHCOCH_3$, $NHOCH_3$, or NHOR (R=alkyl, aryl, or benzyl); optionally wherein the ligand is a compound selected from:

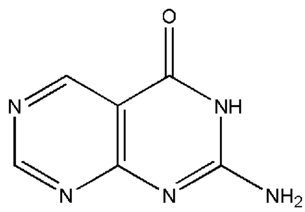
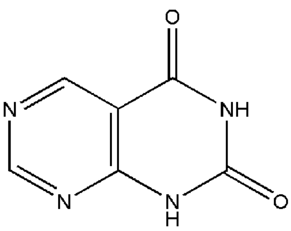
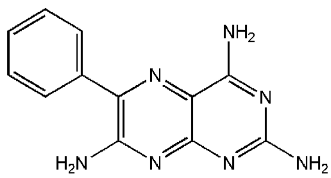

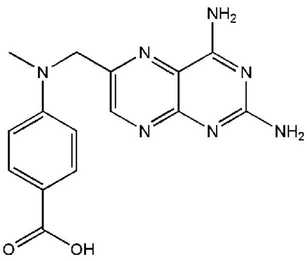 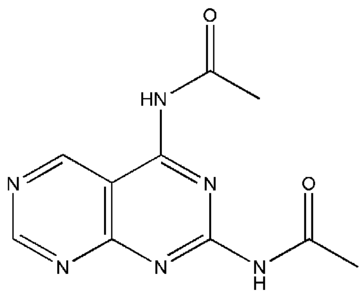 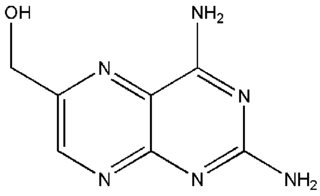

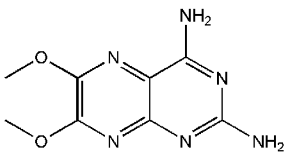 or 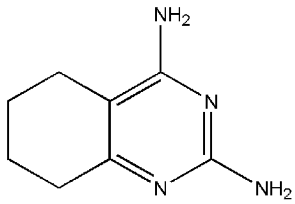 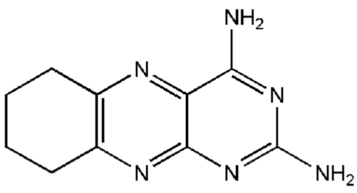

optionally, such a riboswitch ligand wherein both $R_1$ and $R_2$ are $NH_2$;
optionally, such a riboswitch ligand wherein the ligand is a compound selected from:

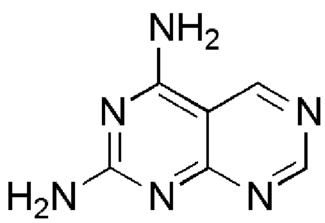 or 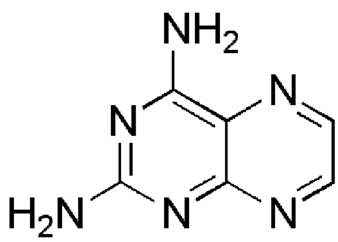

B: a riboswitch ligand defined by Formula II:

II

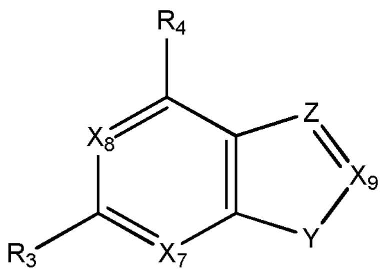

wherein

$R_3$ and $R_4$ are independently selected from a hydrogen bond donor or a hydrogen acceptor;
$X_7$, $X_8$ and $X_9$ are independently selected from CH or any hydrogen bond donor or any hydrogen bond acceptor.
Y is independently selected from N, O, S, NR, where R is independently selected from alkyl, ribose, deoxyribose, dideoxyribose, (hydroxymethyl)morpholino analogues of ribose or 5'-modified ribose;
Z is independently selected from any one of N, C attached to $-CH_2OH$, $-CH_2NH_2$, CN, or $CONH_2$;
optionally, such a riboswitch ligand wherein Z is O;
optionally, such a riboswitch ligand wherein both $R_3$ and $R_4$ are $NH_2$;
optionally, such a riboswitch ligand having a formula :

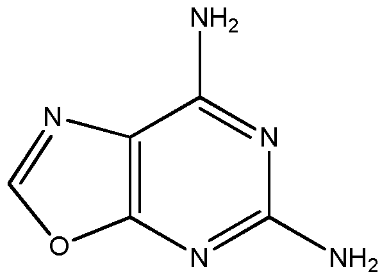

optionally, such a riboswitch ligand wherein $R_3$ and $R_4$ are independently selected from OH, $NH_2$, $NHCOCH_3$, $NHCO_2CH_3$, $NHcCH(CH_2CH_2)$, SH, $NHNHCOCH_3$, $NHOCH_3$, NHCO $CH(CH_2)_3$ NHOR (where R=alkyl, aryl, or benzyl)

optionally, such a riboswitch ligand wherein the ligand is a compound selected from:

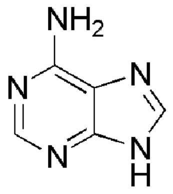 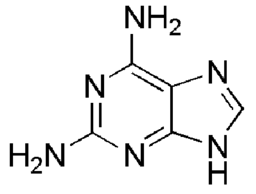 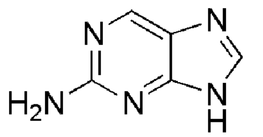

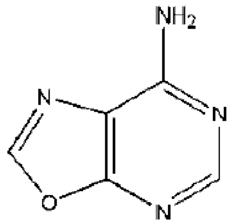 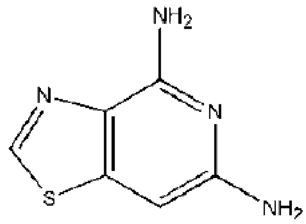 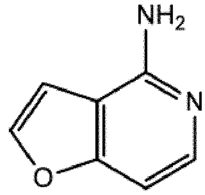

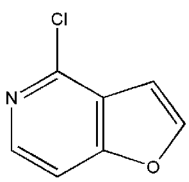 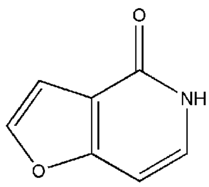 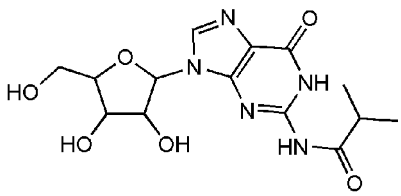 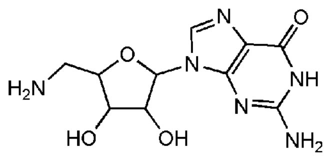

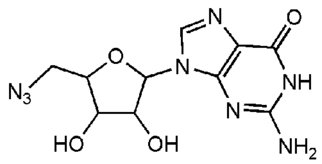 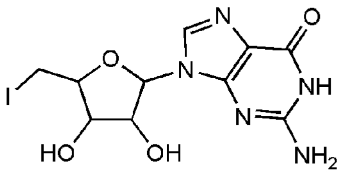

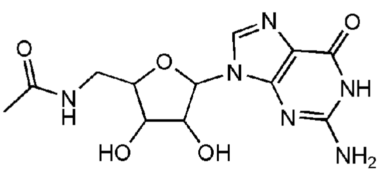 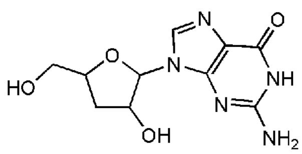 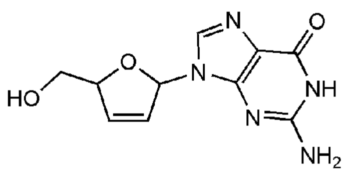

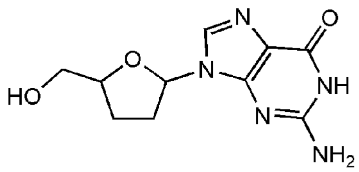 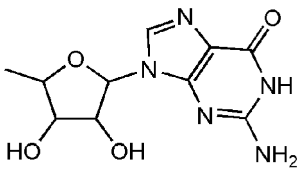 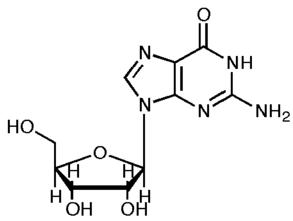

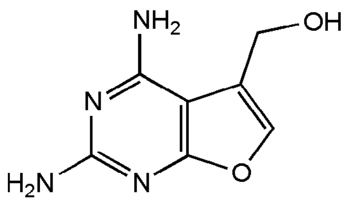 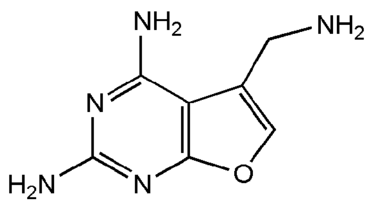 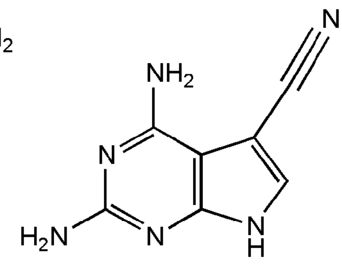

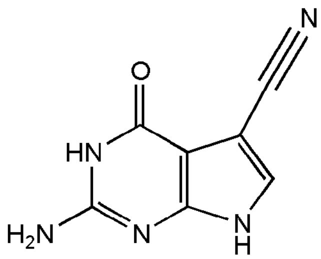 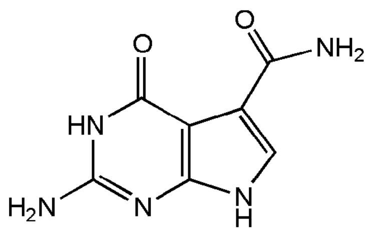 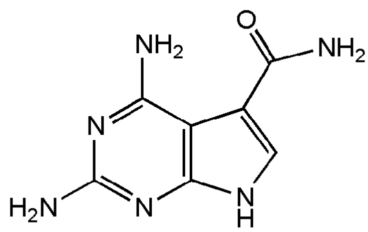

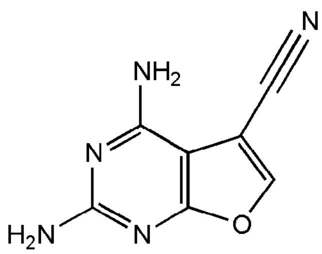 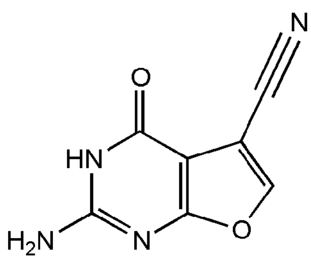 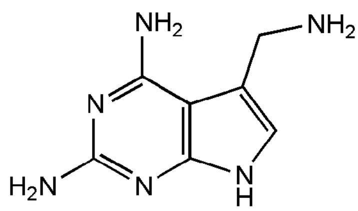

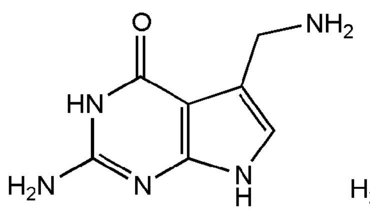 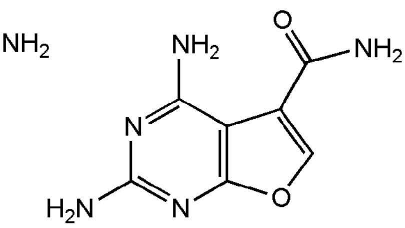 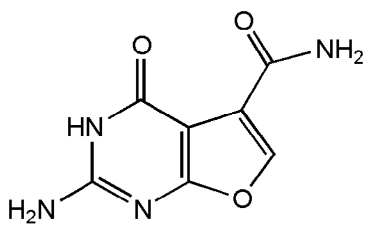

and

C: a riboswitch ligand defined by Formula III

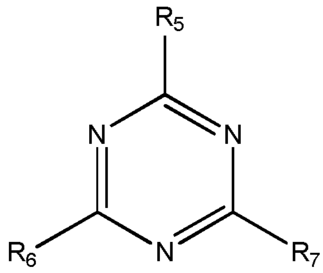

wherein

$R_5$ $R_6$ and $R_7$ is independently selected from aryl or heteroaryl; and $R_5$ is optionally substituted by $C(O)NH_2$, are independently selected from H, $NH_2$, O, OH, $NHCOCH_3$, $NHCO_2CH_3$, $NHcCH(CH_2CH_2)$, SH, $NHNHCOCH_3$, $NHOCH_3$, NHCO $CH(CH_2)_3$ NHOR (where R=alkyl, aryl, or benzyl);
optionally such a riboswitch ligand wherein the ligand is selected from:

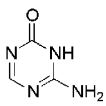 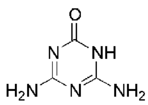 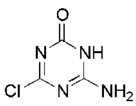 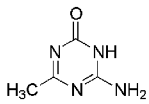 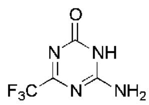 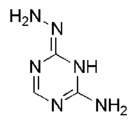 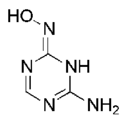 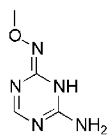

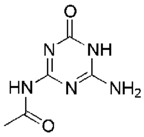 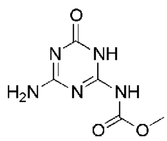 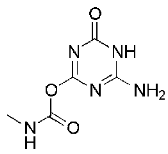 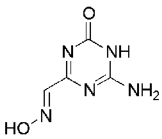 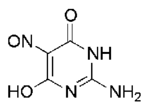 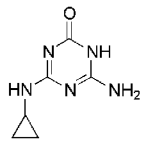 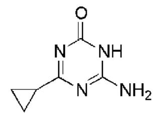

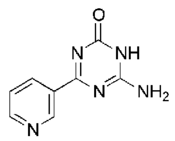 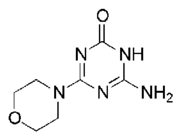 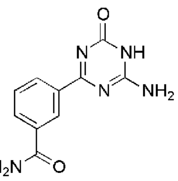 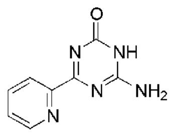 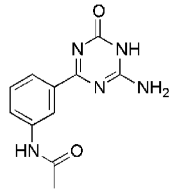 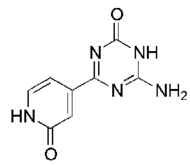

**16.** A system, kit, method, use, cell or expression system according to any one of the previous claims, wherein the riboswitch provided in a genetic construct is:

(a) a purine riboswitch of Figure 11;
(b) an Add-A riboswitch of Figure 13, or a modified Add-A riboswitch having at least, 85, 90, 95, 96, 97, 98 or 99% sequence identity thereto, such as M6, M6', M6" or M6C, M6C' or M6C"; or
(c) a PreQ switch or a modified PreQ switch of Figure 14 or a riboswitch as shown in Figure 16.

**17.** A system, kit, method, use, cell or expression system according to any one of the previous claims, wherein the riboswitch provided in a genetic construct is an orthogonal riboswitch.

## Patentansprüche

**1.** System umfassend

a) ein erstes genetisches Konstrukt umfassend einen Promotor, der die Transkription eines ersten und eines zweiten Nukleinsäuremoleküls steuert, wobei das erste Nukleinsäuremolekül einen Riboswitch kodiert, der mit dem zweiten Nukleinsäuremolekül, das eine regulatorische Sequenz, die eine Polymerase kodiert, umfasst, funktionell verbunden ist; und

b) ein zweites genetisches Konstrukt umfassend ein Nukleinsäuremolekül umfassend eine kodierende Sequenz, deren Expression durch die Polymerase, die vom zweiten Nukleinsäuremolekül kodiert wird, reguliert werden kann;

wobei der Riboswitch ein natürlich vorkommender Riboswitch oder ein modifizierter Riboswitch ist, der eine Sequenzidentität von mindestens 85 % mit einem natürlich vorkommenden Riboswitch aufweist.

**2.** System nach Anspruch 1, wobei die Polymerase, die von der regulatorischen Sequenz des zweiten Nukleinsäuremoleküls kodiert wird, eine virale Polymerase ist, die dazu dient, die Expression der kodierenden Sequenz des zweiten genetischen Konstruktes zu regulieren.

**3.** System nach Anspruch 2, wobei die virale Polymerase ausgewählt ist aus der Gruppe bestehend aus: virale Polymerasen T7, SP6 und T3.

**4.** System nach einem der Ansprüche 1 bis 3, wobei das erste genetische Konstrukt ein erster RNA-Molekül umfasst, das einen Riboswitch umfasst, der funktionell mit einem zweiten RNA-Molekül verbunden ist, das eine regulatorische Sequenz ist oder eine regulatorische Sequenz kodiert.

**5.** System nach einem der Ansprüche 1 bis 4, wobei das zweite genetische Konstrukt DNA oder RNA ist.

**6.** System nach einem der Ansprüche 1 bis 5, wobei das erste genetische Konstrukt einen Spacer umfasst, der funktionell mit dem Riboswitch verbunden ist.

**7.** System nach Anspruch 6, wobei das zweite genetische Konstrukt unabhängig ein Nukleinsäuremolekül, das eine Spacer-Sequenz umfasst, umfassen kann.

**8.** System nach einem der vorstehenden Ansprüche, wobei der Promotor ausgewählt ist aus der Gruppe bestehend aus: T7, T3, Sp6, araC, araBAD, lac-Promotor; lacUCV5, tac; trc; trpR-Promotor, Omp; tet-Promotor; luxR; lambda-Prm-Promotor und pLac-Promotor.

**9.** Teilesatz umfassend ein erstes genetisches Konstrukt und ein zweites genetisches Konstrukt des Systems nach einem der Ansprüche 1 bis 8 und optional einen Liganden eines Riboswitches eines ersten genetischen Konstrukts des Systems.

**10.** Verfahren zum Regulieren der Expression einer kodierenden Sequenz, wobei das Verfahren umfasst: Herstellen des Kontakts einer Zelle oder eines Expressionssystems umfassend ein System nach einem der Ansprüche 1 bis 8 in vitro mit einem Liganden des Riboswitches, wobei die Expression der kodierenden Sequenz durch die Polymerase reguliert wird, die vom zweiten Nukleinsäuremolekül des Systems nach einem der Ansprüche 1 bis 8 kodiert wird.

**11.** Verfahren zum Regulieren der Expression einer kodierenden Sequenz und dadurch:

(a) Hemmen des bakteriellen Zellwachstums;
(b) Erhöhen oder Verringern der Ausbeute des Produkts oder der Produkte einer kodierenden Sequenz;
(c) Verändern der physiologischen Eigenschaften einer Zelle;
(d) Erhöhen der Genproduktion;
(e) Verändern der Zellmorphologie;
(f) Verändern der Empfindlichkeit gegenüber einem Antibiotikum; oder
(g) Erhöhen des Stroms durch einen Biosyntheseweg;

wobei das Verfahren das Herstellen des Kontakts einer Zelle oder eines Expressionssystems umfassend ein System nach einem der Ansprüche 1 bis 8 in vitro mit einem Liganden des Riboswitches umfasst und wobei die Expression der kodierenden Sequenz durch die Polymerase reguliert wird, die vom zweiten Nukleinsäuremolekül des Systems nach einem der Ansprüche 1 bis 8 kodiert wird.

**12.** Verwendung eines Systems nach einem der Ansprüche 1 bis 8 als Instrument zur Arzneimittelforschung oder als Biosensor, wobei die Verwendung das Regulieren der Expression einer kodierenden Sequenz durch Herstellen des Kontakts einer Zelle oder eines Expressionssystems umfassend ein System nach einem der Ansprüche 1 bis 8 in

vitro mit einem Liganden des Riboswitches umfasst, und wobei die Expression der kodierenden Sequenz durch die Polymerase reguliert wird, die vom zweiten Nukleinsäuremolekül des Systems nach einem der Ansprüche 1 bis 8 kodiert wird.

**13.** System nach einem der Ansprüche 1 bis 8 zur Verwendung in der Therapie.

**14.** Zelle oder Expressionssystem umfassend ein erstes genetisches Konstrukt des Systems nach einem der Ansprüche 1 bis 8 und ein zweites genetisches Konstrukt eines Systems nach einem der Ansprüche 1 bis 8 und optional einen Liganden des Riboswitches des ersten genetischen Konstrukts.

**15.** Satz oder Zelle oder Expressionssystem nach Anspruch 9 oder Anspruch 14, wobei der Ligand eine Verbindung ist, die eine Formel aufweist, die ausgewählt wurde aus der Gruppe bestehend aus:

A: einem Riboswitch-Liganden, der definiert ist durch die Formel I:

**I**

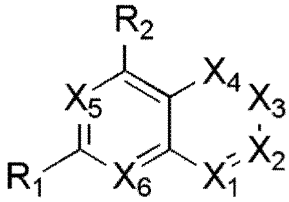

wobei

$R_1$ und $R_2$ unabhängig ausgewählt sind aus einem Donator einer Wasserstoffbrückenbindung oder einem Akzeptor einer Wasserstoffbrückenbindung, zum Beispiel H oder $NH_2$, O, OH; und $X_1$, $X_2$, $X_3$, $X_4$, $X_5$ und $X_6$ unabhängig ausgewählt sind aus einem beliebigen Donator einer Wasserstoffbrückenbindung oder einem beliebigen Akzeptor einer Wasserstoffbrückenbindung oder aus CH oder N;
optional einem solchen Liganden, wobei $R_1$ und $R_2$ unabhängig ausgewählt sind aus O, OH, $NH_2$, $NHCOCH_3$, $NHCO_2CH_3$, $NHcCH(CH_2CH_2)$, SH, $NHNHCOCH_3$, $NHOCH_3$ oder NHOR (R=Alkyl, Aryl oder Benzyl);
wobei der Ligand optional eine Verbindung ist, die ausgewählt ist aus:

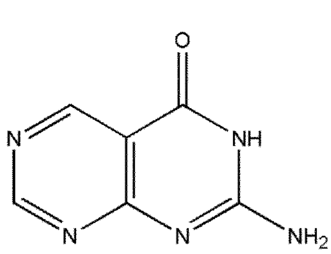

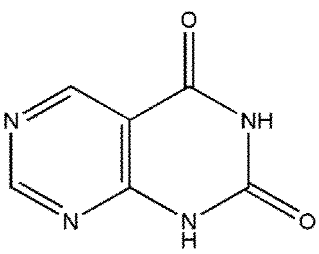

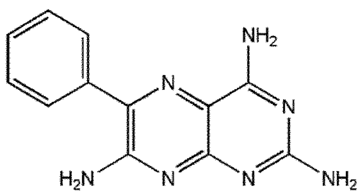

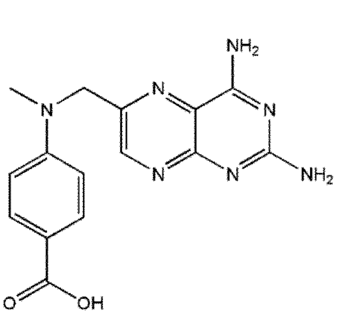

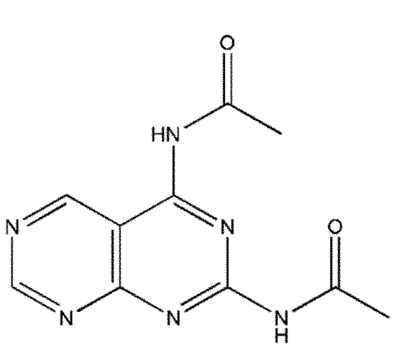

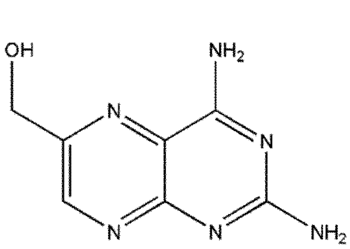

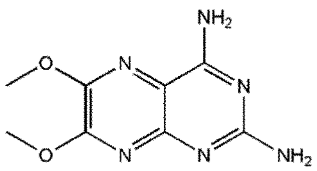 oder 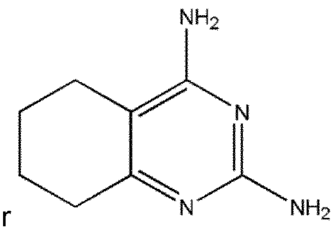 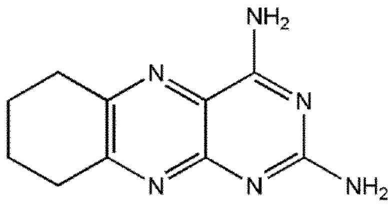

optional einem solchen Riboswitch-Liganden, wobei $R_1$ und $R_2$ $NH_2$ sind;
optional einem solchen Riboswitch-Liganden, wobei der Ligand eine Verbindung ist, die ausgewählt ist aus:

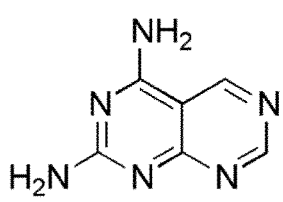 oder 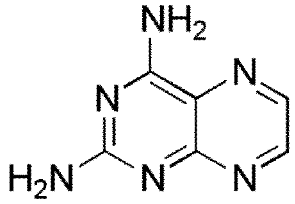

B: einem Riboswitch-Liganden, der definiert ist durch die Formel II:

II

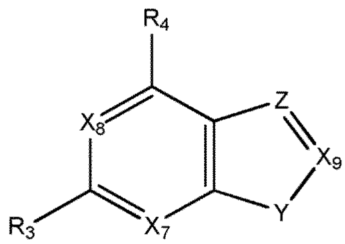

wobei $R_3$ und $R_4$ unabhängig ausgewählt sind aus einem Donator einer Wasserstoffbrückenbindung oder einem Akzeptor einer Wasserstoffbrückenbindung;
$X_7$, $X_8$ und $X_9$ unabhängig ausgewählt sind aus CH oder einem beliebigen Donator einer Wasserstoffbrückenbindung oder einem beliebigen Akzeptor einer Wasserstoffbrückenbindung.
Y ist unabhängig ausgewählt aus N, O, S, NR, wobei R unabhängig ausgewählt ist aus Alkyl, Ribose, Desoxyribose, (Hydroxymethyl)Morpholino-Analoga von Ribose oder 5'-modifizierter Ribose;
Z ist unabhängig ausgewählt aus einem von N, C angelagert an $-CH_2OH$, $-CH_2NH_2$, CN oder $CONH_2$;
optional einem solchen Riboswitch-Liganden, wobei Z 0 ist;
optional einem solchen Riboswitch-Liganden, wobei sowohl $R_3$ als auch $R_4$ $NH_2$ sind;
optional einem solchen Riboswitch-Liganden mit einer Formel:

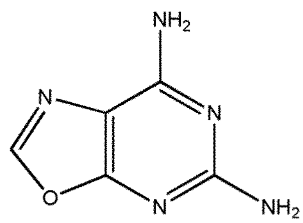

optional einem solchen Riboswitch-Liganden, wobei $R_3$ und $R_4$ unabhängig ausgewählt sind aus OH, $NH_2$, $NHCOCH_3$, $NHCO_2CH_3$, $NHcCH(CH_2CH_2)$, SH, $NHNHCOCH_3$, $NHOCH_3$, $NHCO\ CH(CH_2)_3NHOR$ (mit R=Alkyl, Aryl oder Benzyl);
optional einem solchen Riboswitch-Liganden, wobei der Ligand eine Verbindung ist, die ausgewählt ist aus:

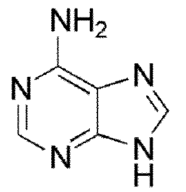
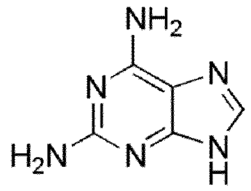
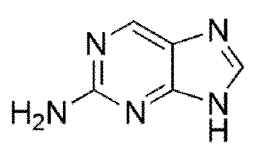

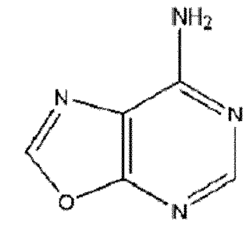
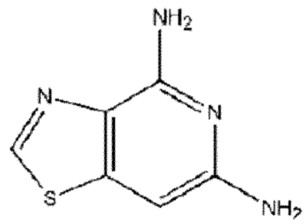
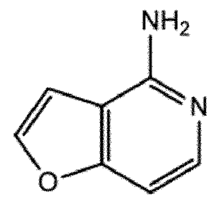

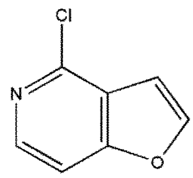
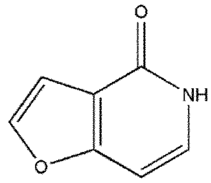
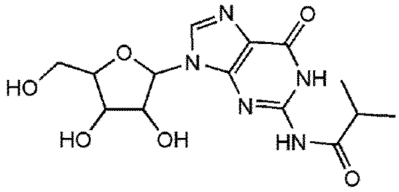
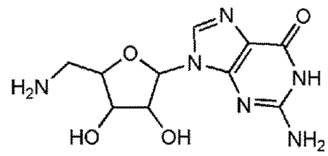

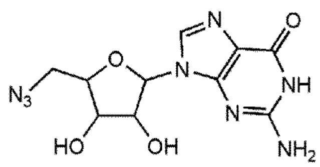
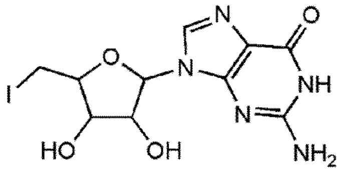

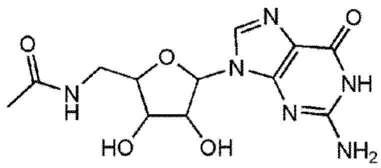
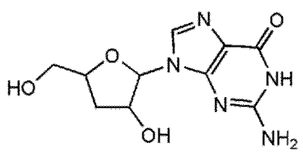
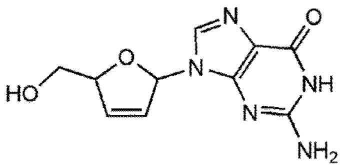

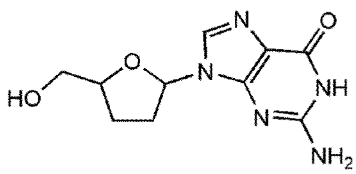
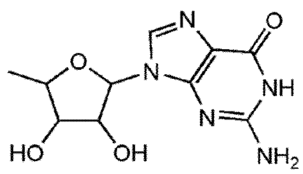
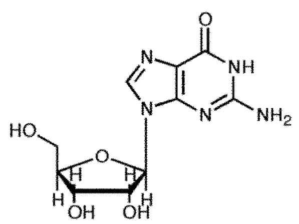

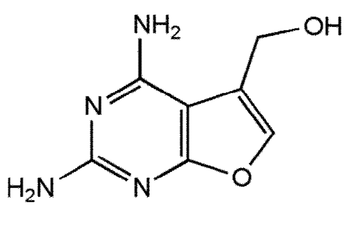
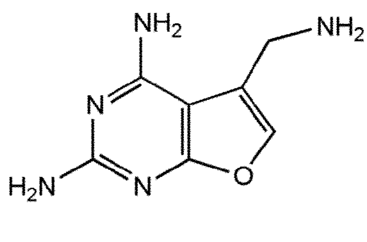
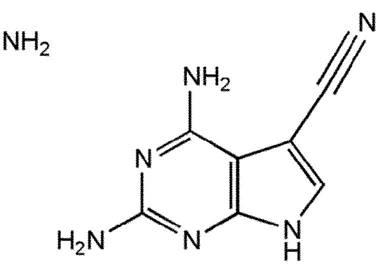

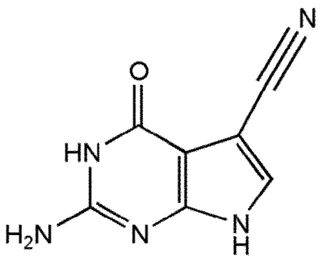 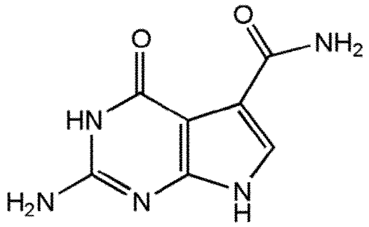 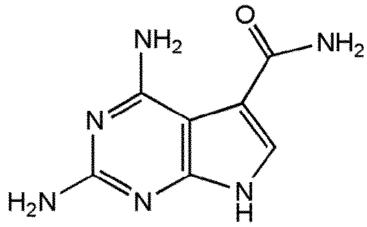

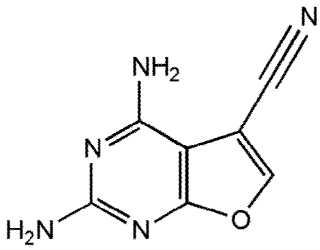 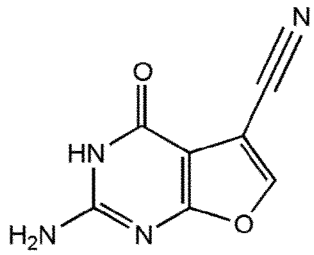 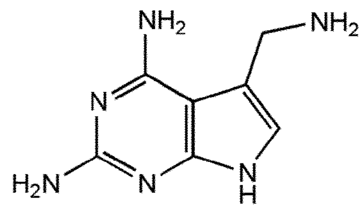

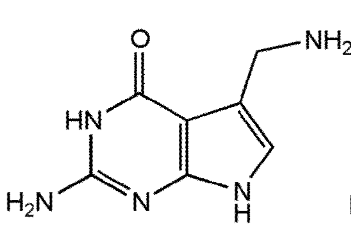 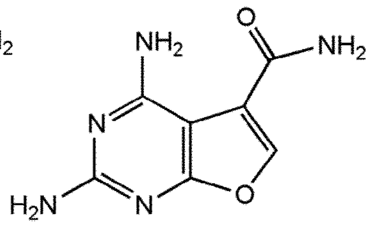 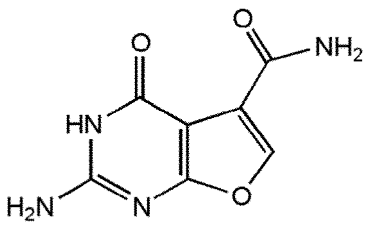

und

C: einem Riboswitch-Liganden, der definiert ist durch die Formel III

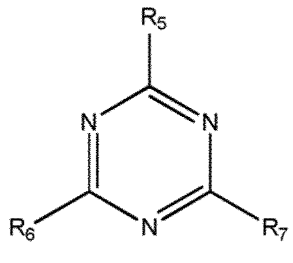

wobei

$R_5$, $R_6$ und $R_7$ unabhängig ausgewählt sind aus Aryl oder Heteroaryl; und $R_5$ optional substituiert ist durch $C(O)NH_2$, unabhängig ausgewählt sind aus H, $NH_2$, O, OH; $NHCOCH_3$, $NHCO_2CH_3$, $NHcCH(CH_2CH_2)$, SH, $NHNHCOCH_3$, $NHOCH_3$, NHCO $CH(CH_2)_3$ NHOR (mit R=Alkyl, Aryl oder Benzyl);

optional einem solchen Riboswitch-Liganden, wobei der Ligand ausgewählt ist aus:

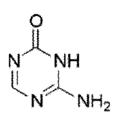 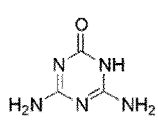 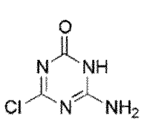 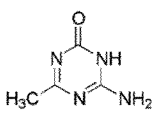 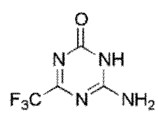 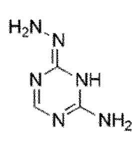 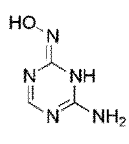 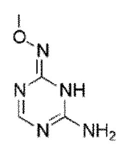

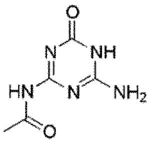 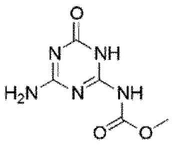 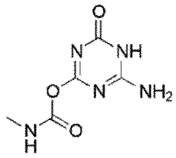 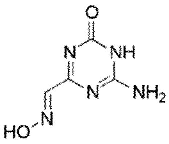 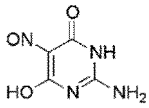 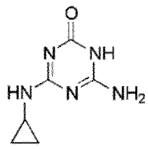 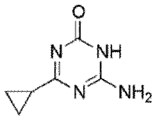

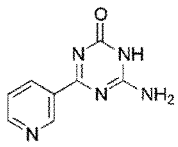 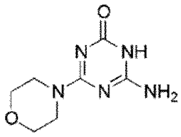 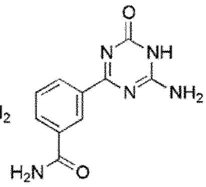 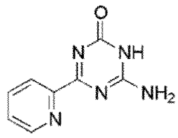 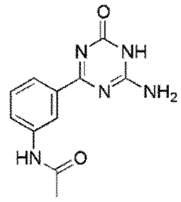 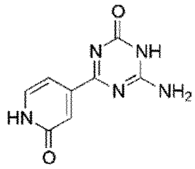

**16.** System, Satz, Verfahren, Verwendung, Zelle oder Expressionssystem nach einem der vorstehenden Ansprüche, wobei der Riboswitch, der in einem genetischen Konstrukt bereitgestellt wird,

(a) ein Purin-Riboswitch aus Figur 11 ist;
(b) ein Add-A-Riboswitch aus Figur 13 oder ein modifizierter Add-A-Riboswitch ist, der eine Sequenzidentität von mindestens 85, 90, 95, 96, 97, 98 oder 99 % mit diesem aufweist, wie M6, M6', M6" oder M6C, M6C' oder M6C"; oder
(c) ein PreQ-Switch oder ein modifizierter PreQ-Switch aus Figur 14 oder ein Riboswitch, wie in Figur 16 dargestellt ist.

**17.** System, Satz, Verfahren, Verwendung, Zelle oder Expressionssystem nach einem der vorstehenden Ansprüche, wobei der Riboswitch, der in einem genetischen Konstrukt bereitgestellt wird, ein orthogonaler Riboswitch ist.

## Revendications

**1.** Système comprenant

a) une première construction génétique comprenant un promoteur régulant la transcription d'une première et d'une seconde molécule d'acide nucléique, la première molécule d'acide nucléique codant un riborégulateur fonctionnellement lié à la seconde molécule d'acide nucléique comprenant une séquence régulatrice codant une polymérase ; et
b) une seconde construction génétique comprenant une molécule d'acide nucléique comprenant une séquence codante dont l'expression peut être régulée par la polymérase codée par la seconde molécule d'acide nucléique ;

ledit riborégulateur étant un riborégulateur naturel ou un riborégulateur modifié présentant une identité de séquence d'au moins 85 % avec un riborégulateur naturel.

**2.** Système selon la revendication 1, ladite polymérase codée par la séquence régulatrice de la seconde molécule d'acide nucléique étant une polymérase virale, qui sert à réguler l'expression de la séquence codante de la seconde construction génétique.

**3.** Système selon la revendication 2, ladite polymérase virale étant choisie dans le groupe constitué par : les polymérases virales : T7, SP6 et T3.

**4.** Système selon l'une quelconque des revendications 1 à 3, ladite première construction génétique comprenant une première molécule d'ARN comprenant un riborégulateur fonctionnellement lié à une seconde molécule d'ARN qui est une séquence régulatrice ou la code.

**5.** Système selon l'une quelconque des revendications 1 à 4, ladite seconde construction génétique étant de l'ADN ou de l'ARN.

**6.** Système selon l'une quelconque des revendications 1 à 5, ladite première construction génétique comprenant un

espaceur fonctionnellement lié au riborégulateur.

7. Système selon la revendication 6, ladite seconde construction génétique pouvant indépendamment comprendre une molécule d'acide nucléique comprenant une séquence d'espaceur.

8. Système selon l'une quelconque des revendications précédentes, ledit promoteur étant choisi dans le groupe constitué par : T7, T3, Sp6, araC, araBAD, le promoteur lac, lacUCV5, tac, trc, le promoteur trpR, Omp, le promoteur tet, luxR, le promoteur lambda Prm et le promoteur pLac.

9. Kit de parties, comprenant une première construction génétique et une seconde construction génétique du système selon les revendications 1 à 8, et éventuellement un ligand de riborégulateur de la première construction génétique du système.

10. Méthode de régulation de l'expression d'une séquence codante, ladite méthode comprenant : la mise en contact d'une cellule ou d'un système d'expression comprenant un système selon l'un quelconque des revendications 1 à 8 in vitro avec un ligand du riborégulateur, l'expression de ladite séquence codante étant régulée par la polymérase codée par la seconde molécule d'acide nucléique du système selon l'une quelconque des revendications 1 à 8.

11. Méthode de régulation de l'expression d'une séquence codante, ce qui permet de :

(a) empêcher la croissance des cellules bactériennes ;
(b) augmenter ou diminuer le rendement d'un produit ou de produits de séquence codante ;
(c) modifier les caractéristiques physiologiques d'une cellule ;
(d) augmenter la production de gènes ;
(e) modifier la morphologie des cellules ;
(f) modifier la sensibilité à un antibiotique ; ou
(g) augmenter un flux à travers une voie biosynthétique ;

ledit procédé comprenant la mise en contact d'une cellule ou d'un système d'expression comprenant un système selon l'une quelconque des revendications 1 à 8 in vitro avec un ligand du riborégulateur, et l'expression de ladite séquence codante étant régulée par la polymérase codée par la seconde molécule d'acide nucléique du système selon l'une quelconque des revendications 1 à 8.

12. Utilisation d'un système selon l'une quelconque des revendications 1 à 8 comme outil pour la découverte de médicaments, ou comme biocapteur, ladite utilisation comprenant la régulation de l'expression d'une séquence codante par la mise en contact d'une cellule ou d'un système d'expression comprenant un système selon l'une quelconque des revendications 1 à 8 in vitro avec un ligand du riborégulateur, et l'expression de ladite séquence codante étant régulée par la polymérase codée par la seconde molécule d'acide nucléique du système selon l'une quelconque des revendications 1 à 8.

13. Système selon l'une quelconque des revendications 1 à 8 destiné à être utilisé en thérapie.

14. Cellule ou système d'expression comprenant une première construction génétique du système selon l'une quelconque des revendications 1 à 8, et une seconde construction génétique d'un système selon l'une quelconque des revendications 1 à 8, et éventuellement un ligand du riborégulateur de la première construction génétique.

15. Kit ou cellule ou système d'expression selon la revendication 9 ou 14, ledit ligand étant un composé présentant une formule choisie dans le groupe constitué par :

A : un ligand de riborégulateur défini par la formule I :

I

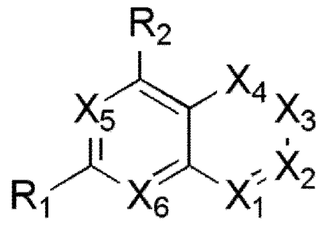

dans laquelle

$R_1$ et $R_2$ sont indépendamment choisis parmi un donneur de liaison hydrogène ou un accepteur de liaison hydrogène, par exemple un atome H ou un groupe $NH_2$, un atome O, un groupe OH ; et
$X_1$, $X_2$, $X_3$, $X_4$, $X_5$ et $X_6$ sont indépendamment choisis parmi un donneur de liaison hydrogène ou un accepteur de liaison hydrogène, ou parmi un groupe CH ou un atome N ;
éventuellement, tel un ligand dans lequel $R_1$ et $R_2$ sont indépendamment choisis parmi un atome O, un groupe OH, $NH_2$, $NHCOCH_3$, $NHCO_2CH_3$, $NHcCH(CH_2CH_2)$, SH, $NHNHCOCH_3$, $NHOCH_3$ ou NHOR (R = un groupe alkyle, aryle ou benzyle) ;
éventuellement ledit ligand étant un composé choisi parmi :

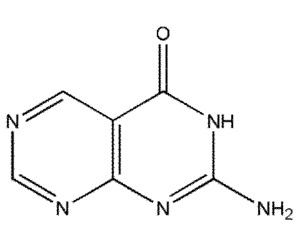
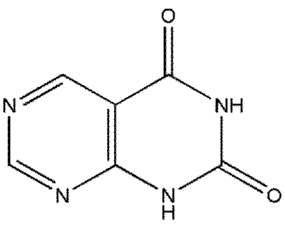
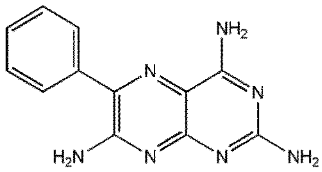
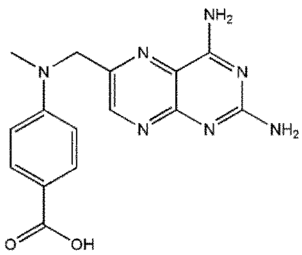
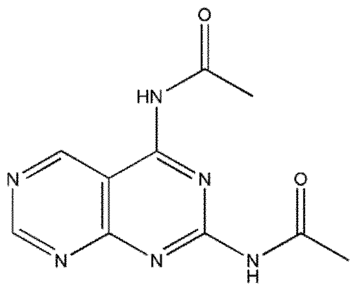
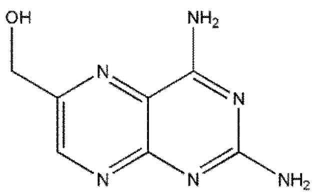
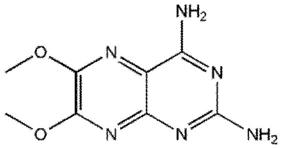
ou
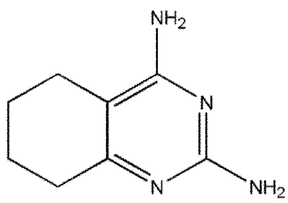
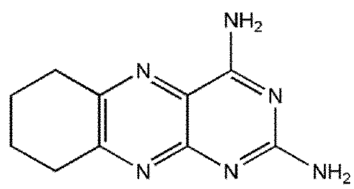

éventuellement, tel un ligand de riborégulateur dans lequel $R_1$ et $R_2$ représentent tous deux un groupe $NH_2$ ;
éventuellement, tel un ligand de riborégulateur, ledit ligand étant un composé choisi parmi :

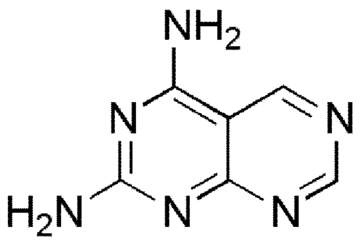

ou

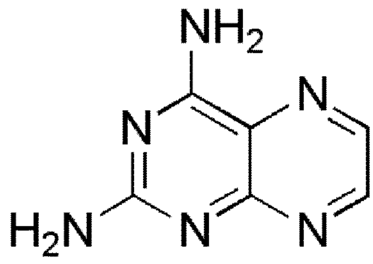

B : un ligand de riborégulateur défini par la formule II :

II

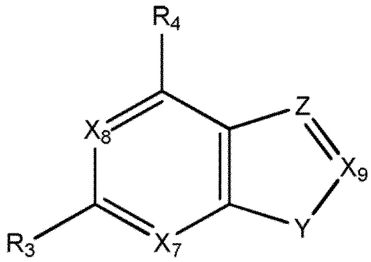

dans laquelle

$R_3$ et $R_4$ sont indépendamment choisis parmi un donneur de liaison hydrogène ou un accepteur d'hydrogène ;
$X_7$, $X_8$ et $X_9$ sont indépendamment choisis parmi un groupe CH ou tout donneur de liaison hydrogène ou tout accepteur de liaison hydrogène ;
Y est indépendamment choisi parmi un atome N, O, S, NR, où R est indépendamment choisi parmi un groupe alkyle, ribose, désoxyribose, didésoxyribose, les analogues (hydroxyméthyl)morpholino de ribose ou de ribose modifiée en 5' ;
Z est indépendamment choisi parmi l'un quelconque d'un atome N, C fixé à $-CH_2OH$, $-CH_2NH_2$, CN ou $CON_2$;
éventuellement tel un ligand de riborégulateur dans lequel Z représente un atome O ;
éventuellement tel un ligand de riborégulateur dans lequel $R_3$ et $R_4$ représentent tous deux un groupe $NH_2$ ;
éventuellement tel un ligand de riborégulateur présentant une formule :

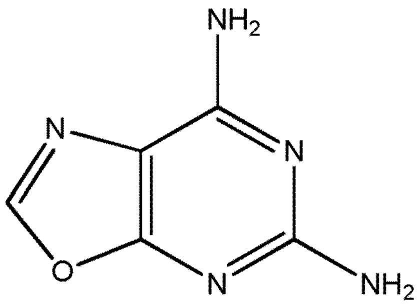

éventuellement, tel un ligand de riborégulateur dans lequel $R_3$ et $R_4$ sont indépendamment choisis parmi un groupe OH, $NH_2$, $NHCOCH_3$, $NHCO_2CH_3$, $NHcCH(CH_2CH_2)$, SH, $NHNHCOCH_3$, $NHOCH_3$, $NH-COCH(CH_2)_3NHOR$ (où R = un groupe alkyle, aryle ou benzyle) ; éventuellement, tel un ligand de riborégulateur, ledit ligand étant un composé choisi parmi :

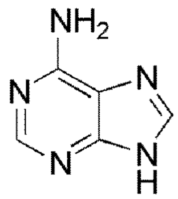 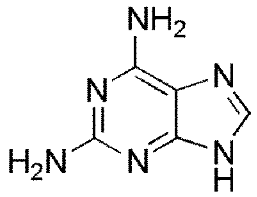 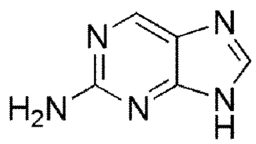

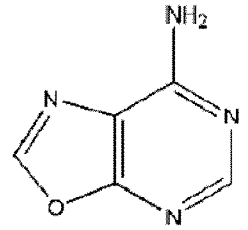 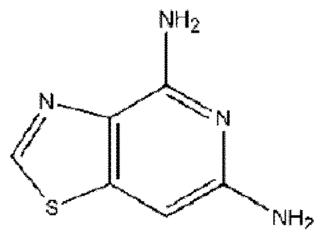 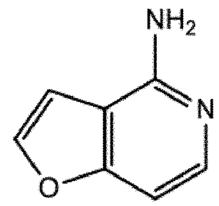

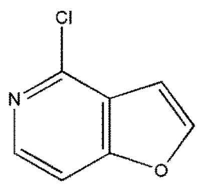 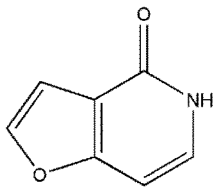 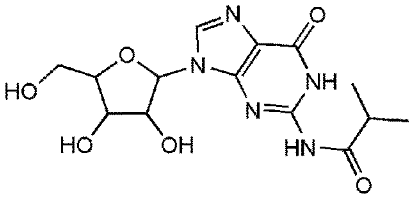 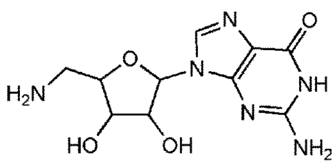

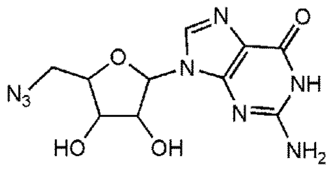 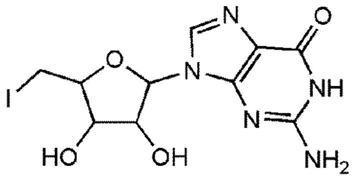

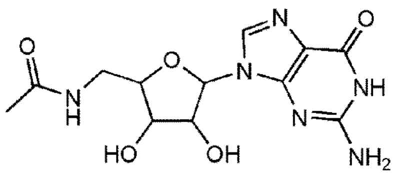 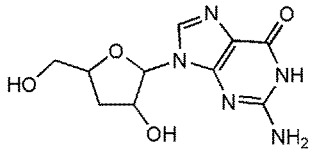 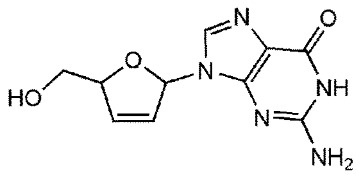

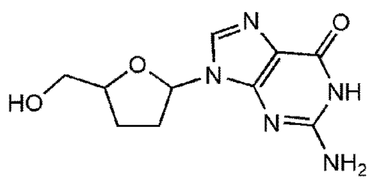 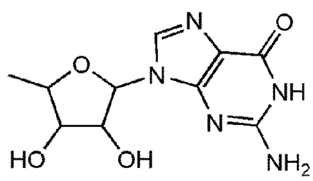 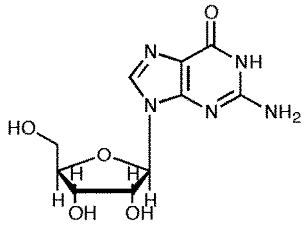

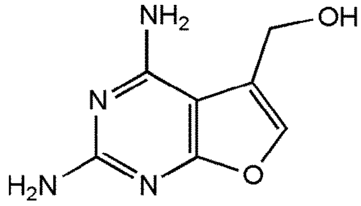 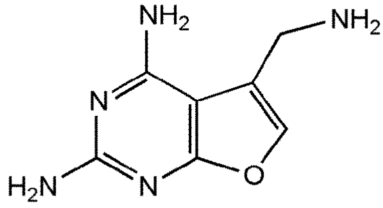 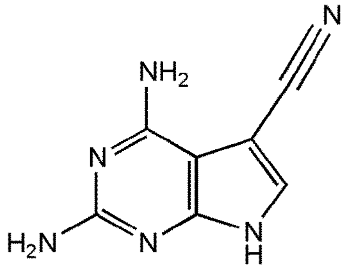

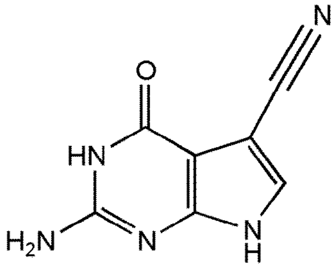 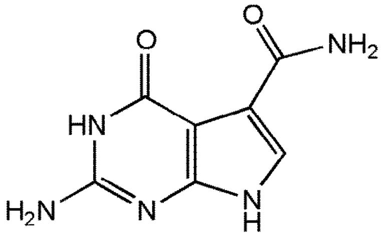 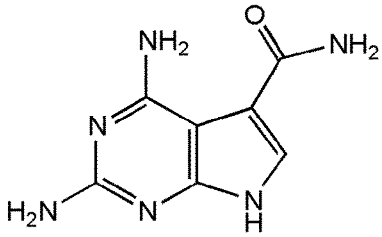

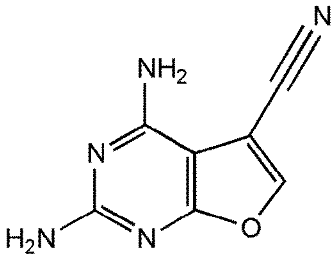 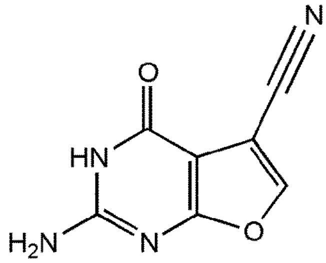 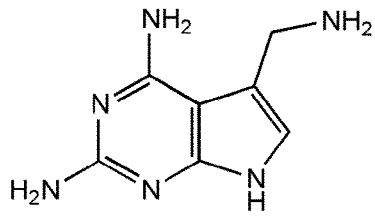

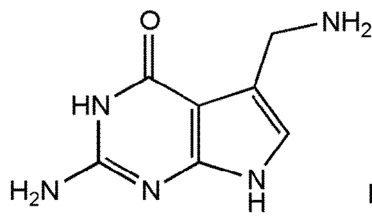 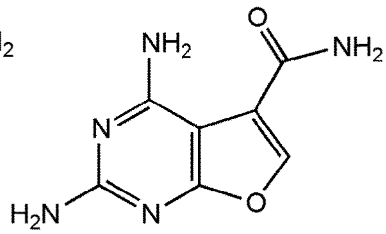 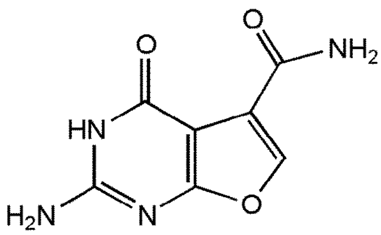

et

C : un ligand de riborégulateur défini par la formule III

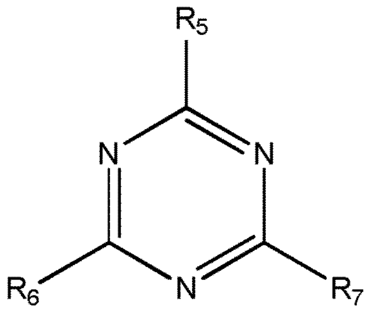

dans laquelle

$R_5$, $R_6$ et $R_7$ sont indépendamment choisis parmi un groupe aryle ou hétéroaryle ; et $R_5$ est éventuellement substitué par $C(O)NH_2$, sont indépendamment choisis parmi un atome H, un groupe $NH_2$, un atome O, un groupe OH, $NHCOCH_3$, $NHCO_2CH_3$, $NHcCH(CH_2CH_2)$, SH, $NHNHCOCH_3$, $NHOCH_3$, NH-$COCH(CH_2)_3NHOR$ (où R = un groupe alkyle, aryle ou benzyle) ; éventuellement, tel un ligand de riborégulateur, ledit ligand étant un composé choisi parmi :

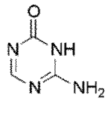 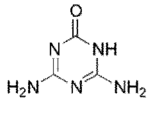 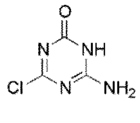 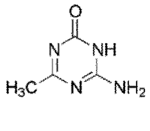 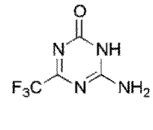 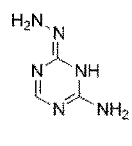 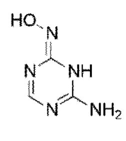 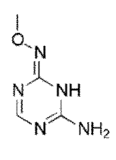

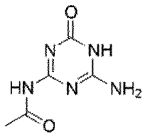 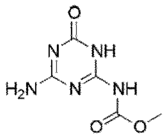 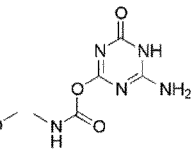 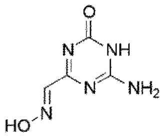 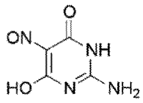 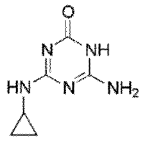 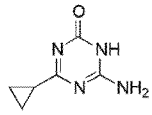

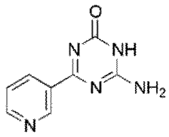 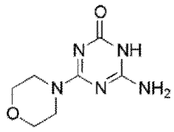 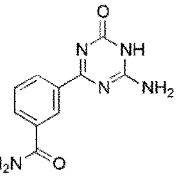 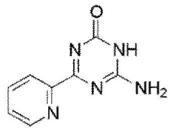 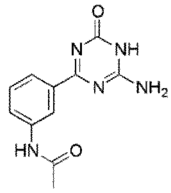 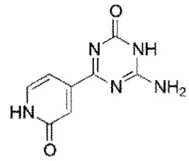

**16.** Système, kit, méthode, utilisation, cellule ou système d'expression selon l'une quelconque des revendications précédentes, ledit riborégulateur fourni dans une construction génétique étant :

(a) un riborégulateur de purine selon la figure 11 ;
(b) un riborégulateur Add-A selon la figure 13, ou un riborégulateur Add-A modifié présentant une identité de séquence d'au moins 85, 90, 95, 96, 97, 98 ou 99 % avec celui-ci, tel que M6, M6', M6" ou M6C, M6C'ou M6C" ; ou
(c) un régulateur PreQ ou un régulateur PreQ modifié selon la figure 14 ou un riborégulateur comme présenté dans la figure 16.

**17.** Système, kit, méthode, utilisation, cellule ou système d'expression selon l'une quelconque des revendications précédentes, ledit riborégulateur fourni dans une construction génétique étant un riborégulateur orthogonal.

FIGURE 1

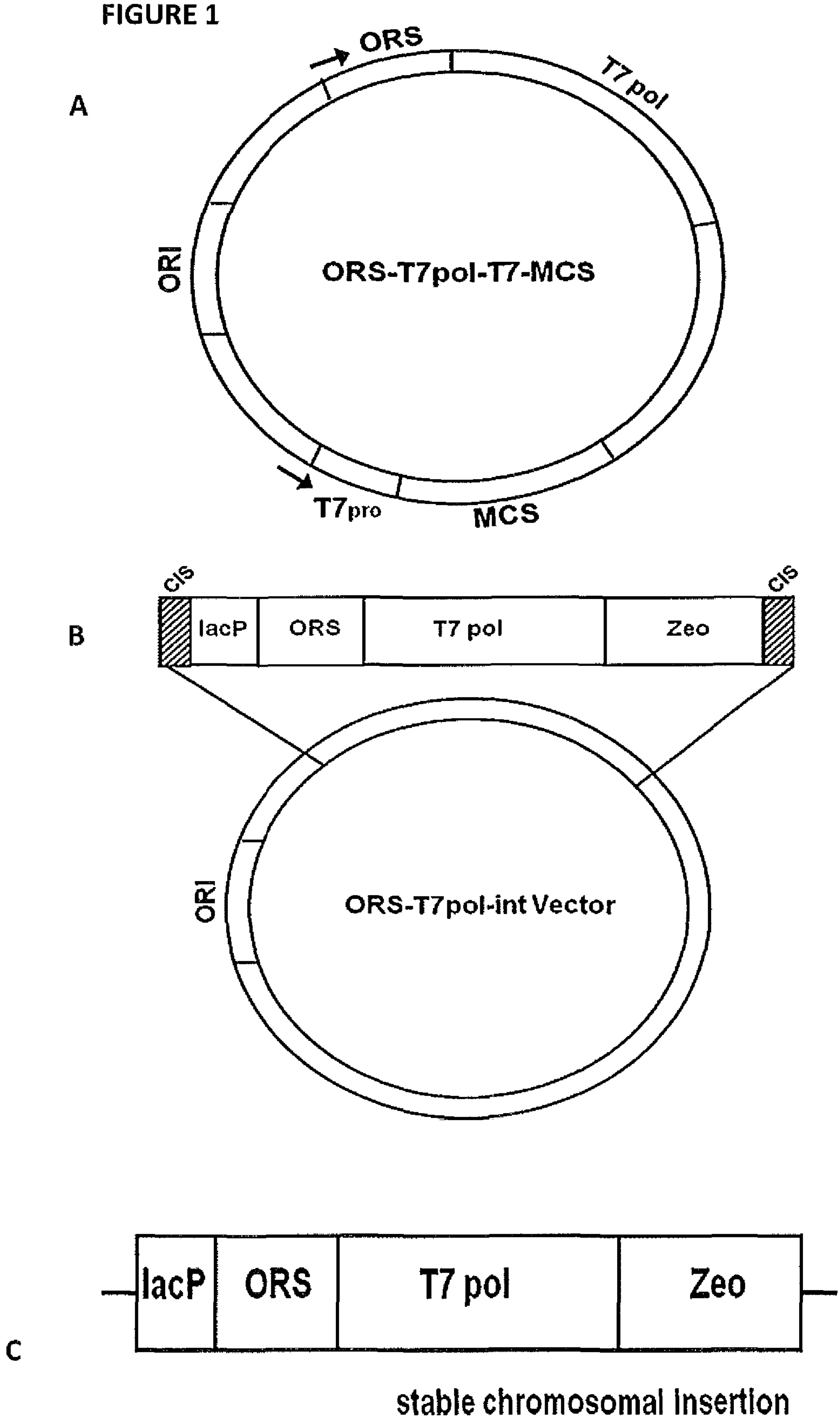
A
ORS
T7 pol
ORI
ORS-T7pol-T7-MCS
T7pro
MCS
B
CIS
lacP
ORS
T7 pol
Zeo
CIS
ORI
ORS-T7pol-int Vector
C
lacP
ORS
T7 pol
Zeo
stable chromosomal insertion

FIGURE 2

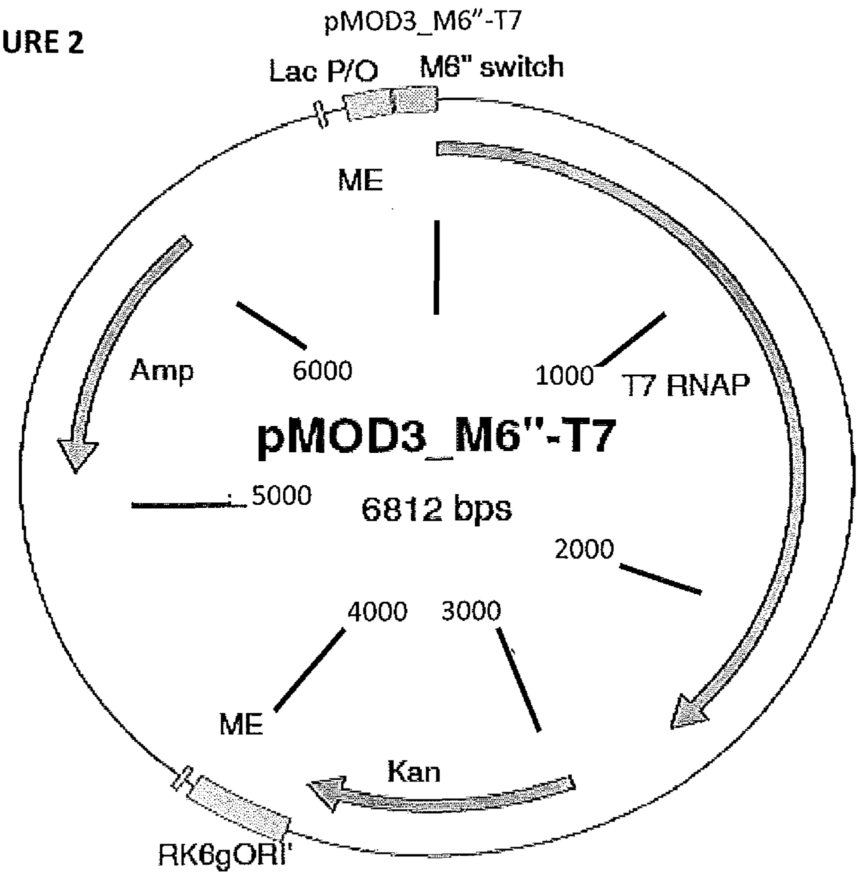
pMOD3_M6"-T7
Lac P/O
M6" switch
ME
Amp
6000
1000
T7 RNAP
pMOD3_M6"-T7
5000
6812 bps
2000
4000
3000
ME
Kan
RK6gORI'

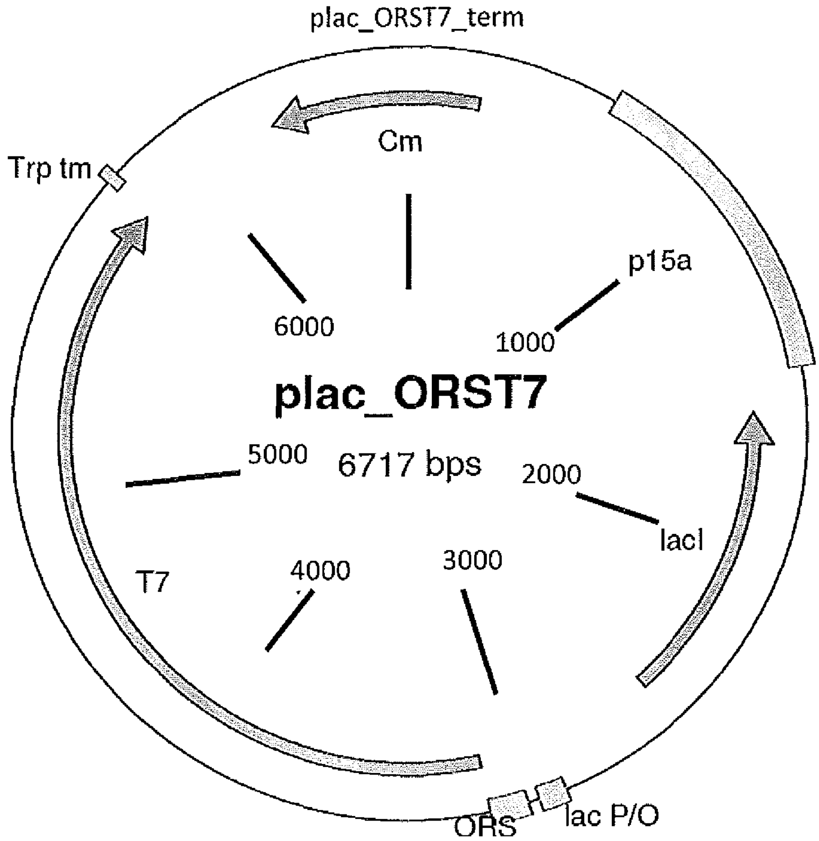
plac_ORST7_term
Cm
Trp tm
p15a
6000
1000
plac_ORST7
5000
6717 bps
2000
lacI
T7
4000
3000
ORS
lac P/O

**FIGURE 2 continued**

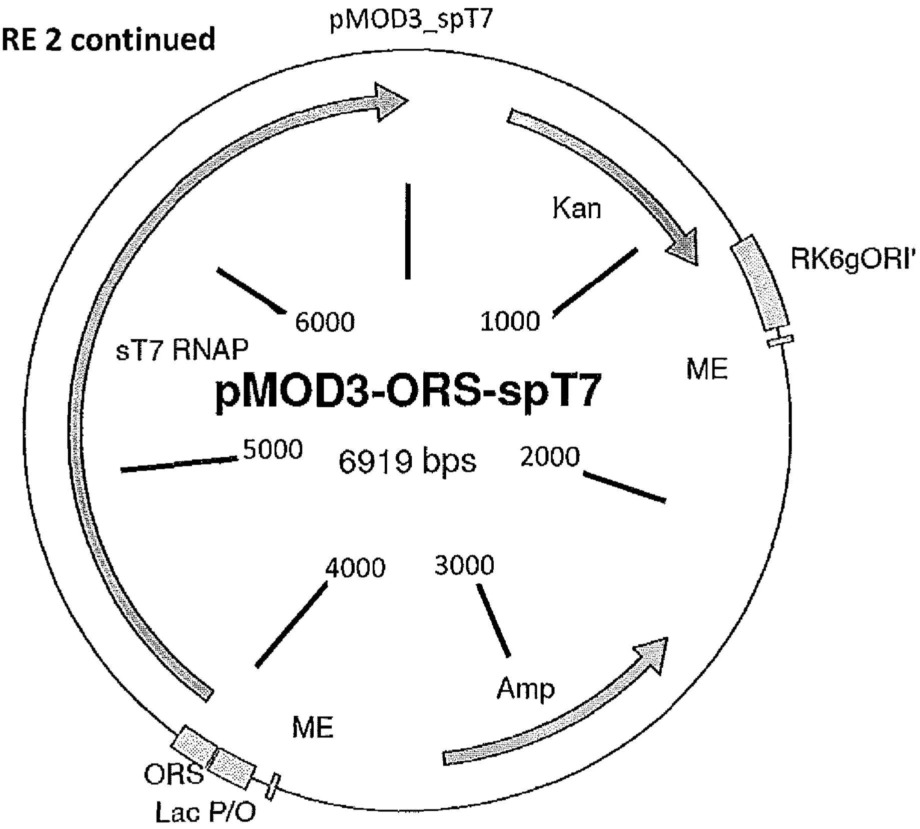


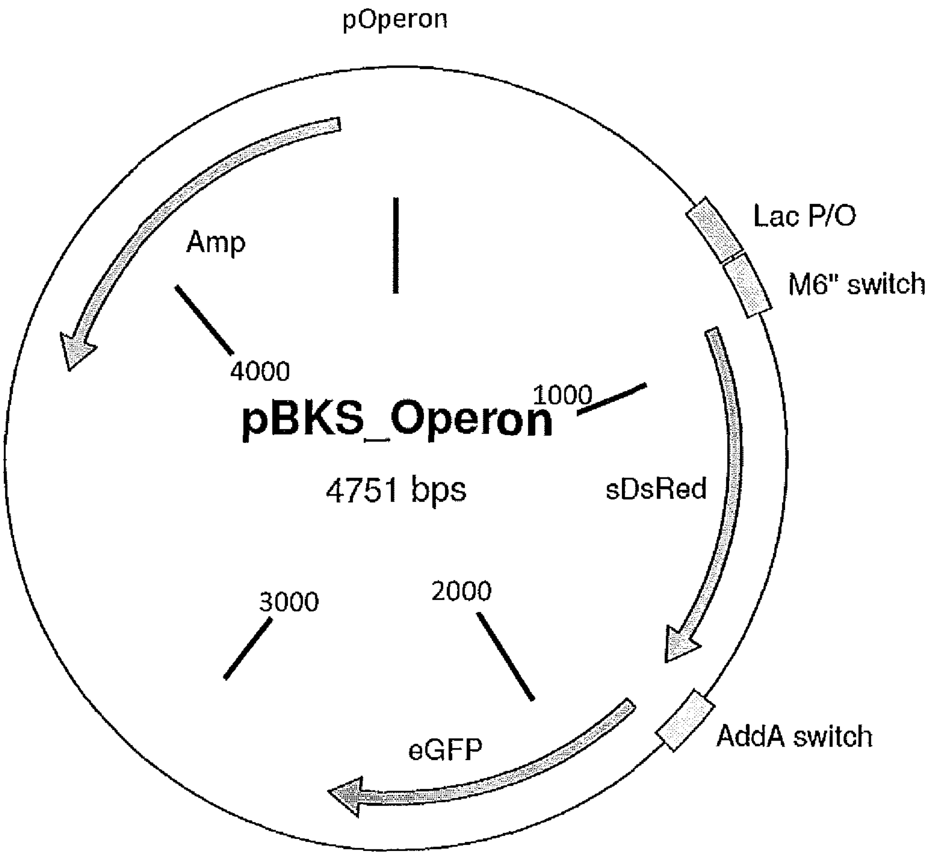

**FIGURE 2 continued**

PACY-ORST7

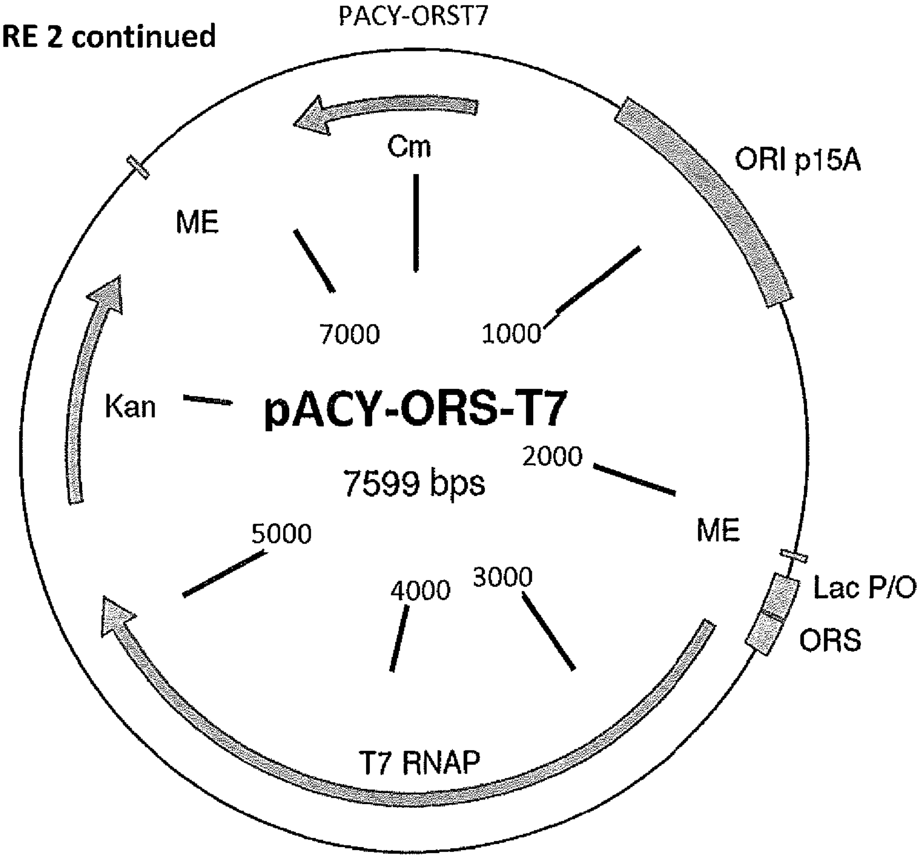


pDual Promoter

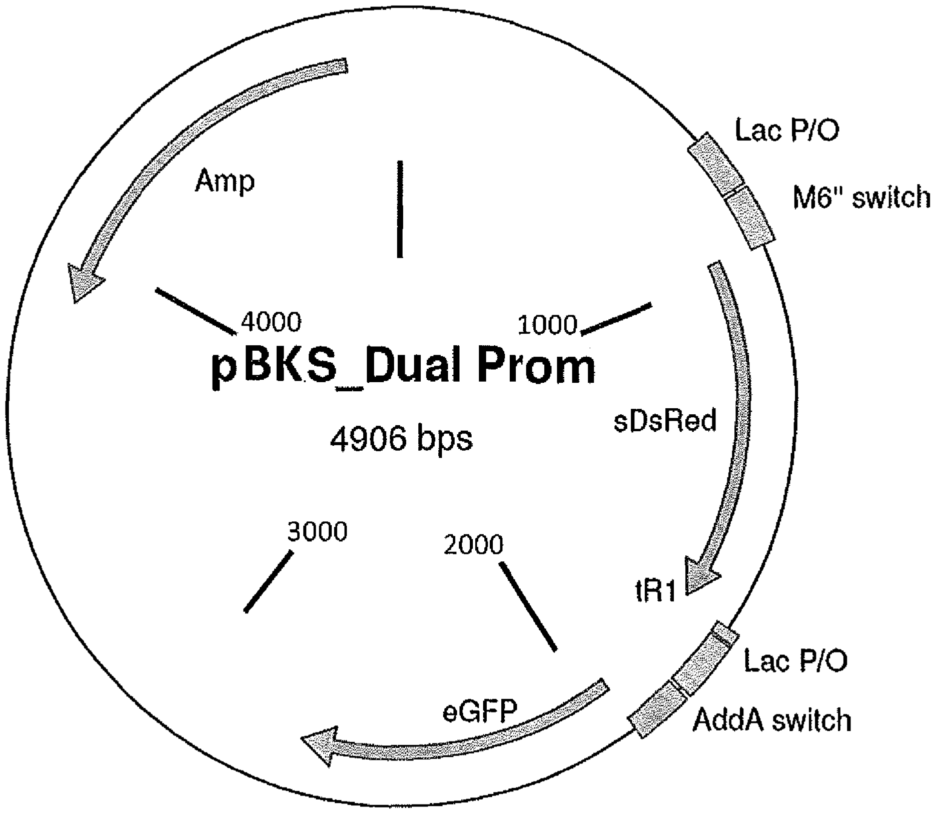

FIGURE 3

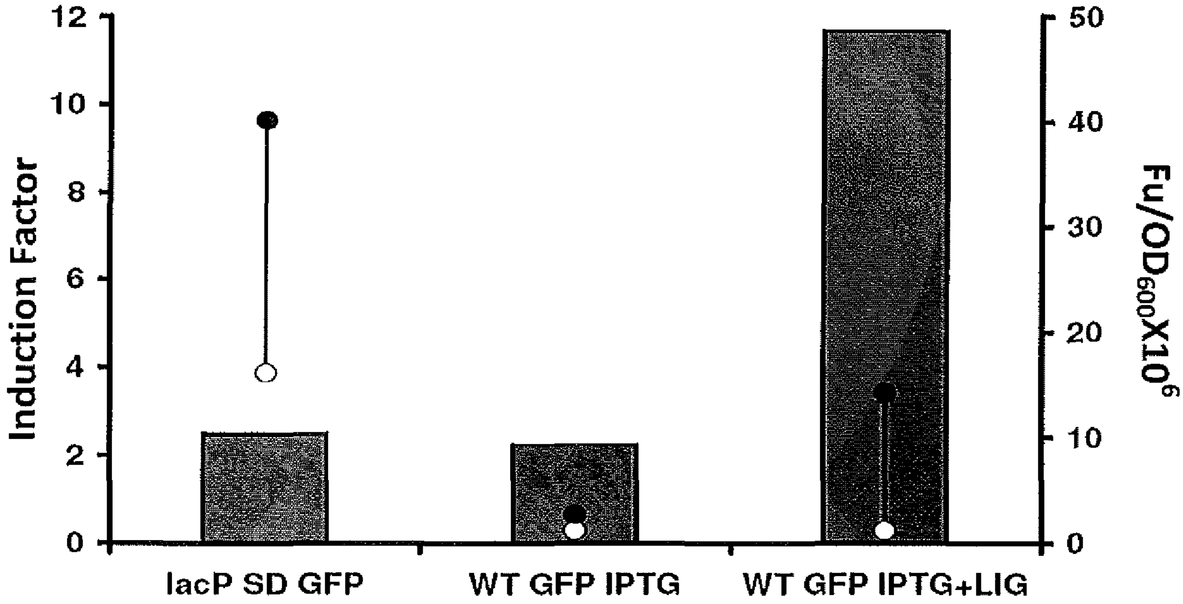


FIGURE 4

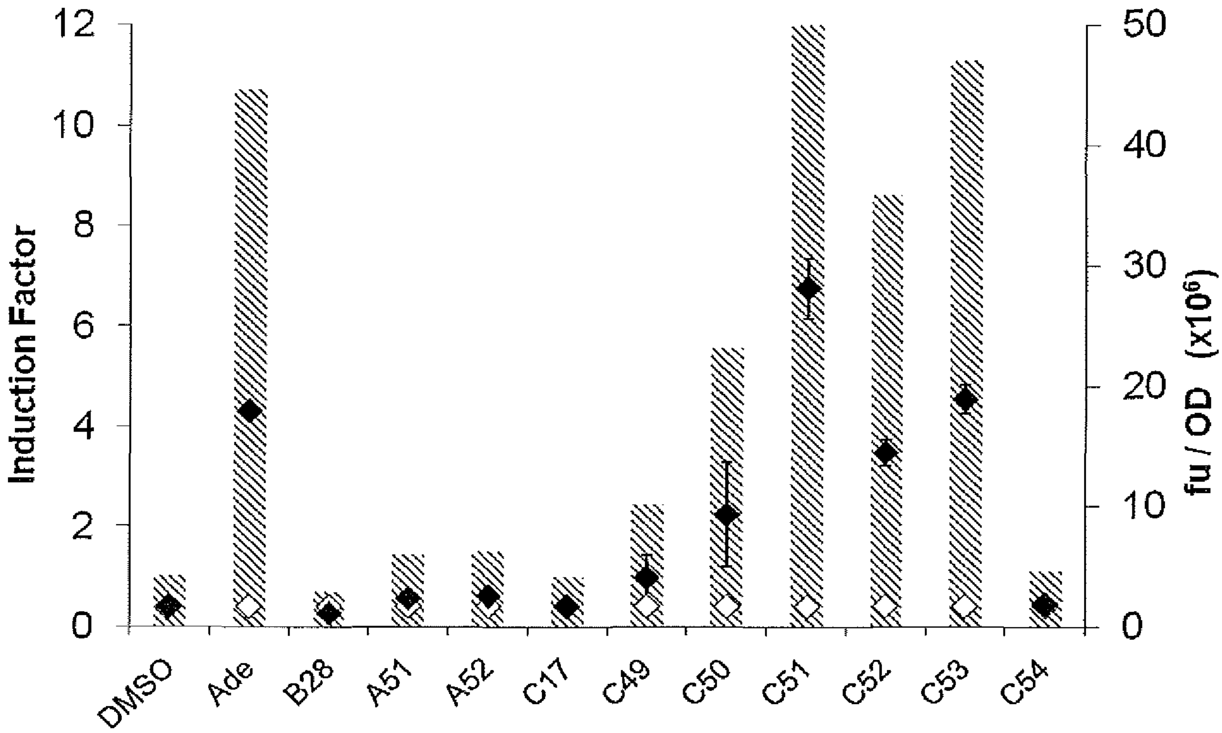

FIGURE 5

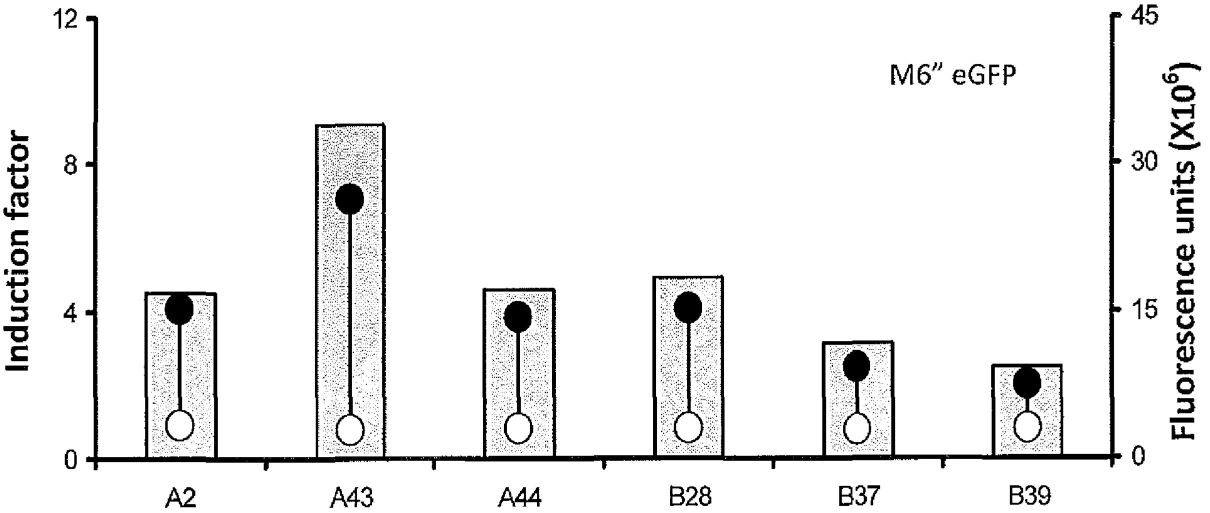


FIGURE 6

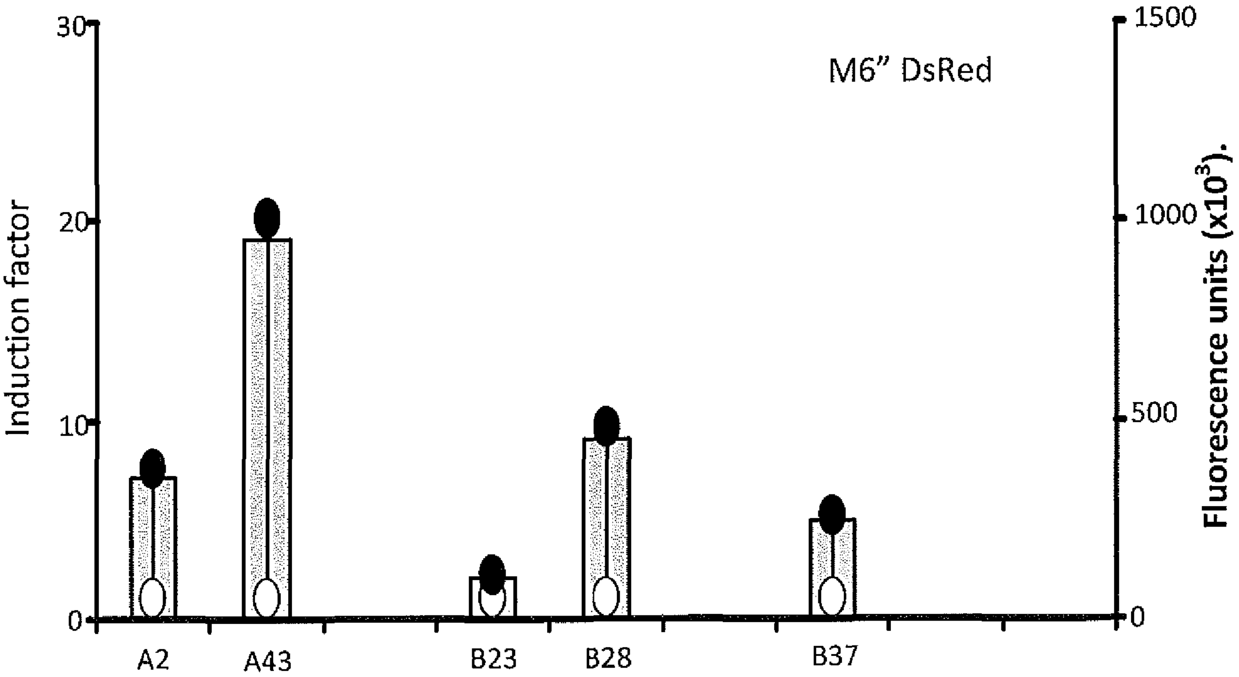

FIGURE 7

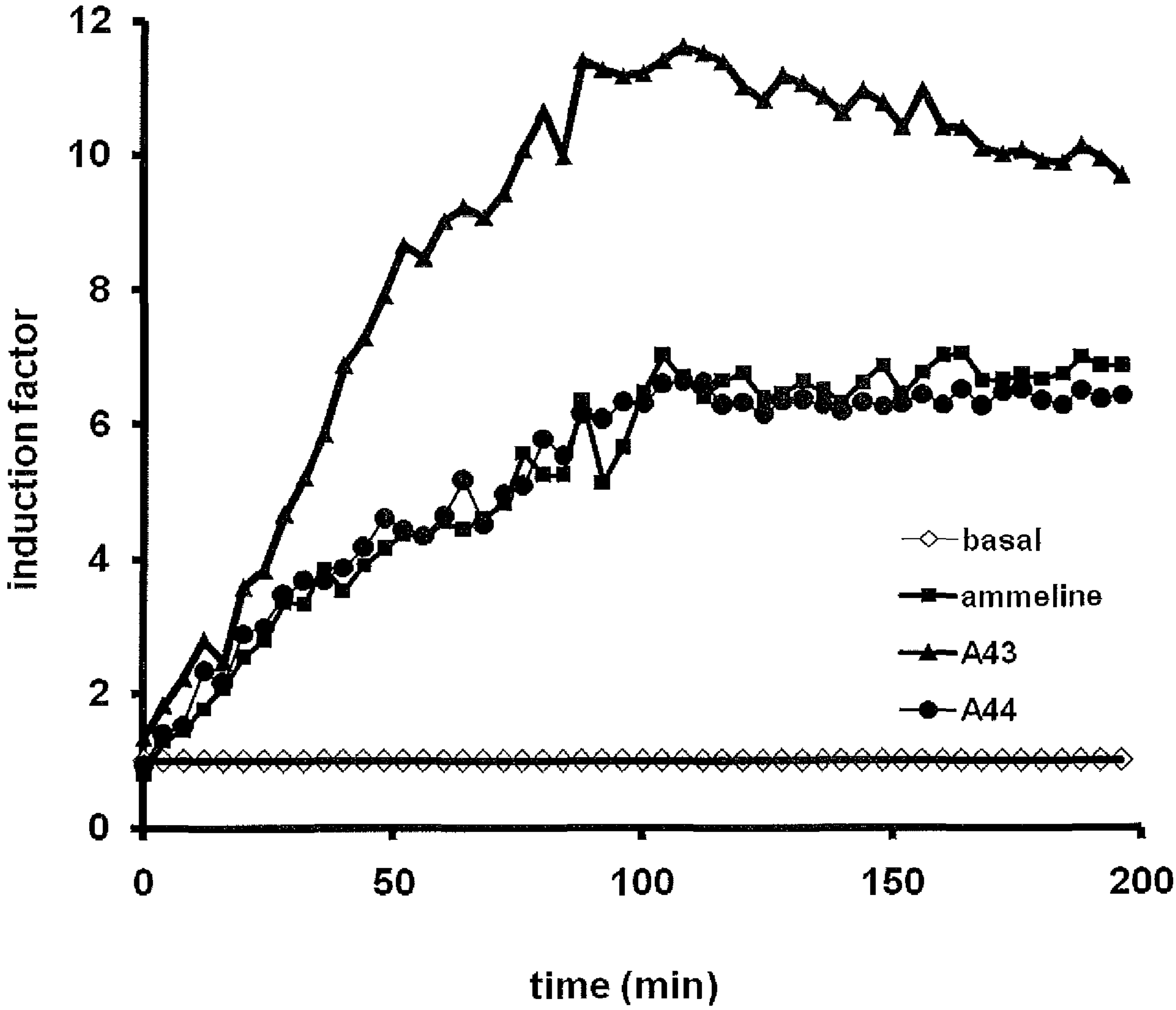
12
10
8
6
4
2
0
induction factor
0
50
100
150
200
time (min)
basal
ammeline
A43
A44

FIGURE 8

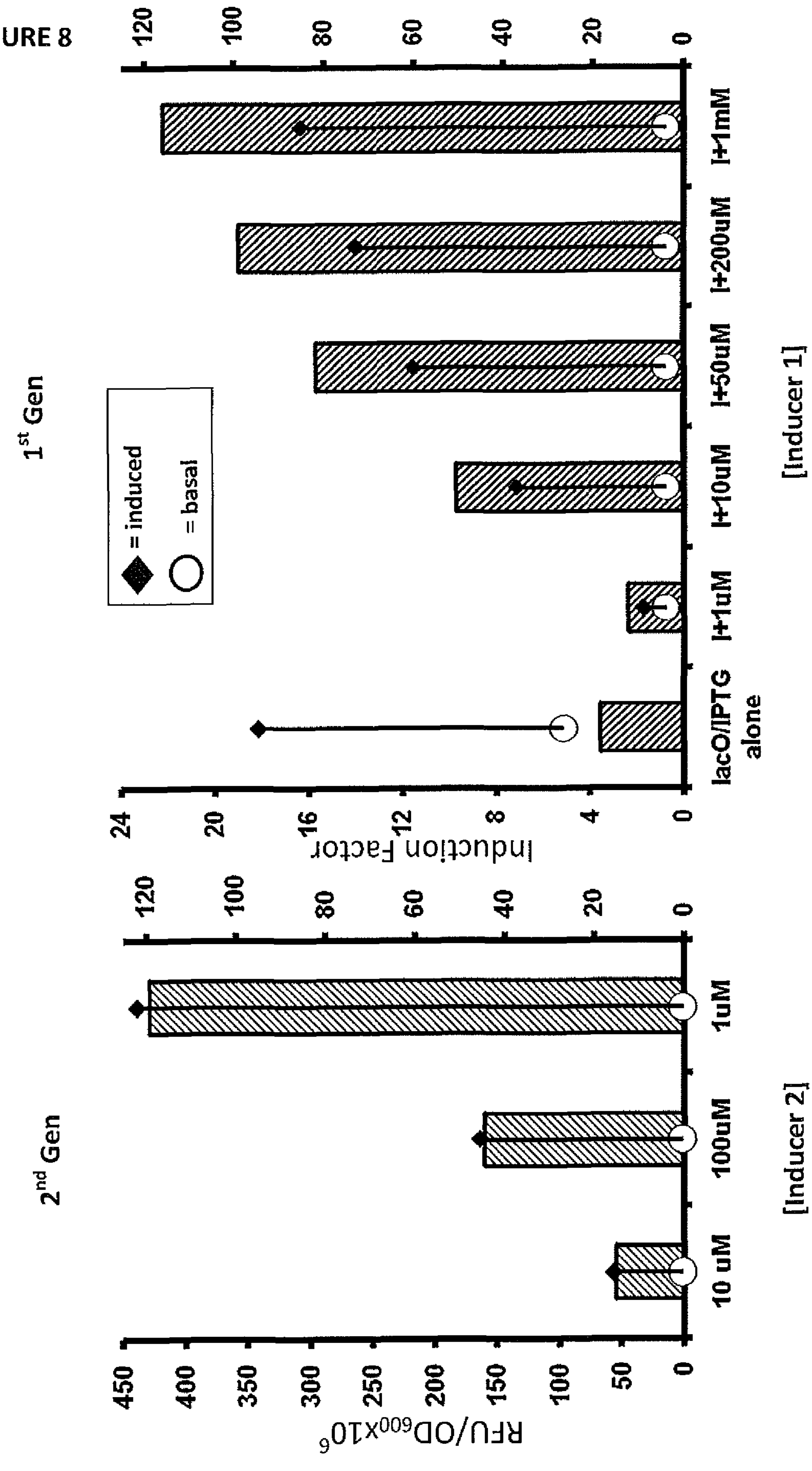
1st Gen
= induced
= basal
Induction Factor
lacO/IPTG alone
I+1uM
I+10uM
I+50uM
I+200uM
I+1mM
[Inducer 1]
2nd Gen
RFU/OD600x10^6
10 uM
100uM
1uM
[Inducer 2]

**FIGURE 9**

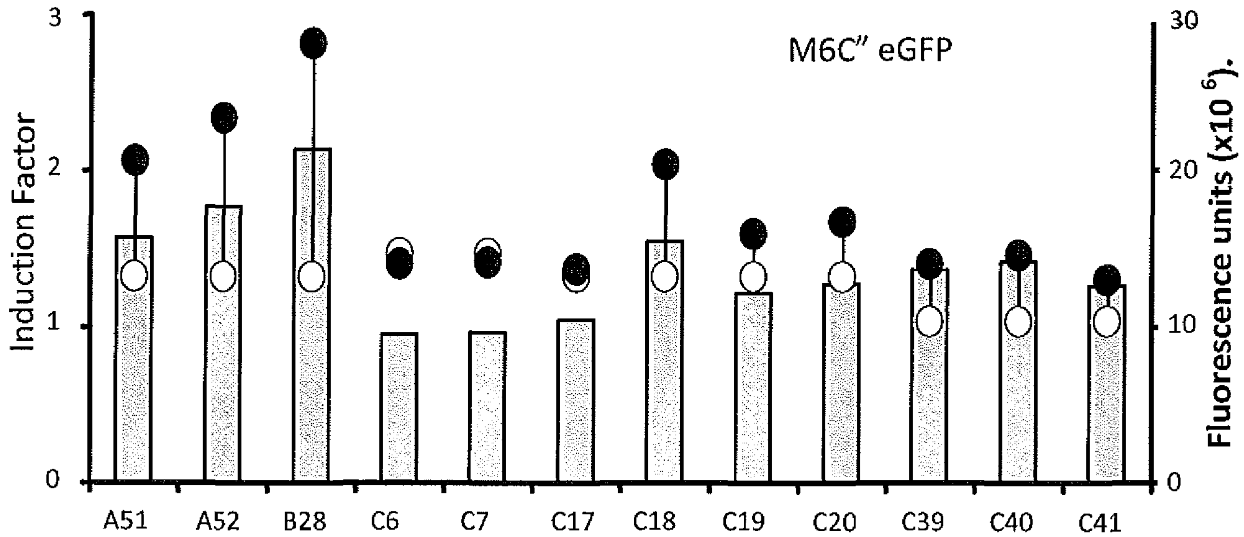


**FIGURE 10**

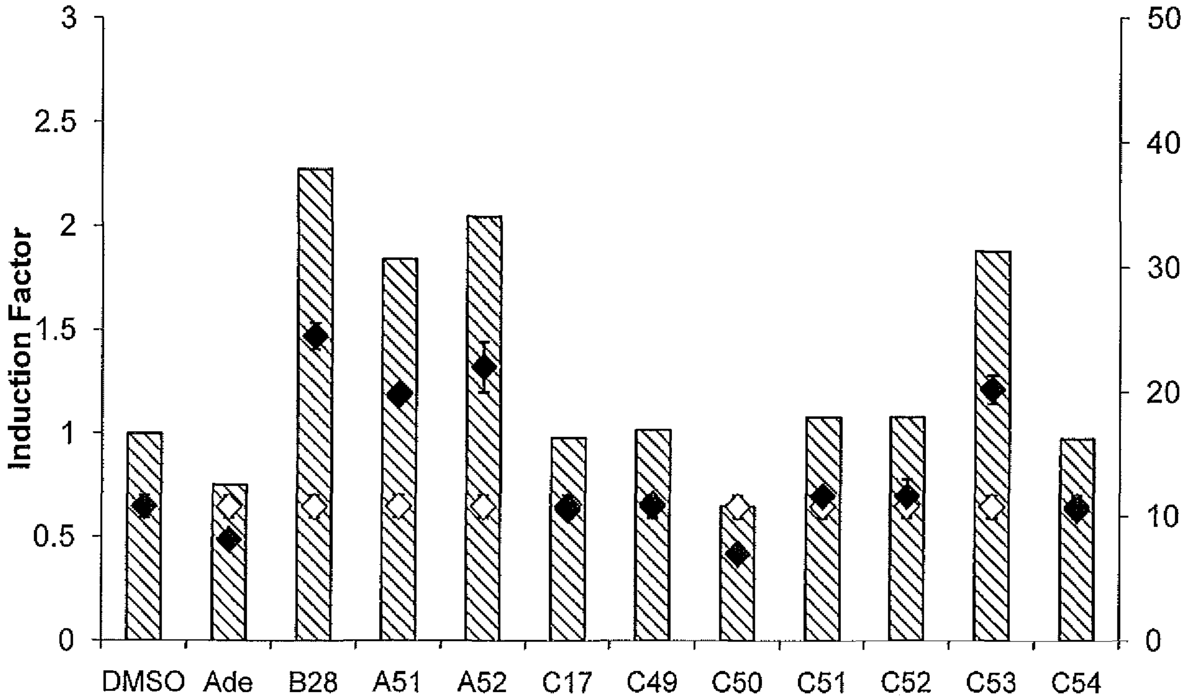

FIGURE 11

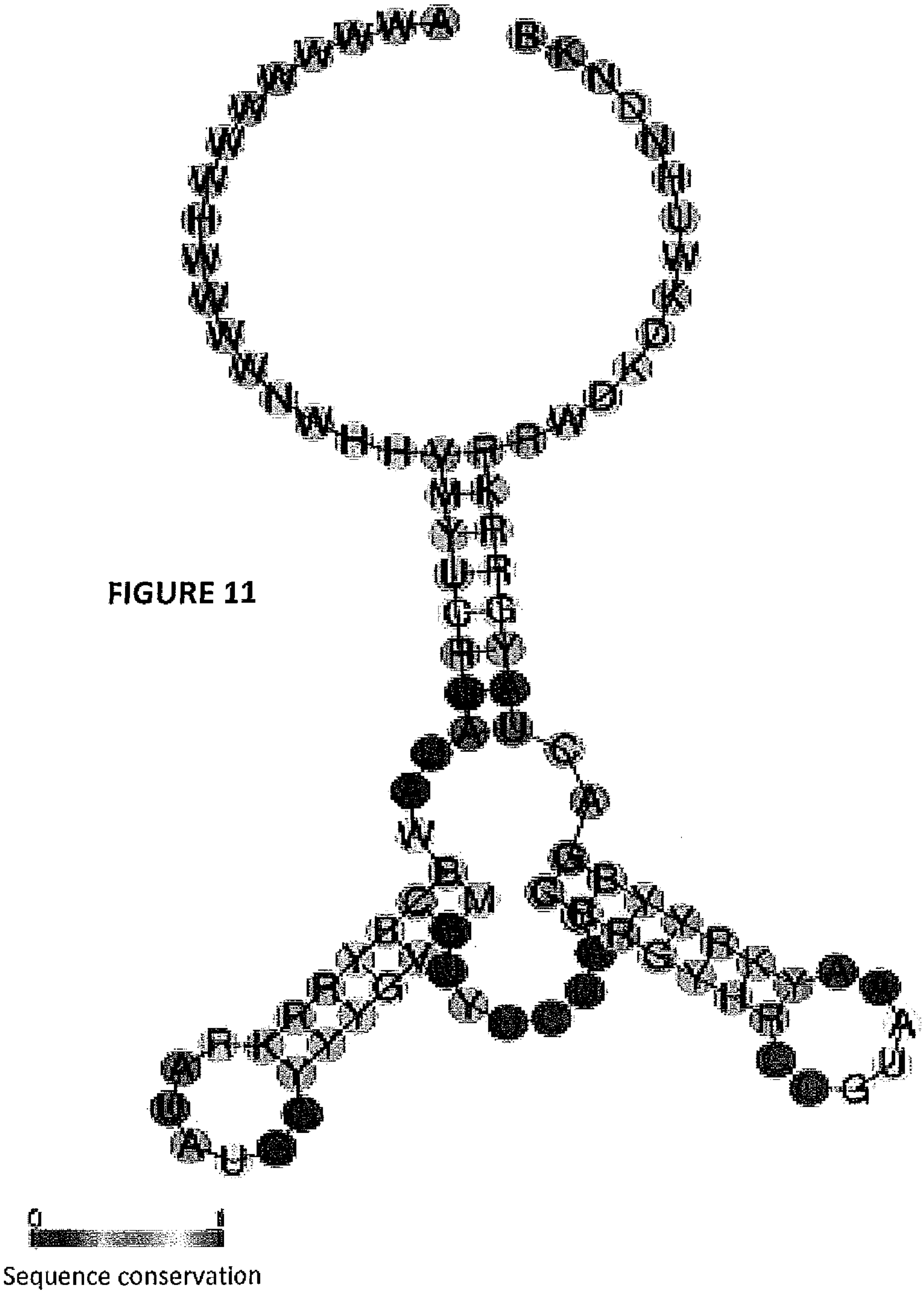

**FIGURE 12** - **Purine aptamer sequences**

>BS2-purE
ACACGACCUCAUAUAAUCUUGGGAAUAUGGCCCAUAAGUUUCUACCCGGCAACCGUAAAUU GCCGGACUAUGCAGGAAAG

>BS3-xpt AGGAACACUCAUAUAAUCGCGUGGAUAUGGCACGCAAGUUUCUACCGGGCA-CCGUAAA-UGUCCGACUAUGGGUGAGCA

>BS5-ydhL AUUAUCACUUGUAUAACCUCAAUAAUAUGGUUUGAGGGUGUCUACCAGGAA-CCGUAAA-AUCCUGAUUACAAAAUUUUG

>CP4-add AUUUUGCUUCGUAUAACUCUAAUGAUAUGGAUUAGAGGUCUCUACCAAGAA-CCGAGAA-UUCUUGAUUACGAAGAAAGC

>VV1-add UCAACGCUUCAUAUAAUCCUAAUGAUAUGGUUUGGGAGUUUCUACCAAGAG-CCUUAAA-CUCUUGAUUAUGAAGUCUGU

**FIGURE 13**

>AddA
CTGGATCCTC AACGCTTCAT ATAATCCTAA TGATATGGTT TGGGAGTTTC TACCAAGAGC
CTTAAACTCT TGATTATGAA GTCTGTCGCT TTATCCGAAA TTTTATAAAG AGAAGACTAT G

>M6
CTGGATCCTC AACGCTTCAT ATAATCCTAA TGATATGGTT TGGGAGCTTC CACCAAGAGC
CTTAAACTCT TGATTATGAA GTCTGTCGCT TTATCCGAAA TTTTATAAAG AGAAGACTAT G

>M6'
CTGGATCCTC AACGCTTCAT ATAATCCTAA TGATATGGTT TAGGAGCTTC CACCAAGAGC
CTTAAACTCT TGATTATGAA GTCTGTCGCT TTATCCGAAA TTTTATAAAG AGAAGACTAT G

>M6"
CTGGATCCTC AACGCTTCAT ATAATCCGAA TGATATGGTT TCGGAGCTTC CACCAAGAGC
CTTAAACTCT TGATTATGAA GTCTGTCGCT TTATCCGAAA TTTTATAAAG AGAAGACTAT G

>M6C
CTGGATCCTC AACGCTTCAT ATAATCCTAA TGATATGGTT TGGGAGCTTC CACCAAGAGC
CTTAAACTCT TGACTATGAA GTCTGTCGCT TTATCCGAAA TTTTATAAAG AGAAGACTAT G

>M6C'
CTGGATCCTC AACGCTTCAT ATAATCCTAA TGATATGGTT TAGGAGCTTC CACCAAGAGC
CTTAAACTCT TGACTATGAA GTCTGTCGCT TTATCCGAAA TTTTATAAAG AGAAGACTAT G

>M6C"
CTGGATCCTC AACGCTTCAT ATAATCCGAA TGATATGGTT TCGGAGCTTC CACCAAGAGC
CTTAAACTCT TGACTATGAA GTCTGTCGCT TTATCCGAAA TTTTATAAAG AGAAGACTAT G

FIGURE 14

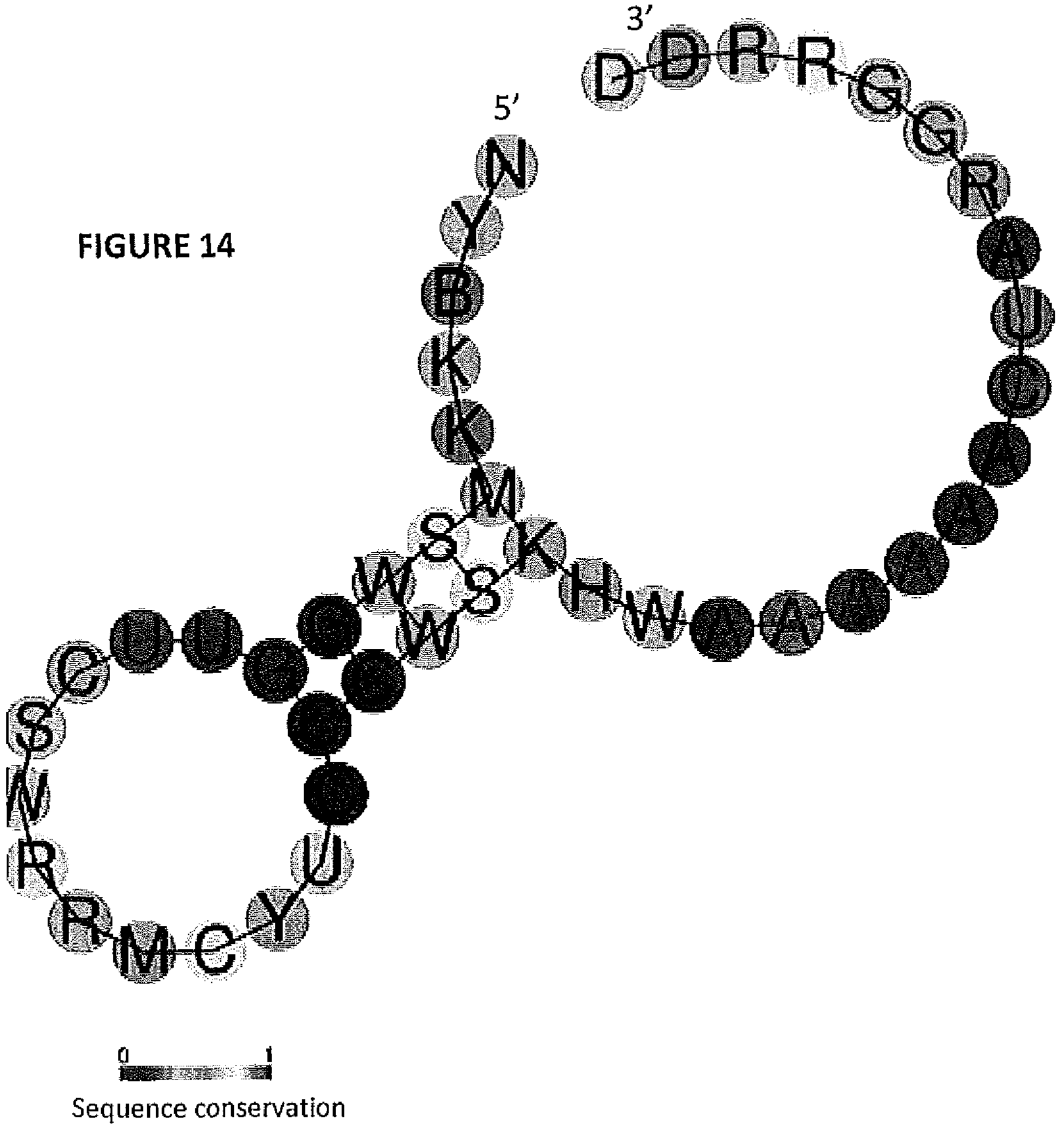

**FIGURE 15**

1 ATAATGAAAC GAACCGTCAC TATAGAGCGA TAATTGATCT AGGAACCGGG
51 GACTGACTTT TTTATTGTCA AGAAAAACAT CATTATGGTA AGATAGCAGA
101 AGTGAAAAAA TTGAAGAAAA TCCGTGCGAT ATGCGGG**AGA GGTTCTAGCT**
151 **ACACCCTCTA TAAAAAACTA AGGACGAGCT GTATCCTTGG ATACGGC**CTT
201 TTTTATGTTT TTCTAGAGCA CCTTCCGAAA AAAGGTGTTT TTTTGCGTGA
251 ATTAGCTGTA GCGATGTCTC TCGCCGGCGT TTTTATTGCG GAGAAGGAGA
301 GGAATCATGA AAAAAGAAAA AGCAATTGTC

## FIGURE 16

1 AGAGRTTCTA GCTACAYYCT CTATAAAARR CTAA

Specifically:

>WT

1 AGAGGTTCTA GCTACACCCT CTATAAAAAA CTAA

>C17U

1 AGAGGTTCTA GCTACATCCT CTATAAAAAA CTAA

>C18U

1 AGAGGTTCTA GCTACACTCT CTATAAAAAA CTAA

>G5A

1 AGAGATTCTA GCTACACCCT CTATAAAAAA CTAA

>U6C

1 AGAGGCTCTA GCTACACCCT CTATAAAAAA CTAA

>C17U, C18U

1 AGAGGTTCTA GCTACATTCT CTATAAAAAA CTAA

>G5A, C18U

1 AGAGATTCTA GCTACACTCT CTATAAAAAA CTAA

>U6C, C17U

1 AGAGGCTCTA GCTACATCCT CTATAAAAAA CTAA

>G5A, C17U, C18U

1 AGAGATTCTA GCTACATTCT CTATAAAAAA CTAA

>U6C, C17U,A29G

1 AGAGGCTCTA GCTACATCCT CTATAAAAGA CTAA

>G5A,U6C, C17U, C18U

1 AGAGACTCTA GCTACATTCT CTATAAAAAA CTAA

## FIGURE 17

AGGAGGAATTAACCATGCAGTGGTGGTGGTGGTGGTGCCATGGAACTTCCCCGGAATAATGT
CATAGTTTCCTTCCTCCAAGGCGGTTAAATACCTTTTTTGAATCTTTGTTGCCGCTTGGAGATC
ATCCAATGACATTGCTTTTTCCTCTCTGGCTTCTTTGAGCCGGATTCCTAGTTCAGTCACAACA
AACCTCGAGCACCACCACCACCACCACTGCATGGTTAATTCCTCCTGAATAACAAGTATTGTT
GGCAGCAGCTCCAGCGCCTTAGATGCTGGCTTTGGCATTTCCTTTTGAAGATTCATGCCGGA
GATCTTTTCAGACACATCATCATGATACGTATTAGGGATATCTTTTTTATGCTCCTCAAACAGC
TGATCCGCATCAAGCCCAACGGCTTCTGCATATTGCTTGATGAATGCCCGAACATAAAAGTAA
AGAGTTTTGCTTAATCGCAAAGCTCTTTTTATTATTATCTTTTA

## FIGURE 18

ATAATGAAAC GAACCGTCAC TATAGAGCGA TAATTGATCT AGGAACCGGG GACTGACTTT
TTTATTGTCA AGAAAAACAT CATTATGGTA AGATAGCAGA AGTGAAAAAA TTGAAGAAAA
TCCGTGCGAT ATGCGGGAGA GGTTCTAGCT ACACCCTCTA TAAAAAACTA AGGACGAGCT
GTATCCTTGG ATACGGCCTT TTTTATGTTT TTCTAGAGCA CCTTCCGAAA AAAGGTGTTT
TTTTGCGTGA ATTAGCTGTA GCGATGTCTC TCGCCGGCGT TTTTATTGCG GAGAAGGAGA
GGAATCATGA AAAAAGAAAA AGCAATTGTC CCAGCTTGTT GATACACTAA TGCTTTTATA
TAGGGAAAAG GTGGTGAACT ACTATGTTTG GAATTGGTGC TAGAGACCTT GGTATAGATC
TTGGAACTGC GAATACGCTT GTTTTTGTAA AAGGAAAAGG AATTGTTGTG AGAGAGCCGT
CAGTTGTCGC TTTGCAGACG GATACGAAAT CGATTGTCGC TGTCGGAAAT GATGCGAAAA
ATATGATTGG ACGGACACCG GGCAACGTGG TGGCTCTTCG CCCGATGAAA
GACGGCGTTA TCGCTGATTA TGAAACAACG GCGACGATGA TGAAATATTA CATCAATCAG
GCCATAAAAA ATAAAGGCAT GTTTGCCAGA AAACCATATG TAATGGTATG TGTCCCATCA
GGCATTACAG CTGTTGAAGA ACGCGCTGTT ATCGATGCGA CAAGACAGGC
GGGAGCGCGT GACGCGTATC CGATTGAAGA GCCTTTTGCC GCAGCAATCG
GAGCCAATCT GCCAGTTTGG GAACCGACTG GAAGCATGGT TGTTGATATC GGGGGCGGTA
CGACAGAAGT TGCGATTATT TCCCTCGGAG
GCATCGTAAC GTCTCAGTCA ATCCGTGTAG CCGGTGATGA GATGGATGAC GCGATTATCA
ACTACATCAG AAAAACGTAC AATCTGATGA TCGGTGACCG TACGGCTGAA GCGATTAAAA
TGGAAATCGG ATCTGCAGAA GCTCCTGAAG AATCCGACAA CATGGAAATC CGCGGCCGCG
ATTTGCTCAC AGGTTTGCCG AAAACAATTG AAATTACAGG AAAAGAGATT TCTAACGCTC
TACGCGACAC TGTATCTACA ATTGTCGAAG CAGTGAAGAG CACACTCGAA AAAACACCGC
CTGAGCTTGC AGCAGATATC ATGGACAGAG GTATAGTGTT AACCGGCGGC GGAGCGCTTT
TGCGCAATTT GGACAAAGTC ATCAGCGAAG AAACAAAAAT GCCGGTCCTT ATCGCCGAAG
ATCCGCTTGA TTGTGTAGCG ATCGGAACAG GGAAAGCACT GGAGCACATC CATCTTTTCA
AAGGGAAAAC TAGATAA

## FIGURE 19

GFP 105nt

>gfp (dG= -16.0kcal/mol, GC 37.1%)

ATGAATGGCATGCTCATGAGTAAAGGAGAAGAACTTTTCACTGGAGTTGTCCCAATTCT
TGTTGAATTAGATGGTGATGTTAATGGGTACAAATTTTCTGTCAGT

>gfp_90 (–13.5, 40%)

ATGAATGCCATGATCATGAGTAAAGGAGAAGAACTTTTCACTGGAGTTGTCCCAATTCTT
GTTGAATTAGATGGTGATGTTAATGGGCCC

>gfp_75 (–11, 37.3%)

ATGAATGCCATGATCATGAGTAAAGGAGAAGAACTTTTCACTGGAGTTGTCCCAATTCTT
GTTGAATTAGATGGT

>gfp_60 (–9.7, 38.6%)

ATGAATGCCATGATCATGAGTAAAGGAGAAGAACTTTTCACTGGAGTTGTCCCAATTCTT

>gfp_45 (–5.4, 37.8%)

ATGAATGCCATGATCATGAGTAAAGGAGAAGAACTTTTCACTGGA

>gfp_30 (–1.9, 36.7%)

ATGAATGCCATGATCATGAGTAAAGGAGAA

>GFP_codon optimizedvariant (-24.2, 56.2%)

ATGATTGAACAAGATGGATTGCACGCAGGTTCTCCGGCCGCTTGGGTGGAGAGGCTAT
TCGGCTATGACTGGGCACAACAGACAATCGGCTGCTCTGATGCCGCC

>DSred (-37.3, 64.2%)

ATGGCCTCCTCCGAGGACGTCATCAAGGAGTTCATGCGCTTCAAGGTGCGCATGGAGG
GCTCCGTGAACGGCCACGAGTTCGAGATCGAGGGCGAGGGCGAGGGC

>lacZ (-22.3, 53.3%)

ATGGCCATGATTACGGATTCACTGGCCGTCGTTTTACAACGTCGTGACTGGGAAAACCC
TGGCGTTACCCAACTTAATCGCCTTGCAGCACATCCCCCTTTCGCC

>T7 (-19.8, 48.6%)

ATGAACACGATTAACATCGCTAAGAACGACTTCTCTGACATCGAACTGGCTGCTATCCC
GTTCAACACTCTGGCTGACCATTACGGTGAGCGTTTAGCTCGCGAA

>T3 (-17.0, 45.7%)

ATGAACATCATCGAAAACATCGAAAAGAATGACTTCTCAGAAATCGAACTGGCTGCTAT
CCCGTTCAACACACTGGCTGACCACTACGGAAGCGCCTTGGCTAAA

>SP6 (-22.0, 44.8%)

ATGCAAGATTTACACGCTATCCAGCTTCAATTAGAAGAAGAGATGTTTAATGGTGGCATT
CGTCGCTTCGAAGCAGATCAACAACGCCAGATTGCAGCAGGTAGC

**FIGURE 20**

>Tandem sequence

ATGAATGCCATGATCATGAGTAAAGGAGAAGAACTTTTCACTGGAGTTGTCCCAATTCTT
GTTGAATTAGATGGTGATGTTAATGGGTACAAATTTTCTGTCAGG

**FIGURE 21**

>5'GFP-plus-3'-DsRED

ATGAATGGCATGCTCATGAGTAAAGGAGAAGAACTTTTCACTGGAGTTGTCCCAATTCTTGTTGA
ATTAGATGGTGATGTTAATGGGTACAAATTTTCTGTCAGTCTCCAAGCTGGACATCACCTCCCAC
AACGAGGACTACACCATCGTGGAGCAGTACGAGCGCGCCGAGGGCCGCCACCACCTGTTCCTG
TAGGCGGCCGCCTCAAC

**FIGURE 22**

>5'GFP-rhoTerm-3'-GFP

ATGAATGGCATGCTCATGAGTAAAGGAGAAGAACTTTTCACTGGAGTTGTCCCAATTCTTGTTGA
ATTAGATGGTGATGTTAATGGGTACAAATTTTCTGTCAGTATAACCCCGCTCTTACACATTCCACT
CCAAGCTGGACATCACCTCCCACAACGAGGACTACACCATCGTGGAGCAGTACGAGCGCGCCG
AGGGCCGCCACCACCTGTTCCTGTAGGCGGCCGCCTCAAC

**FIGURE 23**

>Operon

AATTGTGAGCGGATAACAATTTCACACAGGAAACAGCTGGATCCTCAACGCTTCATATAATCCGA
ATGATATGGTTTCGGAGCTTCCACCAAGAGCCTTAAACTCTTGATTATGAAGTCTGTCGCTTTATC
CGAAATTTTATAAAGAGAAGACTATGAATGCCATGATCATGAGTAAAGGAGAAGAACTTTTCACTG
GAGTTGTCCCAATTCTTGTTGAATTAGATGGTGATGTTAATGGGCAAAAATTCTCTGTCACCATGG
CCTCCTCCGAGGACGTCATCAAGGAGTTCATGCGCTTCAAGGTGCGCATGGAGGGCTCCGTGA
ACGGCCACGAGTTCGAGATCGAGGGCGAGGGCGAGGGCCGCCCCTACGAGGGCACCCAGACC
GCCAAGCTGAAGGTGACCAAGGGCGGCCCCCTGCCCTTCGCCTGGGACATCCTGTCCCCCCAG
TTCCAGTACGGCTCCAAGGTGTACGTGAAGCACCCCGCCGACATCCCCGACTACAAGAAGCTGT
CCTTCCCCGAGGGCTTCAAGTGGGAGCGCGTGATGAACTTCGAGGACGGCGGCGTGGTGACCG
TGACCCAGGACTCCTCCCTGCAGGACGGCTCCTTCATCTACAAGGTGAAGTTCATCGGCGTGAA
CTTCCCCTCCGACGGCCCCGTAATGCAGAAGAAGACTATGGGCTGGGAGGCCTCCACCGAGCG
CCTGTACCCCCGCGACGGCGTGCTGAAGGGCGAGATCCACAAGGCCCTGAAGCTGAAGGACG
GCGGCCACTACCTGGTGGAGTTCAAGTCCATCTACATGGCCAAGAAGCCCGTGCAGCTGCCCG
GCTACTACTACGTGGACTCCAAGCTGGACATCACCTCCCACAACGAGGACTACACCATCGTGGA
GCAGTACGAGCGCGCCGAGGGCCGCCACCACCTGTTCCTGTAGGCGGCCGCCTCAACGCTTCA

**FIGURE 23 CONTINUED**

TATAATCCTAATGATATGGTTTGGGAGTTTCTACCAAGAGCCTTAAACTCTTGATTATGAAGTCTG
TCGCTTTATCCGAAATTTTATAAAGAGAAGACTATGAATGCCATGATCATGAGTAAAGGAGAAGAA
CTTTTCACTGGAGTTGTCCCAATTCTTGTTGAATTAGATGGTGATGTTAATGGGCAAAAATTCTCT
GTCAGGGGAGAGGGTGAAGGTGATGCAACATACGGAAAACTTACCCTTAAATTTATTTGCACTAC
TGGGAAGCTACCTGTTCCATGGCCAACACTTGTCACTACTTTGACTTATGGTGTACAATGCTTCT
CAAGATACCCAGATCACATGAAACAGCATGACTTTCTCAAGAGTGCCATGCCCGAAGGTTATGTA
CAGGAAAGAACTATATTTTACAAAGATGACGGGAACTACAAGACACGTGCTGAAGTCAAGTTTGA
GGGTGATACCCTTGTTAATAGAATCGAGTTAAAAGGTATTGATTTTAAAGAAGATGGAAACATTCT
TGGACACAAAATGGAATACAACTATAACTCACATAATGTATACATCATGGGAGACAAACCAAAGAA
TGGCATCAAAGTTAACTTCAAAATTAGACACAACATTAAAGATGGAAGCGTTCAATTAGCAGACCA
TTATCAACAAAATACTCCAATTGGCGATGGCCCTGTCCTTTTACCAGACAACCATTACCTGTCCAC
ACAATCTGCCCTTTCCCAAGATCCCCACGGAAAGAGAGATCACATGGTCCTTCTTGAGTTTGTTA
CATCTGCTGGGATTACACATGGCATGGATGAACTATACAAATAG

**FIGURE 24**

>DualPromoter

AATTGTGAGCGGATAACAATTTCACACAGGAAACAGCTGGATCCTCAACGCTTCATATAATCCGA
ATGATATGGTTTCGGAGCTTCCACCAAGAGCCTTAAACTCTTGATTATGAAGTCTGTCGCTTTATC
CGAAATTTTATAAAGAGAAGACTATGAATGCCATGATCATGAGTAAAGGAGAAGAACTTTTCACTG
GAGTTGTCCCAATTCTTGTTGAATTAGATGGTGATGTTAATGGGCAAAAATTCTCTGTCACCATGG
CCTCCTCCGAGGACGTCATCAAGGAGTTCATGCGCTTCAAGGTGCGCATGGAGGGCTCCGTGA
ACGGCCACGAGTTCGAGATCGAGGGCGAGGGCGAGGGCCGCCCCTACGAGGGCACCCAGACC
GCCAAGCTGAAGGTGACCAAGGGCGGCCCCCTGCCCTTCGCCTGGGACATCCTGTCCCCCCAG
TTCCAGTACGGCTCCAAGGTGTACGTGAAGCACCCCGCCGACATCCCCGACTACAAGAAGCTGT
CCTTCCCCGAGGGCTTCAAGTGGGAGCGCGTGATGAACTTCGAGGACGGCGGCGTGGTGACCG
TGACCCAGGACTCCTCCCTGCAGGACGGCTCCTTCATCTACAAGGTGAAGTTCATCGGCGTGAA
CTTCCCCTCCGACGGCCCCGTAATGCAGAAGAAGACTATGGGCTGGGAGGCCTCCACCGAGCG
CCTGTACCCCCGCGACGGCGTGCTGAAGGGCGAGATCCACAAGGCCCTGAAGCTGAAGGACG
GCGGCCACTACCTGGTGGAGTTCAAGTCCATCTACATGGCCAAGAAGCCCGTGCAGCTGCCCG
GCTACTACTACGTGGACTCCAAGCTGGACATCACCTCCCACAACGAGGACTACACCATCGTGGA
GCAGTACGAGCGCGCCGAGGGCCGCCACCACCTGTTCCTGTAGGCGGCCGCCATAACCCCGCT
CTTACACATTCCAGCCCCGCAACGCAATTAATGTGAGTTAGCTCACTCATTAGGCACCCCAGGCT
TTACACTTTATGCTTCCGGCTCGTATGTTGTGTGGAATTGTGAGCGGATAACAATTTCACACAGG
AAACAGCTGGATCCTCAACGCTTCATATAATCCTAATGATATGGTTTGGGAGTTTCTACCAAGAG

**FIGURE 24 CONTINUED**

CCTTAAACTCTTGATTATGAAGTCTGTCGCTTTATCCGAAATTTTATAAAGAGAAGACTATGAATG
CCATGATCATGAGTAAAGGAGAAGAACTTTTCACTGGAGTTGTCCCAATTCTTGTTGAATTAGATG
GTGATGTTAATGGGCAAAAATTCTCTGTCAGGGGAGAGGGTGAAGGTGATGCAACATACGGAAA
ACTTACCCTTAAATTTATTTGCACTACTGGGAAGCTACCTGTTCCATGGCCAACACTTGTCACTAC
TTTGACTTATGGTGTACAATGCTTCTCAAGATACCCAGATCACATGAAACAGCATGACTTTCTCAA
GAGTGCCATGCCCGAAGGTTATGTACAGGAAAGAACTATATTTTACAAAGATGACGGGAACTACA
AGACACGTGCTGAAGTCAAGTTTGAGGGTGATACCCTTGTTAATAGAATCGAGTTAAAAGGTATT
GATTTTAAAGAAGATGGAAACATTCTTGGACACAAAATGGAATACAACTATAACTCACATAATGTA
TACATCATGGGAGACAAACCAAAGAATGGCATCAAAGTTAACTTCAAAATTAGACACAACATTAAA
GATGGAAGCGTTCAATTAGCAGACCATTATCAACAAAATACTCCAATTGGCGATGGCCCTGTCCT
TTTACCAGACAACCATTACCTGTCCACACAATCTGCCCTTTCCCAAGATCCCCACGGAAAGAGAG
ATCACATGGTCCTTCTTGAGTTTGTTACATCTGCTGGGATTACACATGGCATGGATGAACTATACA
AATAG

**FIGURE 25**

>ORS_T7

CATTAGGCACCCCAGGCTTTACACTTTATGCTTCCGGCTCGTATGTTGTGTGGAATTGTGAGCGG
ATAACAATTTCACACAGGAAACAGCTGGATCCTCAACGCTTCATATAATCCGAATGATATGGTTTC
GGAGCTTCCACCAAGAGCCTTAAACTCTTGATTATGAAGTCTGTCGCTTTATCCGAAATTTTATAA
AGAGAAGACTATGAACACGATTAACATCGCTAAGAACGACTTCTCTGACATCGAACTGGCTGCTA
TCCCGTTCAACACTCTGGCTGACCATTACGGTGAGCGTTTAGCTCGCGAACAGTTGGCCCTTGA
GCATGAGTCTTACGAGATGGGTGAAGCACGCTTCCGCAAGATGTTTGAGCGTCAACTTAAAGCT
GGTGAGGTTGCGGATAACGCTGCCGCCAAGCCTCTCATCACTACCCTACTCCCTAAGATGATTG
CACGCATCAACGACTGGTTTGAGGAAGTGAAAGCTAAGCGCGGCAAGCGCCCGACAGCCTTCC
AGTTCCTGCAAGAAATCAAGCCGGAAGCCGTAGCGTACATCACCATTAAGACCACTCTGGCTTG
CCTAACCAGTGCTGACAATACAACCGTTCAGGCTGTAGCAAGCGCAATCGGTCGGGCCATTGAG
GACGAGGCTCGCTTCGGTCGTATCCGTGACCTTGAAGCTAAGCACTTCAAGAAAAACGTTGAGG
AACAACTCAACAAGCGCGTAGGGCACGTCTACAAGAAAGCATTTATGCAAGTTGTCGAGGCTGA
CATGCTCTCTAAGGGTCTACTCGGTGGCGAGGCGTGGTCTTCGTGGCATAAGGAAGACTCTATT
CATGTAGGAGTACGCTGCATCGAGATGCTCATTGAGTCAACCGGAATGGTTAGCTTACACCGCC
AAAATGCTGGCGTAGTAGGTCAAGACTCTGAGACTATCGAACTCGCACCTGAATACGCTGAGGC
TATCGCAACCCGTGCAGGTGCGCTGGCTGGCATCTCTCCGATGTTCCAACCTTGCGTAGTTCCT
CCTAAGCCGTGGACTGGCATTACTGGTGGTGGCTATTGGGCTAACGGTCGTCGTCCTCTGGCGC
TGGTGCGTACTCACAGTAAGAAAGCACTGATGCGCTACGAAGACGTTTACATGCCTGAGGTGTA
CAAAGCGATTAACATTGCGCAAAACACCGCATGGAAAATCAACAAGAAAGTCCTAGCGGTCGCC
AACGTAATCACCAAGTGGAAGCATTGTCCGGTCGAGGACATCCCTGCGATTGAGCGTGAAGAAC

**FIGURE 25 CONTINUED**

TCCCGATGAACCGGAAGACATCGACATGAATCCTGAGGCTCTCACCGCGTGGAAACGTGCTGCC
GCTGCTGTGTACCGCAAGGACAAGGCTCGCAAGTCTCGCCGTATCAGCCTTGAGTTCATGCTTG
AGCAAGCCAATAAGTTTGCTAACCATAAGGCCATCTGGTTCCCTTACAACATGGACTGGCGCGGT
CGTGTTTACGCTGTGTCAATGTTCAACCCGCAAGGTAACGATATGACCAAAGGACTGCTTACGCT
GGCGAAAGGTAAACCAATCGGTAAGGAAGGTTACTACTGGCTGAAAATCCACGGTGCAAACTGT
GCGGGTGTCGATAAGGTTCCGTTCCCTGAGCGCATCAAGTTCATTGAGGAAAACCACGAGAACA
TCATGGCTTGCGCTAAGTCTCCACTGGAGAACACTTGGTGGGCTGAGCAAGATTCTCCGTTCTG
CTTCCTTGCGTTCTGCTTTGAGTACGCTGGGGTACAGCACCACGGCCTGAGCTATAACTGCTCC
CTTCCGCTGGCGTTTGACGGGTCTTGCTCTGGCATCCAGCACTTCTCCGCGATGCTCCGAGATG
AGGTAGGTGGTCGCGCGGTTAACTTGCTTCCTAGTGAAACCGTTCAGGACATCTACGGGATTGT
TGCTAAGAAAGTCAACGAGATTCTACAAGCAGACGCAATCAATGGGACCGATAACGAAGTAGTTA
CCGTGACCGATGAGAACACTGGTGAAATCTCTGAGAAAGTCAAGCTGGGCACTAAGGCACTGGC
TGGTCAATGGCTGGCTTACGGTGTTACTCGCAGTGTGACTAAGCGTTCAGTCATGACGCTGGCT
TACGGGTCCAAAGAGTTCGGCTTCCGTCAACAAGTGCTGGAAGATACCATTCAGCCAGCTATTG
ATTCCGGCAAGGGTCTGATGTTCACTCAGCCGAATCAGGCTGCTGGATACATGGCTAAGCTGAT
TTGGGAATCTGTGAGCGTGACGGTGGTAGCTGCGGTTGAAGCAATGAACTGGCTTAAGTCTGCT
GCTAAGCTGCTGGCTGCTGAGGTCAAAGATAAGAAGACTGGAGAGATTCTTCGCAAGCGTTGCG
CTGTGCATTGGGTAACTCCTGATGGTTTCCCTGTGTGGCAGGAATACAAGAAGCCTATTCAGACG
CGCTTGAACCTGATGTTCCTCGGTCAGTTCCGCTTACAGCCTACCATTAACACCAACAAAGATAG
CGAGATTGATGCACACAAACAGGAGTCTGGTATCGCTCCTAACTTTGTACACAGCCAAGACGGTA
GCCACCTTCGTAAGACTGTAGTGTGGGCACACGAGAAGTACGGAATCGAATCTTTTGCACTGATT
CACGACTCCTTCGGTACCATTCCGGCTGACGCTGCGAACCTGTTCAAAGCAGTGCGCGAAACTA
TGGTTGACACATATGAGTCTTGTGATGTACTGGCTGATTTCTACGACCAGTTCGCTGACCAGTTG
CACGAGTCTCAATTGGACAAAATGCCAGCACTTCCGGCTAAAGGTAACTTGAACCTCCGTGACAT
CTTAGAGTCGGACTTCGCGTTCGCGTAA

**FIGURE 26**

**>ORS_sT7**

AATTGTGAGCGGATAACAATTTCACACAGGAAACAGCTGGATCCTCAACGCTTCATATAATCCGA
ATGATATGGTTTCGGAGCTTCCACCAAGAGCCTTAAACTCTTGATTATGAAGTCTGTCGCTTTATC
CGAAATTTTATAAAGAGAAGACTATGAATGCCATGATCATGAGTAAAGGAGAAGAACTTTTCACTG
GAGTTGTCCCAATTCTTGTTGAATTAGATGGTGATGTTAATGGGCAAAAATTCTCTGTCACCATGG
CCACGATTAACATCGCTAAGAACGACTTCTCTGACATCGAACTGGCTGCTATCCCGTTCAACACT
CTGGCTGACCATTACGGTGAGCGTTTAGCTCGCGAACAGTTGGCCCTTGAGCATGAGTCTTACG
AGATGGGTGAAGCACGCTTCCGCAAGATGTTTGAGCGTCAACTTAAAGCTGGTGAGGTTGCGGA
TAACGCTGCCGCCAAGCCTCTCATCACTACCCTACTCCCTAAGATGATTGCACGCATCAACGACT

**FIGURE 26 CONTINUED**

GGTTTGAGGAAGTGAAAGCTAAGCGCGGCAAGCGCCCGACAGCCTTCCAGTTCCTGCAAGAAAT
CAAGCCGGAAGCCGTAGCGTACATCACCATTAAGACCACTCTGGCTTGCCTAACCAGTGCTGAC
AATACAACCGTTCAGGCTGTAGCAAGCGCAATCGGTCGGGCCATTGAGGACGAGGCTCGCTTC
GGTCGTATCCGTGACCTTGAAGCTAAGCACTTCAAGAAAAACGTTGAGGAACAACTCAACAAGC
GCGTAGGGCACGTCTACAAGAAAGCATTTATGCAAGTTGTCGAGGCTGACATGCTCTCTAAGGG
TCTACTCGGTGGCGAGGCGTGGTCTTCGTGGCATAAGGAAGACTCTATTCATGTAGGAGTACGC
TGCATCGAGATGCTCATTGAGTCAACCGGAATGGTTAGCTTACACCGCCAAAATGCTGGCGTAG
TAGGTCAAGACTCTGAGACTATCGAACTCGCACCTGAATACGCTGAGGCTATCGCAACCCGTGC
AGGTGCGCTGGCTGGCATCTCTCCGATGTTCCAACCTTGCGTAGTTCCTCCTAAGCCGTGGACT
GGCATTACTGGTGGTGGCTATTGGGCTAACGGTCGTCGTCCTCTGGCGCTGGTGCGTACTCACA
GTAAGAAAGCACTGATGCGCTACGAAGACGTTTACATGCCTGAGGTGTACAAAGCGATTAACATT
GCGCAAAACACCGCATGGAAAATCAACAAGAAAGTCCTAGCGGTCGCCAACGTAATCACCAAGT
GGAAGCATTGTCCGGTCGAGGACATCCCTGCGATTGAGCGTGAAGAACTCCCGATGAAACCGG
AAGACATCGACATGAATCCTGAGGCTCTCACCGCGTGGAAACGTGCTGCCGCTGCTGTGTACCG
CAAGGACAAGGCTCGCAAGTCTCGCCGTATCAGCCTTGAGTTCATGCTTGAGCAAGCCAATAAG
TTTGCTAACCATAAGGCCATCTGGTTCCCTTACAACATGGACTGGCGCGGTCGTGTTTACGCTGT
GTCAATGTTCAACCCGCAAGGTAACGATATGACCAAAGGACTGCTTACGCTGGCGAAAGGTAAA
CCAATCGGTAAGGAAGGTTACTACTGGCTGAAAATCCACGGTGCAAACTGTGCGGGTGTCGATA
AGGTTCCGTTCCCTGAGCGCATCAAGTTCATTGAGGAAAACCACGAGAACATCATGGCTTGCGC
TAAGTCTCCACTGGAGAACACTTGGTGGGCTGAGCAAGATTCTCCGTTCTGCTTCCTTGCGTTCT
GCTTTGAGTACGCTGGGGTACAGCACCACGGCCTGAGCTATAACTGCTCCCTTCCGCTGGCGTT
TGACGGGTCTTGCTCTGGCATCCAGCACTTCTCCGCGATGCTCCGAGATGAGGTAGGTGGTCGC
GCGGTTAACTTGCTTCCTAGTGAAACCGTTCAGGACATCTACGGGATTGTTGCTAAGAAAGTCAA
CGAGATTCTACAAGCAGACGCAATCAATGGGACCGATAACGAAGTAGTTACCGTGACCGATGAG
AACACTGGTGAAATCTCTGAGAAAGTCAAGCTGGGCACTAAGGCACTGGCTGGTCAATGGCTGG
CTTACGGTGTTACTCGCAGTGTGACTAAGCGTTCAGTCATGACGCTGGCTTACGGGTCCAAAGA
GTTCGGCTTCCGTCAACAAGTGCTGGAAGATACCATTCAGCCAGCTATTGATTCCGGCAAGGGT
CTGATGTTCACTCAGCCGAATCAGGCTGCTGGATACATGGCTAAGCTGATTTGGGAATCTGTGA
GCGTGACGGTGGTAGCTGCGGTTGAAGCAATGAACTGGCTTAAGTCTGCTGCTAAGCTGCTGGC
TGCTGAGGTCAAAGATAAGAAGACTGGAGAGATTCTTCGCAAGCGTTGCGCTGTGCATTGGGTA
ACTCCTGATGGTTTCCCTGTGTGGCAGGAATACAAGAAGCCTATTCAGACGCGCTTGAACCTGAT
GTTCCTCGGTCAGTTCCGCTTACAGCCTACCATTAACACCAACAAAGATAGCGAGATTGATGCAC
ACAAACAGGAGTCTGGTATCGCTCCTAACTTTGTACACAGCCAAGACGGTAGCCACCTTCGTAAG
ACTGTAGTGTGGGCACACGAGAAGTACGGAATCGAATCTTTTGCACTGATTCACGACTCCTTCGG
TACCATTCCGGCTGACGCTGCGAACCTGTTCAAAGCAGTGCGCGAAACTATGGTTGACACATAT
GAGTCTTGTGATGTACTGGCTGATTTCTACGACCAGTTCGCTGACCAGTTGCACGAGTCTCAATT
GGACAAAATGCCAGCACTTCCGGCTAAAGGTAACTTGAACCTCCGTGACATCTTAGAGTCGGAC
TTCGCGTTCGCGTAAG

FIGURE 27

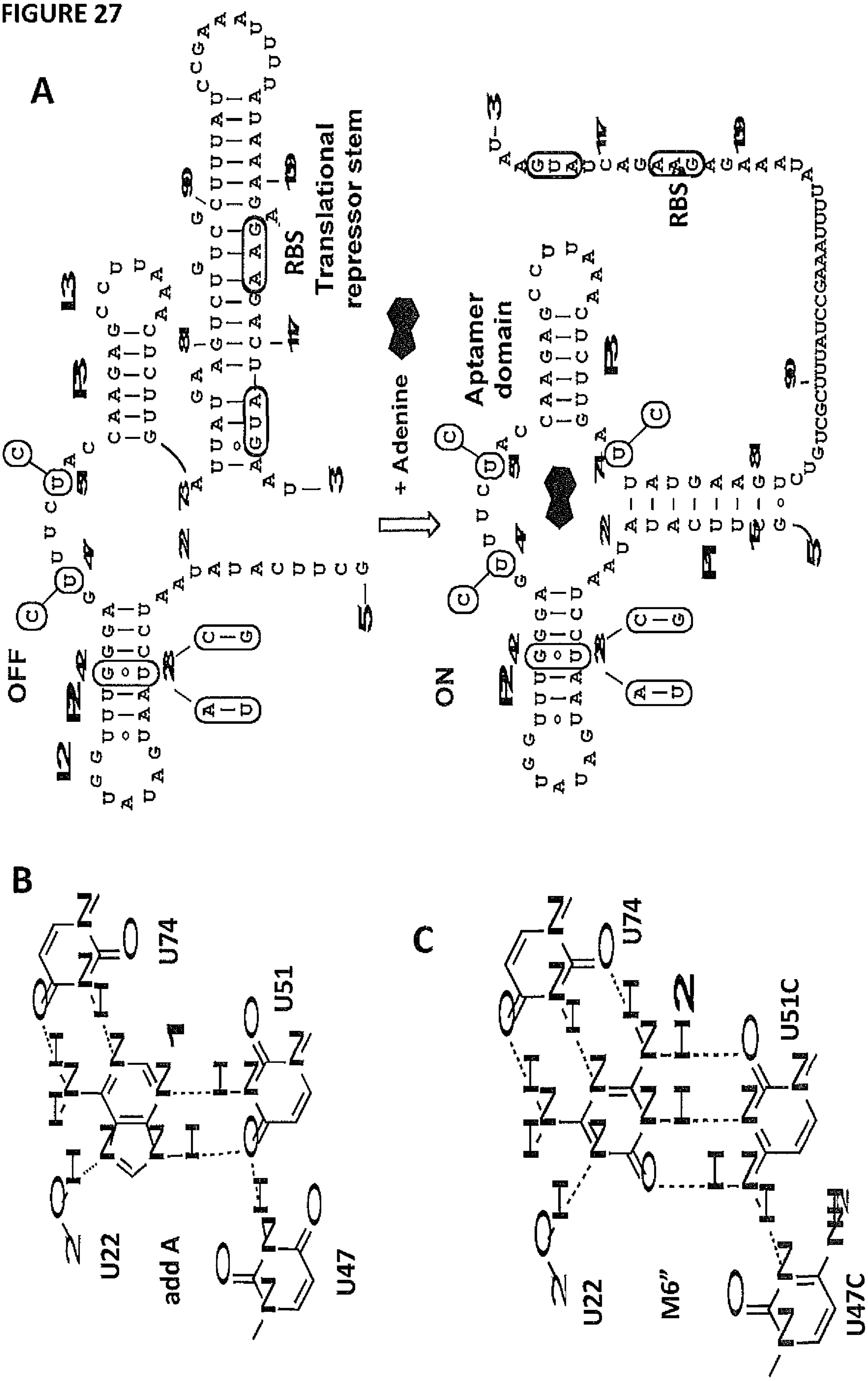
A
OFF
ON
+ Adenine
Aptamer domain
RBS
Translational repressor stem
B
U74
U51
U22
add A
U47
C
U51C
M6"
U47C

FIGURE 27

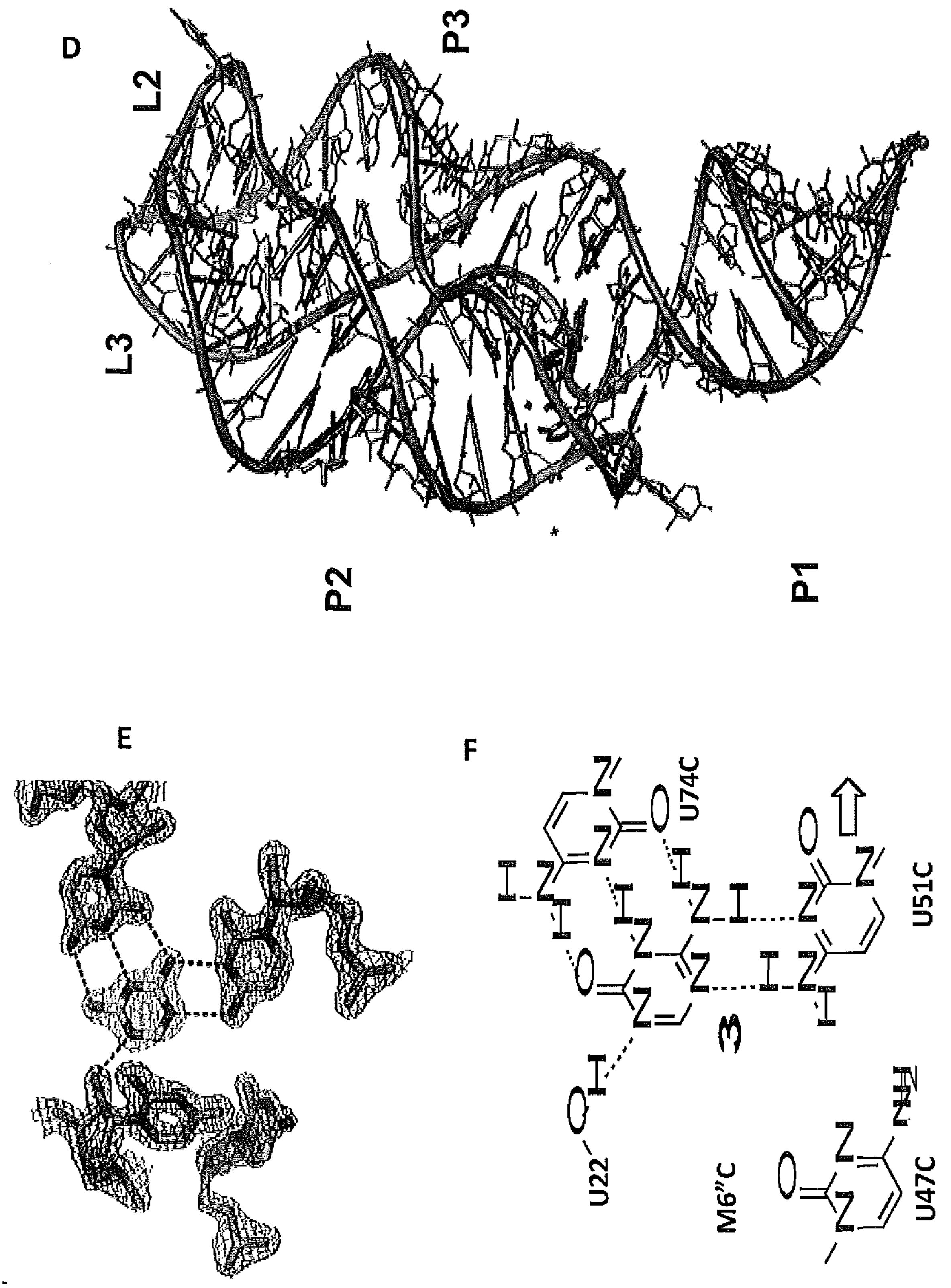

A

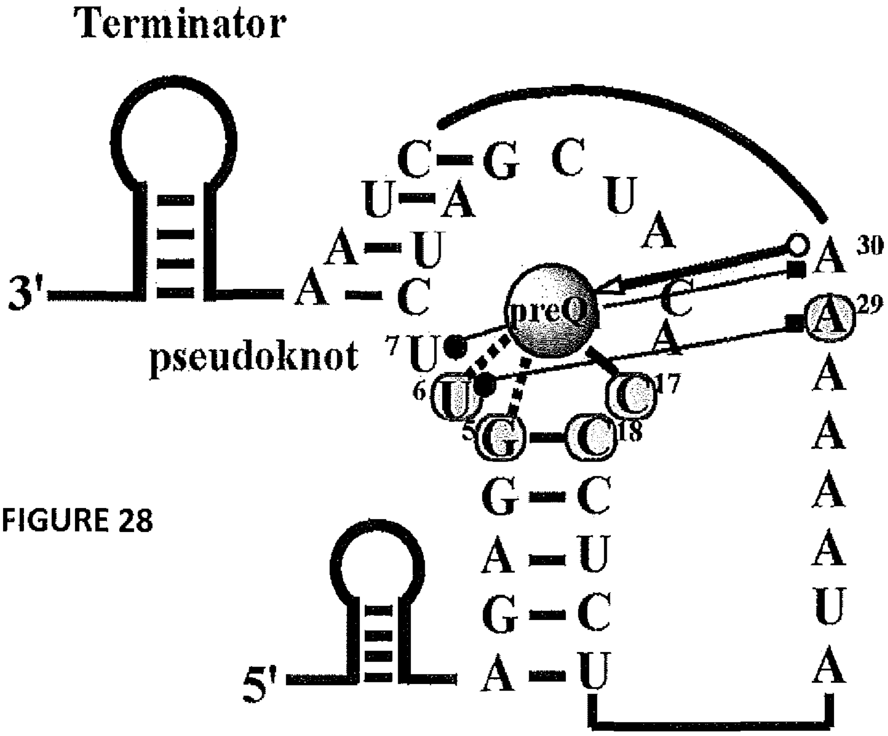
Terminator
3'
5'
pseudoknot
preQ
C G C
U A U
A U A
A C C
7U A
6U C17
5G C18
G C
A U
G C
A U
A30
A29
A
A
A
A
U
A

FIGURE 28

B

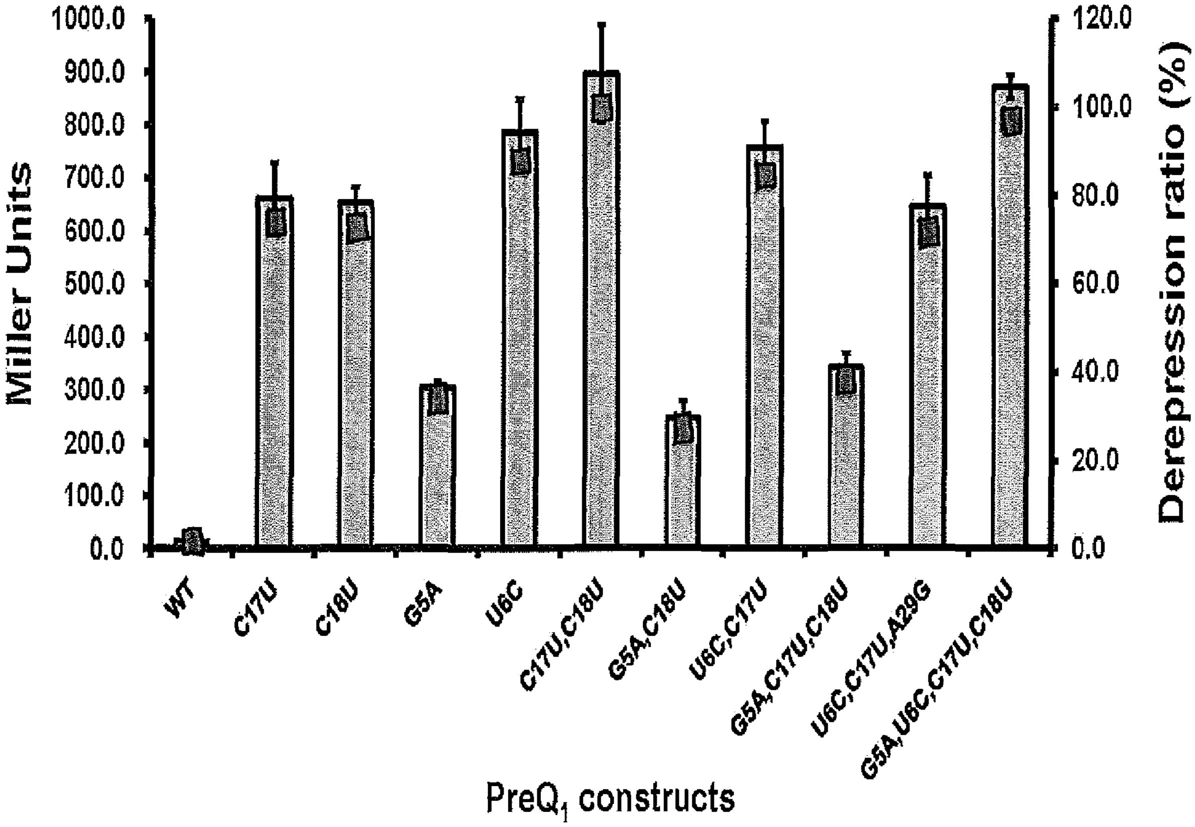
Miller Units
Derepression ratio (%)
1000.0
900.0
800.0
700.0
600.0
500.0
400.0
300.0
200.0
100.0
0.0
120.0
100.0
80.0
60.0
40.0
20.0
0.0
WT
C17U
C18U
G5A
U6C
C17U,C18U
G5A,C18U
U6C,C17U
G5A,C17U,C18U
U6C,C17U,A29G
G5A,U6C,C17U,C18U
$PreQ_1$ constructs

FIGURE 29

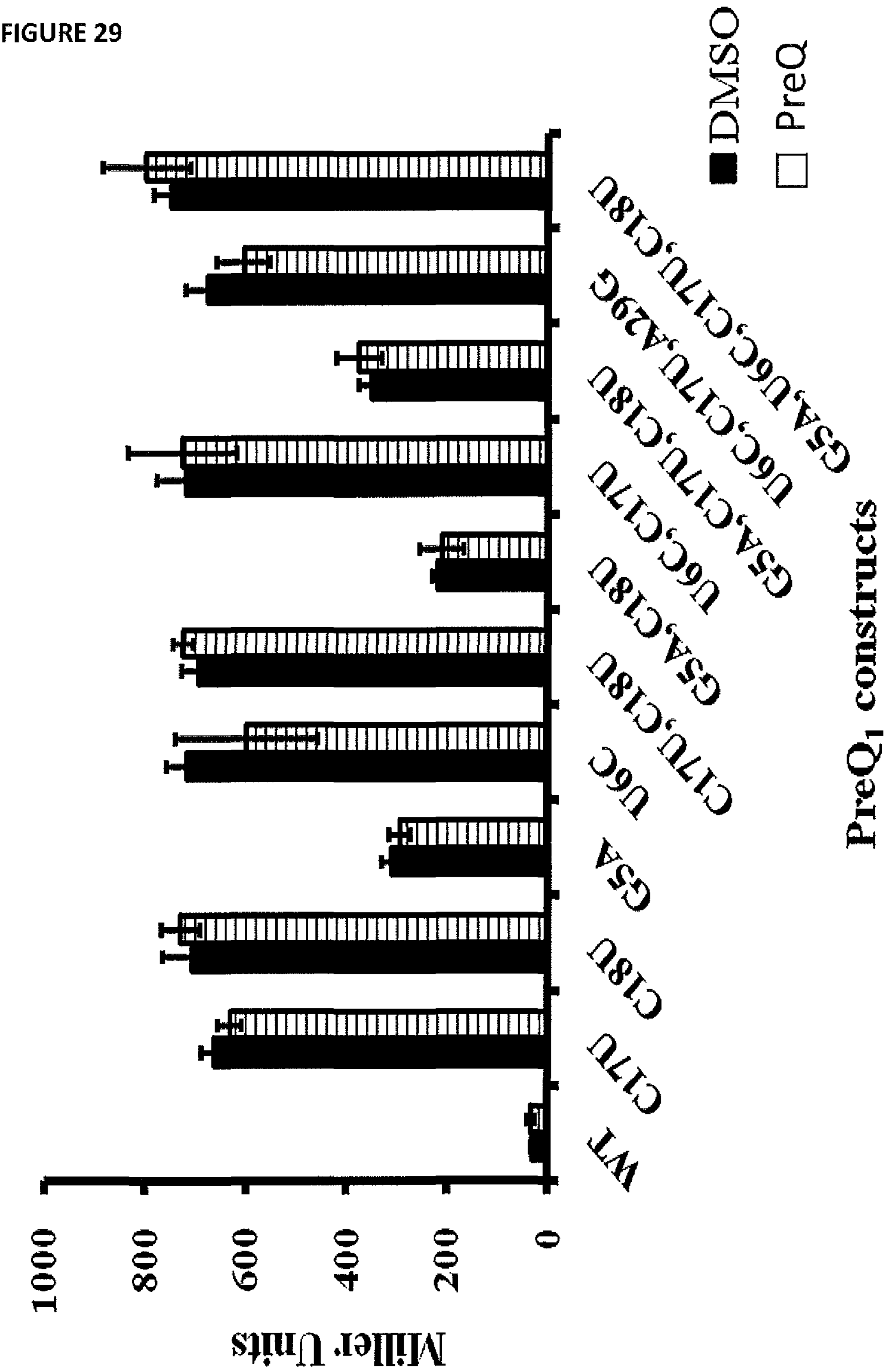
Miller Units
1000
800
600
400
200
0
WT
C17U
C18U
G5A
U6C
C17U,C18U
G5A,C18U
U6C,C17U
U6C,C17U,C18U
G5A,C17U,A29G
G5A,U6C,C17U,C18U
$PreQ_1$ constructs
DMSO
PreQ

FIGURE 30

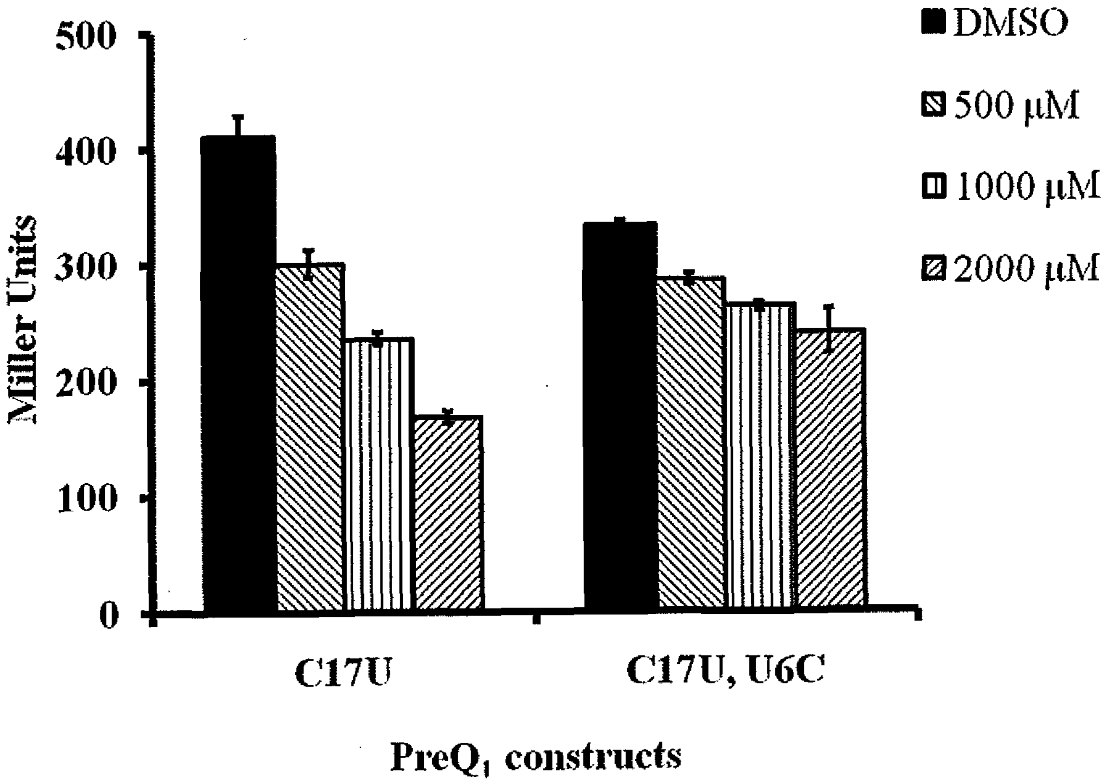


FIGURE 31

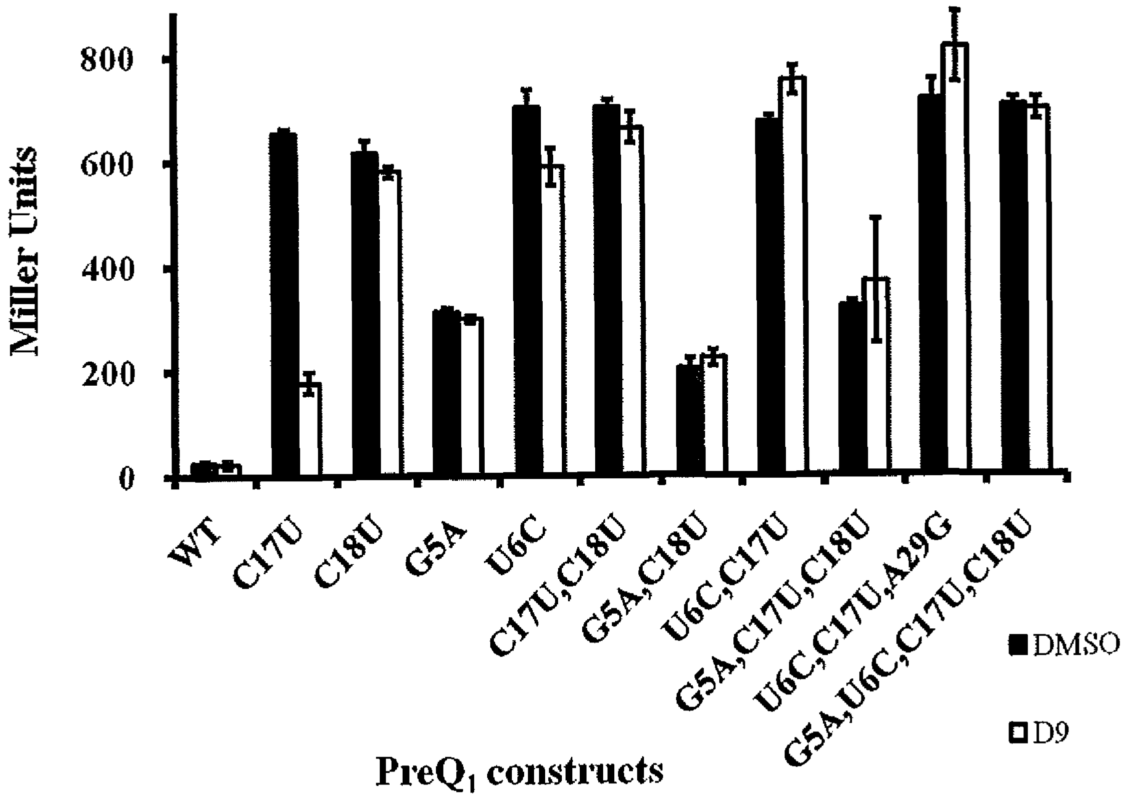

FIGURE 32

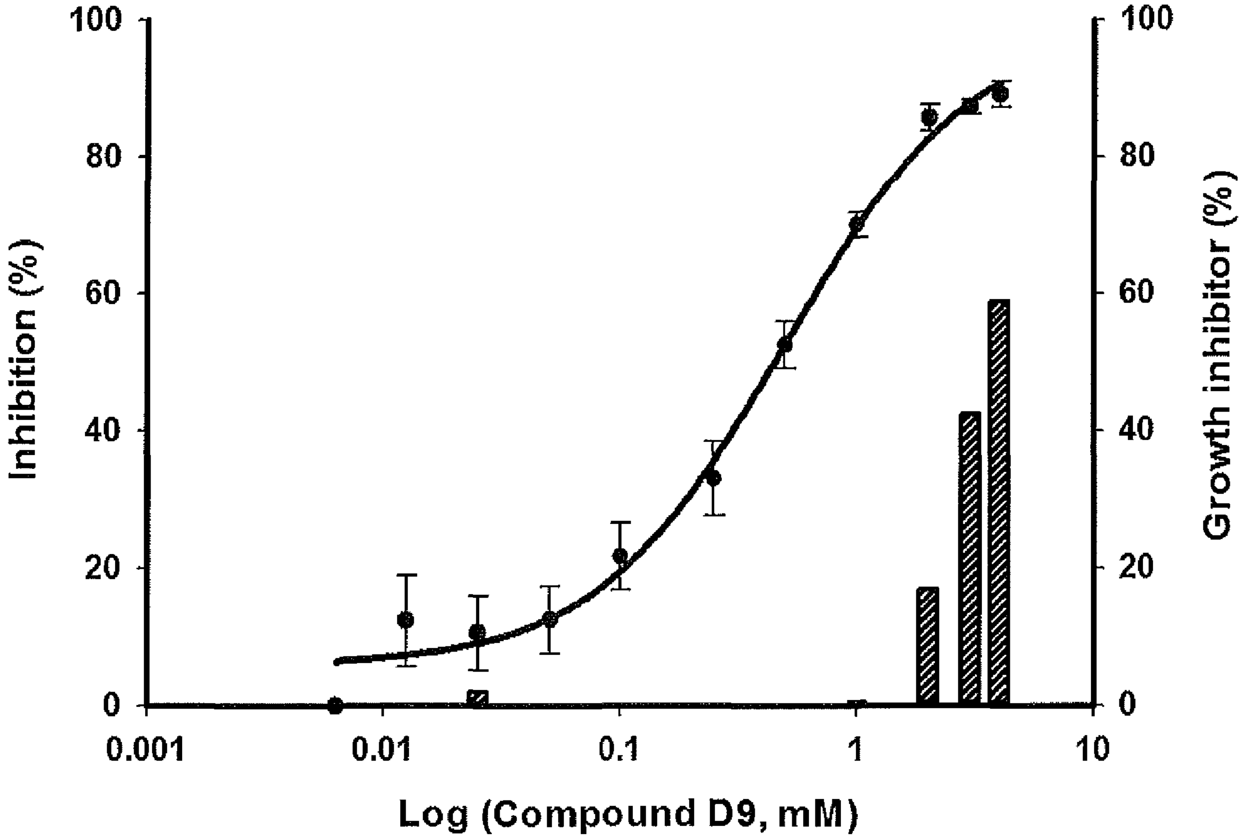


FIGURE 33

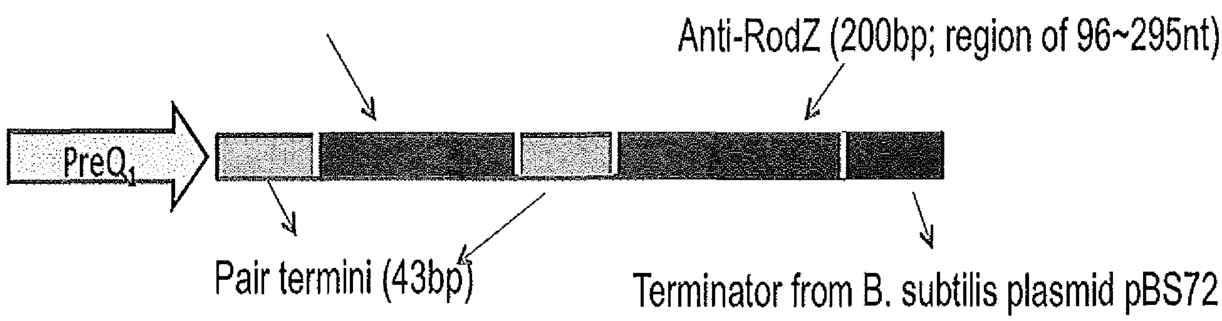

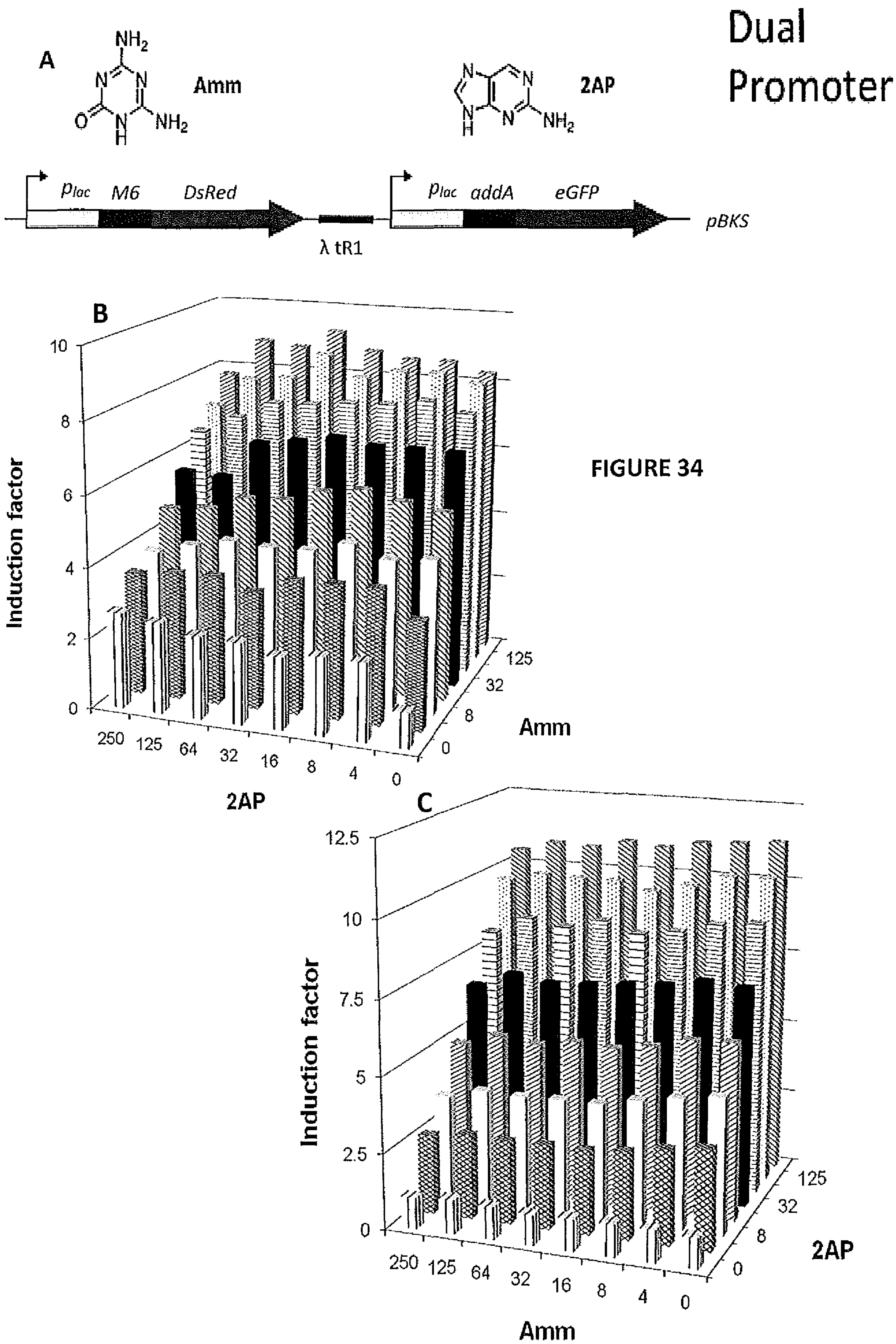
Dual
Promoter
A
Amm
2AP
plac M6 DsRed
λ tR1
plac addA eGFP
pBKS
B
Induction factor
0
2
4
6
8
10
250 125 64 32 16 8 4 0
2AP
0 8 32 125
Amm
C
Induction factor
0
2.5
5
7.5
10
12.5
250 125 64 32 16 8 4 0
Amm
0 8 32 125
2AP

FIGURE 34

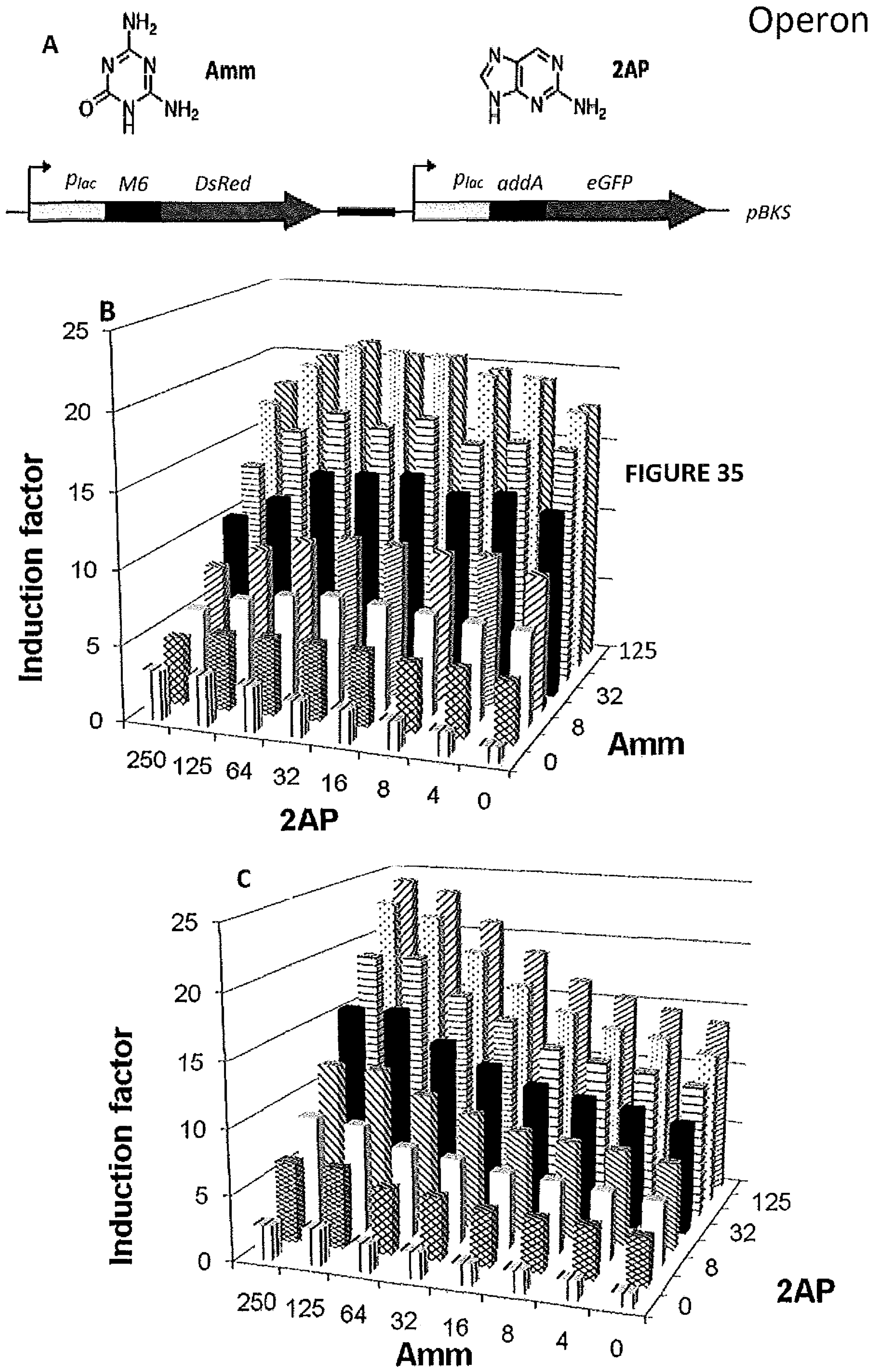
Operon
A
Amm
2AP
plac
M6
DsRed
plac
addA
eGFP
pBKS
B
Induction factor
0
5
10
15
20
25
250
125
64
32
16
8
4
0
2AP
0
8
32
125
Amm
C
Induction factor
0
5
10
15
20
25
250
125
64
32
16
8
4
0
Amm
0
8
32
125
2AP

FIGURE 35

FIGURE 36

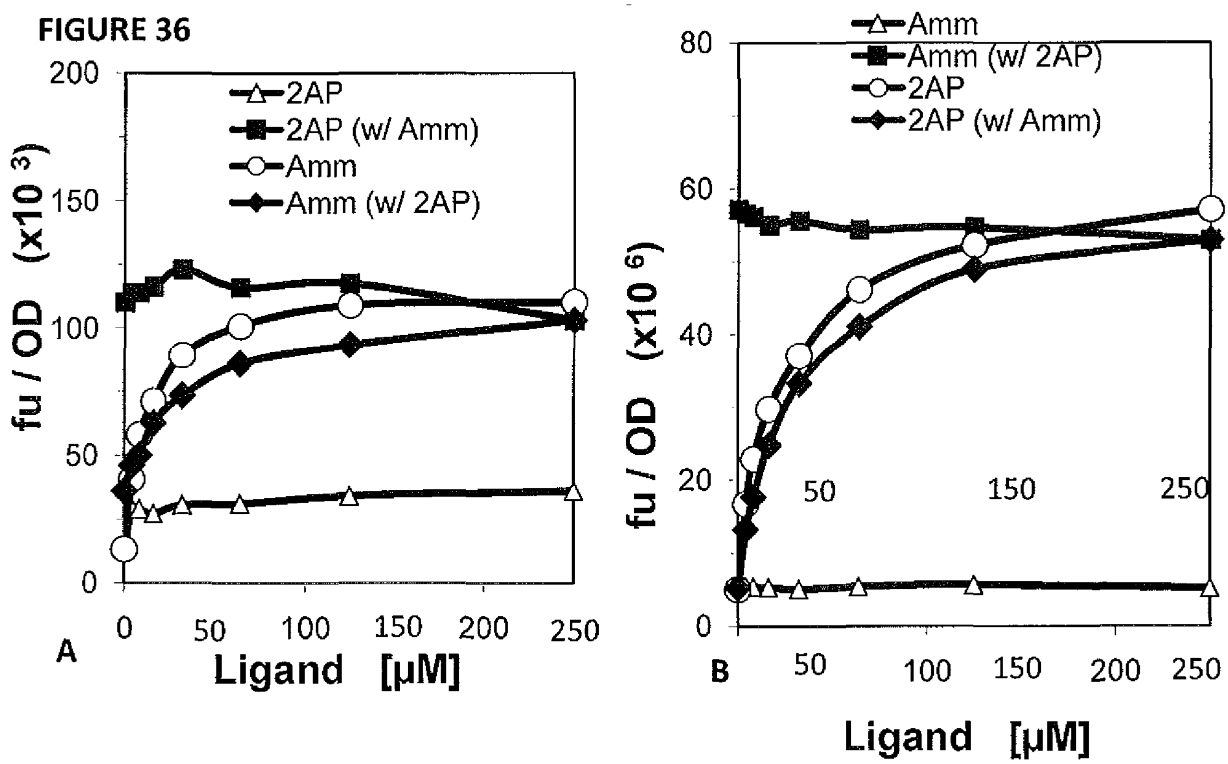


FIGURE 37

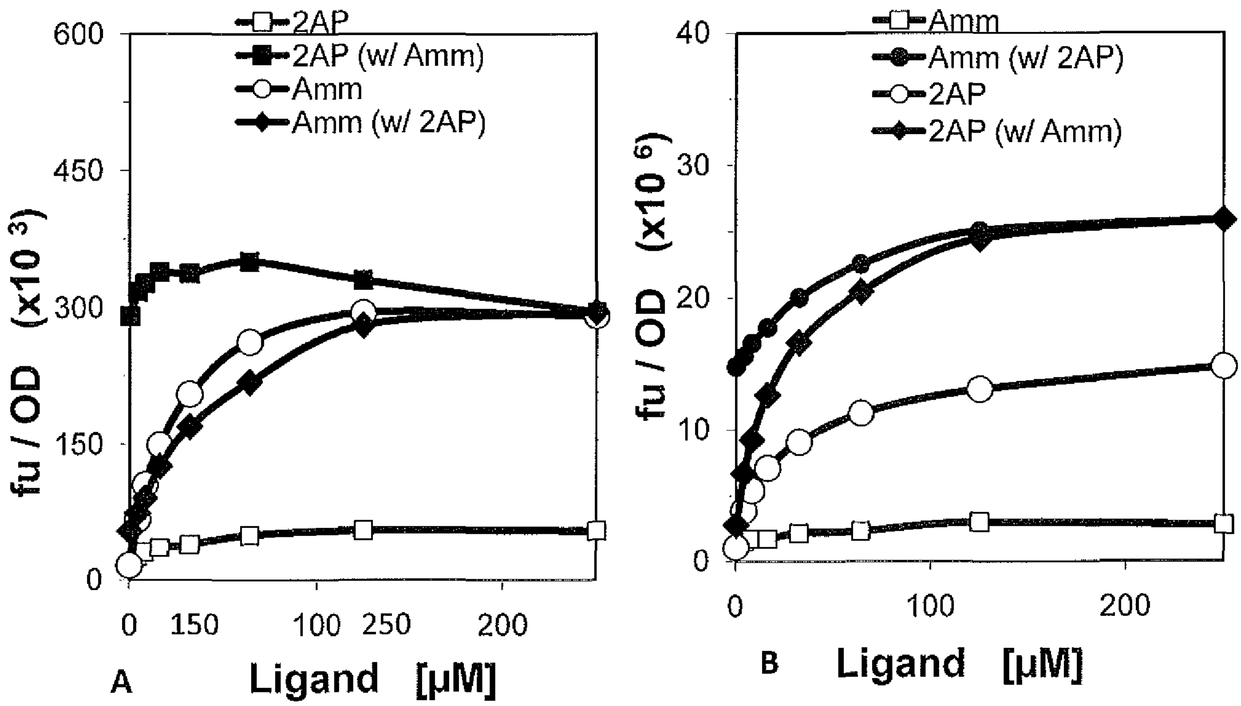

FIGURE 38

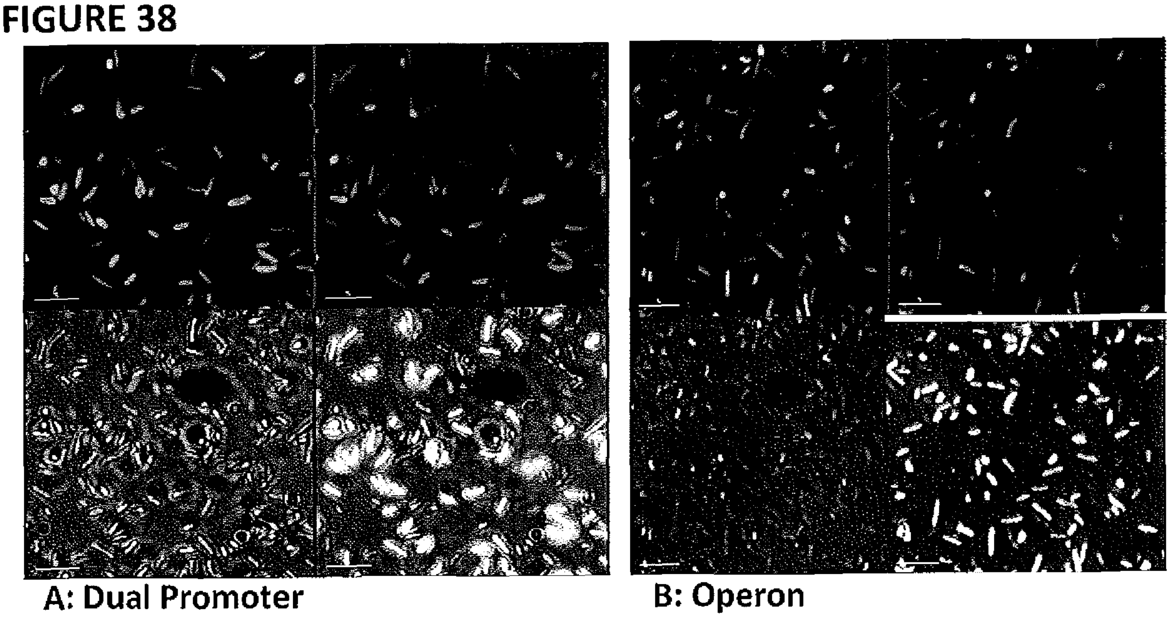

A: Dual Promoter

B: Operon

FIGURE 39

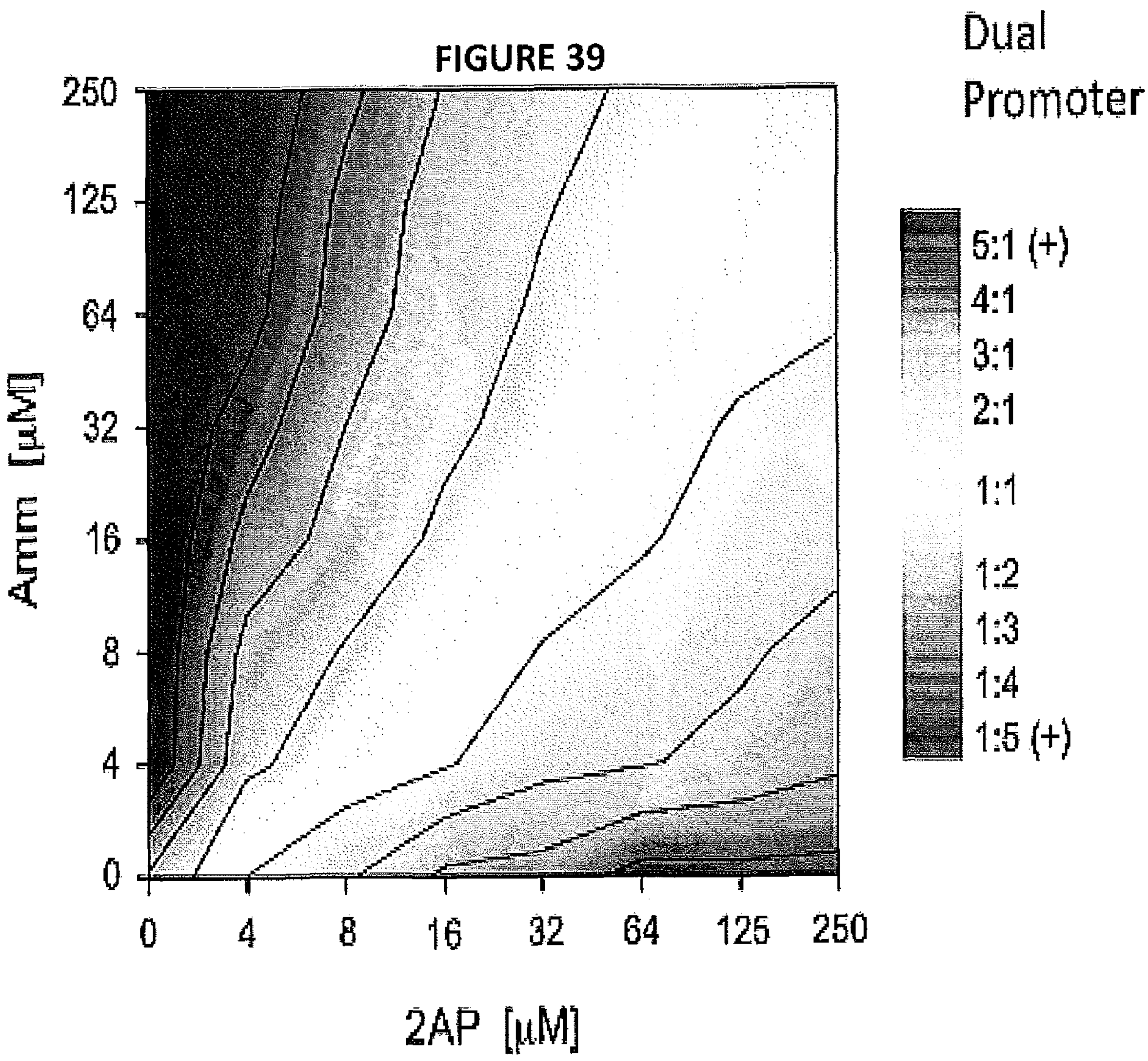

FIGURE 40

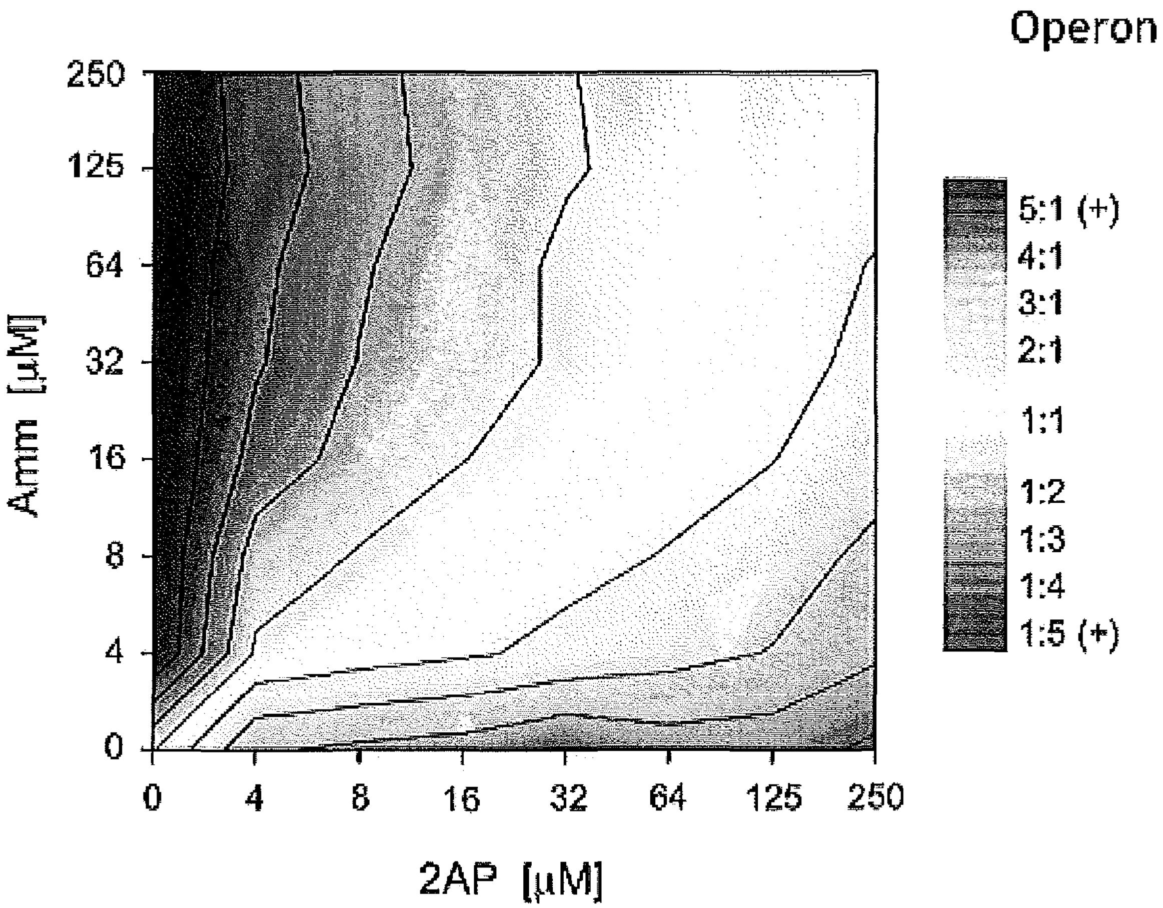
Operon
250
125
64
32
16
8
4
0
Amm [μM]
0
4
8
16
32
64
125
250
2AP [μM]
5:1 (+)
4:1
3:1
2:1
1:1
1:2
1:3
1:4
1:5 (+)

FIGURE 41

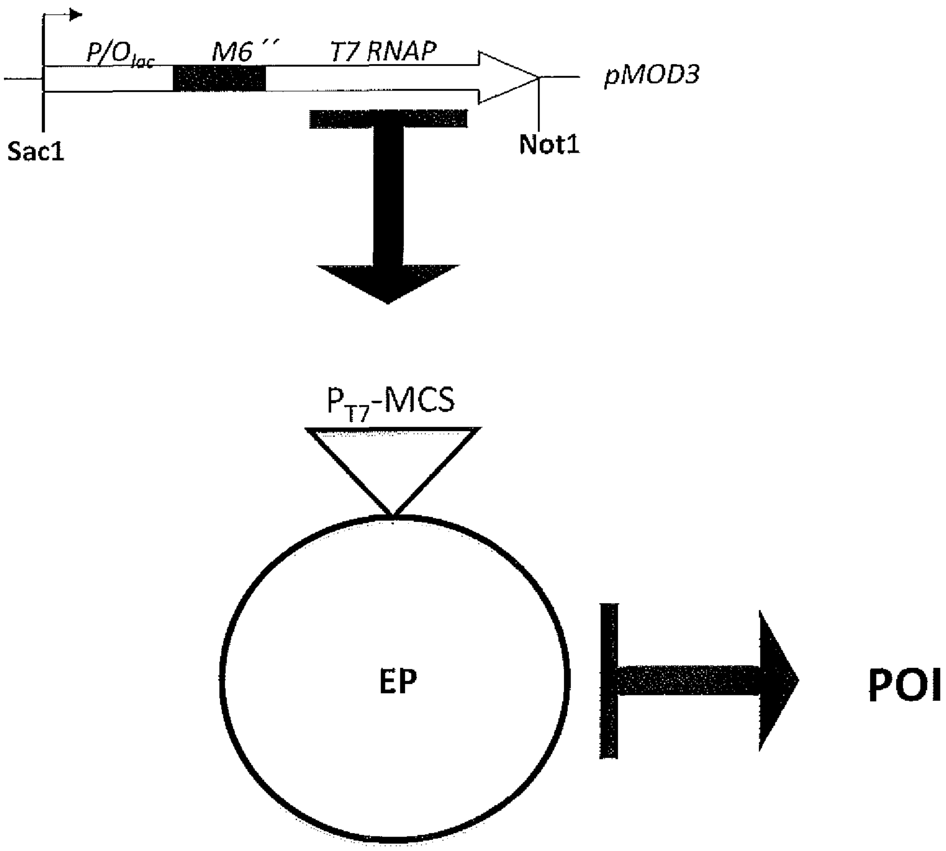


FIGURE 42

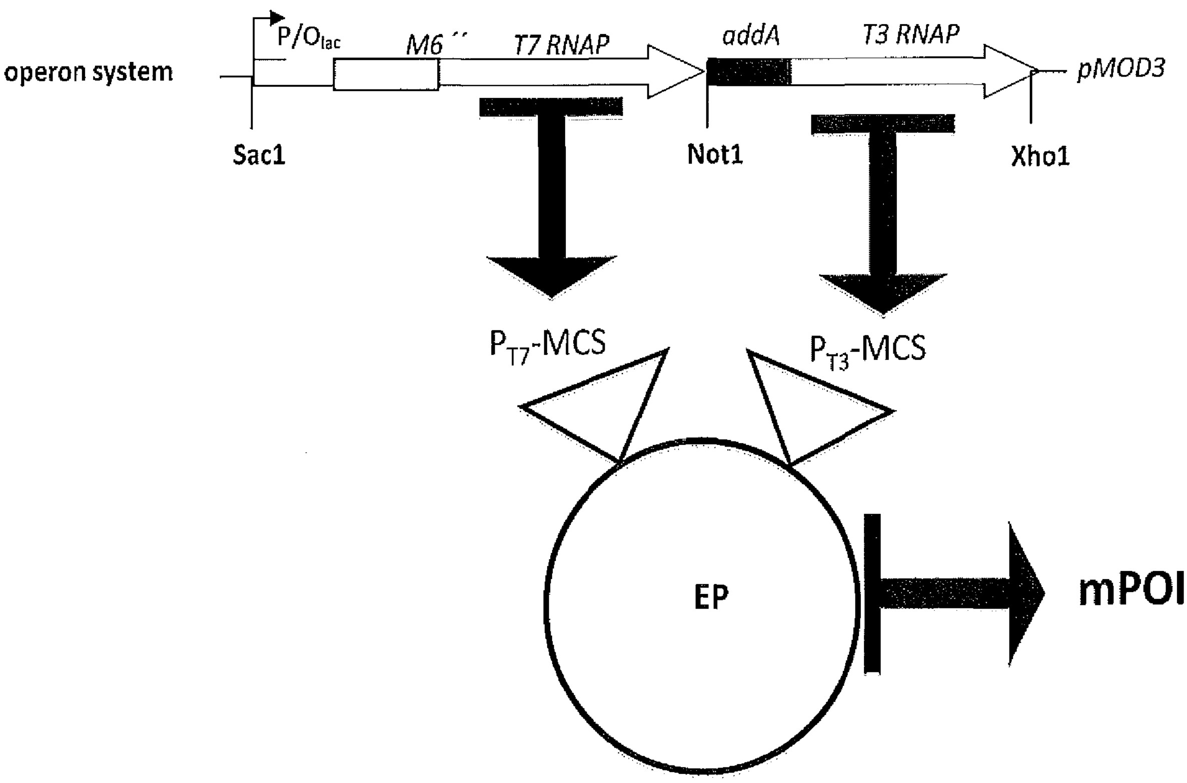

FIGURE 43

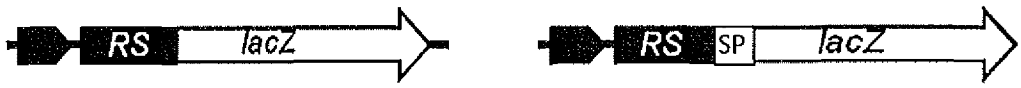
RS
lacZ
RS
SP
lacZ

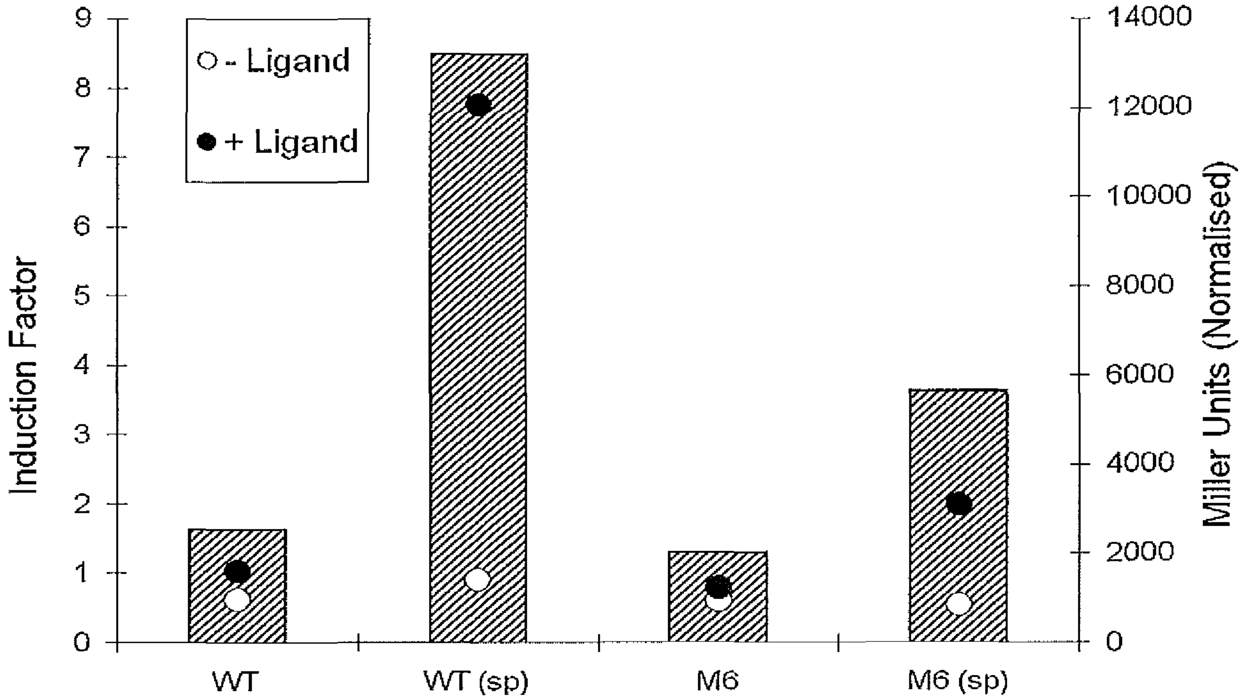
- Ligand
+ Ligand
Induction Factor
Miller Units (Normalised)
0
1
2
3
4
5
6
7
8
9
0
2000
4000
6000
8000
10000
12000
14000
WT
WT (sp)
M6
M6 (sp)

FIGURE 44

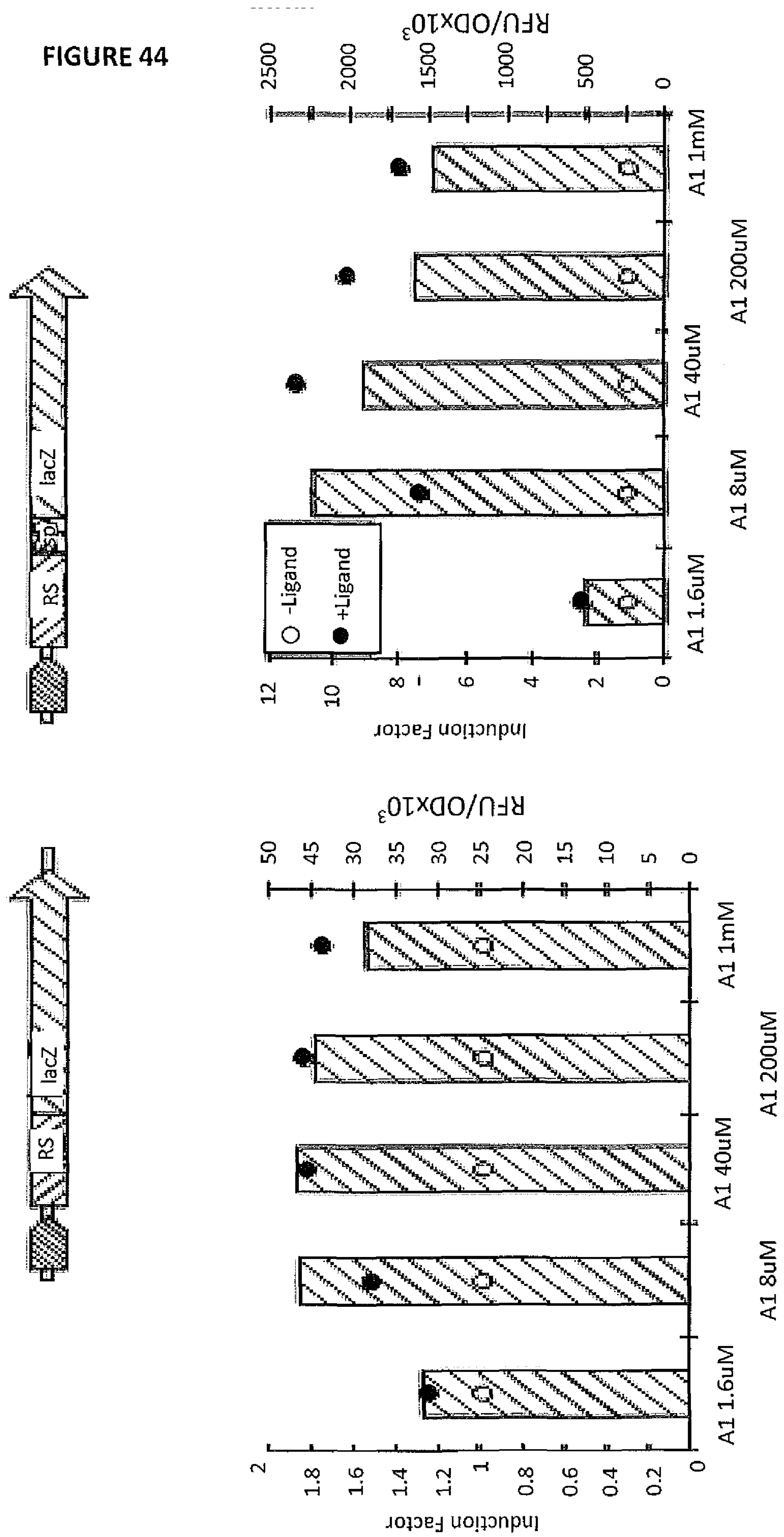
RS
Sp
lacZ
RFU/ODx10^3
Induction Factor
-Ligand
+Ligand
A1 1.6uM
A1 8uM
A1 40uM
A1 200uM
A1 1mM

FIGURE 45

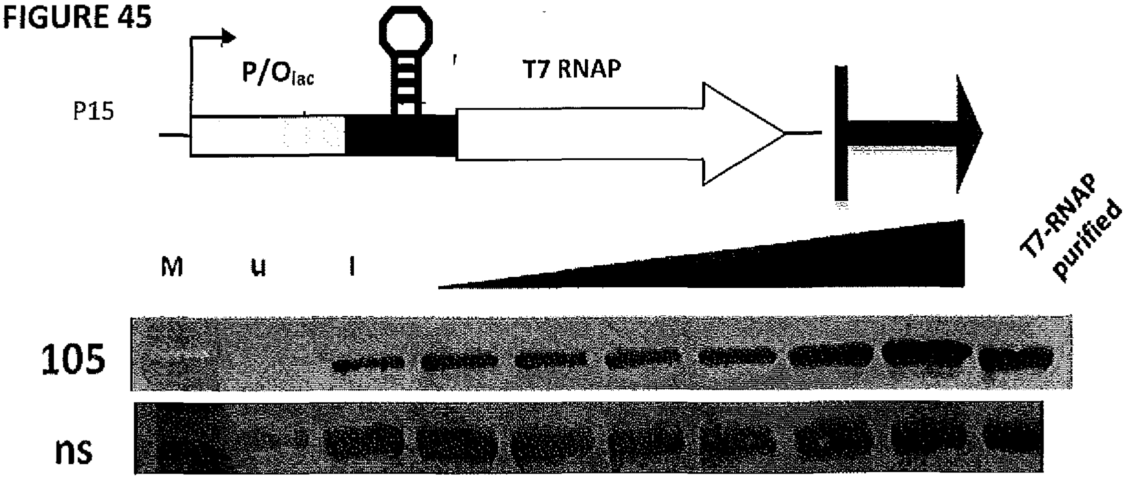


FIGURE 46

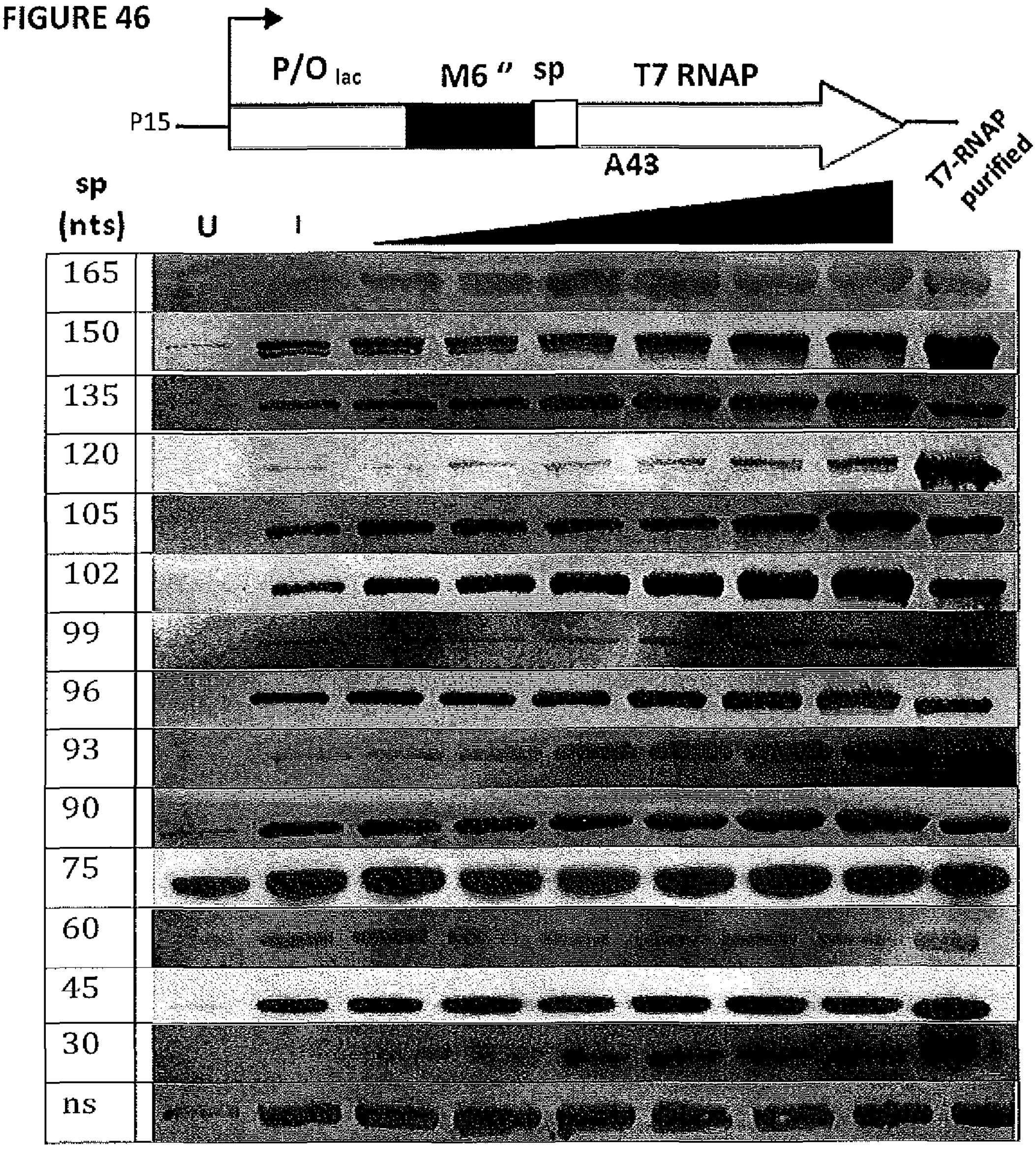

FIGURE 47

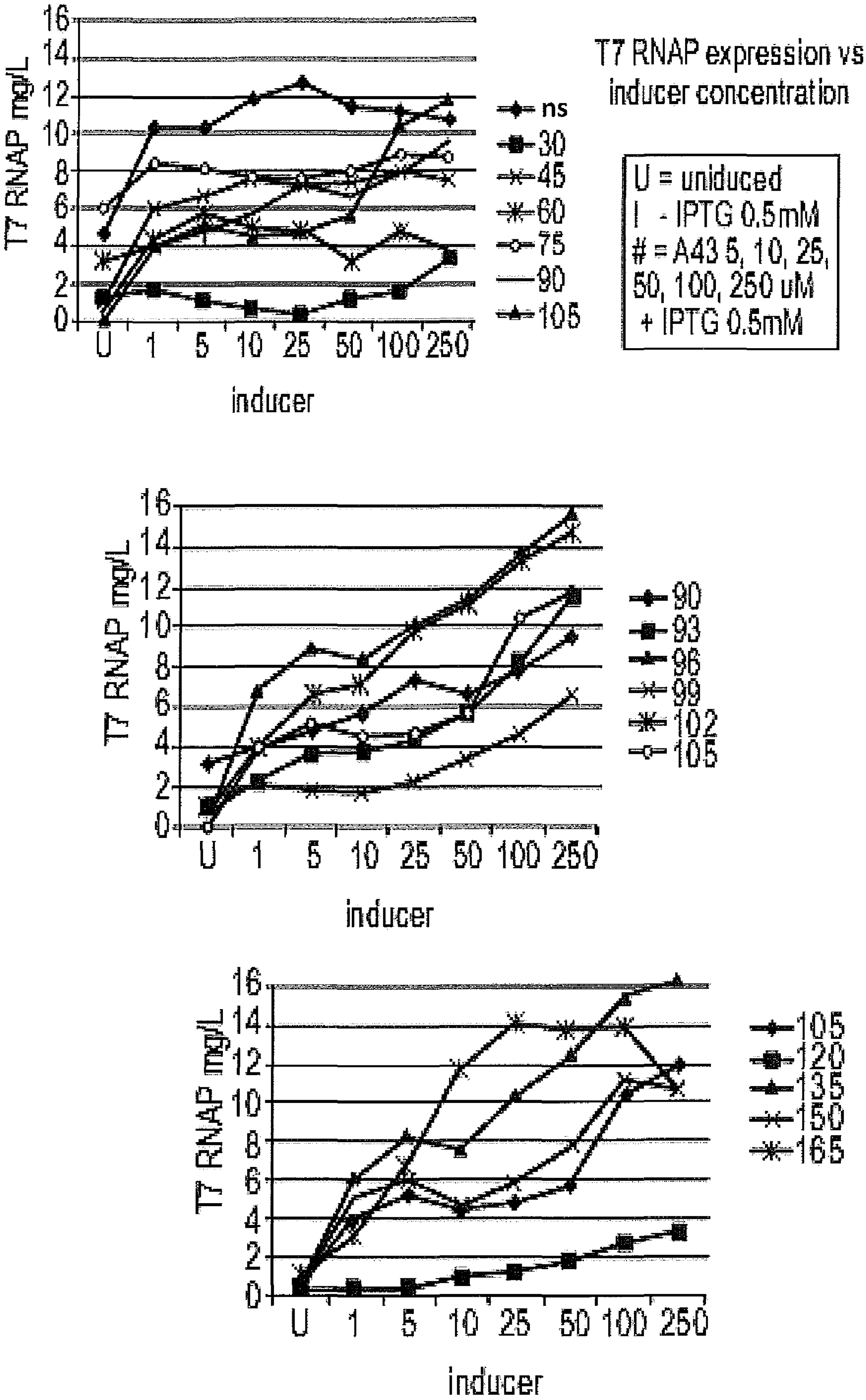
T7 RNAP expression vs inducer concentration
U = uniduced
I - IPTG 0.5mM
# = A43 5, 10, 25, 50, 100, 250 uM + IPTG 0.5mM
T7 RNAP mg/L
inducer
U 1 5 10 25 50 100 250
ns
30
45
60
75
90
105
90
93
96
99
102
105
105
120
135
150
165

FIGURE 48

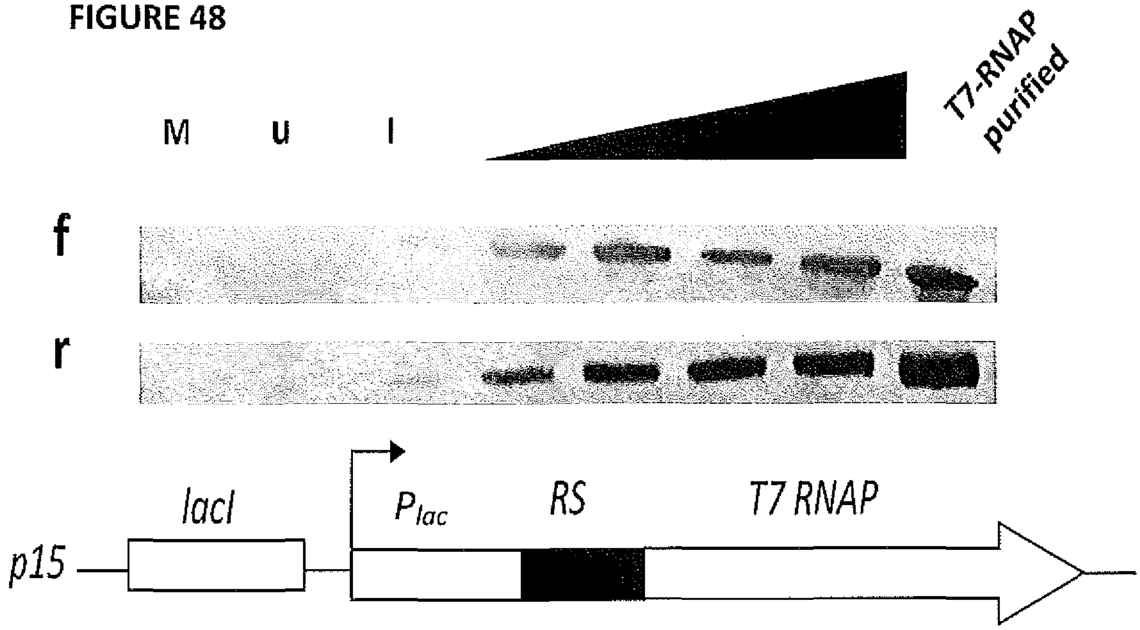


FIGURE 49

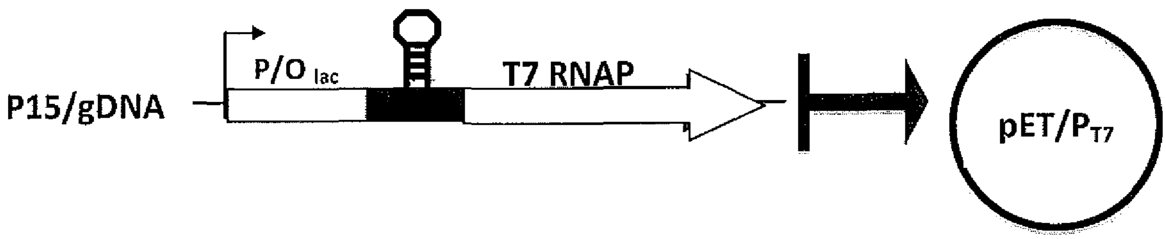


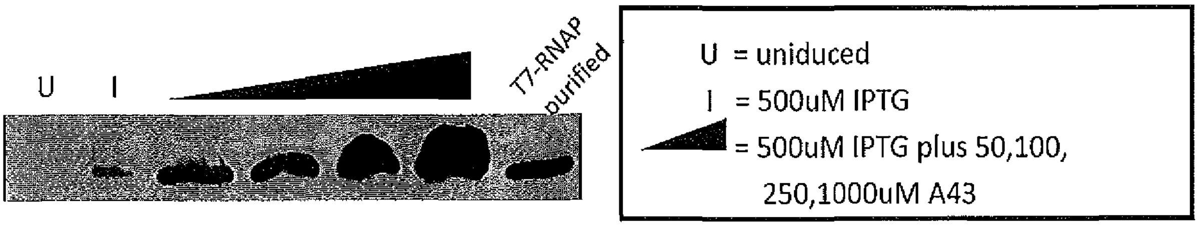

FIGURE 50

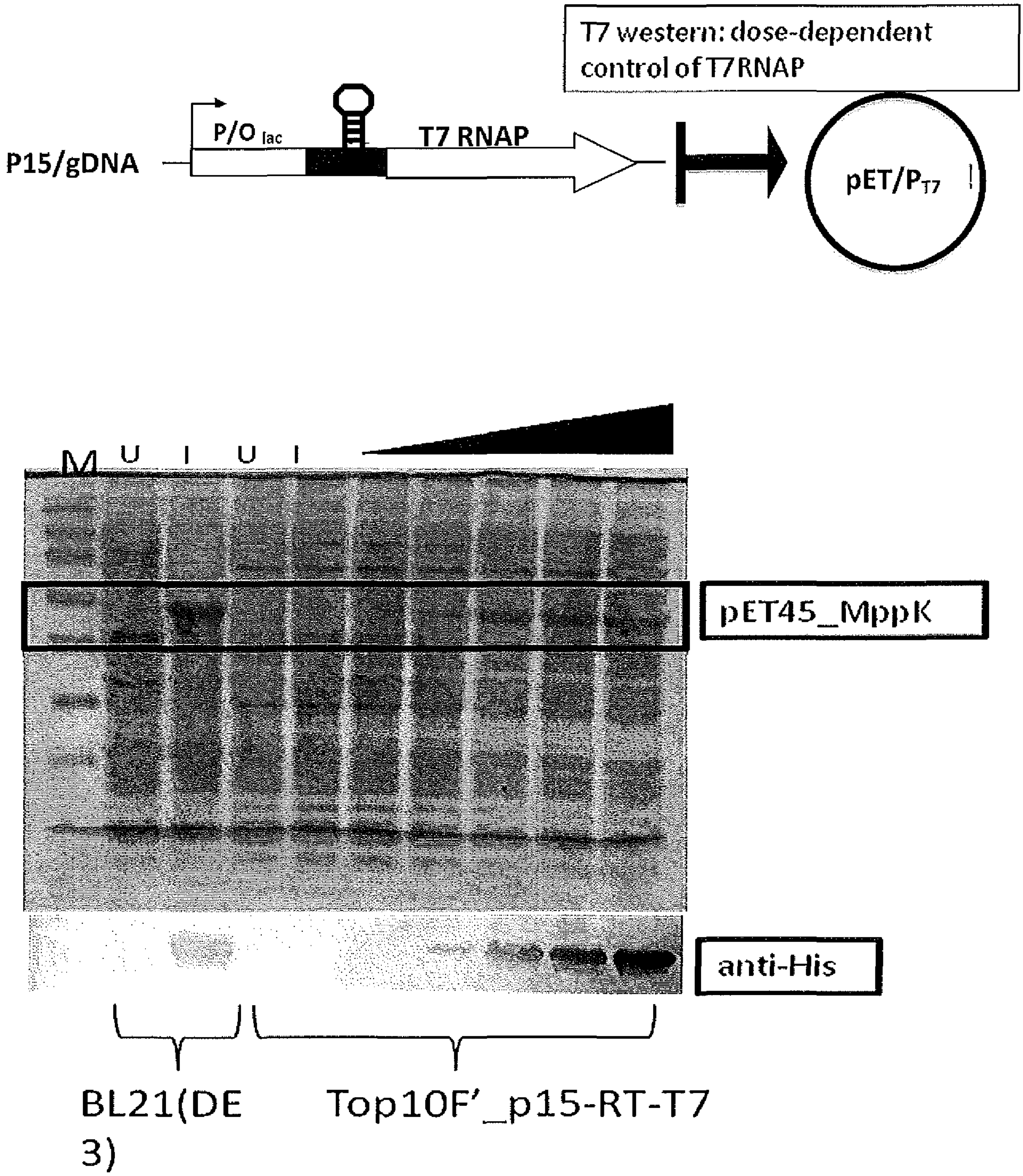
T7 western: dose-dependent control of T7RNAP
P15/gDNA
P/O lac
T7 RNAP
pET/PT7
M
U
I
U
I
pET45_MppK
anti-His
BL21(DE 3)
Top10F'_p15-RT-T7

FIGURE 51

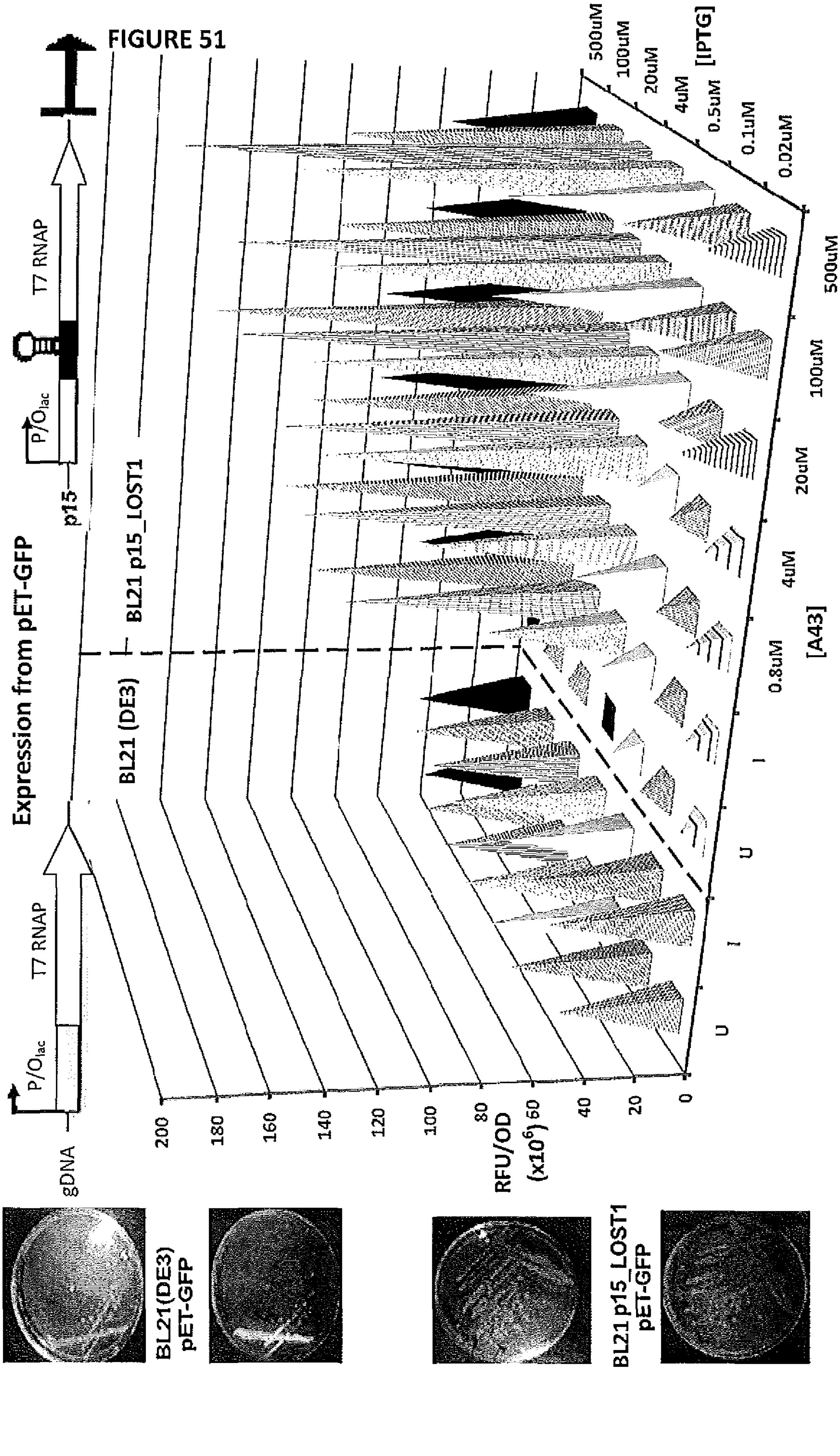
Expression from pET-GFP
gDNA
P/O$_{lac}$
T7 RNAP
p15
P/O$_{lac}$
T7 RNAP
BL21 (DE3)
BL21 p15_LOST1
RFU/OD
(x10$^6$)
0
20
40
60
80
100
120
140
160
180
200
U
I
U
I
0.8uM
4uM
20uM
100uM
500uM
[A43]
500uM
100uM
20uM
4uM
0.5uM
0.1uM
0.02uM
[IPTG]
BL21(DE3)
pET-GFP
BL21 p15_LOST1
pET-GFP

FIGURE 52

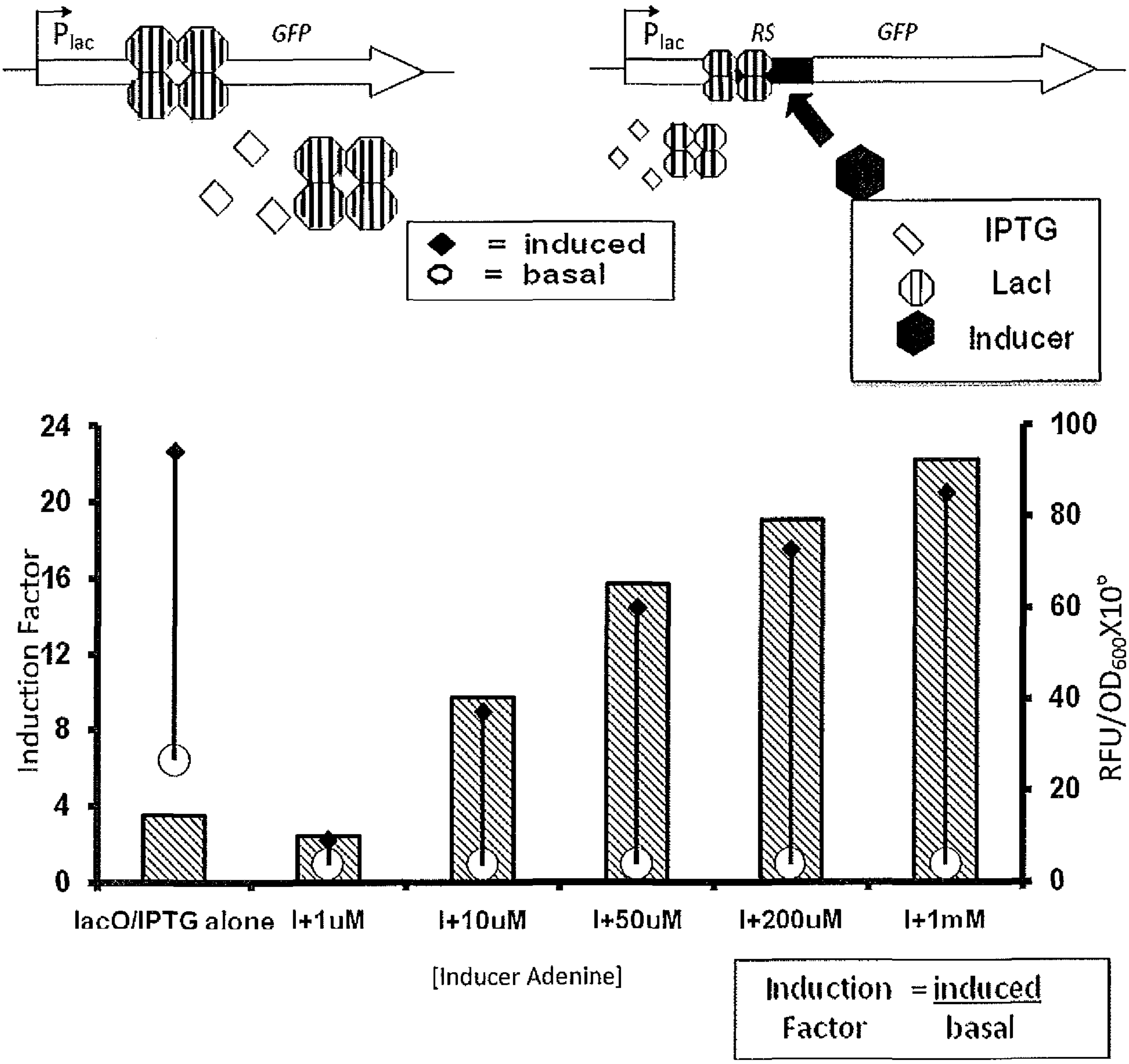
Plac
GFP
Plac
RS
GFP
= induced
= basal
IPTG
LacI
Inducer
24
20
16
12
8
4
0
Induction Factor
100
80
60
40
20
0
RFU/OD600X10^6
lacO/IPTG alone
I+1uM
I+10uM
I+50uM
I+200uM
I+1mM
[Inducer Adenine]
Induction Factor = induced / basal

FIGURE 53

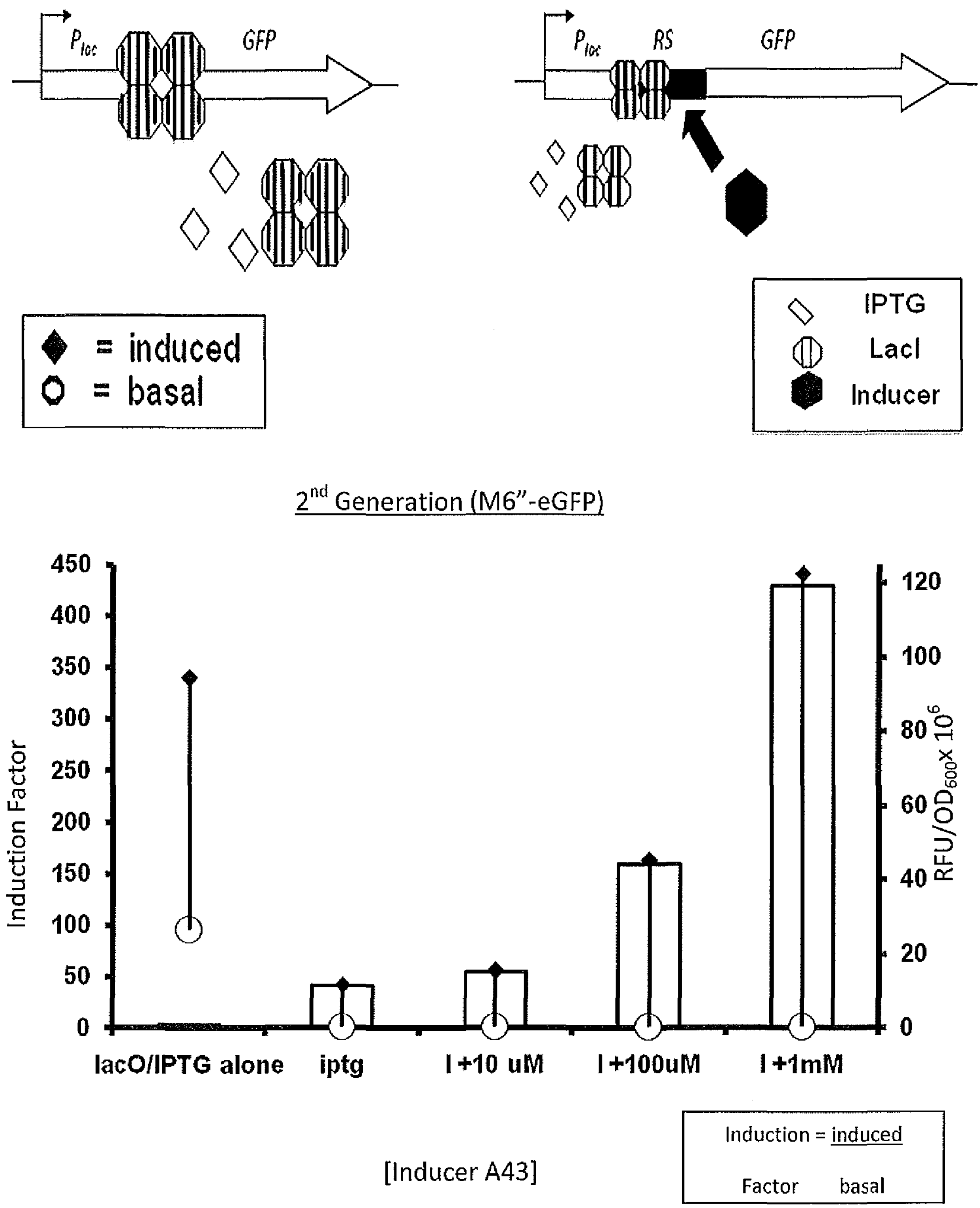

P_lac
GFP
RS
= induced
= basal
IPTG
LacI
Inducer
2nd Generation (M6"-eGFP)
Induction Factor
RFU/OD600X 10^6
450
400
350
300
250
200
150
100
50
0
120
100
80
60
40
20
0
lacO/IPTG alone
iptg
I +10 uM
I +100uM
I +1mM
[Inducer A43]
Induction Factor = induced / basal

FIGURE 54

Cellular level titration:

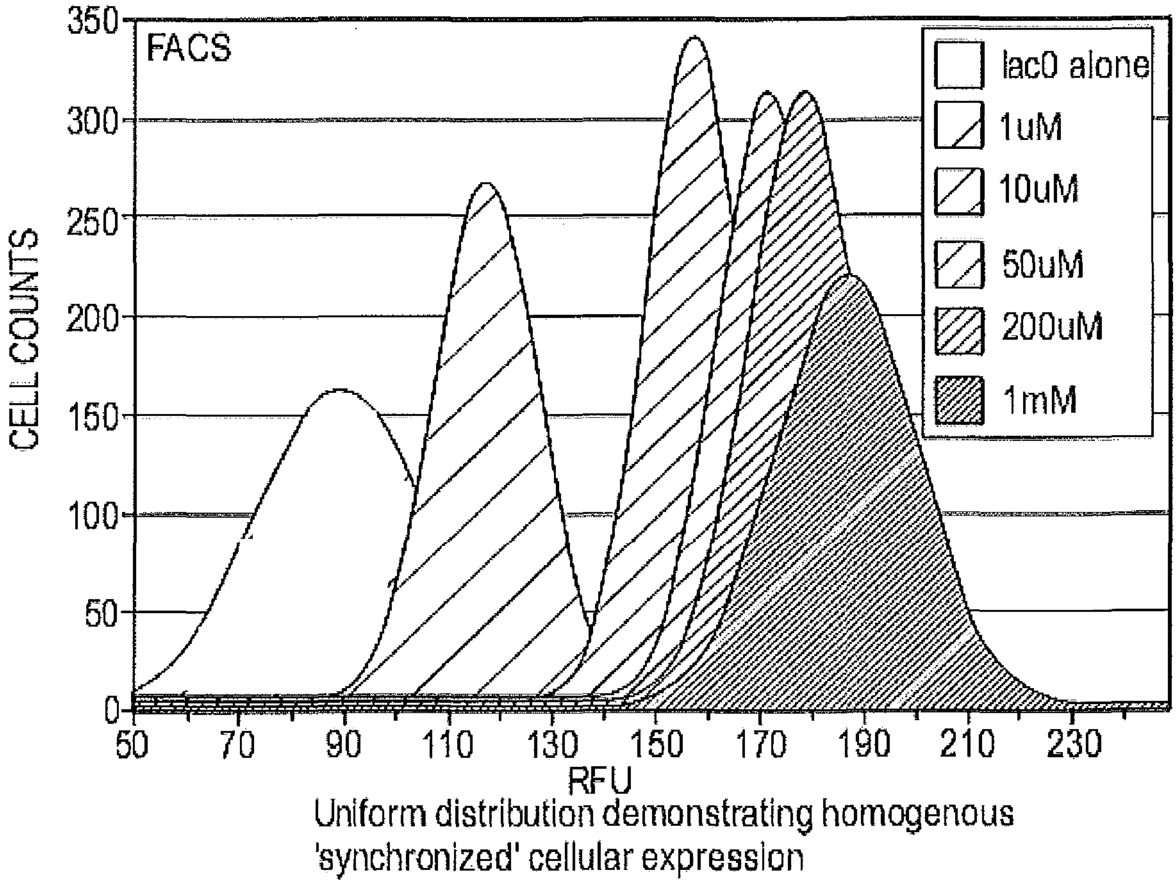


Uniform distribution demonstrating homogenous 'synchronized' cellular expression

FIGURE 55

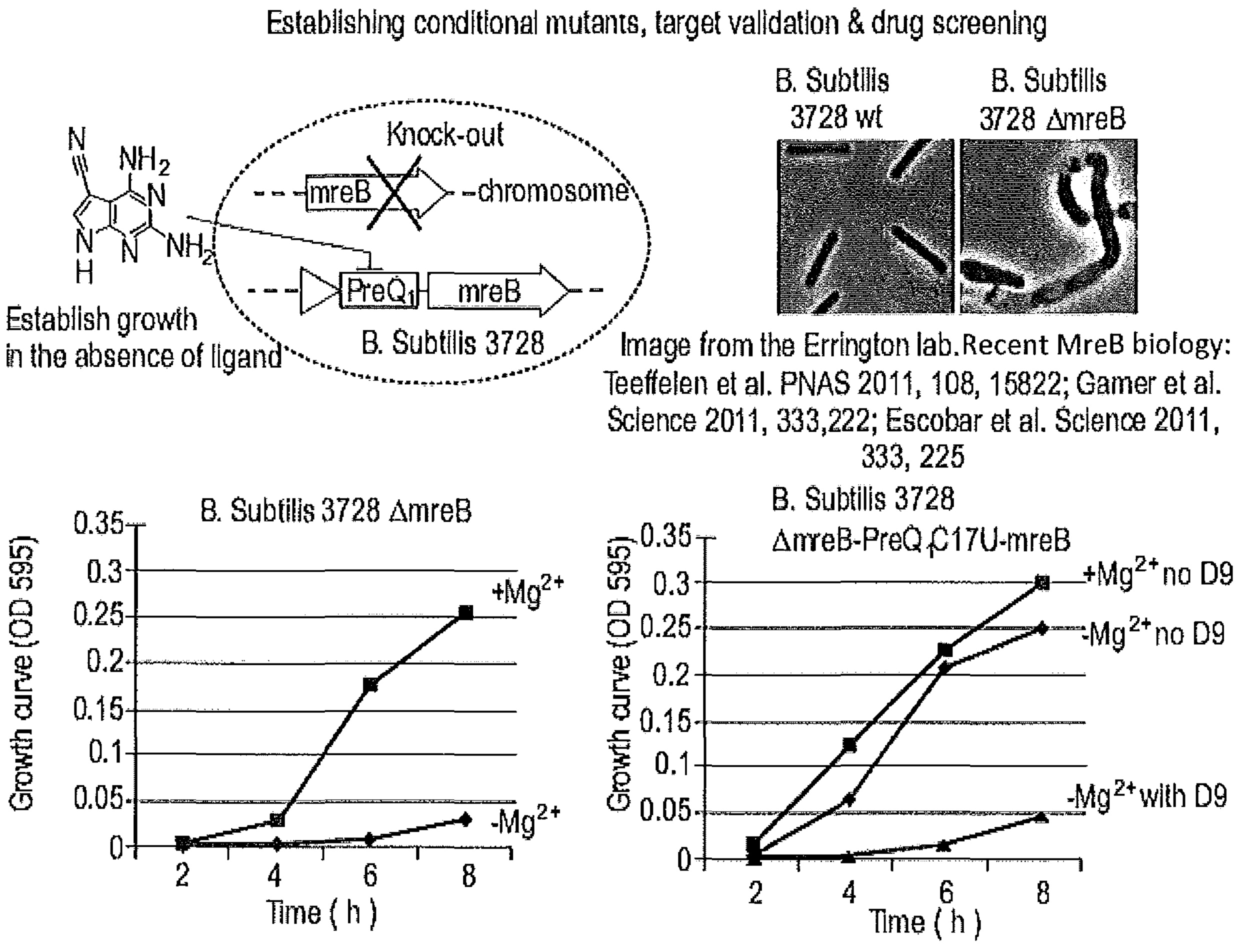
Establishing conditional mutants, target validation & drug screening
Knock-out
mreB
chromosome
PreQ1
mreB
B. Subtilis 3728
Establish growth
in the absence of ligand
B. Subtilis 3728 wt
B. Subtilis 3728 ΔmreB
Image from the Errington lab. Recent MreB biology: Teeffelen et al. PNAS 2011, 108, 15822; Gamer et al. Science 2011, 333,222; Escobar et al. Science 2011, 333, 225
B. Subtilis 3728 ΔmreB
Growth curve (OD 595)
+Mg2+
-Mg2+
Time ( h )
B. Subtilis 3728 ΔmreB-PreQ1C17U-mreB
+Mg2+ no D9
-Mg2+ no D9
-Mg2+ with D9

FIGURE 56

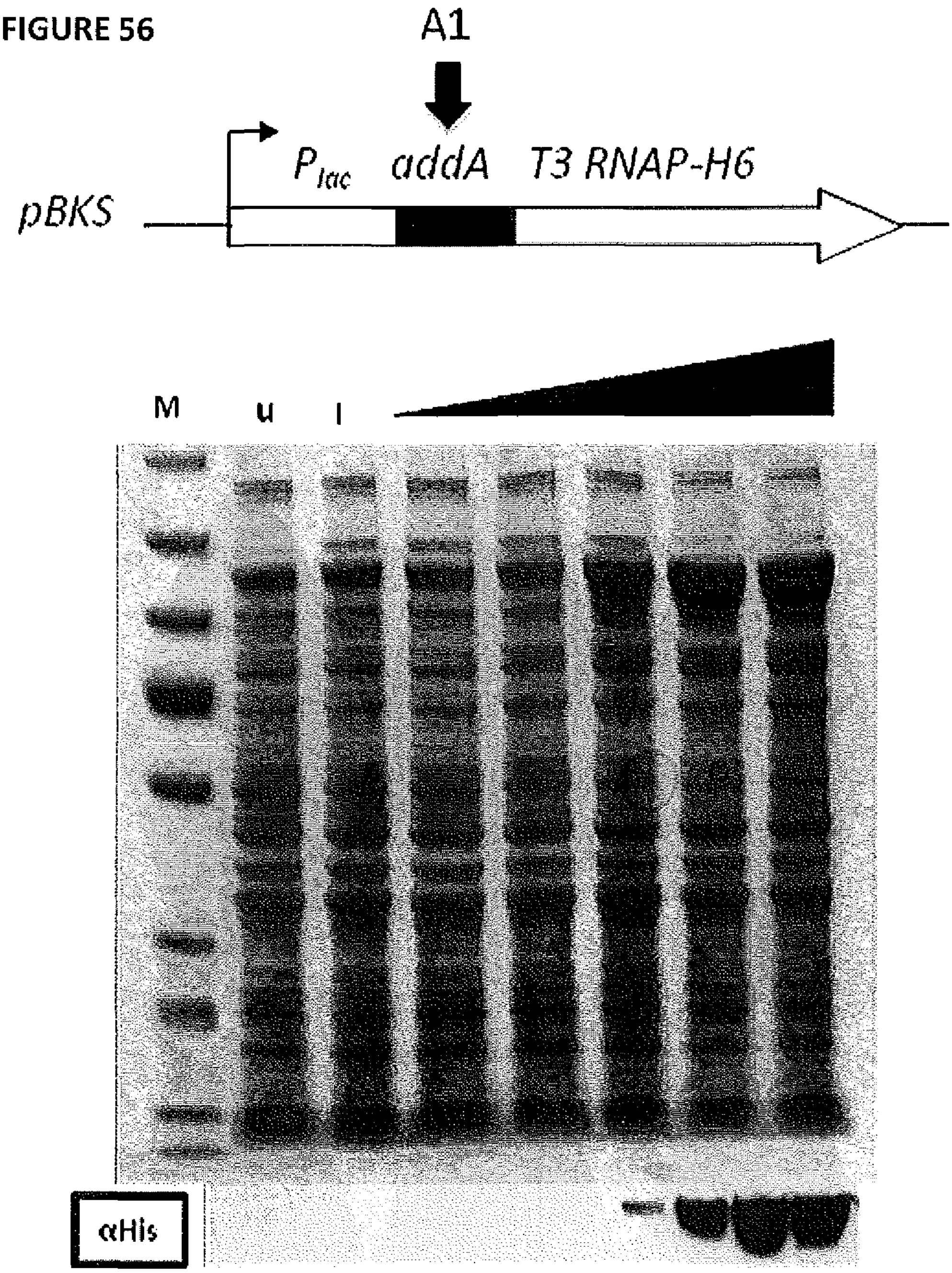

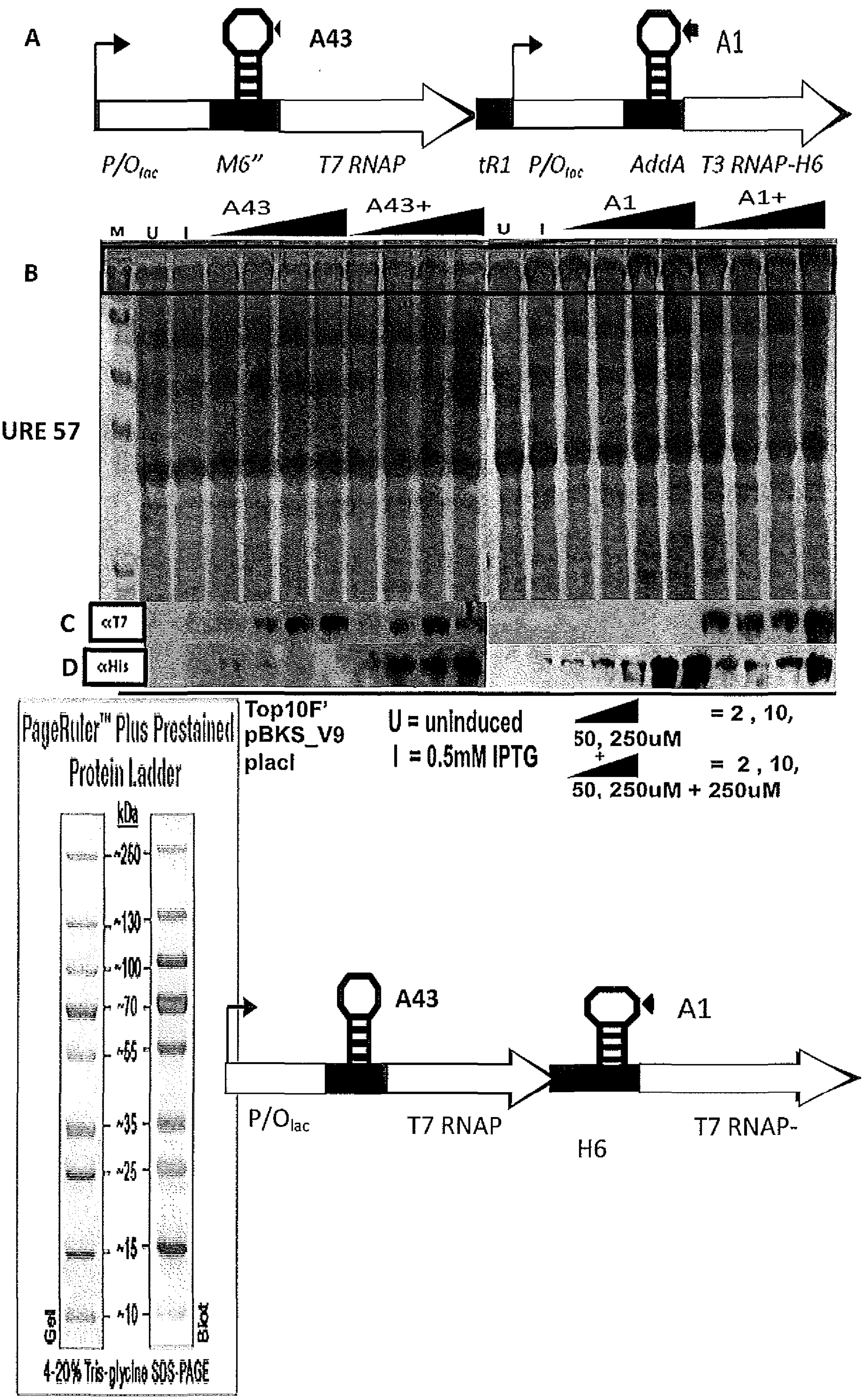
A
A43
A1
P/O$_{lac}$
M6"
T7 RNAP
tR1
P/O$_{lac}$
AddA
T3 RNAP-H6
M
U
I
A43
A43+
U
I
A1
A1+
B
C
αT7
D
αHis
PageRuler™ Plus Prestained Protein Ladder
kDa
~250
~130
~100
~70
~55
~35
~25
~15
~10
Gel
Blot
4-20% Tris-glycine SDS-PAGE
Top10F'
pBKS_V9
placI
U = uninduced
I = 0.5mM IPTG
= 2 , 10, 50, 250uM
+
= 2 , 10, 50, 250uM + 250uM
A43
A1
P/O$_{lac}$
T7 RNAP
H6
T7 RNAP-

FIGURE 57

FIGURE 58

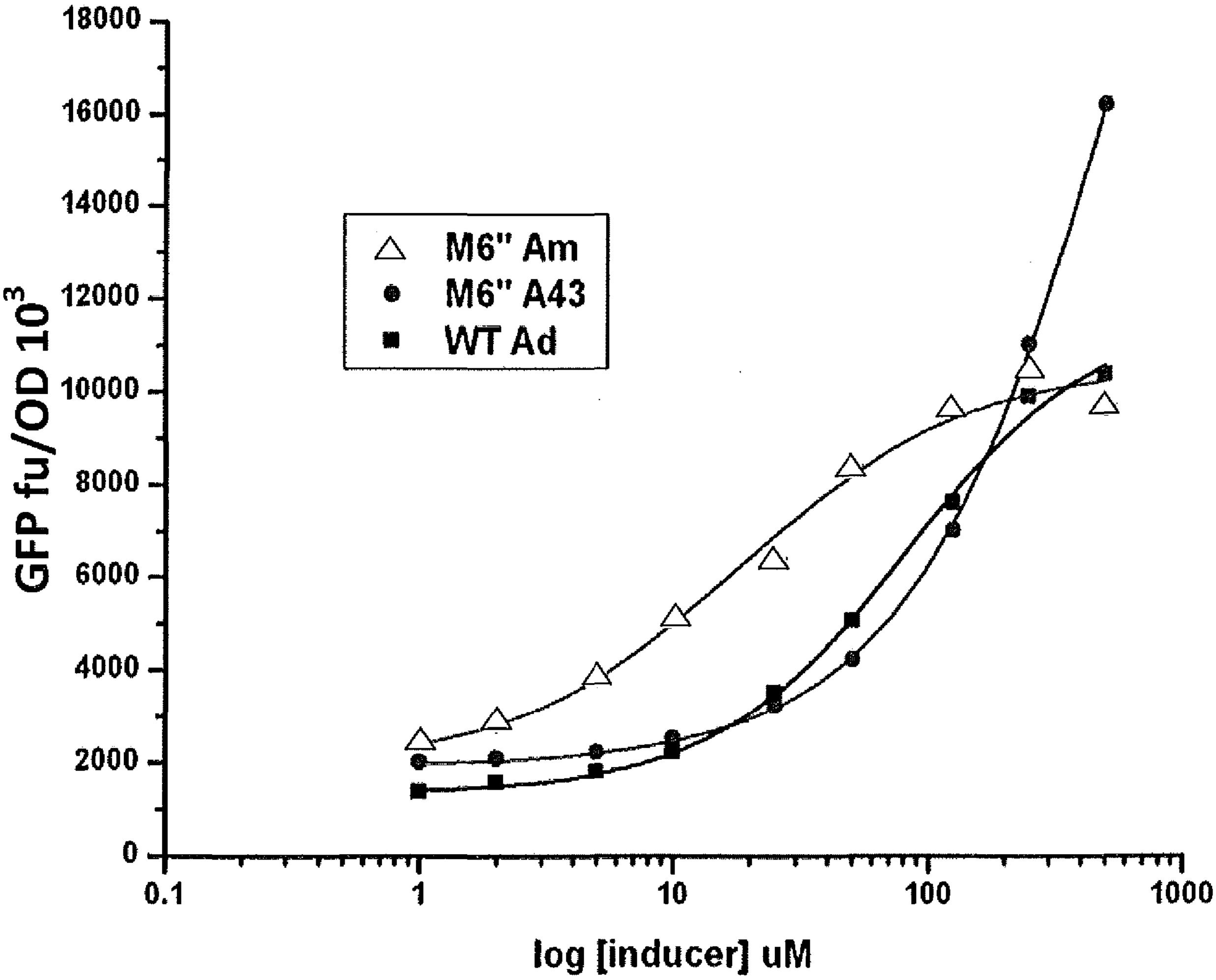
18000
16000
14000
12000
10000
8000
6000
4000
2000
0
GFP fu/OD $10^3$
M6" Am
M6" A43
WT Ad
0.1
1
10
100
1000
log [inducer] uM

FIGURE 59

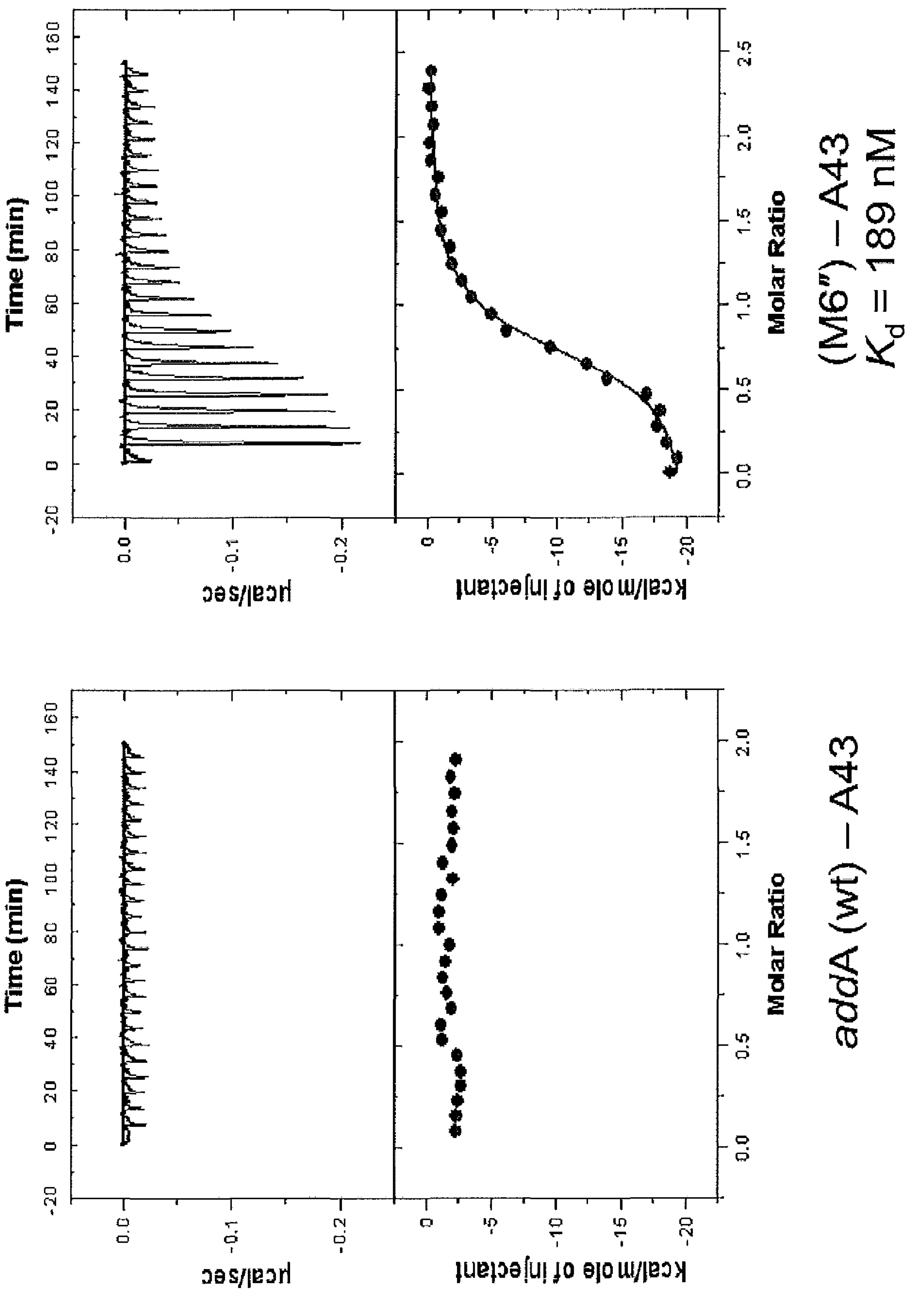
Time (min)
µcal/sec
kcal/mole of injectant
Molar Ratio
(M6") – A43
$K_d$ = 189 nM
addA (wt) – A43

FIGURE 60

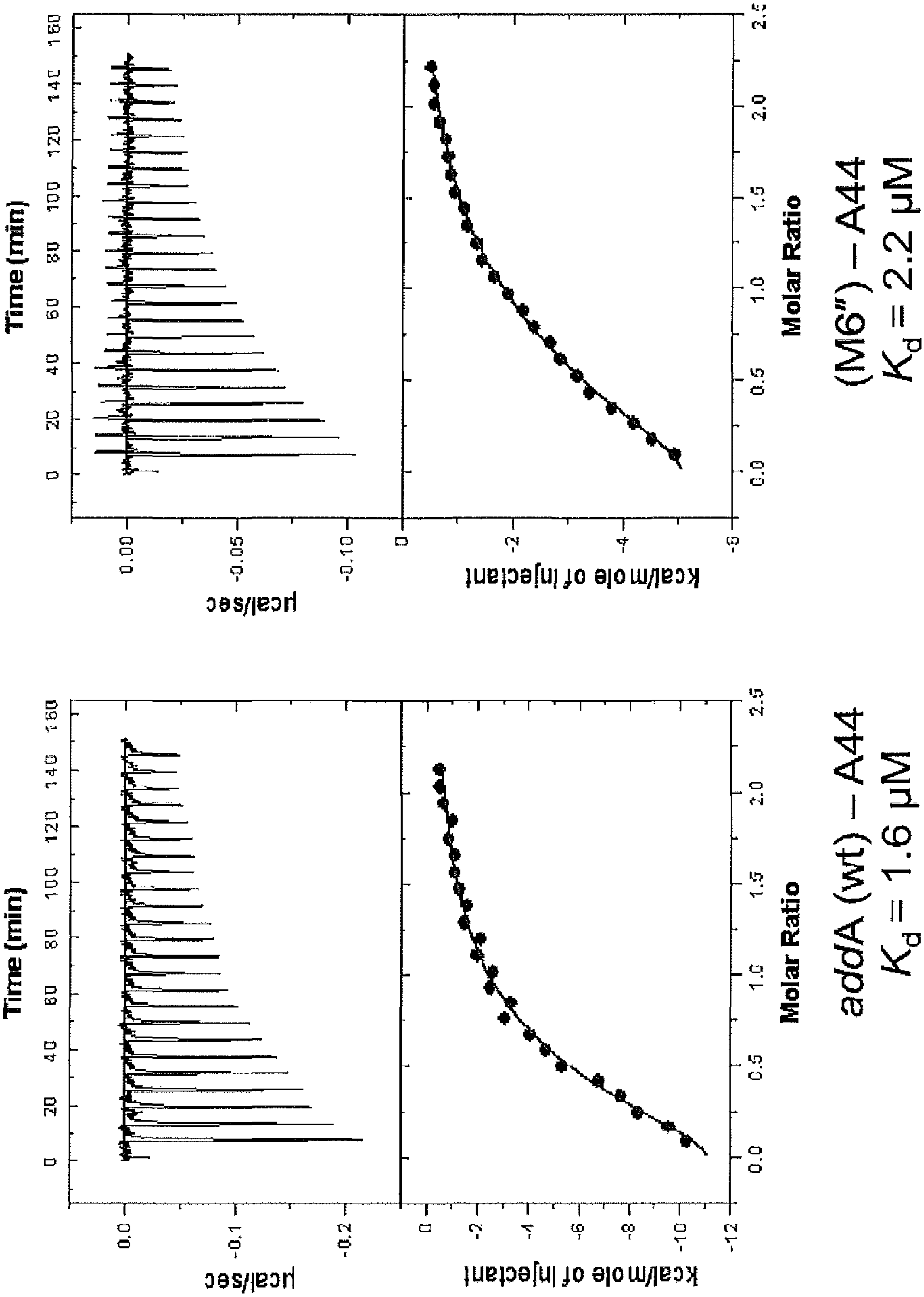
Time (min)
µcal/sec
kcal/mole of injectant
Molar Ratio
(M6") – A44
$K_d$ = 2.2 µM
Time (min)
µcal/sec
kcal/mole of injectant
Molar Ratio
*addA* (wt) – A44
$K_d$ = 1.6 µM

FIGURE 61

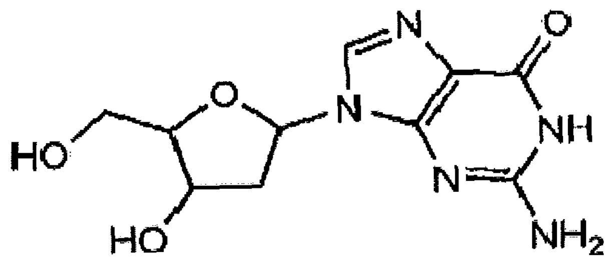

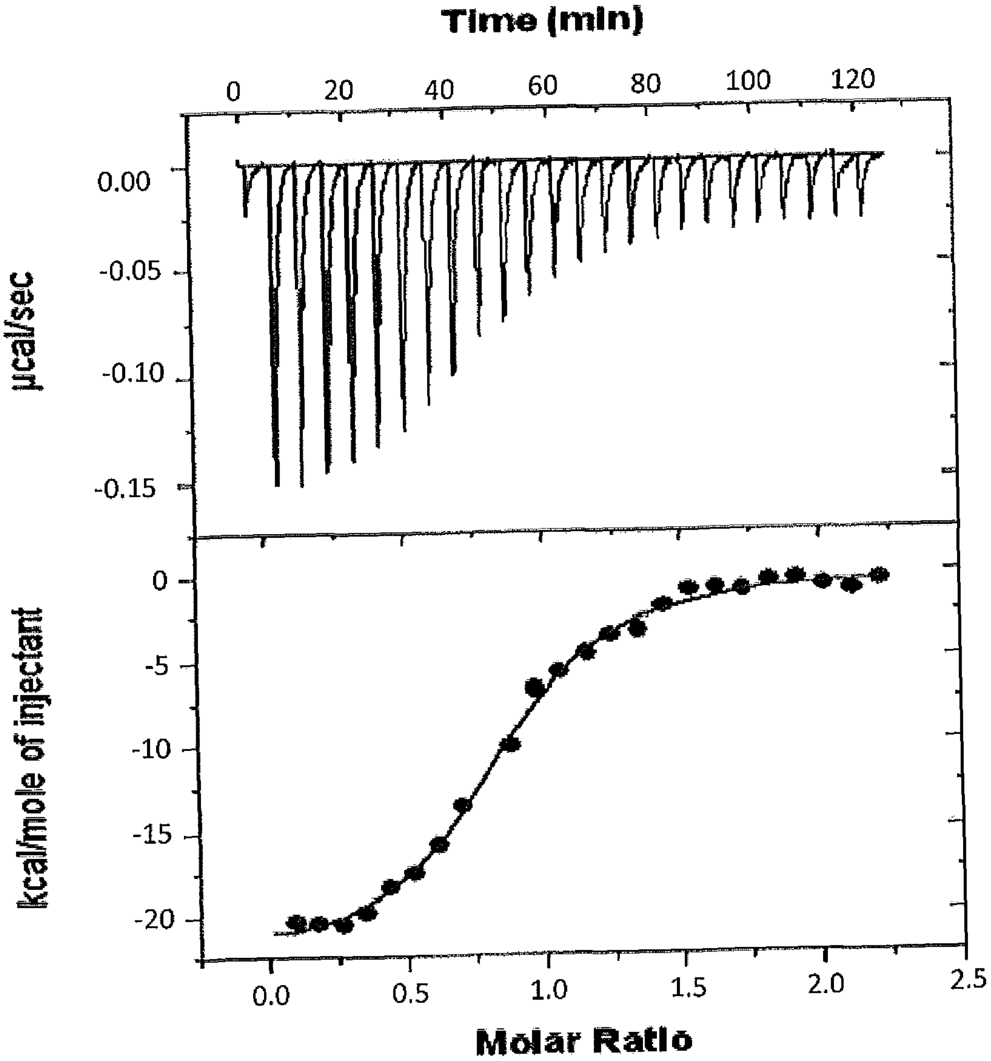


| | | | |
|---|---|---|---|
| **RNA** | M6C" | ***n*** | 0.84 |
| **ligand** | A52 | $K_D$ | 165.07 |
| **model** | onesites | **ΔG** | -9.41 |
| **chi^2** | 178806 | **ΔH** | -22.46 |
| | | **TΔS** | -13.05 |

M6C" : 2'-deoxyguanosine (A52)

FIGURE 61 continued

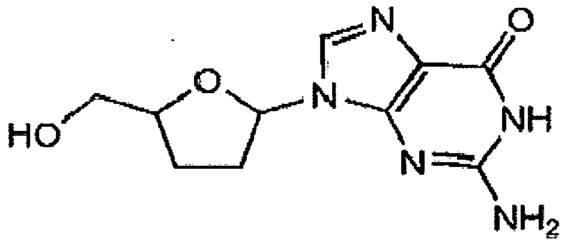

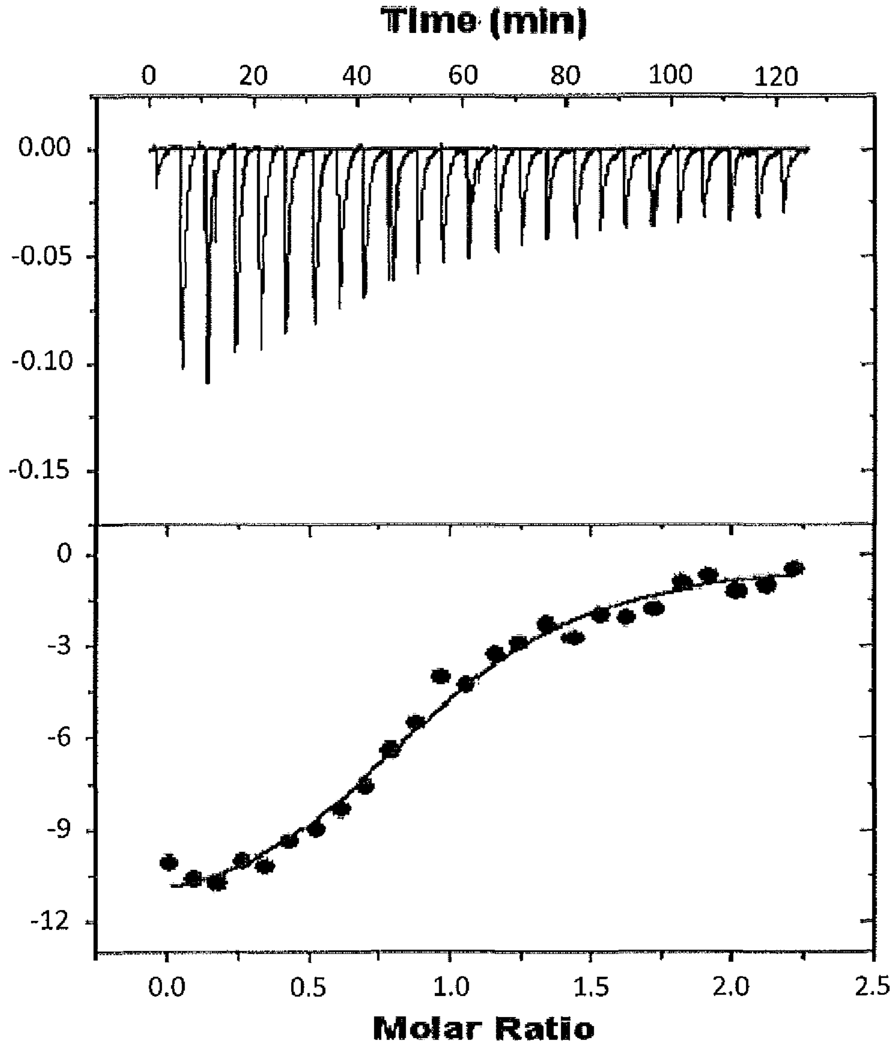


| | | | |
|---|---|---|---|
| **RNA** | M6C" | *n* | 0.94 |
| **ligand** | C44 | $K_D$ | 372.02 |
| **model** | onesites | ΔG | -8.92 |
| **chi^2** | 165636 | ΔH | -12.30 |
| | | TΔS | -3.38 |

M6C" : 2',3'-dideoxyguanosine (C44)

FIGURE 61

continued

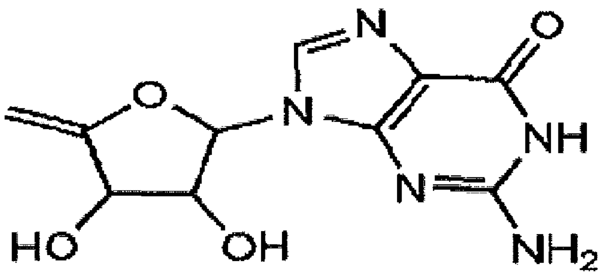

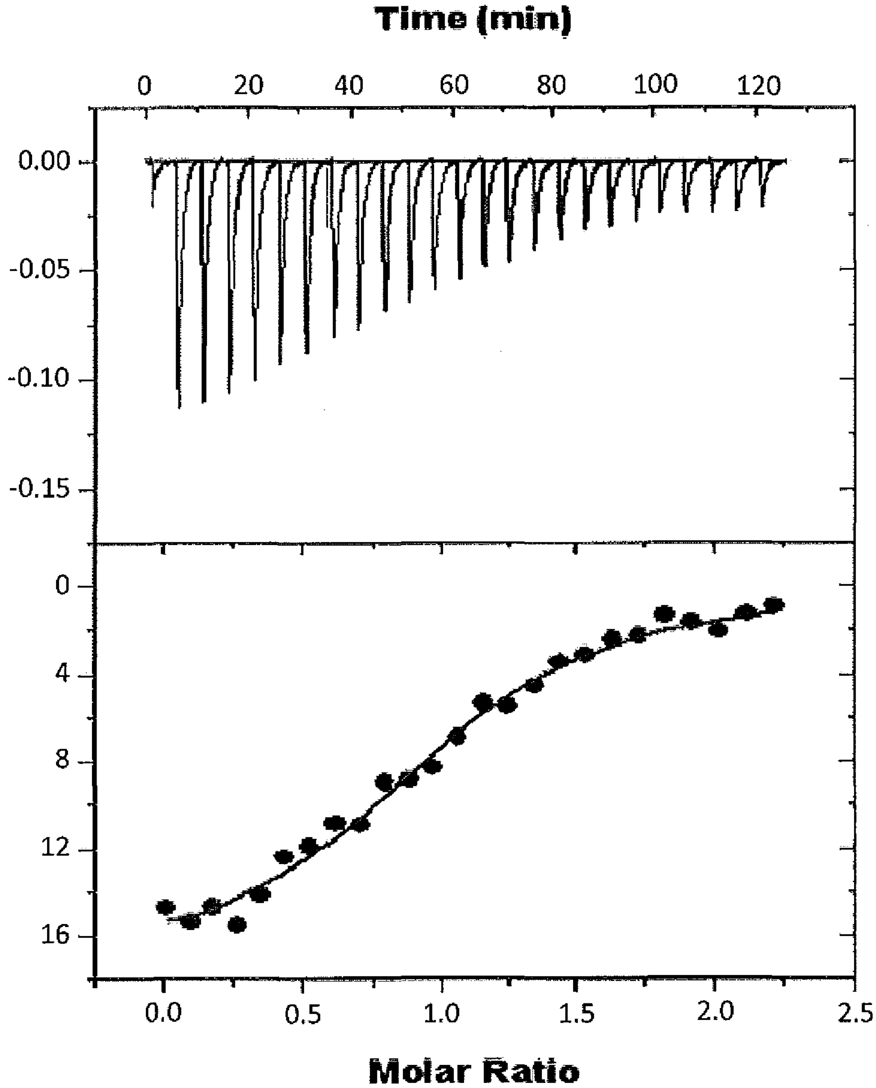


| | | | |
|---|---|---|---|
| **RNA** | M6C" | ***n*** | 1.00 |
| **ligand** | C39 | $K_D$ | 460.41 |
| **model** | onesites | **ΔG** | -8.79 |
| **chi^2** | 267879 | **ΔH** | -17.70 |
| | | **TΔS** | -8.91 |

M6C" : C39

FIGURE 62

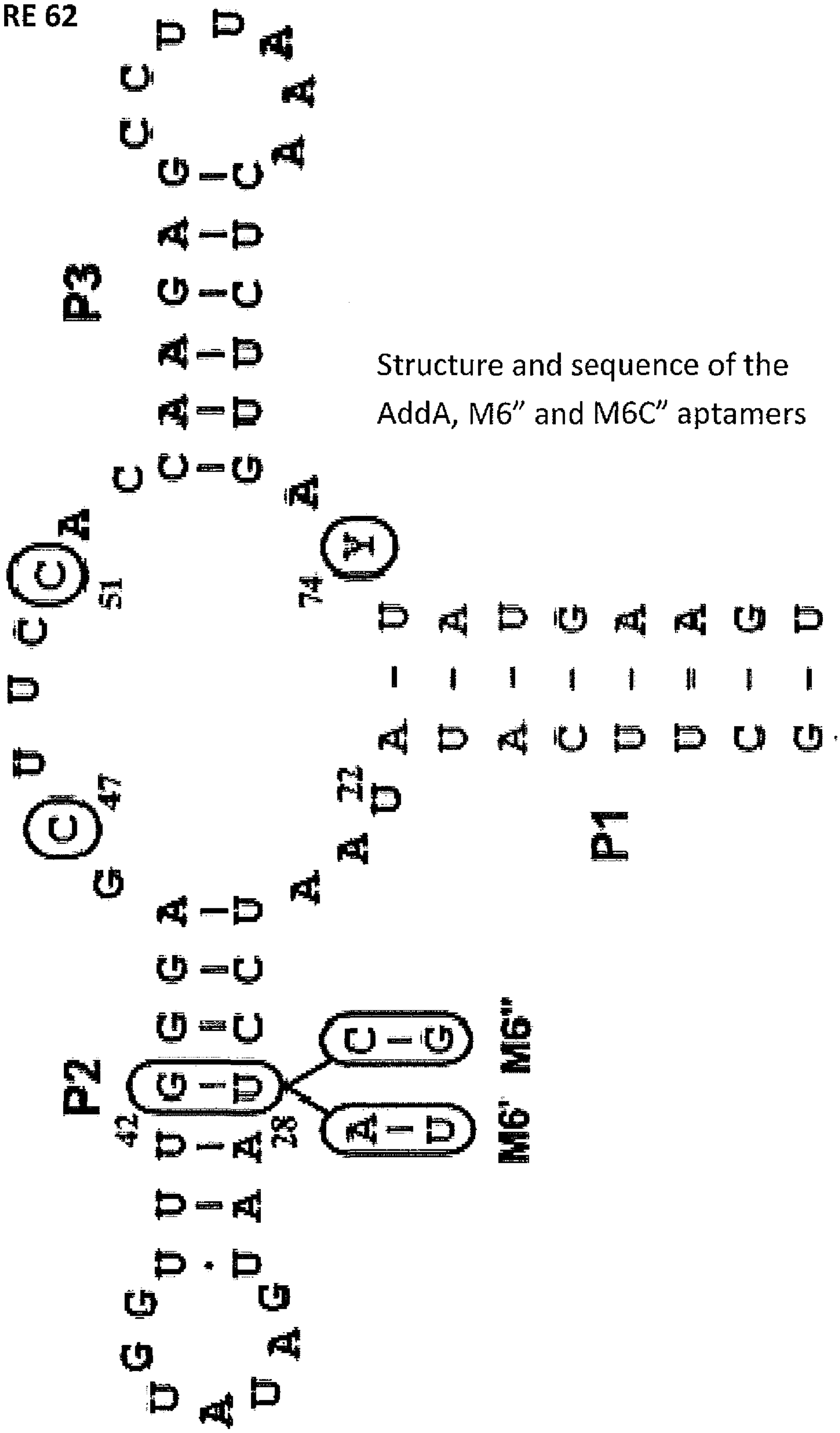


Structure and sequence of the AddA, M6" and M6C" aptamers

**FIGURE 63**

>sp165 (dG = -30.40 kcal/mol, GC 38.2%)

ATGAATGCCATGATCATGAGTAAAGGAGAAGAACTTTTCACTGGAGTTGTCCCAATTCTT

GTTGAATTAGATGGTGATGTTAATGGGTACAAATTTTCTGTCAGTGGAGAGGGTGAAGG
T

GATGCAACATACGGAAAACTTACCCTTAAATTTATTTGCACTACC

>sp150 (dG = -28.00 kcal/mol, GC 39.3%)

ATGAATGCCATGATCATGAGTAAAGGAGAAGAACTTTTCACTGGAGTTGTCCCAATTCTT

GTTGAATTAGATGGTGATGTTAATGGGTACAAATTTTCTGTCAGTGGAGAGGGTGAAGG
T

GATGCAACATACGGAAAACTTACCCTTAAC

>sp135 (dG = -25.50 kcal/mol, GC 40.7%)

ATGAATGCCATGATCATGAGTAAAGGAGAAGAACTTTTCACTGGAGTTGTCCCAATTCTT

GTTGAATTAGATGGTGATGTTAATGGGTACAAATTTTCTGTCAGTGGAGAGGGTGAAGG
T

GATGCAACATACGGC

>sp120 (dG = -23.00 kcal/mol, GC 40.0%)

ATGAATGCCATGATCATGAGTAAAGGAGAAGAACTTTTCACTGGAGTTGTCCCAATTCTT

GTTGAATTAGATGGTGATGTTAATGGGTACAAATTTTCTGTCAGTGGAGAGGGTGAAGG
C

>sp105_cor (dG = -17.20kcal/mol, GC 37.1%)

ATGAATGCCATGATCATGAGTAAAGGAGAAGAACTTTTCACTGGAGTTGTCCCAATTCTT
GTTGAATTAGATGGTGATGTTAATGGGCAAAAATTTTCTGTCACC

>sp102 (dG = -16.80 kcal/mol, GC 38.2%)

ATGAATGCCATGATCATGAGTAAAGGAGAAGAACTTTTCACTGGAGTTGTCCCAATTCTT

**FIGURE 63 CONTINUED**

GTTGAATTAGATGGTGATGTTAATGGGCAAAAATTCTCTGCC

>sp99 (dG = -16.60 kcal/mol, GC 37.4%)

ATGAATGCCATGATCATGAGTAAAGGAGAAGAACTTTTCACTGGAGTTGTCCCAATTCTT

GTTGAATTAGATGGTGATGTTAATGGGCAAAAATTCTCC

>sp96 (dG = -16.20 kcal/mol, GC 37.5%)

ATGAATGCCATGATCATGAGTAAAGGAGAAGAACTTTTCACTGGAGTTGTCCCAATTCTT

GTTGAATTAGATGGTGATGTTAATGGGCAAAAATCC

>sp93 (dG = -14.70 kcal/mol, GC 38.7%)

ATGAATGCCATGATCATGAGTAAAGGAGAAGAACTTTTCACTGGAGTTGTCCCAATTCTT

GTTGAATTAGATGGTGATGTTAATGGGCAAACC

>sp90 (dG = - 13.50 kcal/mol, GC 40.0%

ATGAATGCCATGATCATGAGTAAAGGAGAAGAACTTTTCACTGGAGTTGTCCCAATTCTT

GTTGAATTAGATGGTGATGTTAATGGGCCC

>sp75 (dG = -10.50 kcal/mol, GC 38.7%)

ATGAATGCCATGATCATGAGTAAAGGAGAAGAACTTTTCACTGGAGTTGTCCCAATTCTT

GTTGAATTAGATGCC

>sp60 (dG = -10.60 kcal/mol, GC 41.7%)

ATGAATGCCATGATCATGAGTAAAGGAGAAGAACTTTTCACTGGAGTTGTCCCAATTCC
C

>sp45 (dG = -4.80 kcal/mol, GC 40.0%)

ATGAATGCCATGATCATGAGTAAAGGAGAAGAACTTTTCACTGCC

**FIGURE 63 CONTINUED**

>sp30 (dG = -1.90 kcal/mol, GC 43.3%)

ATGAATGCCATGATCATGAGTAAAGGAGCC

**FIGURE 64 pMAST-h6 sequence**

>pMAST3-h6

AATTCTCTAGACGCAACGCAATTAATGTGAGTTAGCTCACTCATTAGGCACCCCAGGCT
TTACACTTTATGCTTCCGGCTCGTATGTTGTGTGGAATTGTGAGCGGATAACAATTTCAC
ACAGGAAACAGCTGGATCCTCAACGCTTCATATAATCCTAATGATATGGTTTGGGAGTT
TCTACCAAGAGCCTAAACTCTTGATTATGAAGTCTGTCGCTTTATCCGAAATTTTATAAA
GAGAAGACTATGAATGCCATGATCATGAGTAAAGGAGAAGAACTTTTCACTGGAGTTGT
CCCAATTCTTGTTGAATTAGATGGTGATGTTAATGGGCAAAAATTCTCTGTCACCATGGA
CATCATCGAAAACATCGAAAAGAATGACTTCTCAGAAATCGAACTGGCTGCTATCCCGT
TCAACACACTGGCTGACCACTACGGAAGCGCCTTGGCTAAAGAGCAGTTGGCTTTAGA
ACATGAGTCTTATGAGCTAGGCGAGCGCCGCTTCCTCAAGATGCTTGAGCGTCAAGCG
AAAGCTGGTGAGATTGCAGACAACGCAGCCGCTAAGCCGTTACTCGCTACGCTTCTCC
CTAAGTTAACCACACGTATCGTCGAGTGGCTCGAAGAGTACGCATCGAAGAAAGGCCG
CAAGCCTAGCGCATACGCACCGCTCCAGTTACTCAAGCCGGAGGCCTCCGCGTTTATC
ACCCTGAAAGTTATCCTTGCGTCACTAACCAGTACGAACATGACAACCATTCAGGCCGC
TGCTGGTATGCTGGGGAAAGCCATTGAGGACGAGGCACGATTTGGGCGCATCCGTGA
CCTAGAAGCGAAGCACTTCAAGAAGCACGTTGAGGAACAGCTTAACAAGCGCCACGGG
CAAGTCTACAAGAAAGCATTTATGCAGGTGGTCGAGGCCGATATGATTGGTCGAGGTC
TGCTTGGTGGCGAGGCGTGGTCTAGCTGGGATAAAGAAACCACGATGCACGTAGGGAT
TCGCCTGATTGAAATGCTGATTGAATCCACGGGTCTGGTGGAATTACAGCGCCACAAC
GCAGGTAACGCAGGCTCTGACCATGAGGCACTGCAACTGGCCCAAGAGTACGTGGAC
GTATTAGCGAAGCGTGCAGGCGCTCTGGCGGGTATCTCTCCGATGTTCCAGCCGTGTG
TCGTACCGCCGAAACCTTGGGTAGCAATCACAGGGGGCGGCTATTGGGCTAACGGTC
GCAGACCTTTGGCACTCGTTCGCACTCACTCTAAGAAGGGCTTGATGCGCTACGAAGA
CGTTTACATGCCAGAAGTCTACAAGGCTGTGAACCTCGCGCAAAACACCGCATGGAAA
ATCAACAAGAAAGTTCTTGCTGTTGTCAATGAGATTGTTAACTGGAAGAATTGCCCGGT

**FIGURE 64 CONTINUED**

AGCAGACATTCCATCGCTGGAGCGCCAAGAGTTACCGCCTAAGCCTGCGACATTGACA
CCAACGAGGCAGCGCTCAAGGAGTGGAAGAAAGCCGCTGCTGGTATCTATCGCTTGGA
CAAGGCACGAGTGTCTCGCCGTATCAGCTTAGAGTTCATGCTGGAGCAGGCCAACAAG
TTCGCAAGTAAGAAAGCAATCTGGTTCCCTTACAACATGGACTGGCGCGGTCGTGTGTA
CGCTGTGCCGATGTTCAACCCGCAAGGCAACGACATGACGAAAGGTCTGCTGACCCTT
GCTAAAGGCAAGCCAATCGGTGAGGAAGGTTTCTACTGGCTGAAAATCCACGGTGCGA
ACTGTGCGGGTGTTGATAAGGTTCCATTCCCGGAGCGCATCGCGTTCATTGAGAAGCA
CGTAGACGACATTCTGGCTTGCGCTAAAGACCCAATCAATAACACTTGGTGGGCTGAG
CAGGATTCACCGTTCTGTTTCCTCGCGTTTTGCTTCGAGTATGCAGGCGTTACGCACCA
CGGTCTGAGCTACAATTGCTCTCTGCCGCTGGCGTTCGACGGGTCTTGCTCTGGTATC
CAGCACTTCTCCGCGATGCTCCGCGATGAGGTAGGCGGTCGTGCGGTTAACCTGCTG
CCAAGCGAAACCGTGCAGGACATTTACGGCATCGTTGCACAGAAAGTAAACGAGATTC
TCAAACAGGATGCAATCAACGGCACGCCTAACGAGATGATTACCGTGACCGACAAGGA
CACCGGGGAAATCTCAGAGAAGCTCAAACTTGGAACCTCAACGCTGGCGCAACAGTGG
CTGGCATATGGTGTAACCCGTAGCGTAACTAAACGTTCGGTCATGACGCTGGCTTACG
GTTCCAAGGAGTTCGGCTTTCGTCAACAGGTATTGGATGACACCATTCAGCCTGCAATT
GACAGCGGTAAGGGCTTGATGTTCACCCAACCGAACCAAGCGGCTGGCTATATGGCTA
AGCTGATTTGGGATGCGGTAAGCGTGACCGTAGTTGCAGCGGTTGAGGCGATGAACTG
GCTCAAATCTGCCGCTAAGCTGCTGGCTGCTGAGGTCAAGGACAAGAAGACCAAGGAG
ATTCTGCGCCACCGTTGCGCGGTTCACTGGACTACGCCGGACGGCTTCCCGGTCTGG
CAGGAATACCGCAAGCCACTCCAGAAGCGTCTCGATATGATTTTCTTAGGGCAATTCCG
TCTGCAACCGACGATTAATACCCTCAAGGATTCAGGCATTGACGCACACAAGCAGGAG
TCTGGCATCGCTCCTAACTTTGTTCACTCACAGGACGGTAGCCACCTCCGCATGACAGT
CGTTTATGCTCACGAGAAGTATGGCATTGAGTCCTTTGCGCTCATCCATGACAGCTTTG
GGACTATCCCGGCAGACGCTGGTAAGCTCTTTAAGGCTGTGCGTGAAACGATGGTTAT
CACCTATGAGAACAACGATGTGCTGGCAGACTTCTACTCTCAGTTTGCCGACCAGCTAC
ACGAGACCCAACTGGACAAGATGCCTCCGCTTCCGAAGAAAGGAAACCTGAACCTGCA
AGACATTCTCAAGTCTGACTTTGCCTTTGCACACCACCACCACCACCACTAA

**FIGURE 65 pTTv7 (operon) sequence**

>pTTv7

GGAGCTCCGCAACGCAATTAATGTGAGTTAGCTCACTCATTAGGCACCCCAGGCTTTAC
ACTTTATGCTTCCGGCTCGTATGTTGTGTGGAATTGTGAGCGGATAACAATTTCACACA

## FIGURE 65 CONTINUED

GGAAACAGCTGGATCCTCAACGCTTCATATAATCCGAATGATATGGTTTCGGAGCTTCC
ACCAAGAGCCTTAAACTCTTGATTATGAAGTCTGTCGCTTTATCCGAAATTTTATAAAGA
GAAGACTATGAATGCCATGATCATGAGTAAAGGAGAAGAACTTTTCACTGGAGTTGTCC
CAATTCTTGTTGAATTAGATGGTGATGTTAATGGGCAAAAATTCTCTGTCACCATGGCCA
CGATTAACATCGCTAAGAACGACTTCTCTGACATCGAACTGGCTGCTATCCCGTTCAAC
ACTCTGGCTGACCATTACGGTGAGCGTTTAGCTCGCGAACAGTTGGCCCTTGAGCATG
AGTCTTACGAGATGGGTGAAGCACGCTTCCGCAAGATGTTTGAGCGTCAACTTAAAGCT
GGTGAGGTTGCGGATAACGCTGCCGCCAAGCCTCTCATCACTACCCTACTCCCTAAGA
TGATTGCACGCATCAACGACTGGTTTGAGGAAGTGAAAGCTAAGCGCGGCAAGCGCCC
GACAGCCTTCCAGTTCCTGCAAGAAATCAAGCCGGAAGCCGTAGCGTACATCACCATT
AAGACCACTCTGGCTTGCCTAACCAGTGCTGACAATACAACCGTTCAGGCTGTAGCAA
GCGCAATCGGTCGGGCCATTGAGGACGAGGCTCGCTTCGGTCGTATCCGTGACCTTG
AAGCTAAGCACTTCAAGAAAAACGTTGAGGAACAACTCAACAAGCGCGTAGGGCACGT
CTACAAGAAAGCATTTATGCAAGTTGTCGAGGCTGACATGCTCTCTAAGGGTCTACTCG
GTGGCGAGGCGTGGTCTTCGTGGCATAAGGAAGACTCTATTCATGTAGGAGTACGCTG
CATCGAGATGCTCATTGAGTCAACCGGAATGGTTAGCTTACACCGCCAAAATGCTGGC
GTAGTAGGTCAAGACTCTGAGACTATCGAACTCGCACCTGAATACGCTGAGGCTATCG
CAACCCGTGCAGGTGCGCTGGCTGGCATCTCTCCGATGTTCCAACCTTGCGTAGTTCC
TCCTAAGCCGTGGACTGGCATTACTGGTGGTGGCTATTGGGCTAACGGTCGTCGTCCT
CTGGCGCTGGTGCGTACTCACAGTAAGAAAGCACTGATGCGCTACGAAGACGTTTACA
TGCCTGAGGTGTACAAAGCGATTAACATTGCGCAAAACACCGCATGGAAAATCAACAAG
AAAGTCCTAGCGGTCGCCAACGTAATCACCAAGTGGAAGCATTGTCCGGTCGAGGACA
TCCCTGCGATTGAGCGTGAAGAACTCCCGATGAAACCGGAAGACATCGACATGAATCC
TGAGGCTCTCACCGCGTGGAAACGTGCTGCCGCTGCTGTGTACCGCAAGGACAAGGC
TCGCAAGTCTCGCCGTATCAGCCTTGAGTTCATGCTTGAGCAAGCCAATAAGTTTGCTA
ACCATAAGGCCATCTGGTTCCCTTACAACATGGACTGGCGCGGTCGTGTTTACGCTGT
GTCAATGTTCAACCCGCAAGGTAACGATATGACCAAAGGACTGCTTACGCTGGCGAAA
GGTAAACCAATCGGTAAGGAAGGTTACTACTGGCTGAAAATCCACGGTGCAAACTGTG
CGGGTGTCGATAAGGTTCCGTTCCCTGAGCGCATCAAGTTCATTGAGGAAAACCACGA
GAACATCATGGCTTGCGCTAAGTCTCCACTGGAGAACACTTGGTGGGCTGAGCAAGAT
TCTCCGTTCTGCTTCCTTGCGTTCTGCTTTGAGTACGCTGGGGTACAGCACCACGGCCT
GAGCTATAACTGCTCCCTTCCGCTGGCGTTTGACGGGTCTTGCTCTGGCATCCAGCAC

**FIGURE 65 CONTINUED**

TTCTCCGCGATGCTCCGAGATGAGGTAGGTGGTCGCGCGGTTAACTTGCTTCCTAGTG
AAACCGTTCAGGACATCTACGGGATTGTTGCTAAGAAAGTCAACGAGATTCTACAAGCA
GACGCAATCAATGGGACCGATAACGAAGTAGTTACCGTGACCGATGAGAACACTGGTG
AAATCTCTGAGAAAGTCAAGCTGGGCACTAAGGCACTGGCTGGTCAATGGCTGGCTTA
CGGTGTTACTCGCAGTGTGACTAAGCGTTCAGTCATGACGCTGGCTTACGGGTCCAAA
GAGTTCGGCTTCCGTCAACAAGTGCTGGAAGATACCATTCAGCCAGCTATTGATTCCGG
CAAGGGTCTGATGTTCACTCAGCCGAATCAGGCTGCTGGATACATGGCTAAGCTGATTT
GGGAATCTGTGAGCGTGACGGTGGTAGCTGCGGTTGAAGCAATGAACTGGCTTAAGTC
TGCTGCTAAGCTGCTGGCTGCTGAGGTCAAAGATAAGAAGACTGGAGAGATTCTTCGC
AAGCGTTGCGCTGTGCATTGGGTAACTCCTGATGGTTTCCCTGTGTGGCAGGAATACA
AGAAGCCTATTCAGACGCGCTTGAACCTGATGTTCCTCGGTCAGTTCCGCTTACAGCCT
ACCATTAACACCAACAAAGATAGCGAGATTGATGCACACAAACAGGAGTCTGGTATCGC
TCCTAACTTTGTACACAGCCAAGACGGTAGCCACCTTCGTAAGACTGTAGTGTGGGCAC
ACGAGAAGTACGGAATCGAATCTTTTGCACTGATTCACGACTCCTTCGGTACCATTCCG
GCTGACGCTGCGAACCTGTTCAAAGCAGTGCGCGAAACTATGGTTGACACATATGAGT
CTTGTGATGTACTGGCTGATTTCTACGACCAGTTCGCTGACCAGTTGCACGAGTCTCAA
TTGGACAAAATGCCAGCACTTCCGGCTAAAGGTAACTTGAACCTCCGTGACATCTTAGA
GTCGGACTTCGCGTTCGCGTAAGCGGCCGCCTCAACGCTTCATATAATCCTAATGATAT
GGTTTGGGAGTTTCTACCAAGAGCCTTAAACTCTTGATTATGAAGTCTGTCGCTTTATCC
GAAATTTTATAAAGAGAAGACTATGAATGCCATGATCATGAGTAAAGGAGAAGAACTTTT
CACTGGAGTTGTCCCAATTCTTGTTGAATTAGATGGTGATGTTAATGGGCAAAAATTCTC
TGTCACCATGGACATCATCGAAAACATCGAAAAGAATGACTTCTCAGAAATCGAACTGG
CTGCTATCCCGTTCAACACACTGGCTGACCACTACGGAAGCGCCTTGGCTAAAGAGCA
GTTGGCTTTAGAACATGAGTCTTATGAGCTAGGCGAGCGCCGCTTCCTCAAGATGCTTG
AGCGTCAAGCGAAAGCTGGTGAGATTGCAGACAACGCAGCCGCTAAGCCGTTACTCGC
TACGCTTCTCCCTAAGTTAACCACACGTATCGTCGAGTGGCTCGAAGAGTACGCATCGA
AGAAAGGCCGCAAGCCTAGCGCATACGCACCGCTCCAGTTACTCAAGCCGGAGGCCT
CCGCGTTTATCACCCTGAAAGTTATCCTTGCGTCACTAACCAGTACGAACATGACAACC
ATTCAGGCCGCTGCTGGTATGCTGGGGAAAGCCATTGAGGACGAGGCACGATTTGGG
CGCATCCGTGACCTAGAAGCGAAGCACTTCAAGAAG

CACGTTGAGGAACAGCTTAACAAGCGCCACGGGCAAGTCTACAAGAAAGCATTTATGC
AGGTGGTCGAGGCCGATATGATTGGTCGAGGTCTGCTTGGTGGCGAGGCGTGGTCTA
GCTGGGATAAAGAAACCACGATGCACGTAGGGATTCGCCTGATTGAAATGCTGATTGA

**FIGURE 65 CONTINUED**

ATCCACGGGTCTGGTGGAATTACAGCGCCACAACGCAGGTAACGCAGGCTCTGACCAT
GAGGCACTGCAACTGGCCCAAGAGTACGTGGACGTATTAGCGAAGCGTGCAGGCGCT
CTGGCGGGTATCTCTCCGATGTTCCAGCCGTGTGTCGTACCGCCGAAACCTTGGGTAG
CAATCACAGGGGGCGGCTATTGGGCTAACGGTCGCAGACCTTTGGCACTCGTTCGCAC
TCACTCTAAGAAGGGCTTGATGCGCTACGAAGACGTTTACATGCCAGAAGTCTACAAGG
CTGTGAACCTCGCGCAAAACACCGCATGGAAAATCAACAAGAAAGTTCTTGCTGTTGTC
AATGAGATTGTTAACTGGAAGAATTGCCCGGTAGCAGACATTCCATCGCTGGAGCGCC
AAGAGTTACCGCCTAAGCCTGACGACATTGACACCAACGAGGCAGCGCTCAAGGAGTG
GAAGAAAGCCGCTGCTGGTATCTATCGCTTGGACAAGGCACGAGTGTCTCGCCGTATC
AGCTTAGAGTTCATGCTGGAGCAGGCCAACAAGTTCGCAAGTAAGAAAGCAATCTGGTT
CCCTTACAACATGGACTGGCGCGGTCGTGTGTACGCTGTGCCGATGTTCAACCCGCAA
GGCAACGACATGACGAAAGGTCTGCTGACCCTTGCTAAAGGCAAGCCAATCGGTGAGG
AAGGTTTCTACTGGCTGAAAATCCACGGTGCGAACTGTGCGGGTGTTGATAAGGTTCC
ATTCCCGGAGCGCATCGCGTTCATTGAGAAGCACGTAGACGACATTCTGGCTTGCGCT
AAAGACCCAATCAATAACACTTGGTGGGCTGAGCAGGATTCACCGTTCTGTTTCCTCGC
GTTTTGCTTCGAGTATGCAGGCGTTACGCACCACGGTCTGAGCTACAATTGCTCTCTGC
CGCTGGCGTTCGACGGGTCTTGCTCTGGTATCCAGCACTTCTCCGCGATGCTCCGCGA
TGAGGTAGGCGGTCGTGCGGTTAACCTGCTGCCAAGCGAAACCGTGCAGGACATTTAC
GGCATCGTTGCACAGAAAGTAAACGAGATTCTCAAACAGGATGCAATCAACGGCACGC
CTAACGAGATGATTACCGTGACCGACAAGGACACCGGGGAAATCTCAGAGAAGCTCAA
ACTTGGAACCTCAACGCTGGCGCAACAGTGGCTGGCATATGGTGTAACCCGTAGCGTA
ACTAAACGTTCGGTCATGACGCTGGCTTACGGTTCCAAGGAGTTCGGCTTTCGTCAACA
GGTATTGGATGACACCATTCAGCCTGCAATTGACAGCGGTAAGGGCTTGATGTTCACC
CAACCGAACCAAGCGGCTGGCTATATGGCTAAGCTGATTTGGGATGCGGTAAGCGTGA
CCGTAGTTGCAGCGGTTGAGGCGATGAACTGGCTCAAATCTGCCGCTAAGCTGCTGGC
TGCTGAGGTCAAGGACAAGAAGACCAAGGAGATTCTGCGCCACCGTTGCGCGGTTCAC
TGGACTACGCCGGACGGCTTCCCGGTCTGGCAGGAATACCGCAAGCCACTCCAGAAG
CGTCTCGATATGATTTTCTTAGGGCAATTCCGTCTGCAACCGACGATTAATACCCTCAA
GGATTCAGGCATTGACGCACACAAGCAGGAGTCTGGCATCGCTCCTAACTTTGTTCACT
CACAGGACGGTAGCCACCTCCGCATGACAGTCGTTTATGCTCACGAGAAGTATGGCAT
TGAGTCCTTTGCGCTCATCCATGACAGCTTTGGGACTATCCCGGCAGACGCTGGTAAG
CTCTTTAAGGCTGTGCGTGAAACGATGGTTATCACCTATGAGAACAACGATGTGCTGGC

**FIGURE 65 CONTINUED**

AGACTTCTACTCTCAGTTTGCCGACCAGCTACACGAGACCCAACTGGACAAGATGCCTC
CGCTTCCGAAGAAAGGAAACCTGAACCTGCAAGACATTCTCAAGTCTGACTTTGCCTTT
GCACACCACCACCACCACCACTAA

**FIGURE 66 pTTv9 (dual promoter) sequence**

>pTTv9

GGAGCTCCGCAACGCAATTAATGTGAGTTAGCTCACTCATTAGGCACCCCAGGCTTTAC
ACTTTATGCTTCCGGCTCGTATGTTGTGTGGAATTGTGAGCGGATAACAATTTCACACA
GGAAACAGCTGGATCCTCAACGCTTCATATAATCCGAATGATATGGTTTCGGAGCTTCC
ACCAAGAGCCTTAAACTCTTGATTATGAAGTCTGTCGCTTTATCCGAAATTTTATAAAGA
GAAGACTATGAATGCCATGATCATGAGTAAAGGAGAAGAACTTTTCACTGGAGTTGTCC
CAATTCTTGTTGAATTAGATGGTGATGTTAATGGGCAAAAATTCTCTGTCACCATGGCCA
CGATTAACATCGCTAAGAACGACTTCTCTGACATCGAACTGGCTGCTATCCCGTTCAAC
ACTCTGGCTGACCATTACGGTGAGCGTTTAGCTCGCGAACAGTTGGCCCTTGAGCATG
AGTCTTACGAGATGGGTGAAGCACGCTTCCGCAAGATGTTTGAGCGTCAACTTAAAGCT
GGTGAGGTTGCGGATAACGCTGCCGCCAAGCCTCTCATCACTACCCTACTCCCTAAGA
TGATTGCACGCATCAACGACTGGTTTGAGGAAGTGAAAGCTAAGCGCGGCAAGCGCCC
GACAGCCTTCCAGTTCCTGCAAGAAATCAAGCCGGAAGCCGTAGCGTACATCACCATT
AAGACCACTCTGGCTTGCCTAACCAGTGCTGACAATACAACCGTTCAGGCTGTAGCAA
GCGCAATCGGTCGGGCCATTGAGGACGAGGCTCGCTTCGGTCGTATCCGTGACCTTG
AAGCTAAGCACTTCAAGAAAAACGTTGAGGAACAACTCAACAAGCGCGTAGGGCACGT
CTACAAGAAAGCATTTATGCAAGTTGTCGAGGCTGACATGCTCTCTAAGGGTCTACTCG
GTGGCGAGGCGTGGTCTTCGTGGCATAAGGAAGACTCTATTCATGTAGGAGTACGCTG
CATCGAGATGCTCATTGAGTCAACCGGAATGGTTAGCTTACACCGCCAAAATGCTGGC
GTAGTAGGTCAAGACTCTGAGACTATCGAACTCGCACCTGAATACGCTGAGGCTATCG
CAACCCGTGCAGGTGCGCTGGCTGGCATCTCTCCGATGTTCCAACCTTGCGTAGTTCC
TCCTAAGCCGTGGACTGGCATTACTGGTGG

TGGCTATTGGGCTAACGGTCGTCGTCCTCTGGCGCTGGTGCGTACTCACAGTAAGAAA
GCACTGATGCGCTACGAAGACGTTTACATGCCTGAGGTGTACAAAGCGATTAACATTGC

FIGURE 66 CONTINUED

GCAAAACACCGCATGGAAAATCAACAAGAAAGTCCTAGCGGTCGCCAACGTAATCACC
AAGTGGAAGCATTGTCCGGTCGAGGACATCCCTGCGATTGAGCGTGAAGAACTCCCGA
TGAAACCGGAAGACATCGACATGAATCCTGAGGCTCTCACCGCGTGGAAACGTGCTGC
CGCTGCTGTGTACCGCAAGGACAAGGCTCGCAAGTCTCGCCGTATCAGCCTTGAGTTC
ATGCTTGAGCAAGCCAATAAGTTTGCTAACCATAAGGCCATCTGGTTCCCTTACAACAT
GGACTGGCGCGGTCGTGTTTACGCTGTGTCAATGTTCAACCCGCAAGGTAACGATATG
ACCAAAGGACTGCTTACGCTGGCGAAAGGTAAACCAATCGGTAAGGAAGGTTACTACT
GGCTGAAAATCCACGGTGCAAACTGTGCGGGTGTCGATAAGGTTCCGTTCCCTGAGCG
CATCAAGTTCATTGAGGAAAACCACGAGAACATCATGGCTTGCGCTAAGTCTCCACTGG
AGAACACTTGGTGGGCTGAGCAAGATTCTCCGTTCTGCTTCCTTGCGTTCTGCTTTGAG
TACGCTGGGGTACAGCACCACGGCCTGAGCTATAACTGCTCCCTTCCGCTGGCGTTTG
ACGGGTCTTGCTCTGGCATCCAGCACTTCTCCGCGATGCTCCGAGATGAGGTAGGTGG
TCGCGCGGTTAACTTGCTTCCTAGTGAAACCGTTCAGGACATCTACGGGATTGTTGCTA
AGAAAGTCAACGAGATTCTACAAGCAGACGCAATCAATGGGACCGATAACGAAGTAGTT
ACCGTGACCGATGAGAACACTGGTGAAATCTCTGAGAAAGTCAAGCTGGGCACTAAGG
CACTGGCTGGTCAATGGCTGGCTTACGGTGTTACTCGCAGTGTGACTAAGCGTTCAGT
CATGACGCTGGCTTACGGGTCCAAAGAGTTCGGCTTCCGTCAACAAGTGCTGGAAGAT
ACCATTCAGCCAGCTATTGATTCCGGCAAGGGTCTGATGTTCACTCAGCCGAATCAGG
CTGCTGGATACATGGCTAAGCTGATTTGGGAATCTGTGAGCGTGACGGTGGTAGCTGC
GGTTGAAGCAATGAACTGGCTTAAGTCTGCTGCTAAGCTGCTGGCTGCTGAGGTCAAA
GATAAGAAGACTGGAGAGATTCTTCGCAAGCGTTGCGCTGTGCATTGGGTAACTCCTG
ATGGTTTCCCTGTGTGGCAGGAATACAAGAAGCCTATTCAGACGCGCTTGAACCTGATG
TTCCTCGGTCAGTTCCGCTTACAGCCTACCATTAACACCAACAAAGATAGCGAGATTGA
TGCACACAAACAGGAGTCTGGTATCGCTCCTAACTTTGTACACAGCCAAGACGGTAGC
CACCTTCGTAAGACTGTAGTGTGGGCACACGAGAAGTACGGAATCGAATCTTTTGCACT
GATTCACGACTCCTTCGGTACCATTCCGGCTGACGCTGCGAACCTGTTCAAAGCAGTG
CGCGAAACTATGGTTGACACATATGAGTCTTGTGATGTACTGGCTGATTTCTACGACCA
GTTCGCTGACCAGTTGCACGAGTCTCAATTGGACAAAATGCCAGCACTTCCGGCTAAA
GGTAACTTGAACCTCCGTGACATCTTAGAGTCGGACTTCGCGTTCGCGTAAGCGGCCG
CCATAACCCCGCTCTTACACATTCCAGCCCCGCAACGCAATTAATGTGAGTTAGCTCAC
TCATTAGGCACCCCAGGCTTTACACTTTATGCTTCCGGCTCGTATGTTGTGTGGAATTG
TGAGCGGATAACAATTTCACACAGGAAACAGCTGGATCCTCAACGCTTCATATAATCCT
AATGATATGGTTTGGGAGTTTCTACCAAGAGCCTTAAACTCTTGATTATGAAGTCTGTCG
CTTTATCCGAAATTTTATAAAGAGAAGACTATGAATGCCATGATCATGAGTAAAGGAGAA

**FIGURE 66 CONTINUED**

GAACTTTTCACTGGAGTTGTCCCAATTCTTGTTGAATTAGATGGTGATGTTAATGGGCAA
AAATTCTCTGTCACCATGGACATCATCGAAAACATCGAAAAGAATGACTTCTCAGAAATC
GAACTGGCTGCTATCCCGTTCAACACACTGGCTGACCACTACGGAAGCGCCTTGGCTA
AAGAGCAGTTGGCTTTAGAACATGAGTCTTATGAGCTAGGCGAGCGCCGCTTCCTCAA
GATGCTTGAGCGTCAAGCGAAAGCTGGTGAGATTGCAGACAACGCAGCCGCTAAGCC
GTTACTCGCTACGCTTCTCCCTAAGTTAACCACACGTATCGTCGAGTGGCTCGAAGAGT
ACGCATCGAAGAAAGGCCGCAAGCCTAGCGCATACGCACCGCTCCAGTTACTCAAGCC
GGAGG

CCTCCGCGTTTATCACCCTGAAAGTTATCCTTGCGTCACTAACCAGTACGAACATGACA
ACCATTCAGGCCGCTGCTGGTATGCTGGGGAAAGCCATTGAGGACGAGGCACGATTTG
GGCGCATCCGTGACCTAGAAGCGAAGCACTTCAAGAAGCACGTTGAGGAACAGCTTAA
CAAGCGCCACGGGCAAGTCTACAAGAAAGCATTTATGCAGGTGGTCGAGGCCGATATG
ATTGGTCGAGGTCTGCTTGGTGGCGAGGCGTGGTCTAGCTGGGATAAAGAAACCACGA
TGCACGTAGGGATTCGCCTGATTGAAATGCTGATTGAATCCACGGGTCTGGTGGAATTA
CAGCGCCACAACGCAGGTAACGCAGGCTCTGACCATGAGGCACTGCAACTGGCCCAA
GAGTACGTGGACGTATTAGCGAAGCGTGCAGGCGCTCTGGCGGGTATCTCTCCGATGT
TCCAGCCGTGTGTCGTACCGCCGAAACCTTGGGTAGCAATCACAGGGGGCGGCTATTG
GGCTAACGGTCGCAGACCTTTGGCACTCGTTCGCACTCACTCTAAGAAGGGCTTGATG
CGCTACGAAGACGTTTACATGCCAGAAGTCTACAAGGCTGTGAACCTCGCGCAAAACA
CCGCATGGAAAATCAACAAGAAAGTTCTTGCTGTTGTCAATGAGATTGTTAACTGGAAG
AATTGCCCGGTAGCAGACATTCCATCGCTGGAGCGCCAAGAGTTACCGCCTAAGCCTG
ACGACATTGACACCAACGAGGCAGCGCTCAAGGAGTGGAAGAAAGCCGCTGCTGGTAT
CTATCGCTTGGACAAGGCACGAGTGTCTCGCCGTATCAGCTTAGAGTTCATGCTGGAG
CAGGCCAACAAGTTCGCAAGTAAGAAAGCAATCTGGTTCCCTTACAACATGGACTGGC
GCGGTCGTGTGTACGCTGTGCCGATGTTCAACCCGCAAGGCAACGACATGACGAAAG
GTCTGCTGACCCTTGCTAAAGGCAAGCCAATCGGTGAGGAAGGTTTCTACTGGCTGAA
AATCCACGGTGCGAACTGTGCGGGTGTTGATAAGGTTCCATTCCCGGAGCGCATCGCG
TTCATTGAGAAGCACGTAGACGACATTCTGGCTTGCGCTAAAGACCCAATCAATAACAC
TTGGTGGGCTGAGCAGGATTCACCGTTCTGTTTCCTCGCGTTTTGCTTCGAGTATGCAG
GCGTTACGCACCACGGTCTGAGCTACAATTGCTCTCTGCCGCTGGCGTTCGACGGGTC
TTGCTCTGGTATCCAGCACTTCTCCGCGATGCTCCGCGATGAGGTAGGCGGTCGTGCG
GTTAACCTGCTGCCAAGCGAAACCGTGCAGGACATTTACGGCATCGTTGCACAGAAAG
TAAACGAGATTCTCAAACAGGATGCAATCAACGGCACGCCTAACG

**FIGURE 66 CONTINUED**

AGATGATTACCGTGACCGACAAGGACACCGGGGAAATCTCAGAGAAGCTCAAACTTGG
AACCTCAACGCTGGCGCAACAGTGGCTGGCATATGGTGTAACCCGTAGCGTAACTAAA
CGTTCGGTCATGACGCTGGCTTACGGTTCCAAGGAGTTCGGCTTTCGTCAACAGGTATT
GGATGACACCATTCAGCCTGCAATTGACAGCGGTAAGGGCTTGATGTTCACCCAACCG
AACCAAGCGGCTGGCTATATGGCTAAGCTGATTTGGGATGCGGTAAGCGTGACCGTAG
TTGCAGCGGTTGAGGCGATGAACTGGCTCAAATCTGCCGCTAAGCTGCTGGCTGCTGA
GGTCAAGGACAAGAAGACCAAGGAGATTCTGCGCCACCGTTGCGCGGTTCACTGGACT
ACGCCGGACGGCTTCCCGGTCTGGCAGGAATACCGCAAGCCACTCCAGAAGCGTCTC
GATATGATTTTCTTAGGGCAATTCCGTCTGCAACCGACGATTAATACCCTCAAGGATTCA
GGCATTGACGCACACAAGCAGGAGTCTGGCATCGCTCCTAACTTTGTTCACTCACAGG
ACGGTAGCCACCTCCGCATGACAGTCGTTTATGCTCACGAGAAGTATGGCATTGAGTC
CTTTGCGCTCATCCATGACAGCTTTGGGACTATCCCGGCAGACGCTGGTAAGCTCTTTA
AGGCTGTGCGTGAAACGATGGTTATCACCTATGAGAACAACGATGTGCTGGCAGACTT
CTACTCTCAGTTTGCCGACCAGCTACACGAGACCCAACTGGACAAGATGCCTCCGCTT
CCGAAGAAAGGAAACCTGAACCTGCAAGACATTCTCAAGTCTGACTTTGCCTTTGCACA
CCACCACCACCACCACTAA

FIGURE 67

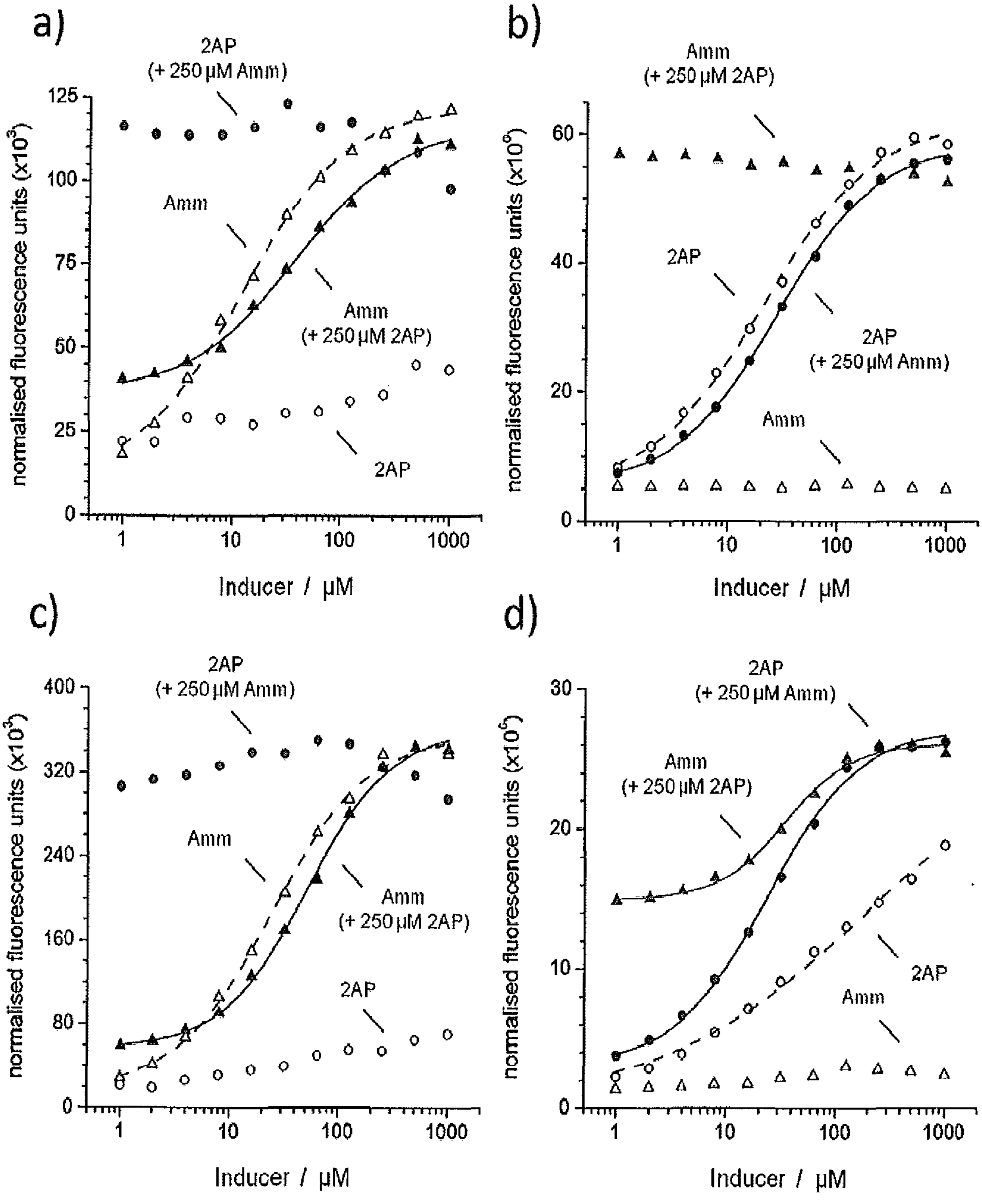
a)
2AP
(+ 250 µM Amm)
Amm
Amm
(+ 250 µM 2AP)
2AP
normalised fluorescence units (x10$^3$)
Inducer / µM
b)
Amm
(+ 250 µM 2AP)
2AP
2AP
(+ 250 µM Amm)
Amm
normalised fluorescence units (x10$^6$)
Inducer / µM
c)
2AP
(+ 250 µM Amm)
Amm
Amm
(+ 250 µM 2AP)
2AP
normalised fluorescence units (x10$^3$)
Inducer / µM
d)
2AP
(+ 250 µM Amm)
Amm
(+ 250 µM 2AP)
2AP
Amm
normalised fluorescence units (x10$^5$)
Inducer / µM

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008116223 A1
- WO 2004027035 A2
- US 2010226901 A1
- US 2009170793 A1


### Non-patent literature cited in the description


- **LYNCH et al.** *Chem Biol,* 2007, vol. 14, 173
- **SINHA et al.** *Nature Chem. Biol.,* 2010, vol. 6, 464
- **OGAWA ; MIZUO.** An Artifical Aptazyme-Based Riboswitch and its Cascading System in E.coli. *Chembiochem,* 2008, vol. 9 (2), 206-209
- **DIXON et al.** Reengineering orthogonally selective riboswitches. *Proceedings of the National Academy of Sciences,* 2010, vol. 107 (7), 2830-2835
- **KIM.** *Biol Cell,* 2008, vol. 100 (1
- **DIXON et al.** *PNAS,* 2010, vol. 107, 2830
- **BURGE et al.** *J. Mol. Biol.,* vol. 268, 78-94
- *Hofacker NAR,* 2003, vol. 1, 3429
- *Zuker NAR,* 2003, vol. 31, 3406
- *Markham NAR,* 2005, vol. 33, W577
- **YAO et al.** *BioInfo,* 2006, vol. 22, 445
- **ALTSCHUL et al.** *J. Mol. Biol.,* 1990, vol. 215, 403
- Wisconsin Genetic Software Package Version 8. Genetics Computer Group. Accelrys Inc
- **HIGGINS et al.** *Gene,* 1998, vol. 73, 237-244
- **YAO et al.** *BioInfo,* 2006, vol. 22, 445
- **ROTH.** *Breaker. Annu Rev Biochem,* 2009, vol. 78, 305
- **BARRICK.** *Breaker Genome Biol,* 2007, vol. 8, R239
- **DIXON et al.** *Proc Natl Acad Sci USA,* 2010, vol. 107, 2830
- Molecular Cloning. **SAMBOOK et al.** A Laboratory Manual. 2000
- **VALDERRAMA-RINCON et al.** *Nature Chemical Biology,* 2012, vol. 8, 434-436
- *Graham. NAR,* 2004, vol. 32, 3093
- **CIAMPI.** *Microbiol,* 2006, vol. 152, 2515
- **DIXON et al.** *Angew. Chem.,* 2012, vol. 51, 3620-3624
- **BEVIS.** *Nat. Biotech,* 2002, vol. 20, 83
- **GRAHAM.** *NAR,* 2004, vol. 32, 3093
- **MANDAL.** *Nat Struct Mol Biol,* 2004, vol. 11, 29
- **ROTH.** *Annu. Rev. Biochem.,* 2009, vol. 78, 305
- **PETERS.** *Proc. Natl. Acad. Sci. USA,* 2009, vol. 106, 15406
- **EPSHTEIN.** *Nature,* 2010, vol. 463, 245
- **DEANA.** *Genes Dev,* 2005, vol. 19, 2526
- **SUDARSAN.** *Science,* 2006, vol. 314, 300
- **WELZ.** *RNA,* 2007, vol. 13, 573
- **WANG et al.** *Nature,* 2006, vol. 441, 358
- **ERRINGTON et al.** *Trends Cell Biol.,* 2003, vol. 13, 577-83
- **MORRIS et al.** *Gene,* 1986, vol. 41, 193-200